# EUROPEAN PATENT APPLICATION

(11) **EP 4 772 531 A2**
(43) Date of publication of application: **08.07.2026**
(21) Application number: 26153982.9
(22) Date of filing: 05.08.2021
(51) Int. Cl.: C07K 14/705

(54) **COMPOSITIONS AND METHODS RELATED TO RECEPTOR PAIRINGS**

(30) Priority: 05.08.2020 US 202063061562 P; 15.09.2020 US 202063078745 P; 11.01.2021 US 202163135884 P
(62) Divisional of application: 21867327.5
(71) Applicant: Synthekine, Inc., Menlo Park, CA 94025 (US)
(72) Inventor: KASTELEIN, Robert, California 94025 (US); LUPARDUS, Patrick J, California 94025 (US); ROKKAM, Deepti, California 94025 (US)
(74) Representative: J A Kemp LLP

(57) **Abstract**

Provided herein are receptor binding proteins that bind to either natural cytokine receptor pairs or non-natural cytokine receptor pairs to create signalling diversity beyond natural receptor pairings.

## Description

### CROSS-REFERNCE TO RELATED PATENT APPLICATIONS

This application claims priority to U.S. Provisional Application No. 63/061,562, filed August 5, 2020, U.S. Provisional Application No. 63/078,745, filed September 15, 2020, and U.S. Provisional Application No. 63/135,884, filed January 11, 2021, the disclosures of which are hereby incorporated by reference in their entirety for all purposes.

### BACKGROUND OF THE DISCLOSURE

Cytokine and growth-factor ligands typically signal through homodimeric or heterodimeric cell surface receptors via Janus Kinase (JAK/TYK), or Receptor Tyrosine Kinase (RTK)-mediated transphosphorylation. However, the number of receptor dimer pairings occurring in nature is limited to those driven by natural ligands encoded within the genome.

In some instance, cytokines act as multispecific (*e.g.*, bispecific or trispecific) ligands. Cytokines determine which receptors are included in the dimers by binding to the extracellular domain of each of the two receptors. Cytokines thus act to bridge or crosslink the receptors in a signaling complex. Cytokine receptor domain or subunit association leads to, among other effects, the activation of an intracellular JAK/STAT signaling pathway, which includes one or more of the four Janus Kinases (JAK1-3 and TYK2) (Ihle, Nature 377(6550):591-4, 1995; O'Shea and Plenge, Immunity 36(4):542-50, 2012) and several signal transducer and activator of transcription (STATs 1-6) proteins (Delgoffe, et al., Curr Opin Immunol. 23(5):632-8, 2011; Levy and Darnell, Nat Rev Mol Cell Biol. 3(9):651-62, 2002; Murray, J Immunol. 178(5):2623-9, 2007). While cytokines typically bind specifically to the extracellular domains of cell surface receptors, the JAK/TYK/STAT signaling modules are found in many combinations in endogenous cytokine receptor signaling complexes.

Given that the a ligand determines the composition of receptor domains or subunits in a receptor complex and the intracellular JAK/TYK and RTK enzymes are degenerate, the number of cytokine and growth factor receptor dimer pairings that occur in nature represents only a fraction of the total number of signaling-competent receptor pairings theoretically allowed by the system. For example, the human genome encodes for approximately forty different JAK/STAT cytokine receptors. In principle, approximately 1600 unique homodimeric and heterodimeric cytokine receptor pairs could be generated with the potential to signal through different JAK/TYK/STAT combinations (Bazan, Proc Natl Acad Sci U S A. 87(18):6934-8, 1990; Huising et al., J Endocrinol. 189(1):1-25, 2006). However, as of the present knowledge, the human genome encodes for less than fifty different cytokine ligands (Bazan, Proc Natl Acad Sci U S A. 87(18):6934-8, 1990; Huising et al., J Endocrinol. 189(1):1-25, 2006), limiting the scope of cytokine receptor complexes signaling to those that can be assembled by the natural ligands.

### SUMMARY OF THE DISCLOSURE

In one aspect, provided herein is an IL12 receptor (IL12R) binding protein that specifically binds to IL12Rβ1 and IL12Rβ2, wherein the binding protein causes the multimerization of IL12Rβ1 and IL12Rβ2 and the multimerization results in the association of intracellular domains of IL12Rβ1 and IL12Rβ2 and intraceullar signaling, and wherein the binding protein comprises a single-domain antibody (sdAb) that specifically binds to IL12Rβ1 (an anti-IL12Rβ1 sdAb) and a sdAb that specifically binds to IL12Rβ2 (an anti-IL12Rβ2 sdAb).

In some embodiments, the anti-IL12Rβ1 sdAb is a V_{H}H antibody (an anti IL12Rβ1 V_{H}H antibody) and/or the anti-IL12Rβ2 sdAb is a V_{H}H antibody (an anti IL12Rβ2 V_{H}H antibody). In some embodiments, the anti-IL12Rβ1 sdAb and the anti-IL12Rβ2 sdAb are joined directly or via a peptide linker. In some embodiments, the peptide linker comprises between 1 and 50 amino acids. In some embodiments, the IL12R binding protein has a reduced Eₘₐₓ compared to IL12. In some embodiments, the IL12R binding protein has an increased Eₘₐₓ compared to IL12. In some embodiments, the IL12R binding protein has a similar potency compared to that of IL12.

In another aspect, the disclosure provides a method for treating neoplastic diseases, such as cancer in a subject in need thereof, the method comprising the step of administering to the subject the IL12R binding protein as described herein, wherein the IL12R binding protein binds to and activates natural killer, CD4⁺ T cells, and/or CD8⁺ T cells. In some embodiments, the cancer is a solid tumor cancer.

In another aspect, the disclosure provides an IL27 receptor (IL27R) binding protein that specifically binds to IL27Rα subunit (IL27Rα) and glycoprotein 130 subunit (gp130), wherein the binding protein causes the multimerization of IL27Rα and gp130 and the multimerization results in the association of intracellular domains of IL27Rα and gp130 and intraceullar signaling, and wherein the binding protein comprises a single-domain antibody (sdAb) that specifically binds to IL27Rα (an anti-IL27Rα sdAb) and a sdAb that specifically binds to gp130 (an anti-gp130 sdAb).

In some embodiments, the anti-IL27Rα sdAb is a V_{H}H antibody (an anti IL27Rα V_{H}H antibody) and/or the anti-gp130 sdAb is a V_{H}H antibody (an anti gp130 V_{H}H antibody). In some embodiments, the anti-IL27Rα sdAb and the anti-gp130 sdAb are joined directly or via a peptide linker. In some embodiments, the peptide linker comprises between 1 and 50 amino acids.

In another aspect, the disclosure provides a method for treating neoplastic diseases, such as cancer in a subject in need thereof, comprising administering to the subject the IL27R binding protein described herein, wherein the IL27R binding protein binds to and activates CD8⁺ T cells, CD4⁺ T cells, and/or T regulatory (Treg) cells. In some embodiments, the IL27R binding protein binds to and activates CD8⁺ T cells. In some embodiments, the IL27R binding protein binds to and activates CXCR5⁺ CD8⁺ T cells. In some embodiments, the cancer is a solid tumor cancer.

In another aspect, the disclosure provides an IL10 receptor (IL10R) binding protein that specifically binds to IL10R α subunit (IL10Rα, also referred to herein as IL10R1) and IL10Rβ (also referred to herein as IL10R2), wherein the binding protein causes the multimerization of IL10Rα and IL10Rβ and the multimerization results in the association of intracellular domains of IL10Rα and IL10Rβ and intraceullar signaling, and wherein the binding protein comprises a single-domain antibody (sdAb) that specifically binds to IL10Rα (an anti-IL10Rα sdAb) and a sdAb that specifically binds to IL10Rβ (an anti-IL10Rβ sdAb).

In some embodiments, the anti-IL10Rα sdAb is a V_{H}H antibody (an anti IL10Rα V_{H}H antibody) and/or the anti-IL10Rβ sdAb is a V_{H}H antibody (an anti IL10Rβ V_{H}H antibody). In some embodiments, the anti-IL10Rα sdAb and the anti-IL10Rβ sdAb are joined by a peptide linker. In some embodiments, the peptide linker comprises between 1 and 50 amino acids.

In another aspect, the disclosure provides a method for treating neoplastic diseases, such as cancer in a subject in need thereof, comprising administering to the subject the IL10R binding protein described herein, wherein the IL10R binding protein binds to and activates CD8⁺ T cells, CD4⁺ T cells, macrophages, and/or Treg cells. In some embodiments, the IL10R binding protein provides longer therapeutic efficacy than a pegylated IL10. In some embodiments, the cancer is a solid tumor cancer.

In other aspects, the IL10R binding proteins described herein can als be used to treat inflammatory diseases, such as Crohn's disease and ulcerative colitis, and autoimmune diseases, such as psoriasis, rheumatoid arthritis, and multiple sclerosis.

In another aspect, the disclosure provides an interferon (IFN) λ receptor (IFNλR) binding protein that specifically binds to IL10Rβ and IL28 receptor (IL28R) α subunit (IL28Rα), wherein the binding protein causes the multimerization of IL10Rβ and IL28Rα and downstream signaling, and wherein the binding protein comprises a single-domain antibody (sdAb) that specifically binds to IL10Rβ (an anti-IL10Rβ sdAb) and a sdAb that specifically binds to IL28Rα (an anti-IL28Rα sdAb).

In some embodiments, the anti-IL10Rβ sdAb is a V_{H}H antibody (an anti-IL10Rβ V_{H}H antibody) and/or the anti-IL28Rα sdAb is a V_{H}H antibody (an anti IL28Rα V_{H}H antibody). In some embodiments, the anti-IL10Rβ sdAb and the anti-IL28Rα sdAb are joined directly or via a peptide linker. In some embodiments, the peptide linker comprises between 1 and 50 amino acids.

In another aspect, the disclosure features a method for treating an infectious disease in a subject in need thereof, comprising administering to the subject an IFNλR binding protein described herein, wherein the IFNλR binding protein binds to and activates macrophages. CD8⁺ T cells, CD4⁺ T cells, Treg cells, dendritic cells, and/or epithelial cells. In some embodiments, the IFNλR binding protein binds to and activates macrophages. In some embodiments, the infectious disease is influenza, hepatitis B, hepatitis C, or human immunodeficiency virus (HIV) infection.

In another aspect, the disclosure provides a binding protein that specifically binds to IL10Rα and IL2Ry, wherein the binding protein causes the multimerization of IL10Rα and IL2Rγ and downstream signaling, and wherein the binding protein comprises a sdAb that specifically binds to IL10Rα (an anti-IL10Rα sdAb) and a sdAb that specifically binds to IL2Rγ (an anti-IL2Ry sdAb).

In some embodiments, the anti-IL10Rα sdAb is a V_{H}H antibody (an anti-IL10Rα V_{H}H antibody) and/or the anti-IL2Rγ sdAb is a V_{H}H antibody (an anti IL2Ry V_{H}H antibody). In some embodiments, the anti-IL10Rα sdAb and the anti-IL2Rγ sdAb are joined directly or via a peptide linker. In some embodiments, the peptide linker comprises between 1 and 50 amino acids.

In another aspect, the disclosure provides a method for treating neoplastic diseases, such as cancer in a subject in need thereof, comprising administering to the subject the binding protein that specifically binds to IL10Rα and IL2Rγ described herein, wherein the binding protein binds to and activates CD8⁺ T cells and/or CD4⁺ T cells. In some embodiments, the method does not cause anemia.

In another aspect, the disclosure provides a binding protein that specifically binds to a first receptor and a second receptor, wherein the first receptor is interferon y receptor 1 (IFNyR1) or IL28Rα and the second receptor is preferentially expressed on myeloid cells and/or T cells, wherein the binding protein causes the multimerization of the first receptor and the second receptor and their downstream signaling, and wherein the binding protein comprises a single-domain antibody (sdAb) that specifically binds to the first receptor and a sdAb that specifically binds to the second receptor.

In some embodiments, the sdAb that specifically binds to a first receptor is an anti-IFNγR1 V_{H}H antibody. In some embodiments, the sdAb that specifically binds to a first receptor is an anti-IL28Rα V_{H}H antibody. In some embodiments, the first receptor is IFNγR1 and the second receptor is IL2Ry. In some embodiments, the first receptor is IL28Rα and the second receptor is IL2Rγ. In some embodiments, the sdAb that specifically binds to the first receptor and the sdAb that specifically binds to the second receptor are joined directly or via a peptide linker. In some embodiments, the peptide linker comprises between 1 and 50 amino acids.

In another aspect, the disclosure provides a method for treating neoplastic diseases, such as cancer in a subject in need thereof, comprising administering to the subject the binding protein that binds to a first receptor (*e.g.,* IFNγR1 or IL28Rα) and a second receptor (*e.g.,* a receptor preferentially expressed on myeloid cells and/or T cells) described herein, wherein the binding protein binds to and activates myeloid cells and/or T cells. In some embodiments, the binding protein binds to and activates macrophages. In some embodiments, the binding protein binds to and activates CD8⁺ T cells and/or CD4⁺ T cells.

### DETAILED DESCRIPTION OF THE DISCLOSURE

### I. INTRODUCTION

The present disclosure provides compositions useful in the pairing of cellular receptors to generate desirable effects useful in treatment of diseases. In general, binding proteins are provided that comprise at least a first domain that binds to a first receptor and a second domain that binds to a second receptor, such that upon contacting with a cell expressing the first and second receptors, the binding protein causes the functional association of the first and second receptors, thereby triggering their interaction and resulting in downstream signaling. In some embodiments, the first and second receptors occur in proximity in response to certain cytokine binding and are referred to herein as "natural" cytokine receptor pairs. In other embodiments, the binding proteins described herein bind to two receptors that do not naturally interact via binding to a naturally occurring cytokine and are referred to herein as "unnatural" cytokine receptor pairs.

Several advantages flow from the binding proteins described herein. In the case of natural cytokine receptor pairs, the natural cytokines cause the natural cytokine receptor pairs to come into proximity (*i.e.,* by their simultaneous binding of a cytokine). However, when some of these natural cytokines are used as therapeutics in mammalian, particularly human, subjects they may also trigger a number of adverse and undesirable effects by a variety of mechanisms including the presence of the natural cytokine receptor on other cell types and the binding to those same receptor pairs on the other cell types can cause unwanted effects or trigger undesired signaling. The present disclosure is directed to manipulating the multiple effects of cytokines so that desired therapeutic signaling occurs, particularly in a desired cellular or tissue subtype, while minimizing undesired activity and/or intracellular signaling.

In some embodiment, the binding proteins described herein are designed such that the binding proteins provide the maximal desired signaling from the natural cytokine receptor pairs on the desired cell types, while the signaling from the receptors on other undesired cell types is weak such that reduced or no toxic effects result from the other undesired cell types. This can be achieved, for example, by selection of binding proteins having differing affinities or causing different Eₘₐₓ for their target receptors as compared to the affinity of a natural cytokine for the same receptors. Because different cell types respond to the binding of ligands to its cognate receptor with different sensitivity, by modulating the affinity of the ligand for the receptor compared to natural cytokine binding facilitates the stimulation of desired activities while reducing undesired activities on non-target cells. To measure downstream signaling activity, a number of methods are available. For example, in some embodiments, one can measure JAK/STAT signaling by the presence of phosphorylated receptors and/or phosphorylated STATs. In other embodiments, the expression of one or more downstream genes, whose expression levels can be affected by the level of downstream signalinging caused by the binding protein, can also be measured.

In other embodiments, the binding proteins described herein provide novel signaling including, but not limited to, by bringing two receptors into proximity that generally do not interact to a significant or measurable degree under natural conditions, or signaling in specific target cell types, by binding to unnatural cytokine receptor pairs. As an example of the latter, one can obtain beneficial signaling caused by binding to the interferon y receptor 1 (IFNyR1) or IL28Rα and a second receptor that is uniquely or preferentially expressed on myeloid or T-cells, while avoiding or reducing binding of the same receptors *(e.g.,* IFNγR1 or IL28Rα) expressed in other cells in a human by contacting the target cells with a binding protein that comprises a first domain that specifically binds to IFNγR1 or IL28Rα and a second domain that specifically binds to a receptor uniquely or preferentially expressed on myeloid or T-cells, thereby targeting activation of IFNγR1 or IL28Rα by targeting the binding protein to these target cells (myeloid or T-cells) and limiting binding to other cells. The various receptor binding proteins described herein can be designed and tailored to bind to specific receptors, or domains or subunits thereof, that are highly expressed on the cell surface of different cell types. By binding two separate receptors, these receptor binding proteins provide a way to selectively activate or inhibit specific cell types that provide therapeutic and/or prophylactic activity useful in the treatment and/or prevention of diseases such as neoplastic diseases, such as cancer, and infectious diseases.

### II. DEFINITIONS

As used herein, the term "antibody" refers collectively to: (a) glycosylated and non-glycosylated immunoglobulins (including but not limited to mammalian immunoglobulin classes IgG1, IgG2, IgG3 and IgG4) that specifically binds to target molecule and (b) immunoglobulin derivatives including but not limited to IgG(1-4)deltaC_{H}2, F(ab')₂, Fab, ScFv, V_{H}, V_{L}, tetrabodies, triabodies, diabodies, dsFv, F(ab')₃, scFv-Fc and (scFv)₂ that competes with the immunoglobulin from which it was derived for binding to the target molecule. The term antibody is not restricted to immunoglobulins derived from any particular mammalian species and includes murine, human, equine, and camelids antibodies (e.g., human antibodies).

The term antibody also includes so called "single-domain antibodies" or "sdAbs," as well as "heavy chain antibodies" or "VuHs," which are further defined herein. V_{H}Hs can be obtained from immunization of camelids (including camels, llamas, and alpacas (see, *e.g.,* Hamers-Casterman, et al. (1993) Nature 363:446-448) or by screening libraries *(e.g.,* phage libraries) constructed in V_{H}H frameworks. Antibodies having a given specificity may also be derived from non-mammalian sources such as V_{H}Hs obtained from immunization of cartilaginous fishes including, but not limited to, sharks. The term "antibody" encompasses antibodies isolatable from natural sources or from animals following immunization with an antigen and as well as engineered antibodies including monoclonal antibodies, bispecific antibodies, trispecific, chimeric antibodies, humanized antibodies, human antibodies, CDR-grafted, veneered, or deimmunized (*e.g.*, to remove T-cell epitopes) antibodies. The term "human antibody" includes antibodies obtained from human beings as well as antibodies obtained from transgenic mammals comprising human immunoglobulin genes such that, upon stimulation with an antigen the transgenic animal produces antibodies comprising amino acid sequences characteristic of antibodies produced by human beings.

The term antibody includes both the parent antibody and its derivatives such as affinity matured, veneered, CDR grafted, humanized, camelized (in the case of V_{H}Hs), or binding molecules comprising binding domains of antibodies (*e.g*., CDRs) in non-immunoglobulin scaffolds.

The term "antibody" should not be construed as limited to any particular means of synthesis and includes naturally occurring antibodies isolatable from natural sources and as well as engineered antibodies molecules that are prepared by "recombinant" means including antibodies isolated from transgenic animals that are transgenic for human immunoglobulin genes or a hybridoma prepared therefrom, antibodies isolated from a host cell transformed with a nucleic acid construct that results in expression of an antibody, antibodies isolated from a combinatorial antibody library including phage display libraries. In one embodiment, an "antibody" is a mammalian immunoglobulin. In some embodiments, the antibody is a "full length antibody" comprising variable and constant domains providing binding and effector functions.

The term antibody includes antibody conjugates comprising modifications to prolong duration of action such as fusion proteins or conjugation to polymers (*e.g*., PEGylated).

As used herein, the term "binding protein" refers to a protein that can bind to one or more cell surface receptors or domains or subunits thereof. In some embodiments, a binding protein specifically binds to two different receptors (or domains or subunits thereof) such that the receptors (or domains or subunits) are maintained in proximity to each other such that the receptors (or domains or subunits), including domains thereof (*e.g*., intracellular domains) interact with each other and result in downstream signaling.

As used herein, the term "CDR" or "complementarity determining region" is intended to mean the non-contiguous antigen combining sites found within the variable region of both heavy and light chain immunoglobulin polypeptides. CDRs have been described by Kabat et al., J. Biol. Chem. 252:6609-6616 (1977); Kabat et al., U.S. Dept. of Health and Human Services, "Sequences of proteins of immunological interest" (1991) (also referred to herein as Kabat 1991); by Chothia et al., J. Mol. Biol. 196:901-917 (1987) (also referred to herein as Chothia 1987); and MacCallum et al., J. Mol. Biol. 262:732-745 (1996), where the definitions include overlapping or subsets of amino acid residues when compared against each other. Nevertheless, application of either definition to refer to a CDR of an antibody or grafted antibodies or variants thereof is intended to be within the scope of the term as defined and used herein. For purposes of the present disclosure, unless otherwise specifically identified, the positioning of CDRs2 and 3 in the variable region of an antibody follows Kabat numbering or simply, "Kabat." The positioning of CDR1 in the variable region of an antibody follows a hybrid of Kabat and Chothia numbering schemes.

As used herein, the term "conservative amino acid substitution" refers to an amino acid replacement that changes a given amino acid to a different amino acid with similar biochemical properties (*e.g.,* charge, hydrophobicity, and size). For example, the amino acids in each of the following groups can be considered as conservative amino acids of each other: (1) hydrophobic amino acids: alanine, isoleucine, leucine, tryptophan, phenylalanine, valine, proline, and glycine; (2) polar amino acids: glutamine, asparagine, histidine, serine, threonine, tyrosine, methionine, and cysteine; (3) basic amino acids: lysine and arginine; and (4) acidic amino acids: aspartic acid and glutamic acid.

As used herein, the term "interferon λ receptor" or "IFNλR" refers to a heterodimeric receptor formed by IL10Rβ receptor and IL28 receptor α (IL28Rα) and bound by the ligand IFNλ. Subunit IL28Rα is also referred to as IFNLR1 (IFNλ receptor 1). The human sequence of IL10Rβ is listed as UniProt ID NO. Q08334. The human sequence of IL28Rα is listed as UniProt ID NO. Q8IU57.

As used herein, the term "interferon y receptor 1" or "IFNyR1" refers to a subunit of the heterodimeric IFNγR that is formed by subunit IFNγR1 and subunit IFNyR2 and bound by the ligand IFNγ. The amino acid sequence of the human IFNγR1 polypeptide is known and listed as UniProt ID NO. P15260.

As used herein, the term "interleukin 12 receptor" or "IL12R" refers to a heterodimeric receptor formed by subunit IL12R β1 (IL12Rβ1) and subunit IL12R β2 (IL12Rβ2) and bound by its cognate ligand IL12. The amino acid sequence of human IL12Rβ1 is known and listed as UniProt ID NO. P42701. The amino acid sequence of human IL12Rβ2 is known and listed as UniProt ID NO. Q99665.

As used herein, the term "interleukin 27 receptor" or "IL27R" refers to a heterodimeric receptor formed by subunits IL27R α (IL27Rα) and glycoprotein 130 (gp130) and bound by the ligand IL27. The human sequence of IL27Rα is listed as UniProt ID NO. Q6UWB1. The human sequence of gp130 is listed as UniProt ID NO. Q13514.

As used herein, the term "interleukin 10 receptor" or "IL10R" refers to a tetrameric receptor formed by two IL10R α subunits (IL10Rα) and two IL10R β subunits (IL10Rβ) and bound by the ligand IL10. The amino acid sequence of human IL10Rα is listed as UniProt ID NO. Q13651. The amino acid sequence of human IL10Rβ is listed as UniProt ID NO. Q08334.

As used herein, the term "interleukin 2 receptor γ" or "IL2Ry" refers to the y subunit of the trimeric IL2R. IL2Rγ is also known as CD132. The amino acid sequence of human IL2Rγ is listed as UniProt ID NO. P31785.

As used herein, the term "linker" refers to a linkage between two elements, *e.g.,* protein domains. A linker can be a covalent bond or a peptide linker. The term "bond" refers to a chemical bond, *e.g.,* an amide bond or a disulfide bond, or any kind of bond created from a chemical reaction, *e.g.,* chemical conjugation. The term "peptide linker" refers to an amino acid or polyeptide that may be employed to link two protein domains to provide space and/or flexibility between the two protein domains.

As used herein, the term "multimerization" refers to two or more cell surface receptors, or domains or subunits thereof, being brought in close proximity to each other such that the receptors, or domains or subunits thereof, can interact with each other and cause downstream signaling.

As used herein, the term "proximity" refers to the spatial proximity or physical distance between two cell surface receptors, or domains or subunits thereof, after a binding protein described herein binds to the two cell surface receptors, or domains or subunits thereof. In some embodiments, after the binding protein binds to the cell surface receptors, or domains or subunits thereof, the spatial proximity between the cell surface receptors, or domains or subunits thereof, can be, *e.g.,* less than about 500 angstroms, such as *e.g.,* a distance of about 5 angstroms to about 500 angstroms. In some embodiments, the spatial proximity amounts to less than about 5 angstroms, less than about 20 angstroms, less than about 50 angstroms, less than about 75 angstroms, less than about 100 angstroms, less than about 150 angstroms, less than about 250 angstroms, less than about 300 angstroms, less than about 350 angstroms, less than about 400 angstroms, less than about 450 angstroms, or less than about 500 angstroms. In some embodiments, the spatial proximity amounts to less than about 100 angstroms. In some embodiments, the spatial proximity amounts to less than about 50 angstroms. In some embodiments, the spatial proximity amounts to less than about 20 angstroms. In some embodiments, the spatial proximity amounts to less than about 10 angstroms. In some embodiments, the spatial proximity ranges from about 10 to 100 angstroms, from about 50 to 150 angstroms, from about 100 to 200 angstroms, from about 150 to 250 angstroms, from about 200 to 300 angstroms, from about 250 to 350 angstroms, from about 300 to 400 angstroms, from about 350 to 450 angstroms, or about 400 to 500 angstroms. In some embodiments, the spatial proximity amounts to less than about 250 angstroms, alternatively less than about 200 angstroms, alternatively less than about 150 angstroms, alternatively less than about 120 angstroms, alternatively less than about 100 angstroms, alternatively less than about 80 angstroms, alternatively less than about 70 angstroms, or alternatively less than about 50 angstroms.

As used herein, the term "downstream signaling" refers to the cellular signaling process that is caused by the interaction of two or more cell surface receptors that are brought into proximity of each other.

As used herein, the term "percent (%) sequence identity" used in the context of nucleic acids or polypeptides, refers to a sequence that has at least 50% sequence identity with a reference sequence. Alternatively, percent sequence identity can be any integer from 50% to 100%. In some embodiments, a sequence has at least 50%, 55%, 60%, 65%, 70%, 75%, 80%, 85%, 90%, 91%, 92%, 93%, 94%, 95%, 96%, 97%, 98%, or 99% sequence identity to the reference sequence as determined with BLAST using standard parameters, as described below.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or altemative parameters can be designated. The sequence comparison algorithm then calculates the percent sequence identities for the test sequences relative to the reference sequence, based on the program parameters.

A comparison window includes reference to a segment of any one of the number of contiguous positions, *e.g*., a segment of at least 10 residues. In some embodiments, the comparison window has from 10 to 600 residues, *e.g.,* about 10 to about 30 residues, about 10 to about 20 residues, about 50 to about 200 residues, or about 100 to about 150 residues, in which a sequence may be compared to a reference sequence of the same number of contiguous positions after the two sequences are optimally aligned.

Algorithms that are suitable for determining percent sequence identity and sequence similarity are the BLAST and BLAST 2.0 algorithms, which are described in Altschul *et al.*

(1990) J. Mol. Biol. 215: 403-410 and Altschul et al. (1977) Nucleic Acids Res. 25: 3389-3402, respectively. Software for performing BLAST analyses is publicly available through the National Center for Biotechnology Information (NCBI) web site. The algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al. supra).* These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues: always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word size (W) of 28, an expectation (E) of 10, M=1, N=-2, and a comparison of both strands. For amino acid sequences, the BLASTP program uses as defaults a word size (W) of 3, an expectation (E) of 10, and the BLOSUM62 scoring matrix (*see* Henikoff & Henikoff, Proc. Natl. Acad. Sci. USA 89:10915 (1989)).

The BLAST algorithm also performs a statistical analysis of the similarity between two sequences (*see, e.g.,* Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, an amino acid sequence is considered similar to a reference sequence if the smallest sum probability in a comparison of the test amino acid sequence to the reference amino acid sequence is less than about 0.01, more preferably less than about 10⁻⁵, and most preferably less than about 10⁻²⁰.

As used herein, the term "single-domain antibody" or "sdAb" refers to an antibody having a single monomeric variable antibody domain. A sdAb is able to bind selectively to a specific antigen. A V_{H}H antibody, further defined below, is an example of a sdAb.

As used herein, the term "specifically bind" refers to the degree of selectivity or affinity for which one molecule binds to another. In the context of binding pairs (*e.g*., a binding protein described herein/receptor, a ligand/receptor, antibody/antigen, antibody/ligand, antibody/receptor binding pairs), a first molecule of a binding pair is said to specifically bind to a second molecule of a binding pair when the first molecule of the binding pair does not bind in a significant amount to other components present in the sample. A first molecule of a binding pair is said to specifically bind to a second molecule of a binding pair when the affinity of the first molecule for the second molecule is at least two-fold greater, alternatively at least five times greater, alternatively at least ten times greater, alternatively at least 20-times greater, or alternatively at least 100-times greater than the affinity of the first molecule for other components present in the sample.

In a particular embodiment, a V_{H}H in a bispecific V_{H}H² binding protein described herein binds to a receptor (e.g., the first receptor or the second receptor of the natural or non-natural receptor pairs) if the equilibrium dissociation constant between the V_{H}H and the receptor is greater than about 10⁶ M, alternatively greater than about 10⁸ M, alternatively greater than about 10¹⁰ M, alternatively greater than about 10¹¹ M, alternatively greater than about 10¹⁰ M, greater than about 10¹² M as determined by, e.g., Scatchard analysis (Munsen, et al. 1980 Analyt. Biochem. 107:220-239). Specific binding may be assessed using techniques known in the art including but not limited to competition ELISA, BIACORE^{®} assays and/or KINEXA^{®} assays.

As used herein, the term "subject", "recipient", "individual", or "patient", refers to any mammalian subject for whom diagnosis, treatment, or therapy is desired, particularly humans. These terms can also be used interchangeably herein. "Mammal" for purposes of treatment refers to any animal classified as a mammal, including humans, domestic and farm animals, and zoo, sports, or pet animals, such as dogs, horses, cats, cows, sheep, goats, pigs, etc. In some embodiments, the mammal is a human being.

The terms "treat", "treating", treatment" and the like refer to a course of action (such as administering a binding protein described herein, or a pharmaceutical composition comprising same) initiated with respect to a subject after a disease, disorder or condition, or a symptom thereof, has been diagnosed, observed, or the like in the subject so as to eliminate, reduce, suppress, mitigate, or ameliorate, either temporarily or permanently, at least one of the underlying causes of such disease, disorder, or condition afflicting a subject, or at least one of the symptoms associated with such disease, disorder, or condition. The treatment includes a course of action taken with respect to a subject suffering from a disease where the course of action results in the inhibition (e.g., arrests the development of the disease, disorder or condition or ameliorates one or more symptoms associated therewith) of the disease in the subject.

As used herein the terms "prevent", "preventing", "prevention" and the like refer to a course of action initiated with respect to a subject prior to the onset of a disease, disorder, condition or symptom thereof so as to prevent, suppress, inhibit or reduce, either temporarily or permanently, a subject's risk of developing a disease, disorder, condition or the like (as determined by, for example, the absence of clinical symptoms) or delaying the onset thereof, generally in the context of a subject predisposed due to genetic, experiential or environmental factors to having a particular disease, disorder or condition. In certain instances, the terms "prevent", "preventing", "prevention" are also used to refer to the slowing of the progression of a disease, disorder or condition from a present its state to a more deleterious state.

As used herein, the term "V_{H}H" is a type of sdAb that has a single monomeric heavy chain variable antibody domain. Such antibodies can be found in or produced from Camelid mammals (e.g., camels, llamas) which are naturally devoid of light chains.

As used herein, the term "V_{H}H²" refers to two V_{H}Hs that are joined together by way of a linker *(e.g.,* a covalent bond or a peptide linker). A "bispecific V_{H}H²" refers to a V_{H}H² that has a first V_{H}H binding to a first receptor, or domain or subunit thereof, and a second V_{H}H binding to a second receptor, or domain or subunit thereof.

### III. COMPOSITIONS AND METHODS

The disclosure describes various receptor binding proteins that bind to either natural cytokine receptor pairs or domains or subunits thereof, or non-natural cytokine receptor pairs or domains or subunits thereof to create signaling diversity not observed with natural receptor pairings. The various receptor binding proteins can be screened for binding to receptor pairs or domains or subunits thereof and for signal transduction in therapeutically relevant cell types.

### Receptor Binding Proteins that Bind to Natural Receptor Pairs

### IL12 receptor binding proteins

The IL12 receptor (IL12R) includes subunits IL12Rβ1 and IL12Rβ2. Provided herein is an IL12R binding protein that specifically binds to IL12Rβ1 and IL12Rβ2. In some embodiments, the IL12R binding protein binds to a mammalian cell expressing both IL12Rβ1 and IL12Rβ2. In some embodiments, the IL12R binding protein can be a bispecific V_{H}H² as described below. In other embodiments, the IL12R binding protein can include a first domain that is a V_{H}H and a second domain which can be a fragment of IL12 or, for example, a scFv.

The IL12R binding protein can be a bispecific V_{H}H² that has a first V_{H}H binding to IL12Rβ1 (an anti-IL12Rβ1 V_{H}H antibody) and a second V_{H}H binding to IL12Rβ2 (an anti-IL12Rβ2 V_{H}H antibody) and causes the dimerization of the two receptor subunits and downstream signaling when bound to a cell expressing IL12Rβ1 and IL12Rβ2, *e.g.,* a natural killer or a T cell *(e.g.,* a CD4⁺ T cells, and/or a CD8⁺ T cell).

A linker can be used to join the anti-IL12Rβ1 V_{H}H antibody and the anti-IL12Rβ2 V_{H}H antibody. For example, a linker can simply be a covalent bond or a peptide linker. A peptide linker can include between 1 and 50 amino acids (e.g., between 2 and 50, between 5 and 50, between 10 and 50, between 15 and 50, between 20 and 50, between 25 and 50, between 30 and 50, between 35 and 50, between 40 and 50, between 45 and 50, between 2 and 45, between 2 and 40, between 2 and 35, between 2 and 30, between 2 and 25, between 2 and 20, between 2 and 15, between 2 and 10, between 2 and 5 amino acids). A peptide linker joining the anti-IL12Rβ1 V_{H}H antibody and the anti-IL12Rβ2 V_{H}H antibody can be a flexible glycine-serine linker. A linker can also be a chemical linker, such as a synthetic polymer, e.g., a polyethylene glycol (PEG) polymer.

The anti-IL12Rβ1 V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:105-111.

The anti-IL12Rβ2 V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:58-63.

The anti-IL12Rβ2 V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:112-117.

In some embodiments, an IL12 receptor binding protein described herein can have an anti-IL12Rβ1 V_{H}H, a linker, and an anti-IL12Rβ2 V_{H}H as listed in Table 1 below.

| **Table 1. IL12 Receptor Binding Protein Constructs** | | | |
|---|---|---|---|
| **SEQ ID NO of anti-IL12Rβ1 V_{H}H at N-terminus** | **SEQ ID NO of linker** | **SEQ ID NO of anti-IL12Rβ2 V_{H}H at C-terminus** | **Sequence of IL12 receptor binding protein (SEQ ID NO)** |
| 105 | 23 | 112 | 131 |
| 105 | 23 | 113 | 132 |
| 105 | 23 | 114 | 133 |
| 105 | 23 | 115 | 134 |
| 105 | 23 | 116 | 135 |
| 105 | 23 | 117 | 136 |
| 106 | 23 | 112 | 137 |
| 106 | 23 | 113 | 138 |
| 106 | 23 | 114 | 139 |
| 106 | 23 | 115 | 140 |
| 106 | 23 | 116 | 141 |
| 106 | 23 | 117 | 142 |
| 107 | 23 | 112 | 143 |
| 107 | 23 | 113 | 144 |
| 107 | 23 | 114 | 145 |
| 107 | 23 | 115 | 146 |
| 107 | 23 | 116 | 147 |
| 107 | 23 | 117 | 148 |
| 108 | 23 | 112 | 149 |
| 108 | 23 | 113 | 150 |
| 108 | 23 | 114 | 151 |
| 108 | 23 | 115 | 152 |
| 108 | 23 | 116 | 153 |
| 108 | 23 | 117 | 154 |
| 109 | 23 | 112 | 155 |
| 109 | 23 | 113 | 156 |
| 109 | 23 | 114 | 157 |
| 109 | 23 | 115 | 158 |
| 109 | 23 | 116 | 159 |
| 109 | 23 | 117 | 160 |
| 112 | 23 | 105 | 161 |
| 112 | 23 | 106 | 162 |
| 112 | 23 | 107 | 163 |
| 112 | 23 | 108 | 164 |
| 112 | 23 | 109 | 165 |
| 113 | 23 | 105 | 166 |
| 113 | 23 | 106 | 167 |
| 113 | 23 | 107 | 168 |
| 113 | 23 | 108 | 169 |
| 113 | 23 | 109 | 170 |
| 114 | 23 | 105 | 171 |
| 114 | 23 | 106 | 172 |
| 114 | 23 | 107 | 173 |
| 114 | 23 | 108 | 174 |
| 114 | 23 | 109 | 175 |
| 115 | 23 | 105 | 176 |
| 115 | 23 | 106 | 177 |
| 115 | 23 | 107 | 178 |
| 115 | 23 | 108 | 179 |
| 115 | 23 | 109 | 180 |
| 116 | 23 | 105 | 181 |
| 116 | 23 | 106 | 182 |
| 116 | 23 | 107 | 183 |
| 116 | 23 | 108 | 184 |
| 116 | 23 | 109 | 185 |
| 117 | 23 | 105 | 186 |
| 117 | 23 | 106 | 187 |
| 117 | 23 | 107 | 188 |
| 117 | 23 | 108 | 189 |
| 117 | 23 | 109 | 190 |

In some embodiments, the IL12R binding protein has a reduced Eₘₐₓ compared to the Eₘₐₓ caused by IL12. Eₘₐₓ reflects the maximum response level in a cell type that can be obtained by a ligand *(e.g.,* a binding protein described herein or the native cytokine *(e.g.,* IL12)). In some embodiments, the IL12R binding protein described herein has at least 1% (e.g., between 1% and 100%, between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ caused by IL12. In some embodiments, by varying the linker length of the IL12R binding protein, the Eₘₐₓ of the IL12R binding protein can be changed. The IL12R binding protein can cause Eₘₐₓ in the most desired cell types (*e.g*., CD8⁺ T cells), and a reduced Eₘₐₓ in other cell types (*e.g.,* natural killer cells). In some embodiments, the Eₘₐₓ in natural killer cells caused by an IL12R binding protein described herein is between 1% and 100% *(e.g.,* between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ in T cells *(e.g.,* CD8⁺ T cells) caused by the IL12R binding protein. In other embodiments, the Eₘₐₓ of the IL12R binding protein described herein is greater (*e.g.,* at least 1%, 5%, 10%, 15%, 20%, 25%, 30%, 35%, 40%, 45%, or 50% greater) than the Eₘₐₓ of the natural ligand, IL12.

An IL12R binding protein described herein are useful in the treatment of neoplastic diseases, such as cancer *(e.g.,* a solid tumor cancer: *e.g.,* non-small-cell lung carcinoma (NSCLC), renal cell carcinoma (RCC), or melanoma) in a subject in need thereof. The IL12R binding protein binds to and activates natural killer, CD4⁺ T cells, and/or CD8⁺ T cells. The IL12R binding protein can trigger different levels of downstream signaling in different cell types. For example, by varying the length of the linker between the anti-IL12Rβ1 V_{H}H antibody and the anti-IL12Rβ2 V_{H}H antibody in the IL12R binding protein, the IL12R binding protein can cause a higher level of downstream signaling in desired cell types compared to undesired cell types. In some embodiments, by varying the linker length, an IL12R binding protein can cause a higher level of downstream signaling in T cells *(e.g.,* CD8⁺ T cells) compared to the level of downstream signaling in natural killer cells, a cell type that expresses both IL12Rβ1 and IL12Rβ2 receptors but when activated too potently can give rise to toxicities. In other embodiments, different anti-IL12Rβ1 V_{H}H antibodies with different binding affinities and different anti-IL12Rβ2 V_{H}H antibodies with different binding affinities can be combined to make different IL12R binding proteins. Further, the orientation of the two antibodies in the binding protein can also be changed to make a different binding protein *(i.e.,* anti-IL12Rβ1 V_{H}H antibody-linker-anti-IL12Rβ2 V_{H}H antibody, or anti-IL12Rβ2 V_{H}H antibody-linker-anti-IL12Rβ1 V_{H}H antibody). Different IL12R binding proteins can be screened to find the ideal binding protein that causes a higher level of downstream signaling in desired cell types compared to undesired cell types. In some embodiments, IL12R binding proteins can be partial agonists that have different activities on different cell types, *e.g.,* T cells versus natural killer cells. For example, the selective activation of T cells over natural killer cells is desirable to avoid the toxicity associated with IL12 activated natural killer cells. In some embodiments IL12R binding protein is a partial agonist, where the partial agonist activates T cells selectively over NK cells. In some embodiments, the level of downstream signaling in T cells (*e.g.,* CD8⁺ T cells) is at least 1.1, 1.5, 2, 3, 5, or 10 times of the level of downstream signaling in natural killer cells.

### IL27 receptor binding proteins

The IL27 receptor (IL27R) includes IL27Rα subunit (IL27Rα) and glycoprotein 130 subunit (gp130). Provided herein is an IL27R binding protein that specifically binds to IL27Rα and gp130. In some embodiments, the IL27R binding protein binds to a mammalian cell expressing both IL27Rα and gp130. In some embodiments, the IL27R binding protein can be a bispecific V_{H}H² as described below. In other embodiments, the IL27R binding protein can include a first domain that is a V_{H}H and a second domain which can be a fragment of IL27 or, for example, a scFv.

The IL27R binding protein can be a bispecific V_{H}H² that has a first V_{H}H binding to IL27Rα (an anti-IL27Rα V_{H}H antibody) and a second V_{H}H binding to gp130 (an anti-gp130 V_{H}H antibody) and causes the dimerization of the two receptor subunits and downstream signaling when bound to a cell expressing IL27Rα and gp130, *e.g.,* a CD8⁺ T cells, a CD4⁺ T cells, and/or a T regulatory (Treg) cell.

A linker can be used to join the anti-IL27Rα V_{H}H antibody and the anti-gp130 V_{H}H antibody. For example, a linker can simply be a covalent bond or a peptide linker. A peptide linker can include between 1 and 50 amino acids (*e.g.,* between 2 and 50, between 5 and 50, between 10 and 50, between 15 and 50, between 20 and 50, between 25 and 50, between 30 and 50, between 35 and 50, between 40 and 50, between 45 and 50, between 2 and 45, between 2 and 40, between 2 and 35, between 2 and 30, between 2 and 25, between 2 and 20, between 2 and 15, between 2 and 10, between 2 and 5 amino acids). A peptide linker joining the anti-IL27Rα V_{H}H antibody and the anti-gp130 V_{H}H antibody can be a flexible glycine-serine linker. A linker can also be a chemical linker, such as a synthetic polymer, e.g., a polyethylene glycol (PEG) polymer.

The anti-IL27Rα V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:70-75.

The anti-IL27Rα V_{H}H antibody can have a sequence having at least 90% (*e.g*., 91%, 92%, 93%, 94%, 95%, 96%, 97%, 98%, 99%, or 100%) sequence identity to the sequence of any one of SEQ ID NOS:125-130.

The anti-gp130 V_{H}H antibody can have a sequence having at least 90% (*e.g*., 91%, 92%, 93%, 94%, 95%, 96%, 97%, 98%, 99%, or 100%) sequence identity to the sequence of any one of SEQ ID NOS:24-29.

The anti-gp130 V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:83-89.

In some embodiments, the IL27R binding protein has a reduced Eₘₐₓ compared to the Eₘₐₓ caused by IL27. Eₘₐₓ reflects the maximum response level in a cell type that can be obtained by a ligand *(e.g.,* a binding protein described herein or the native cytokine *(e.g.,* IL27)). In some embodiments, the IL27R binding protein described herein has at least 1% (*e.g.,* between 1% and 100%, between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ caused by IL27. In other embodiments, the Eₘₐₓ of the IL27R binding protein described herein is greater (*e.g.,* at least 1%, 5%, 10%, 15%, 20%, 25%, 30%, 35%, 40%, 45%, or 50% greater) than the Eₘₐₓ of the natural ligand, IL27. In some embodiments, by varying the linker length of the IL27R binding protein, the Eₘₐₓ of the IL27R binding protein can be changed. The IL27R binding protein can cause Eₘₐₓ in the most desired cell types, and a reduced Eₘₐₓ in other cell types.

An IL27R binding protein described herein are useful in the treatment of neoplastic diseases, such as cancer *(e.g.,* a solid tumor cancer; *e.g.,* non-small-cell lung carcinoma (NSCLC), renal cell carcinoma (RCC), or melanoma) and/or infectious diseases (e.g., bacterial infections and viral infections (*e.g*., viral infections caused by hepatitis C virus (HCV), human papillomavirus (HPV), or human immunodeficiency virus (HIV)) in a subject in need thereof. The IL27R binding protein binds to and activates CD8⁺ T cells, CD4⁺ T cells, and/or T regulatory (Treg) cells. The IL27R binding protein can trigger different levels of downstream signaling in different cell types. For example, by varying the length of the linker between the anti-IL27Rα V_{H}H antibody and the anti-gp130 V_{H}H antibody in the IL27R binding protein, the IL27R binding protein can cause a higher level of downstream signaling in desired cell types compared to undesired cell types. In some embodiments, by varying the linker length, an IL27R binding protein can cause a higher level of downstream signaling in T cells (*e.g*., CD8⁺ T cells) compared to the level of downstream signaling in other cells. In other embodiments, different anti-IL27Rα V_{H}H antibodies with different binding affinities and different anti-gp130 V_{H}H antibodies with different binding affinities can be combined to make different IL27R binding proteins. Further, the orientation of the two antibodies in the binding protein can also be changed to make a different binding protein (*i.e.,* anti-IL27Rα V_{H}H antibody-linker-anti-gp130 V_{H}H antibody, or anti-gp130 V_{H}H antibody-linker-anti-IL27Rα V_{H}H antibody). Different IL27R binding proteins can be screened to find the ideal binding protein that causes a higher level of downstream signaling in desired cell types compared to undesired cell types. In some embodiments, the level of downstream signaling in T cells *(e.g.,* CD8⁺ T cells) is at least 1.1, 1.5, 2, 3, 5, or 10 times of the level of downstream signaling in other cells.

In particular, the IL27R binding protein binds to and activates CD8⁺ T cells. In some embodiments, the IL27R binding protein binds to and activates CXCR5⁺ CD8⁺ T cells. It is known that IL27 can promote and sustain a rapid division of memory-like CXCR5⁺ CD8⁺ T cells during, for example, viral infection. The CXCR5⁺ CD8⁺ T cells can sustain T cell responses during persistent infection or cancer and drive the proliferative burst of CD8⁺ T cells after anti-PD1 treatment. Accordingly, an IL27R binding protein described herein is useful to sustain and augment self-renewing T cells in chronic infections and neoplastic diseases, such as cancer.

### IL10 receptor binding proteins

The IL10 receptor (IL10R) includes IL10R α subunit (IL10Rα) and IL10Rβ subunit (IL10Rβ). Provided herein is an IL10R binding protein that specifically binds to IL10Rα and IL10Rβ. In some embodiments, the IL10R binding protein binds to a mammalian cell expressing both IL10Rα and IL10Rβ. In some embodiments, the IL10R binding protein can be a bispecific V_{H}H ² as described below. In other embodiments, the IL10R binding protein can include a first domain that is a V_{H}H and a second domain which can be a fragment of IL10 or, for example, a scFv.

The IL10R binding protein can be a bispecific V_{H}H² that has a first V_{H}H binding to IL10Rα (an anti-IL10Rα V_{H}H antibody) and a second V_{H}H binding to IL10Rβ (an anti-IL10Rβ V_{H}H antibody) and causes the dimerization of the two receptor subunits and downstream signaling when bound to a cell expressing IL10Rα and IL10Rβ, *e.g*., a T cell (*e.g*., a CD8⁺ T cell or a CD4⁺ T cell), a macrophage, and/or a Treg cell.

A linker can be used to join the anti-IL10Rα V_{H}H antibody and the anti-IL10Rβ V_{H}H antibody. For example, a linker can simply be a covalent bond or a peptide linker. A peptide linker can include between 1 and 50 amino acids (*e.g*., between 2 and 50, between 5 and 50, between 10 and 50, between 15 and 50, between 20 and 50, between 25 and 50, between 30 and 50, between 35 and 50, between 40 and 50, between 45 and 50, between 2 and 45, between 2 and 40, between 2 and 35, between 2 and 30, between 2 and 25, between 2 and 20, between 2 and 15, between 2 and 10, between 2 and 5 amino acids). A peptide linker joining the anti-IL10Rα V_{H}H antibody and the anti-IL10Rβ V_{H}H antibody can be a flexible glycine-serine linker. A linker can also be a chemical linker, such as a synthetic polymer, e.g., a polyethylene glycol (PEG) polymer.

The anti-IL10Rα V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:44-50.

The anti-IL10Rα V_{H}H antibody can have a sequence comprising: a CDR1 having at least 90% *(e.g.,* 91%, 92%, 93%, 94%, 95%, 96%, 97%, 98%, 99%, or 100%) sequence identity, or having 0, 1, 2, or 3 amino acid changes, optionally conservative amino acid changes relative, to the sequence of any one of SEQ ID NOS: 388, 391, 394, 397, 400, 403, and 406; a CDR2 having at least 90% *(e.g.,* 91%, 92%, 93%, 94%, 95%, 96%, 97%, 98%, 99%, or 100%) sequence identity, or having 0, 1, 2, or 3 amino acid changes, optionally conservative amino acid changes relative, to the sequence of any one of SEQ ID NOS: 389, 392, 395, 398, 401, 404, and 407; and a CDR3 having at least 90% *(e.g.,* 91%, 92%, 93%, 94%, 95%, 96%, 97%, 98%, 99%, or 100%) sequence identity, or having 0, 1, 2, or 3 amino acid changes, optionally conservative amino acid changes relative, to the sequence of any one of SEQ ID NOS: 390, 393, 396, 399, 402, 405, and 408.

The anti-IL10Rβ V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:51-57.

The anti-IL10Rβ V_{H}H antibody can have a sequence comprising: a CDR1 having at least 90% *(e.g.,* 91%, 92%, 93%, 94%, 95%, 96%, 97%, 98%, 99%, or 100%) sequence identity, or having 0, 1, 2, or 3 amino acid changes, optionally conservative amino acid changes relative, to the sequence of any one of SEQ ID NOS: 409, 412, 415, 418, 421, 424, and 427: a CDR2 having at least 90% *(e.g.,* 91%, 92%, 93%, 94%, 95%, 96%, 97%, 98%, 99%, or 100%) sequence identity, or having 0, 1, 2, or 3 amino acid changes, optionally conservative amino acid changes relative, to the sequence of any one of SEQ ID NOS: 410, 413, 416, 419, 422, 425, and 428; and a CDR3 having at least 90% (*e.g*., 91%, 92%, 93%, 94%, 95%, 96%, 97%, 98%, 99%, or 100%) sequence identity, or having 0, 1, 2, or 3 amino acid changes, optionally conservative amino acid changes relative, to the sequence of any one of SEQ ID NOS: 411, 414, 417, 420, 423, 426, and 429.

The anti-IL10Rβ V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:99-104.

In some embodiments, the IL10R binding protein has a reduced Eₘₐₓ compared to the Eₘₐₓ caused by IL10. Eₘₐₓ reflects the maximum response level in a cell type that can be obtained by a ligand *(e.g.,* a binding protein described herein or the native cytokine *(e.g.,* IL10)). In some embodiments, the IL10R binding protein described herein has at least 1% (*e.g.,* between 1% and 100%, between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ caused by IL10. In some embodiments, by varying the linker length of the IL10R binding protein, the Eₘₐₓ of the IL10R binding protein can be changed. The IL10R binding protein can cause Eₘₐₓ in the most desired cell types (e.g., CD8⁺ T cells), and a reduced Eₘₐₓ in other cell types (*e.g.,* marcophages). In some embodiments, the Eₘₐₓ in macrophages caused by an IL10R binding protein described herein is between 1% and 100% (e.g., between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ in T cells *(e.g.,* CD8⁺ T cells) caused by the IL10R binding protein. In other embodiments, the Eₘₐₓ of the IL10R binding protein described herein is greater *(e.g.,* at least 1%, 5%, 10%, 15%, 20%, 25%, 30%, 35%, 40%, 45%, or 50% greater) than the Eₘₐₓ of the natural ligand, IL10.

In some embodiments, the present disclosure provides examples of IL10 receptor binding proteins comprising anti-IL10Rα V_{H}H, an optional linker, and an anti-IL10Rβ2 V_{H}H. In some embodiments, the N-terminal V_{H}H of the IL-10 binding molecule is anti-IL10Rα V_{H}H and the C-terminal V_{H}H of the IL-10 receptor binding protein is anti-IL10Rβ V_{H}H, optionally comprising a linker between the V_{H}Hs. In some embodiments, the N-terminal V_{H}H of the IL-10 receptor binding protein is an anti-IL10Rβ V_{H}H and the C-terminal V_{H}H of the IL-10 receptor binding protein is anti-IL10Rα V_{H}H, optionally comprising a linker between the V_{H}Hs. In some embodiments, the IL-10 receptor binding protein may provide a purification handle such as but not limited to the Ala-Ser-His-His-His-His-His-His ("ASH6", SEQ ID NO:430) purification handle to facilitate purification of the receptor binding protein by chelating peptide immobilized metal affinity chromatography ("CP-IMAC, as described in United States Patent No 4,569,794).

A series of ninety-eight IL10 receptor binding proteins comprising anti-IL10Rα V_{H}H, a linker, and an anti-IL10Rβ2 V_{H}H and an ASH6 purification handle (SEQ ID NOs: 192-289) were prepared in substantial accordance with Examples 1-4 herein and evaluated for IL-10 activity in substantial accordance with Examples 5 and 6 herein. The arrangement of V_{H}H, linker and purification handle elements of these ninety-eight IL-10 receptor binding proteins is provided in Table 2 below.

| **Table 2. IL10 Receptor Binding Proteins** | | | | |
|---|---|---|---|---|
| **IL10 Receptor Binding Protein (SEQ ID NO:)** | **N-terminal anti-IL10 V_{H}H (SEQ ID NO:)** | **Linker (SEQ ID NO:)** | **C-terminal anti-IL10 V_{H}H (SEQ ID NO:)** | **C-terminal purification handle (SEQ ID NO:)** |
| 192 | 44 | 23 | 51 | 430 |
| 193 | 44 | 23 | 52 | 430 |
| 194 | 44 | 23 | 53 | 430 |
| 195 | 44 | 23 | 54 | 430 |
| 196 | 44 | 23 | 55 | 430 |
| 197 | 44 | 23 | 56 | 430 |
| 198 | 44 | 23 | 57 | 430 |
| 199 | 45 | 23 | 51 | 430 |
| 200 | 45 | 23 | 52 | 430 |
| 201 | 45 | 23 | 53 | 430 |
| 202 | 45 | 23 | 54 | 430 |
| 203 | 45 | 23 | 55 | 430 |
| 204 | 45 | 23 | 56 | 430 |
| 205 | 45 | 23 | 57 | 430 |
| 206 | 46 | 23 | 51 | 430 |
| 207 | 46 | 23 | 52 | 430 |
| 208 | 46 | 23 | 53 | 430 |
| 209 | 46 | 23 | 54 | 430 |
| 210 | 46 | 23 | 55 | 430 |
| 211 | 46 | 23 | 56 | 430 |
| 212 | 46 | 23 | 57 | 430 |
| 213 | 47 | 23 | 51 | 430 |
| 214 | 47 | 23 | 52 | 430 |
| 215 | 47 | 23 | 53 | 430 |
| 216 | 47 | 23 | 54 | 430 |
| 217 | 47 | 23 | 55 | 430 |
| 218 | 47 | 23 | 56 | 430 |
| 219 | 47 | 23 | 57 | 430 |
| 220 | 48 | 23 | 51 | 430 |
| 221 | 48 | 23 | 52 | 430 |
| 222 | 48 | 24 | 53 | 430 |
| 223 | 48 | 24 | 54 | 430 |
| 224 | 48 | 24 | 55 | 430 |
| 225 | 48 | 24 | 56 | 430 |
| 226 | 48 | 24 | 57 | 430 |
| 227 | 49 | 24 | 51 | 430 |
| 228 | 49 | 24 | 52 | 430 |
| 229 | 49 | 24 | 53 | 430 |
| 230 | 49 | 24 | 54 | 430 |
| 231 | 49 | 24 | 55 | 430 |
| 232 | 49 | 24 | 56 | 430 |
| 233 | 49 | 24 | 57 | 430 |
| 234 | 50 | 24 | 51 | 430 |
| 235 | 50 | 24 | 52 | 430 |
| 236 | 50 | 24 | 53 | 430 |
| 237 | 50 | 24 | 54 | 430 |
| 238 | 50 | 24 | 55 | 430 |
| 239 | 50 | 24 | 56 | 430 |
| 240 | 50 | 24 | 57 | 430 |
| 241 | 51 | 24 | 44 | 430 |
| 242 | 51 | 24 | 45 | 430 |
| 243 | 51 | 24 | 46 | 430 |
| 244 | 51 | 24 | 47 | 430 |
| 245 | 51 | 24 | 48 | 430 |
| 246 | 51 | 24 | 49 | 430 |
| 247 | 51 | 24 | 50 | 430 |
| 248 | 52 | 24 | 44 | 430 |
| 249 | 52 | 24 | 45 | 430 |
| 250 | 52 | 24 | 46 | 430 |
| 251 | 52 | 24 | 47 | 430 |
| 252 | 52 | 24 | 48 | 430 |
| 253 | 52 | 24 | 49 | 430 |
| 254 | 52 | 24 | 50 | 430 |
| 255 | 53 | 24 | 44 | 430 |
| 256 | 53 | 24 | 45 | 430 |
| 257 | 53 | 24 | 46 | 430 |
| 258 | 53 | 24 | 47 | 430 |
| 259 | 53 | 24 | 48 | 430 |
| 260 | 53 | 24 | 49 | 430 |
| 261 | 53 | 24 | 50 | 430 |
| 262 | 54 | 24 | 44 | 430 |
| 263 | 54 | 24 | 45 | 430 |
| 264 | 54 | 24 | 46 | 430 |
| 265 | 54 | 24 | 47 | 430 |
| 266 | 54 | 24 | 48 | 430 |
| 267 | 54 | 24 | 49 | 430 |
| 268 | 54 | 24 | 50 | 430 |
| 269 | 55 | 24 | 44 | 430 |
| 270 | 55 | 24 | 45 | 430 |
| 271 | 55 | 24 | 46 | 430 |
| 272 | 55 | 24 | 47 | 430 |
| 273 | 55 | 24 | 48 | 430 |
| 274 | 55 | 24 | 49 | 430 |
| 275 | 55 | 24 | 50 | 430 |
| 276 | 56 | 24 | 44 | 430 |
| 277 | 56 | 24 | 45 | 430 |
| 278 | 56 | 24 | 46 | 430 |
| 279 | 56 | 24 | 47 | 430 |
| 280 | 56 | 24 | 48 | 430 |
| 281 | 56 | 24 | 49 | 430 |
| 282 | 56 | 24 | 50 | 430 |
| 283 | 57 | 24 | 44 | 430 |
| 284 | 57 | 24 | 45 | 430 |
| 285 | 57 | 24 | 46 | 430 |
| 286 | 57 | 24 | 47 | 430 |
| 287 | 57 | 24 | 48 | 430 |
| 288 | 57 | 24 | 49 | 430 |
| 289 | 57 | 24 | 50 | 430 |

As provided in more detail in the Example 3 herein, nucleic acid sequences encoding SEQ ID Nos: 192-289 were synthesized as SEQ ID Nos: 290-387 respectively and were inserted into a recombinant expression vector and expressed in HEK293 cells in 24 well place format and purified in substantial accordance with Example 4. The supernatants containing the IL-10 receptor binding proteins of SEQ ID Nos: 192-298 were evaluated for activity with unstimulated and wild-type human IL-10 as controls in substantial accordance with Examples 5 and 6 herein. The results of these experiments are provided in Table 3 below.

| Table 3. IL10 Receptor Binding Protein Activity | | |
|---|---|---|
| TestArticle (SEQ ID NO:) | Abs 630 (25 nM) | Abs 630 (100 nM) |
| Unstimulated | 0.58 | 0.59 |
| WildType hIL10 | 2.08 | 2.04 |
| 192 | 0.49 | 0.39 |
| 193 | 0.45 | 0.36 |
| 194 | 0.49 | 0.38 |
| 195 | 1.85 | 1.13 |
| 196 | 0.49 | 0.39 |
| 197 | 0.44 | 0.34 |
| 198 | 1.38 | 0.40 |
| 199 | 1.77 | 1.02 |
| 200 | 1.52 | 0.67 |
| 201 | 0.54 | 0.46 |
| 202 | 0.49 | 0.39 |
| 203 | 0.75 | 0.53 |
| 204 | 0.53 | 0.41 |
| 205 | 0.46 | 0.37 |
| 206 | 1.41 | 0.73 |
| 207 | 1.93 | 1.65 |
| 208 | 0.47 | 0.38 |
| 209 | 0.52 | 0.41 |
| 210 | 0.46 | 0.37 |
| 211 | 0.51 | 0.36 |
| 212 | 0.46 | 0.36 |
| 213 | 1.19 | 1.00 |
| 214 | 1.61 | 1.18 |
| 215 | 0.49 | 0.39 |
| 216 | 0.49 | 0.37 |
| 217 | 0.66 | 0.69 |
| 218 | 0.44 | 0.36 |
| 219 | 0.48 | 0.39 |
| 220 | 0.45 | 0.34 |
| 221 | 0.48 | 0.39 |
| 222 | 0.46 | 0.39 |
| 223 | 0.90 | 0.51 |
| 224 | 0.50 | 0.44 |
| 225 | 0.48 | 0.39 |
| 226 | 0.49 | 0.37 |
| 227 | 1.73 | 0.59 |
| 228 | 0.78 | 0.47 |
| 229 | 0.54 | 0.43 |
| 230 | 0.49 | 0.39 |
| 231 | 0.72 | 0.46 |
| 232 | 0.54 | 0.38 |
| 233 | 0.46 | 0.36 |
| 234 | 0.84 | 0.38 |
| 235 | 0.47 | 0.37 |
| 236 | 2.08 | 2.11 |
| 237 | 2.05 | 1.91 |
| 238 | 1.98 | 2.09 |
| 239 | 1.92 | 1.93 |
| 240 | 1.96 | 2.06 |
| 241 | 0.59 | 0.35 |
| 242 | 0.69 | 0.49 |
| 243 | 0.44 | 0.34 |
| 244 | 0.48 | 0.39 |
| 245 | 0.45 | 0.37 |
| 246 | 0.51 | 0.44 |
| 247 | 0.48 | 0.40 |
| 248 | 0.48 | 0.39 |
| 249 | 0.47 | 0.39 |
| 250 | 0.49 | 0.42 |
| 251 | 0.51 | 0.41 |
| 252 | 0.48 | 0.39 |
| 253 | 0.45 | 0.38 |
| 254 | 0.50 | 0.45 |
| 255 | 0.47 | 0.36 |
| 256 | 0.54 | 0.41 |
| 257 | 0.46 | 0.40 |
| 258 | 0.46 | 0.41 |
| 259 | 0.64 | 0.38 |
| 260 | 0.61 | 0.44 |
| 261 | 0.49 | 0.42 |
| 262 | 0.47 | 0.56 |
| 263 | 0.52 | 0.54 |
| 264 | 0.44 | 0.34 |
| 265 | 0.48 | 0.39 |
| 266 | 0.45 | 0.36 |
| 267 | 0.50 | 0.41 |
| 268 | 0.47 | 0.36 |
| 269 | 0.49 | 0.54 |
| 270 | 1.43 | 1.14 |
| 271 | 0.50 | 0.44 |
| 272 | 0.54 | 0.45 |
| 273 | 0.49 | 0.40 |
| 274 | 0.51 | 0.41 |
| 275 | 0.49 | 0.42 |
| 276 | 0.46 | 0.51 |
| 277 | 0.60 | 0.48 |
| 278 | 0.45 | 0.36 |
| 279 | 0.46 | 0.37 |
| 280 | 0.50 | 0.43 |
| 281 | 0.52 | 0.44 |
| 282 | 0.43 | 0.35 |
| 283 | 0.45 | 0.35 |
| 284 | 0.46 | 0.36 |
| 285 | 0.46 | 0.38 |
| 286 | 0.43 | 0.35 |
| 287 | 0.43 | 0.34 |
| 288 | 0.54 | 0.65 |
| 289 | 0.56 | 0.61 |

| Table 3. IL10 Receptor Binding Protein Activity | | |
|---|---|---|
| TestArticle (SEQ ID NO:) | Abs 630 (25 nM) | Abs 630 (100 nM) |
| Unstimulated | 0.58 | 0.59 |
| WildType hIL10 | 2.08 | 2.04 |
| 192 | 0.49 | 0.39 |
| 193 | 0.45 | 0.36 |
| 194 | 0.49 | 0.38 |
| 195 | 1.85 | 1.13 |
| 196 | 0.49 | 0.39 |
| 197 | 0.44 | 0.34 |
| 198 | 1.38 | 0.40 |
| 199 | 1.77 | 1.02 |
| 200 | 1.52 | 0.67 |
| 201 | 0.54 | 0.46 |
| 202 | 0.49 | 0.39 |
| 203 | 0.75 | 0.53 |
| 204 | 0.53 | 0.41 |
| 205 | 0.46 | 0.37 |
| 206 | 1.41 | 0.73 |
| 207 | 1.93 | 1.65 |
| 208 | 0.47 | 0.38 |
| 209 | 0.52 | 0.41 |
| 210 | 0.46 | 0.37 |
| 211 | 0.51 | 0.36 |
| 212 | 0.46 | 0.36 |
| 213 | 1.19 | 1.00 |
| 214 | 1.61 | 1.18 |
| 215 | 0.49 | 0.39 |
| 216 | 0.49 | 0.37 |
| 217 | 0.66 | 0.69 |
| 218 | 0.44 | 0.36 |
| 219 | 0.48 | 0.39 |
| 220 | 0.45 | 0.34 |
| 221 | 0.48 | 0.39 |
| 222 | 0.46 | 0.39 |
| 223 | 0.90 | 0.51 |
| 224 | 0.50 | 0.44 |
| 225 | 0.48 | 0.39 |
| 226 | 0.49 | 0.37 |
| 227 | 1.73 | 0.59 |
| 228 | 0.78 | 0.47 |
| 229 | 0.54 | 0.43 |
| 230 | 0.49 | 0.39 |
| 231 | 0.72 | 0.46 |
| 232 | 0.54 | 0.38 |
| 233 | 0.46 | 0.36 |
| 234 | 0.84 | 0.38 |
| 235 | 0.47 | 0.37 |
| 236 | 2.08 | 2.11 |
| 237 | 2.05 | 1.91 |
| 238 | 1.98 | 2.09 |
| 239 | 1.92 | 1.93 |
| 240 | 1.96 | 2.06 |
| 241 | 0.59 | 0.35 |
| 242 | 0.69 | 0.49 |
| 243 | 0.44 | 0.34 |
| 244 | 0.48 | 0.39 |
| 245 | 0.45 | 0.37 |
| 246 | 0.51 | 0.44 |
| 247 | 0.48 | 0.40 |
| 248 | 0.48 | 0.39 |
| 249 | 0.47 | 0.39 |
| 250 | 0.49 | 0.42 |
| 251 | 0.51 | 0.41 |
| 252 | 0.48 | 0.39 |
| 253 | 0.45 | 0.38 |
| 254 | 0.50 | 0.45 |
| 255 | 0.47 | 0.36 |
| 256 | 0.54 | 0.41 |
| 257 | 0.46 | 0.40 |
| 258 | 0.46 | 0.41 |
| 259 | 0.64 | 0.38 |
| 260 | 0.61 | 0.44 |
| 261 | 0.49 | 0.42 |
| 262 | 0.47 | 0.56 |
| 263 | 0.52 | 0.54 |
| 264 | 0.44 | 0.34 |
| 265 | 0.48 | 0.39 |
| 266 | 0.45 | 0.36 |
| 267 | 0.50 | 0.41 |
| 268 | 0.47 | 0.36 |
| 269 | 0.49 | 0.54 |
| 270 | 1.43 | 1.14 |
| 271 | 0.50 | 0.44 |
| 272 | 0.54 | 0.45 |
| 273 | 0.49 | 0.40 |
| 274 | 0.51 | 0.41 |
| 275 | 0.49 | 0.42 |
| 276 | 0.46 | 0.51 |
| 277 | 0.60 | 0.48 |
| 278 | 0.45 | 0.36 |
| 279 | 0.46 | 0.37 |
| 280 | 0.50 | 0.43 |
| 281 | 0.52 | 0.44 |
| 282 | 0.43 | 0.35 |
| 283 | 0.45 | 0.35 |
| 284 | 0.46 | 0.36 |
| 285 | 0.46 | 0.38 |
| 286 | 0.43 | 0.35 |
| 287 | 0.43 | 0.34 |
| 288 | 0.54 | 0.65 |
| 289 | 0.56 | 0.61 |

As can be seen from the data provided above, IL-10 receptor binding proteins demonstrated significant IL-10 activity in the IL-10 activity assay (Example 4). In particular, IL-10 activity was categorized as low (above unstimulated and A₆₃₀< 1), medium (A₆₃₀ 1-1.5) and high (A₆₃₀ >1.5) based on absorbance readings. From the above data, 11 IL10R binding proteins demonstrated high activity (SEQ ID Nos: 194, 209, 210, 211, 213, 218, 226, 233, 238, 244 and 250), 4 with medium activity (SEQ ID Nos: 203, 205, 207, and 269) and 8 V_{H}Hs with low activity (SEQ ID Nos: 212, 217, 219, 224, 227, 237, 239, and 249). In some embodiments, the present disclosure provides the IL10R binding protein wherein the IL10R binding protein comprises, from amino to carboxy, a first anti-IL10R sdAb joined via a linker to a second anti-IL10R sdAb, according to the following Table 4:

| Table 4. | |
|---|---|
| first anti-IL10R sdAb SEQ ID | second anti-IL10R sdAb SEQ ID |
| 48 | 57 |
| 49 | 56 |
| 50 | 55 |
| 52 | 46 |
| 47 | 51 |
| 51 | 47 |
| 46 | 55 |
| 46 | 56 |
| 47 | 56 |
| 46 | 54 |
| 44 | 53 |
| 55 | 44 |
| 46 | 52 |
| 45 | 57 |
| 45 | 55 |
| 47 | 55 |
| 50 | 54 |
| 48 | 55 |
| 46 | 57 |
| 47 | 57 |
| 50 | 56 |
| 49 | 51 |
| 52 | 45 |
| 53 | 44 |
| 54 | 47 |

and wherein the IL10R binding protein further optionally comprises a linker is selected from the group consisting of SEQ ID Nos:1-23.

IL10R binding proteins described herein are useful in the treatment of neoplastic diseases, such as cancer *(e.g.,* a solid tumor cancer; *e.g.,* non-small-cell lung carcinoma (NSCLC), renal cell carcinoma (RCC), or melanoma) in a subject in need thereof. The IL10R binding protein binds to and activates CD8⁺ T cells, CD4⁺ T cells, macrophages, and/or Treg cells. In some embodiments, the IL10R binding protein described herein can provide a longer therapeutic efficacy (e.g., lower effective dose, reduced toxicity) than a wild-type or pegylated IL10. The IL10R binding protein can trigger different levels of downstream signaling in different cell types. For example, by varying the length of the linker between the anti-IL10Rα V_{H}H antibody and the anti-IL10Rβ V_{H}H antibody in the IL10R binding protein, the IL10R binding protein can cause a higher level of downstream signaling in desired cell types compared to undesired cell types. In some embodiments the IL10R binding protein can be a partial agonist that selectively activate T cells (*e.g*., CD8⁺ T cells) over macrophages. In some embodiments, activated T cells have an upregulation of IFNgamma. In some embodiments, an IL10R binding protein that is a partial agonist can suppress autoimmune inflammatory diseases such as ulcerative colitis and Crohn's disease. In some embodiments, by varying the linker length, an IL10R binding protein can cause a higher level of downstream signaling in T cells *(e.g.,* CD8⁺ T cells) compared to the level of downstream signaling in macrophages, a cell type that expresses both IL10Rα and IL10Rβ receptors but when activated too potently can cause anemia. When the downstream signaling in macrophages is activated to a high level, these activated macrophages can then eliminate aging red blood cells, causing anemia. An IL10R binding protein can cause a higher level of downstream signaling in T cells (e.g., CD8⁺ T cells) compared to the level of downstream signaling in macrophages, such that anemia is avoided. In other embodiments, different anti-IL10Rα V_{H}H antibodies with different binding affinities and different anti-IL10Rβ V_{H}H antibodies with different binding affinities can be combined to make different IL10R binding proteins. Further, the orientation of the two antibodies in the binding protein can also be changed to make a different binding protein (*i.e.,* anti-IL10Rα V_{H}H antibody-linker-anti-IL10Rβ V_{H}H antibody, or anti-IL10Rβ V_{H}H antibody-linker-anti-IL10Rα V_{H}H antibody). Different IL10R binding proteins can be screened to find the ideal binding protein that causes a higher level of downstream signaling in desired cell types compared to undesired cell types. In some embodiments, the level of downstream signaling in T cells *(e.g.,* CD8⁺ T cells) is at least 1.1, 1.5, 2, 3, 5, or 10 times of the level of downstream signaling in macrophages.

### IFNλ receptor binding proteins

The interferon (IFN) λ receptor (IFNλR) includes IL10Rβ and IL28 receptor (IL28R) α subunit (IL28Rα). Provided herein is an IFNλR binding protein that specifically binds to IL10Rβ and IL28Rα. In some embodiments, the IFNλR binding protein binds to a mammalian cell expressing both IL10Rβ and IL28Rα. In some embodiments, the IFNλR binding protein can be a bispecific V_{H}H² as described below. In other embodiments, the IFNλR binding protein can include a first domain that is a V_{H}H and a second domain which can be a fragment of IFNλ or, for example, a scFv.

The IFNλR binding protein can be a bispecific V_{H}H² that has a first V_{H}H binding to IL10Rβ (an anti-IL10Rβ V_{H}H antibody) and a second V_{H}H binding to IL28Rα (an anti-IL28Rα V_{H}H antibody) and causes the dimerization of the two receptor subunits and downstream signaling when bound to a cell expressing IL10Rβ and IL28R, *e.g*., a macrophage, a T cell (*e.g.,* a CD8⁺ T cell or a CD4⁺ T cell), a Treg cell, a dendritic cell, and/or an epithelial cell.

A linker can be used to join the anti-IL10Rβ V_{H}H antibody and the anti-IL28Rα V_{H}H antibody. For example, a linker can simply be a covalent bond or a peptide linker. A peptide linker can include between 1 and 50 amino acids (*e.g*., between 2 and 50, between 5 and 50, between 10 and 50, between 15 and 50, between 20 and 50, between 25 and 50, between 30 and 50, between 35 and 50, between 40 and 50, between 45 and 50, between 2 and 45, between 2 and 40, between 2 and 35, between 2 and 30, between 2 and 25, between 2 and 20, between 2 and 15, between 2 and 10, between 2 and 5 amino acids). A peptide linker joining the anti-IL10Rβ V_{H}H antibody and the anti-IL28Rα V_{H}H antibody can be a flexible glycine-serine linker. A linker can also be a chemical linker, such as a synthetic polymer, *e.g.*, a polyethylene glycol (PEG) polymer.

The anti-IL10Rβ V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:51-57.

The anti-IL10Rβ V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:99-104.

The anti- IL28Rα V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:76-82.

In some embodiments, the IFNλR binding protein has a reduced Eₘₐₓ compared to the Eₘₐₓ caused by IFNλ. Eₘₐₓ reflects the maximum response level in a cell type that can be obtained by a ligand *(e.g.,* a binding protein described herein or the native cytokine *(e.g.,* IFNλ)). In some embodiments, the IFNλR binding protein described herein has at least 1% (e.g., between 1% and 100%, between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ caused by IFNλ. In other embodiments, the Eₘₐₓ of the IFNλR binding protein described herein is greater (e.g., at least 1%, 5%, 10%, 15%, 20%, 25%, 30%, 35%, 40%, 45%, or 50% greater) than the Eₘₐₓ of the natural ligand, IFNλ. In some embodiments, by varying the linker length of the IFNλR binding protein, the Eₘₐₓ of the IFNλR binding protein can be changed. The IFNλR binding protein can cause Eₘₐₓ in the most desired cell types (e.g., macrophages), and a reduced Eₘₐₓ in other cell types.

The IFNλR binding proteins of the present disclosure are useful in the treatment of an infectious disease in a subject in need thereof. The IFNλR binding protein binds to and activates macrophages, CD8⁺ T cells, CD4⁺ T cells, Treg cells, dendritic cells, and/or epithelial cells. In particular, the IFNλR binding protein binds to and activates macrophages. Examples of infectious diseases include, but are not limited to, influenza, hepatitis B, hepatitis C, and human immunodeficiency virus (HIV) infection. In some embodiments, the IFNλR binding protein can protect Kuppfer cells in the liver against the effects of an infectious disease. The IFNλR binding protein can trigger different levels of downstream signaling in different cell types. For example, by varying the length of the linker between the anti-IL10Rβ V_{H}H antibody and the anti-IL28Rα V_{H}H antibody in the IFNλR binding protein, the IFNλR binding protein can cause a higher level of downstream signaling in desired cell types (*e.g*., macrophages) compared to undesired cell types. In some embodiments, by varying the linker length, an IFNλR binding protein results in the modulation of downstream signaling in macrophages compared to the level of downstream signaling in other cell types. In other embodiments, different anti-IL10Rβ V_{H}H antibodies with different binding affinities and different anti-IL28Rα V_{H}H antibodies with different binding affinities can be combined to make different IFNλR binding proteins. Further, the orientation of the two antibodies in the binding protein can also be changed to make a different binding protein *(i.e.,* anti-IL10Rβ V_{H}H antibody-linker-anti-IL28Rα V_{H}H antibody, or anti-IL28Rα V_{H}H antibody-linker-anti-IL10Rβ V_{H}H antibody). Different IFNλR binding proteins can be screened to find the ideal binding protein that causes a higher level of downstream signaling in desired cell types compared to undesired cell types. In some embodiments, the level of downstream signaling in macrophages is at least 1.1, 1.5, 2, 3, 5, or 10 times of the level of downstream signaling in other cell types.

### IL23 receptor binding proteins

The IL23 receptor (IL23R) includes IL12R β1 subunit (IL12Rβ1) and IL23R subunit. Provided herein is an IL23R binding protein that specifically binds to IL12Rβ1 and IL23R. In some embodiments, the IL23R binding protein binds to a mammalian cell expressing both IL12Rβ1 and IL23R. In some embodiments, the IL23R binding protein can be a bispecific V_{H}H² as described below. In other embodiments, the IL23R binding protein can include a first domain that is a V_{H}H and a second domain which can be a fragment of IL23 or, for example, a scFv.

The IL23R binding protein can be a bispecific V_{H}H² that has a first V_{H}H binding to IL12Rβ1 (an anti-IL12Rβ1 V_{H}H antibody) and a second V_{H}H binding to IL23R (an anti-IL23R V_{H}H antibody) and causes the dimerization of the two receptor subunits and downstream signaling when bound to a cell expressing IL12Rβ1 and IL23R, *e.g.,* a T cell (*e.g.,* a CD8⁺ T cell or a CD4⁺ T cell), a macrophage, and/or a Treg cell.

A linker can be used to join the anti-IL12Rβ1 V_{H}H antibody and the anti-IL23R V_{H}H antibody. For example, a linker can simply be a covalent bond or a peptide linker. A peptide linker can include between 1 and 50 amino acids (*e.g*., between 2 and 50, between 5 and 50, between 10 and 50, between 15 and 50, between 20 and 50, between 25 and 50, between 30 and 50, between 35 and 50, between 40 and 50, between 45 and 50, between 2 and 45, between 2 and 40, between 2 and 35, between 2 and 30, between 2 and 25, between 2 and 20, between 2 and 15, between 2 and 10, between 2 and 5 amino acids). A peptide linker joining the anti-IL12Rβ1 V_{H}H antibody and the anti-IL23R V_{H}H antibody can be a flexible glycine-serine linker. A linker can also be a chemical linker, such as a synthetic polymer, *e.g.,* a polyethylene glycol (PEG) polymer.

The anti-IL12Rβ1 V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:105-111.

The anti-IL23R V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:64-69.

The anti-IL23R V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:118-124.

In some embodiments, the IL23R binding protein has a reduced Eₘₐₓ compared to the Eₘₐₓ caused by IL23. Eₘₐₓ reflects the maximum response level in a cell type that can be obtained by a ligand *(e.g.,* a binding protein described herein or the native cytokine (*e.g*., IL23)). In some embodiments, the IL23R binding protein described herein has at least 1% (e.g., between 1% and 100%, between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ caused by IL23. In some embodiments, by varying the linker length of the IL23R binding protein, the Eₘₐₓ of the IL23R binding protein can be changed. The IL23R binding protein can cause Eₘₐₓ in the most desired cell types (*e.g*., CD8⁺ T cells), and a reduced Eₘₐₓ in other cell types (e.g., marcophages). In some embodiments, the Eₘₐₓ in macrophages caused by an IL23R binding protein described herein is between 1% and 100% (*e.g.,* between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ in T cells (e.g., CD8⁺ T cells) caused by the IL23R binding protein. In other embodiments, the Eₘₐₓ of the IL23R binding protein described herein is greater (*e.g.,* at least 1%, 5%, 10%, 15%, 20%, 25%, 30%, 35%, 40%, 45%, or 50% greater) than the Eₘₐₓ of the natural ligand, IL23.

An IL23R binding protein described herein are useful in wound healing. Particularly, the IL23R binding protein described herein plays an important role in initiating wound healing, e.g., healing of keratinocyte layer of the skin. The IL23R binding protein binds to and activates CD8⁺ T cells, CD4⁺ T cells, macrophages, and/or Treg cells. The IL23R binding protein can trigger different levels of downstream signaling in different cell types. For example, by varying the length of the linker between the anti-IL12Rβ1 V_{H}H antibody and the anti-IL23R V_{H}H antibody in the IL23R binding protein, the IL23R binding protein can cause a higher level of downstream signaling in desired cell types compared to undesired cell types. In some embodiments the IL23R binding protein can be a partial agonist that selectively activate T cells (*e.g.,* CD8⁺ T cells) over macrophages. In other embodiments, different anti-IL12Rβ1 V_{H}H antibodies with different binding affinities and different anti-IL23R V_{H}H antibodies with different binding affinities can be combined to make different IL23R binding proteins. Further, the orientation of the two antibodies in the binding protein can also be changed to make a different binding protein (*i.e.,* anti-IL12Rβ1 V_{H}H antibody-linker-anti-IL23R V_{H}H antibody, or anti-IL23R V_{H}H antibody-linker-anti-IL12Rβ1 V_{H}H antibody). Different IL23R binding proteins can be screened to find the ideal binding protein that causes a higher level of downstream signaling in desired cell types compared to undesired cell types. In some embodiments, the level of downstream signaling in T cells (*e.g*., CD8⁺ T cells) is at least 1.1, 1.5, 2, 3, 5, or 10 times of the level of downstream signaling in macrophages.

### IL2 receptor binding proteins

The IL2 receptor (IL2R) includes CD25 subunit (CD25: also called IL2R α subunit), CD122 subunit (CD122; also called IL2R β subunit), and CD132 subunit (CD132; also called IL2R y subunit). Provided herein is an IL2R binding protein that specifically binds to CD122 and CD132. In some embodiments, the IL2R binding protein binds to a mammalian cell expressing both CD122 and CD132. In some embodiments, the IL2R binding protein can be a bispecific V_{H}H² as described below. In other embodiments, the IL2R binding protein can include a first domain that is a V_{H}H and a second domain which can be a fragment of IL2 or, for example, a scFv.

The IL2R binding protein can be a bispecific V_{H}H² that has a first V_{H}H binding to CD122 (an anti-CD122 V_{H}H antibody) and a second V_{H}H binding to CD132 (an anti-CD132 V_{H}H antibody) and causes the dimerization of the two receptor subunits and downstream signaling when bound to a cell expressing CD122 and CD132, *e.g.,* a T cell *(e.g.,* a CD8⁺ T cell or a CD4⁺ T cell), a macrophage, and/or a Treg cell.

A linker can be used to join the anti-CD122 V_{H}H antibody and the anti-CD132 V_{H}H antibody. For example, a linker can simply be a covalent bond or a peptide linker. A peptide linker can include between 1 and 50 amino acids (e.g., between 2 and 50, between 5 and 50, between 10 and 50, between 15 and 50, between 20 and 50, between 25 and 50, between 30 and 50, between 35 and 50, between 40 and 50, between 45 and 50, between 2 and 45, between 2 and 40, between 2 and 35, between 2 and 30, between 2 and 25, between 2 and 20, between 2 and 15, between 2 and 10, between 2 and 5 amino acids). A peptide linker joining the anti-CD122 V_{H}H antibody and the anti-CD132 V_{H}H antibody can be a flexible glycine-serine linker. A linker can also be a chemical linker, such as a synthetic polymer, *e.g.,* a polyethylene glycol (PEG) polymer.

The anti-CD122 V_{H}H antibody can have a sequence having at least 90% (*e.g.,* 91%, 92%, 93%, 94%, 95%, 96%, 97%, 98%, 99%, or 100%) sequence identity to the sequence of any one of SEQ ID NOS:30-37.

The anti-CD122 V_{H}H antibody can have a sequence having at least 90% (*e.g*., 91%, 92%, 93%, 94%, 95%, 96%, 97%, 98%, 99%, or 100%) sequence identity to the sequence of any one of SEQ ID NOS:90 and 91.

The anti-CD132 V_{H}H antibody can have a sequence having at least 90% (*e.g.,* 91%, 92%, 93%, 94%, 95%, 96%, 97%, 98%, 99%, or 100%) sequence identity to the sequence of any one of SEQ ID NOS:38-43.

The anti-CD132 V_{H}H antibody can have a sequence having at least 90% (*e.g.,* 91%, 92%, 93%, 94%, 95%, 96%, 97%, 98%, 99%, or 100%) sequence identity to the sequence of any one of SEQ ID NOS:92-98.

In some embodiments, the IL2R binding protein has a reduced Eₘₐₓ compared to the Eₘₐₓ caused by IL2. Eₘₐₓ reflects the maximum response level in a cell type that can be obtained by a ligand (*e.g.,* a binding protein described herein or the native cytokine (*e.g.,* IL2)). In some embodiments, the IL2R binding protein described herein has at least 1% (*e.g.*, between 1% and 100%, between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ caused by IL2. In some embodiments, by varying the linker length of the IL2R binding protein, the Eₘₐₓ of the IL2R binding protein can be changed. The IL2R binding protein can cause Eₘₐₓ in the most desired cell types (*e.g.,* CD8⁺ T cells), and a reduced Eₘₐₓ in other cell types (*e.g.,* marcophages). In some embodiments, the Eₘₐₓ in macrophages caused by an IL2R binding protein described herein is between 1% and 100% (*e.g*., between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ in T cells *(e.g.,* CD8⁺ T cells) caused by the IL2R binding protein. In other embodiments, the Eₘₐₓ of the IL2R binding protein described herein is greater *(e.g.,* at least 1%, 5%, 10%, 15%, 20%, 25%, 30%, 35%, 40%, 45%, or 50% greater) than the Eₘₐₓ of the natural ligand, IL2.

An IL2R binding protein described herein are useful in the treatment of neoplastic diseases, such as cancer *(e.g.,* a solid tumor cancer: *e.g*., non-small-cell lung carcinoma (NSCLC), renal cell carcinoma (RCC), melanoma, kidney cancer, or lung cancer) in a subject in need thereof. The IL2R binding protein binds to and activates CD8⁺ T cells. CD4⁺ T cells, macrophages, and/or Treg cells. The IL2R binding protein can trigger different levels of downstream signaling in different cell types. For example, by varying the length of the linker between the anti-CD122 V_{H}H antibody and the anti-CD132 V_{H}H antibody in the IL2R binding protein, the IL2R binding protein can cause a higher level of downstream signaling in desired cell types compared to undesired cell types. In some embodiments, the IL2R binding protein can be a partial agonist that selectively activate T cells (*e.g*., CD8⁺ T cells) over macrophages. In some embodiments, an IL2R binding protein that is a partial agonist can suppress autoimmune inflammatory diseases such as lupus, type-2 diabetes, ulcerative colitis, and Crohn's disease. In some embodiments, by varying the linker length, an IL2R binding protein can cause a higher level of downstream signaling in T cells *(e.g.,* CD8⁺ T cells) compared to the level of downstream signaling in other cell types. In other embodiments, different anti-CD122 V_{H}H antibodies with different binding affinities and different anti-CD132 V_{H}H antibodies with different binding affinities can be combined to make different IL2R binding proteins. Further, the orientation of the two antibodies in the binding protein can also be changed to make a different binding protein *(i.e.,* anti-CD122 V_{H}H antibody-linker-anti-CD132 V_{H}H antibody, or anti-CD132 V_{H}H antibody-linker-anti-CD122 V_{H}H antibody). Different IL2R binding proteins can be screened to find the ideal binding protein that causes a higher level of downstream signaling in desired cell types compared to undesired cell types. In some embodiments, the level of downstream signaling in T cells *(e.g.,* CD8⁺ T cells) is at least 1.1, 1.5, 2, 3, 5, or 10 times of the level of downstream signaling in other cell types.

### IL22 receptor binding proteins

The IL22 receptor (IL22R) includes IL22R1 subunit (IL22R1) and IL10Rβ subunit (IL10Rβ). While IL10Rβ is expressed on a wide range of cells and especially immune cells including monocytes, T cells, B cells and NK cells, in contrast, the expression of the IL22R1 subunit of the IL22 receptor complex is primarily observed in non-immune tissues including the skin, small intestine, liver, colon, lung, kidney, and pancreas, see, e.g., Wolk, et al. (2004) Immunity 21(2):241-254. Provided herein is an IL22R binding protein that specifically binds to IL22R1 and IL10Rβ. In some embodiments, the IL22R binding protein binds to a mammalian cell expressing both IL22R1 and IL10Rβ. In some embodiments, the IL22R binding protein can be a bispecific V_{H}H² as described below. In other embodiments, the IL22R binding protein can include a first domain that is a V_{H}H and a second domain which can be a fragment of IL22 or, for example, a scFv.

The IL22R binding protein can be a bispecific V_{H}H² that has a first V_{H}H binding to IL22R1 (an anti-IL22R1 V_{H}H antibody) and a second V_{H}H binding to IL10Rβ (an anti-IL10Rβ V_{H}H antibody) and causes the dimerization of the two receptor subunits and downstream signaling when bound to a cell expressing IL22R1 and IL10Rβ, *e.g*., an epithelial cell. IL22R is expressed on tissue cells, and it is absent on immune cells. IL22R1 is almost exclusively expressed on cells of non-hematopoietic origin such as epithelial, renal tubular, and pancreatic ductal cells.

A linker can be used to join the anti-IL22R1 V_{H}H antibody and the anti-IL10Rβ V_{H}H antibody. For example, a linker can simply be a covalent bond or a peptide linker. A peptide linker can include between 1 and 50 amino acids (*e.g*., between 2 and 50, between 5 and 50, between 10 and 50, between 15 and 50, between 20 and 50, between 25 and 50, between 30 and 50, between 35 and 50, between 40 and 50, between 45 and 50, between 2 and 45, between 2 and 40, between 2 and 35, between 2 and 30, between 2 and 25, between 2 and 20, between 2 and 15, between 2 and 10, between 2 and 5 amino acids). A peptide linker joining the anti-IL22R1 V_{H}H antibody and the anti-IL10Rβ V_{H}H antibody can be a flexible glycine-serine linker. A linker can also be a chemical linker, such as a synthetic polymer, e.g., a polyethylene glycol (PEG) polymer.

The anti-IL10Rβ V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:51-57.

The anti-IL10Rβ V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:99-104.

In some embodiments, the IL22R binding protein has a reduced Eₘₐₓ compared to the Eₘₐₓ caused by IL22. Eₘₐₓ reflects the maximum response level in a cell type that can be obtained by a ligand *(e.g.,* a binding protein described herein or the native cytokine *(e.g.,* IL22)). In some embodiments, the IL22R binding protein described herein has at least 1% (e.g., between 1% and 100%, between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ caused by IL22. In some embodiments, by varying the linker length of the IL22R binding protein, the Eₘₐₓ of the IL22R binding protein can be changed.

The IL22R binding protein can cause Eₘₐₓ in the most desired cell types (e.g., epithelial cells, IL22R1 expressing tumor cells, and a reduced Eₘₐₓ in other cell types). In some embodiments, the Eₘₐₓ in macrophages caused by an IL22R binding protein described herein is between 1% and 100% (e.g., between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ in epithelials cells caused by the IL22R binding protein. In other embodiments, the Eₘₐₓ of the IL22R binding protein described herein is greater (e.g., at least 1%, 5%, 10%, 15%, 20%, 25%, 30%, 35%, 40%, 45%, or 50% greater) than the Eₘₐₓ of the natural ligand, IL22.

The biological activity of IL22 is modulated by a specific endogenous antagonist, IL22 binding protein (IL22BP) which is regarded as a soluble, neutralizing decoy receptor for IL22. As wild-type IL22 possesses a higher affinity with respect to IL22BP as compared with the IL22 receptor complex, IL22BP is supposed to control IL22 biological activity *in vivo.* In one embodiment, the IL22R binding proteins of the present disclosure may provide preferential binding to the IL22 receptor complex versus the IL22BP avoiding the endogenous antagonism and modulation of IL22 activity derived from the presence of the endogenous IL22BP. In some embodiments, an IL22R binding protein described herein exhibits between 1% and 100% *(e.g.,* between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the affinity of the natural ligand, IL22, for the IL22BP.

An IL22R binding protein described herein are useful in the treatment of neoplastic diseases, such as cancer *(e.g.,* a solid tumor cancer: *e.g*., non-small-cell lung carcinoma (NSCLC), renal cell carcinoma (RCC), melanoma, kidney cancer, or lung cancer) in a subject in need thereof. The IL22R binding protein binds to and activates epithelial cells. The IL22R binding protein can trigger different levels of downstream signaling in the target cell. For example, by varying the length of the linker between the anti-IL22R1 V_{H}H antibody and the anti-IL10Rβ V_{H}H antibody in the IL22R binding protein, the IL22R binding protein can cause a differing (e.g., higher or lower) level of downstream signaling in desired cell types compared to undesired cell types. In some embodiments, the IL22R binding protein can be a partial agonist that selectively activate epithelial cells. In some embodiments, an IL22R binding protein that is a partial agonist is useful in the treatment or prevention of diseases such as psoriasis, graft-versus-host disease, inflammatory diseases of the lung and airway such as lung fibrosis, ventilator induced lung injury, neoplastic disease (e.g., IL22R1-expressing tumors), liver fibrosis, diseases associated with liver injury such as alcohol toxicity (acute or chronic) steatosis,, and pancreatitis, lupus, type-2 diabetes, ulcerative colitis, and Crohn's disease. In some embodiments, by varying the linker length, an IL22R binding protein can cause a higher level of downstream signaling in epithelial cells compared to the level of downstream signaling in other cell types. In other embodiments, different anti-IL22R1 V_{H}H antibodies with different binding affinities and different anti-IL10Rβ V_{H}H antibodies with different binding affinities can be combined to make different IL22R binding proteins. Further, the orientation of the two antibodies in the binding protein can also be changed to make a different binding protein *(i.e.,* anti-IL22R1 V_{H}H antibody-linker-anti-IL10Rβ V_{H}H antibody, or anti-IL10Rβ V_{H}H antibody-linker-anti-IL22R1 V_{H}H antibody). Different IL22R binding proteins can be screened to find the ideal binding protein that causes a higher level of downstream signaling in desired cell types compared to undesired cell types. In some embodiments, the level of downstream signaling in the target cell is at least 1.1, 1.5, 2, 3, 5, or 10 times of the level of downstream signaling in other cell types or cells derived from different tissues.

### Receptor Binding Proteins that Bind to Non-Natural Receptor Pairs

### Receptor binding proteins that bind IL10Rα and IL2Rγ

Provided herein is a binding protein that specifically binds to IL10Rα and IL2Rγ. In some embodiments, the binding protein binds to a mammalian cell expressing both IL10Rα and IL2Rγ. In some embodiments, the binding protein is a bispecific V_{H}H² that has a first V_{H}H that specifically binds to the extracellular domain of IL10Rα (an anti-IL10Rα V_{H}H antibody) and a second V_{H}H that specifically binds to the extracellular domain of IL2Rγ (an anti-IL2Rγ V_{H}H antibody) and causes the dimerization of the two receptor subunits and downstream signaling when bound to a cell expressing IL10Rα and IL2Ry, *e.g*., a T cell (*e.g*., a CD8⁺ T cell and/or a CD4⁺ T cell). In some embodiments, a binding protein that specifically binds to IL10Rα and IL2Rγ can be a bispecific V_{H}H² as described below. In other embodiments, the binding protein can include a first domain that is a V_{H}H and a second domain which can be a fragment of IL10Rα or IL2Rγ or, for example, a scFv.

A linker can be used to join the anti-IL10Rα V_{H}H antibody and the anti-IL2Rγ V_{H}H antibody. For example, a linker can simply be a covalent bond or a peptide linker. A peptide linker can include between 1 and 50 amino acids (*e.g*., between 2 and 50, between 5 and 50, between 10 and 50, between 15 and 50, between 20 and 50, between 25 and 50, between 30 and 50, between 35 and 50, between 40 and 50, between 45 and 50, between 2 and 45, between 2 and 40, between 2 and 35, between 2 and 30, between 2 and 25, between 2 and 20, between 2 and 15, between 2 and 10, between 2 and 5 amino acids). A peptide linker joining the anti-IL10Rα V_{H}H antibody and the anti-IL2Rγ V_{H}H antibody can be a flexible glycine-serine linker. A linker can also be a chemical linker, such as a synthetic polymer, e.g., a polyethylene glycol (PEG) polymer.

The anti-IL10Rα V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:44-50.

The anti-IL2Ry V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:38-43.

The anti-IL2Ry V_{H}H antibody can have a sequence having at least 90% *(e.g.,* 91%, 92%, 93%, 94%, 95%, 96%, 97%, 98%, 99%, or 100%) sequence identity to the sequence of any one of SEQ ID NOS:92-98.

In some embodiments, the binding protein that specifically binds to IL10Rα and IL2Rγ has a reduced Eₘₐₓ compared to the Eₘₐₓ of IL10. Eₘₐₓ reflects the maximum response level in a cell type that can be obtained by a ligand *(e.g.,* a binding protein described herein or the native cytokine (e.g., IL10)). In some embodiments, the binding protein that specifically binds to IL10Rα and IL2Rγ described herein has at least 1% (e.g., between 1% and 100%, between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ caused by IL10. In some embodiments, by varying the linker length of the binding protein that specifically binds to IL10Rα and IL2Ry, the Eₘₐₓ of the binding protein can be changed. The binding protein can cause Eₘₐₓ in the most desired cell types CD8⁺ T cells. In some embodiments, the Eₘₐₓ in CD8⁺ T cells caused by a binding protein that specifically binds to IL10Rα and IL2Rγ is between 1% and 100% (e.g., between 10% and 100%, between 20% and 100%, between 30% and 100%, between 40% and 100%, between 50% and 100%, between 60% and 100%, between 70% and 100%, between 80% and 100%, between 90% and 100%, between 1% and 90%, between 1% and 80%, between 1% and 70%, between 1% and 60%, between 1% and 50%, between 1% and 40%, between 1% and 30%, between 1% and 20%, or between 1% and 10%) of the Eₘₐₓ in other T cells caused by the binding protein. In other embodiments, the Eₘₐₓ of the binding protein that specifically binds to IL10Rα and IL2Rγ is greater (e.g., at least 1%, 5%, 10%, 15%, 20%, 25%, 30%, 35%, 40%, 45%, or 50% greater) than the Eₘₐₓ of the natural ligand.

A binding protein that binds to IL10Rα and IL2Ry as described herein is useful in the treatment of disease in a subject in need thereof including but not limited to the treatment of neoplastic diseases, such as cancer *(e.g.,* a solid tumor cancer; *e.g.,* non-small-cell lung carcinoma (NSCLC), renal cell carcinoma (RCC), or melanoma). The binding protein binds to and activates CD8⁺ T cells and/or CD4⁺ T cells. In certain embodiments, the method does not cause anemia. It is known that IL10 has activities on macrophages and T cells. In some embodiments, the method provided herein uses a binding protein of the present disclosure that binds to IL10Rα and IL2Rγ resulting in the selective activation of T cells relative to activation of macrophages. The selective activation of T cells relative to macrophages is beneficial because IL10-activated macrophages can phagocytose aging red blood cells, which manifests itself as anemia in a patient receiving IL10. Binding proteins as described herein that provide for the selective substantial activation of T cells while providing a minimal activation of macrophages result in a molecule that produces lower side effects, such as anemia, relative to the native IL10 ligand. Other problems and toxicities related to IL10 activation are described in, *e.g.,* Fioranelli and Grazia, *J Integr Cardiol* 1(1):2-6, 2014. Such problems can be avoided by using a binding protein of the present disclosure that specifically binds to IL10Rα and IL2Rγ.

In some embodiments, the binding protein that binds to IL 10Rα and IL2Rγ can trigger different levels of downstream signaling in different cell types. For example, by varying the length of the linker between the anti-IL10Rα V_{H}H antibody and the anti-IL2Rγ V_{H}H antibody in the binding protein, the downstream signaling of the binding protein is modulated in CD8⁺ T cells compared to other T cells. In other embodiments, different anti-IL10Rα V_{H}H antibodies with different binding affinities and different anti-IL2Rγ V_{H}H antibodies with different binding affinities can be combined to make different binding proteins. Further, the orientation of the two antibodies in the binding protein can also be changed to make a different binding protein *(i.e.,* anti-IL10Rα V_{H}H antibody-linker-anti-IL2Rγ V_{H}H antibody, or anti-IL2Rγ V_{H}H antibody-linker-anti-IL10Rα V_{H}H antibody). Different binding proteins can be screened to find the ideal binding protein that causes a higher level of downstream signaling in desired cell types compared to undesired cell types. In some embodiments, the level of downstream signaling in CD8⁺ T cells is at least 1.1, 1.5, 2, 3, 5, or 10 times of the level of downstream signaling in other T cells.

### Receptor binding proteins that bind IFNγR1 or IL28Rα and myeloid cells and/or T cells

Provided herein is also a binding protein that specifically binds to a first receptor and a second receptor, in which the first receptor is interferon y receptor 1 (IFNyR1) or IL28Rα and the second receptor is preferentially expressed on myeloid cells and/or T cells. In some embodiments, the binding protein binds to a mammalian cell expressing both the first receptor and the second receptor. For example, a binding protein can selectively trigger downstream signaling in T cells if the binding protein binds to IFNγR1 as the first receptor and IL2Rγ as the second receptor expressed on T cells. In some embodiments, the binding protein can be a bispecific V_{H}H² as described below. In other embodiments, the binding protein can include a first domain that is a V_{H}H and a second domain which can be a fragment of IFNyR1 or IL28Rα or, for example, a scFv.

In one embodiment, the binding protein is a bispecific V_{H}H² having a first V_{H}H binding that specifically binds to the first receptor (*e.g*., an anti-IFNγR1 V_{H}H antibody or an anti-IL28Rα V_{H}H antibody) and a second V_{H}H that specifically binds to to the second receptor and causes the dimerization of the two receptors and downstream signaling when bound to a cell expressing IFNγR1 or IL28Rα and a cell expressing the second receptor, *e.g.,* a myeloid cell and/or T cell.

A linker can be used to join the two V_{H}Hs. For example, a linker can simply be a covalent bond or a peptide linker. A peptide linker can include between 1 and 50 amino acids (e.g., between 2 and 50, between 5 and 50, between 10 and 50, between 15 and 50, between 20 and 50, between 25 and 50, between 30 and 50, between 35 and 50, between 40 and 50, between 45 and 50, between 2 and 45, between 2 and 40, between 2 and 35, between 2 and 30, between 2 and 25, between 2 and 20, between 2 and 15, between 2 and 10, between 2 and 5 amino acids). A peptide linker joining the two V_{H}Hs can be a flexible glycine-serine linker. A linker can also be a chemical linker, such as a synthetic polymer, *e.g*., a polyethylene glycol (PEG) polymer.

The anti- IL28Rα V_{H}H antibody can have a sequence having at least 90% (*e.g.,* 91%, 92%, 93%, 94%, 95%, 96%, 97%, 98%, 99%, or 100%) sequence identity to the sequence of any one of SEQ ID NOS:76-82.

In certain embodiments of the binding protein described herein, the binding protein binds to the first receptor IFNγR1 and the second receptor IL2Rγ. In particular embodiments, the binding protein can activate T cells and avoid activating macrophages. In other embodiments, different antibodies with different binding affinities to the first receptor and different antibodies with different binding affinities to the second receptor can be combined to make different binding proteins. Further, the orientation of the two antibodies in the binding protein can also be changed to make a different binding protein (*i.e.,* V_{H}H antibody to the first receptor-linker-V_{H}H antibody to the second receptor, or V_{H}H antibody to the second receptor-linker-V_{H}H antibody to the first receptor). Different binding proteins can be screened to find the ideal binding protein that causes a higher level of downstream signaling in desired cell types compared to undesired cell types. In some embodiments, the level of downstream signaling in T cells is at least 1.1, 1.5, 2, 3, 5, or 10 times of the level of downstream signaling in macrophages.

In certain embodiments of the binding protein described herein, the binding protein binds to the first receptor IL28Rα and the second receptor IL2Ry.

The binding protein described herein are useful in the treatment of neoplastic diseases, such as cancer (*e.g.,* a solid tumor cancer; *e.g.,* non-small-cell lung carcinoma (NSCLC), renal cell carcinoma (RCC), or melanoma) in a subject in need thereof. In some embodiments, the binding protein binds to and activates myeloid cells and/or T cells. In particular embodiments, the binding protein binds to and activates macrophages. In particular embodiments, the binding protein binds to and activates CD8⁺ T cells and/or CD4⁺ T cells.

### IV. SINGLE-DOMAIN ANTIBODY AND V_{H}H

A single-domain antibody (sdAb) is an antibody containing a single monomeric variable antibody domain. Like a full-length antibody, it is able to bind selectively to a specific antigen. The complementary determining regions (CDRs) of sdAbs are within a single-domain polypeptide. Single-domain antibodies can be engineered from heavy-chain antibodies found in camelids, which are referred to as V_{H}Hs. Cartilaginous fishes also have heavy-chain antibodies (IgNAR, "immunoglobulin new antigen receptor"), from which single-domain antibodies referred to as V_{NARS} can be obtained. The dimeric variable domains from common immunoglobulin G (IgG) from humans or mice can also be split into monomers to make sdAbs. Although most research into sdAbs is currently based on heavy chain variable domains, sdAbs derived from light chains have also been shown to bind specifically to target, see, *e.g.,* Moller et al., J Biol Chem. 285(49):38348-38361, 2010. In some embodiments, a sdAb is composed of a single monomeric light chain variable antibody domain.

A sdAb can be a heavy chain antibody (V_{H}H). A V_{H}H is a type of sdAb that has a single monomeric heavy chain variable antibody domain. Similar to a traditional antibody, a V_{H}H is able to bind selectively to a specific antigen. A binding protein described herein can include two V_{H}Hs (*e.g.,* V_{H}H²) joined together by a linker (*e.g.,* a peptide linker). The binding protein can be a bispecific V_{H}H² that includes a first V_{H}H binding to a first receptor or domain or subunit thereof and a second V_{H}H binding to a second receptor or domain or subunit thereof, in which the two V_{H}Hs are joined by a linker.

An exemplary V_{H}H has a molecular weight of approximately 12-15 kDa which is much smaller than traditional mammalian antibodies (150-160 kDa) composed of two heavy chains and two light chains. V_{H}Hs can be found in or produced from *Camelidae* mammals (*e.g*., camels, llamas, dromedary, alpaca, and guanaco) which are naturally devoid of light chains. Descriptions of sdAbs and V_{H}HS can be found in, *e.g.,* De Greve et al., Curr Opin Biotechnol. 61:96-101, 2019: Ciccarese, et al., Front Genet. 10:997, 2019; Chanier and Chames, Antibodies (Basel) 8(1), 2019: and De Vlieger et al., Antibodies (Basel) 8(1), 2018.

To prepare a binding protein that is a bispecific V_{H}H², in some embodiments, the two V_{H}Hs can be synthesized separately, then joined together by a linker. Alternatively, the bispecific V_{H}H² can be synthesized as a fusion protein. V_{H}Hs having different binding activities and receptor targets can be paired to make a bispecific V_{H}H². The binding proteins can be screened for signal transduction on cells carrying one or both relevant receptors.

### V. LINKERS

As previously described, the binding domains of the binding proteins of the present disclosure may be joined contiguously (e.g., the C-terminal amino acid of the first V_{H}H in the binding protein to the N-terminal amino acid of the second V_{H}H in the binding protein) or the binding domains of the binding protein may optionally be joined via a linker. A linker is a linkage between two elements, *e.g.,* protein domains. In a bispecific V_{H}H² binding protein described herein, a linker is a linkage between the two V_{H}Hs in the binding protein. A linker can be a covalent bond or a peptide linker. In some embodiments, the two V_{H}Hs in a binding protein are joined directly (*i.e.,* via a covalent bond). The length of the linker between two V_{H}Hs in a binding protein can be used to modulate the proximity of the two V_{H}Hs of the binding protein. By varying the length of the linker, the overall size and length of the binding protein can be tailored to bind to specific cell receptors or domains or subunits thereof. For example, if the binding protein is designed to bind to two receptors or domains or subunits thereof that are located close to each other on the same cell, then a short linker can be used. In another example, if the binding protein is designed to bind to two receptors or domains or subunits there of that are located on two different cells, then a long linker can be used.

In some embodiments, the linker is a peptide linker. A peptide linker can include between 1 and 50 amino acids (*e.g.,* between 2 and 50, between 5 and 50, between 10 and 50, between 15 and 50, between 20 and 50, between 25 and 50, between 30 and 50, between 35 and 50, between 40 and 50, between 45 and 50, between 2 and 45, between 2 and 40, between 2 and 35, between 2 and 30, between 2 and 25, between 2 and 20, between 2 and 15, between 2 and 10, between 2 and 5 amino acids). A linker can also be a chemical linker, such as a synthetic polymer, *e.g*., a polyethylene glycol (PEG) polymer.

In some embodiments, a linker joins the C-terminus of the first V_{H}H in the binding protein to the N-terminus of the second V_{H}H in the binding protein. In other embodiments, a linker joins the C-terminus of the second V_{H}H in the binding protein to the N-terminus of the first V_{H}H in the binding protein.

Suitable peptide linkers are known in the art, and include, for example, peptide linkers containing flexible amino acid residues such as glycine and serine. In certain embodiments, a peptide linker can contain motifs, *e.g.,* multiple or repeating motifs, of GS, GGS, GGGGS (SEQ ID NO:1), GGGGGS (SEQ ID NO:2), GGSG (SEQ ID NO:3), or SGGG (SEQ ID NO:4). In certain embodiments, a peptide linker can contain 2 to 12 amino acids including motifs of GS, *e.g.,* GS, GSGS (SEQ ID NO:5), GSGSGS (SEQ ID NO:6), GSGSGSGS (SEQ ID NO:191), GSGSGSGSGS (SEQ ID NO:7), or GSGSGSGSGSGS (SEQ ID NO:8). In certain other embodiments, a peptide linker can contain 3 to 12 amino acids including motifs of GGS, *e.g.,* GGS, GGSGGS (SEQ ID NO:9), GGSGGSGGS (SEQ ID NO:10), and GGSGGSGGSGGS (SEQ ID NO:11). In yet other embodiments, a peptide linker can contain 4 to 20 amino acids including motifs of GGSG (SEQ ID NO:3), *e.g*., GGSGGGSG (SEQ ID NO:12), GGSGGGSGGGSG (SEQ ID NO:13), GGSGGGSGGGSGGGSG (SEQ ID NO:14), or GGSGGGSGGGSGGGSGGGSG (SEQ ID NO:15). In other embodiments, a peptide linker can contain motifs of GGGGS (SEQ ID NO:1), *e.g*., GGGGSGGGGS (SEQ ID NO:16) or GGGGSGGGGSGGGGS (SEQ ID NO:17).

### VI. MODIFICATIONS TO EXTEND DURATION OF ACTION IN VIVO

The binding proteins described herein can be modified to provide for an extended lifetime *in vivo* and/or extended duration of action in a subject. In some embodiments, the binding protein can be conjugated to carrier molecules to provide desired pharmacological properties such as an extended half-life. In some embodiments, the binding protein can be covalently linked to the Fc domain of IgG, albumin, or other molecules to extend its half-life, *e.g.,* by pegylation, glycosylation, and the like as known in the art.

In some embodiments, the binding protein is conjugated to a functional domain of an Fc-fusion chimeric polypeptide molecule. Fc fusion conjugates have been shown to increase the systemic half-life of biopharmaceuticals, and thus the biopharmaceutical product can require less frequent administration. Fc binds to the neonatal Fc receptor (FcRn) in endothelial cells that line the blood vessels, and, upon binding, the Fc fusion molecule is protected from degradation and re-released into the circulation, keeping the molecule in circulation longer. This Fc binding is believed to be the mechanism by which endogenous IgG retains its long plasma half-life. More recent Fc-fusion technology links a single copy of a biopharmaceutical to the Fc region of an antibody to optimize the pharmacokinetic and pharmacodynamic properties of the biopharmaceutical as compared to traditional Fc-fusion conjugates. The "Fc region" useful in the preparation of Fc fusions can be a naturally occurring or synthetic polypeptide that is homologous to an IgG C-terminal domain produced by digestion of IgG with papain. IgG Fc has a molecular weight of approximately 50 kDa. The binding protein described herein can be conjugated to the entire Fc region, or a smaller portion that retains the ability to extend the circulating half- life of a chimeric polypeptide of which it is a part. In addition, full-length or fragmented Fc regions can be variants of the wild-type molecule. In a typical presentation, each monomer of the dimeric Fc can carry a heterologous polypeptide, the heterologous polypeptides being the same or different.

In some embodiments, when the binding protein described herein is to be administered in the format of an Fc fusion, particularly in those situations when the polypeptide chains conjugated to each subunit of the Fc dimer are different, the Fc fusion may be engineered to possess a "knob-into-hole modification." The knob-into-hole modification is more fully described in Ridgway, et al. (1996) Protein Engineering 9(7):617-621 and United States Patent No. 5,731,168, issued March 24, 1998. The knob-into-hole modification refers to a modification at the interface between two immunoglobulin heavy chains in the CH3 domain, wherein: i) in a CH3 domain of a first heavy chain, an amino acid residue is replaced with an amino acid residue having a larger side chain (*e.g.,* tyrosine or tryptophan) creating a projection from the surface ("knob"), and ii) in the CH3 domain of a second heavy chain, an amino acid residue is replaced with an amino acid residue having a smaller side chain (*e.g.,* alanine or threonine), thereby generating a cavity ("hole") at interface in the second CH3 domain within which the protruding side chain of the first CH3 domain ("knob") is received by the cavity in the second CH3 domain. In one embodiment, the "knob-into-hole modification" comprises the amino acid substitution T366W and optionally the amino acid substitution S354C in one of the antibody heavy chains, and the amino acid substitutions T366S, L368A, Y407V and optionally Y349C in the other one of the antibody heavy chains. Furthermore, the Fc domains may be modified by the introduction of cysteine residues at positions S354 and Y349 which results in a stabilizing disulfide bridge between the two antibody heavy chains in the Fc region (Carter, et al. (2001) Immunol Methods 248, 7-15). The knob-into-hole format is used to facilitate the expression of a first polypeptide on a first Fc monomer with a "knob" modification and a second polypeptide on the second Fc monomer possessing a "hole" modification to facilitate the expression of heterodimeric polypeptide conjugates.

In some embodiments, the binding protein can be conjugated to one or more watersoluble polymers. Examples of water soluble polymers useful in the practice of the present disclosure include polyethylene glycol (PEG), poly-propylene glycol (PPG), polysaccharides (polyvinylpyrrolidone, copolymers of ethylene glycol and propylene glycol, poly(oxyethylated polyol), polyolefinic alcohol,), polysaccharides), poly-alpha-hydroxy acid), polyvinyl alcohol (PVA), polyphosphazene, polyoxazolines (POZ), poly(N-acryloylmorpholine), or a combination thereof.

In some embodiments, binding protein can be conjugated to one or more polyethylene glycol molecules or "PEGylated." Although the method or site of PEG attachment to the binding protein may vary, in certain embodiments the PEGylation does not alter, or only minimally alters, the activity of the binding protein.

In some embodiments, selective PEGylation of the binding protein, for example, by the incorporation of non-natural amino acids having side chains to facilitate selective PEG conjugation, may be employed. Specific PEGylation sites can be chosen such that PEGylation of the binding protein does not affect its binding to the target receptors.

In certain embodiments, the increase in half-life is greater than any decrease in biological activity. PEGs suitable for conjugation to a polypeptide sequence are generally soluble in water at room temperature, and have the general formula R(O-CH₂-CH₂)ₙO-R, where R is hydrogen or a protective group such as an alkyl or an alkanol group, and where n is an integer from 1 to 1000. When R is a protective group, it generally has from 1 to 8 carbons. The PEG conjugated to the polypeptide sequence can be linear or branched. Branched PEG derivatives, "star-PEGs" and multi-armed PEGs are contemplated by the present disclosure.

A molecular weight of the PEG used in the present disclosure is not restricted to any particular range. The PEG component of the binding protein can have a molecular mass greater than about 5kDa, greater than about 10kDa, greater than about 15kDa, greater than about 20kDa, greater than about 30kDa, greater than about 40kDa, or greater than about 50kDa. In some embodiments, the molecular mass is from about 5kDa to about 10kDa, from about 5kDa to about 15kDa, from about 5kDa to about 20kDa, from about 10kDa to about 15kDa, from about 10kDa to about 20kDa, from about 10kDa to about 25kDa, or from about 10kDa to about 30kDa. Linear or branched PEG molecules having molecular weights from about 2,000 to about 80,000 daltons, alternatively about 2,000 to about 70,000 daltons, alternatively about 5,000 to about 50,000 daltons, alternatively about 10,000 to about 50,000 daltons, alternatively about 20,000 to about 50,000 daltons, alternatively about 30,000 to about 50,000 daltons, alternatively about 20,000 to about 40,000 daltons, or alternatively about 30,000 to about 40,000 daltons. In one embodiment of the disclosure, the PEG is a 40kD branched PEG comprising two 20 kD arms.

The present disclosure also contemplates compositions of conjugates wherein the PEGs have different n values, and thus the various different PEGs are present in specific ratios. For example, some compositions comprise a mixture of conjugates where n=1, 2, 3 and 4. In some compositions, the percentage of conjugates where n=1 is 18-25%, the percentage of conjugates where n=2 is 50-66%, the percentage of conjugates where n=3 is 12-16%, and the percentage of conjugates where n=4 is up to 5%. Such compositions can be produced by reaction conditions and purification methods known in the art. Chromatography may be used to resolve conjugate fractions, and a fraction is then identified which contains the conjugate having, for example, the desired number of PEGs attached, purified free from unmodified protein sequences and from conjugates having other numbers of PEGs attached.

PEGs suitable for conjugation to a polypeptide sequence are generally soluble in water at room temperature, and have the general formula R(O-CH₂-CH₂)ₙO-R, where R is hydrogen or a protective group such as an alkyl or an alkanol group, and where n is an integer from 1 to 1000. When R is a protective group, it generally has from 1 to 8 carbons.

Two widely used first generation activated monomethoxy PEGs (mPEGs) are succinimdyl carbonate PEG (SC-PEG: see, *e.g.,* Zalipsky, et al. (1992) Biotehnol. Appl. Biochem 15:100-114) and benzotriazole carbonate PEG (BTC-PEG; see, *e.g.,* Dolence, et al. US Patent No. 5,650,234), which react preferentially with lysine residues to form a carbamate linkage but are also known to react with histidine and tyrosine residues. Use of a PEG-aldehyde linker targets a single site on the N-terminus of a polypeptide through reductive amination.

Pegylation most frequently occurs at the α-amino group at the N-terminus of the polypeptide, the epsilon amino group on the side chain of lysine residues, and the imidazole group on the side chain of histidine residues. Since most recombinant polypeptides possess a single alpha and a number of epsilon amino and imidazole groups, numerous positional isomers can be generated depending on the linker chemistry. General PEGylation strategies known in the art can be applied herein.

The PEG can be bound to a binding protein of the present disclosure via a terminal reactive group (a "spacer") which mediates a bond between the free amino or carboxyl groups of one or more of the polypeptide sequences and polyethylene glycol. The PEG having the spacer which can be bound to the free amino group includes N-hydroxysuccinylimide polyethylene glycol, which can be prepared by activating succinic acid ester of polyethylene glycol with N-hydroxysuccinylimide.

In some embodiments, the PEGylation of the binding proteins is facilitated by the incorporation of non-natural amino acids bearing unique side chains to facilitate site specific PEGylation. The incorporation of non-natural amino acids into polypeptides to provide functional moieties to achieve site specific PEGylation of such polypeptides is known in the art. See *e.g.,* Ptacin et al., PCT International Application No. PCT/US2018/045257 filed August 3, 2018 and published February 7, 2019 as International Publication Number WO 2019/028419Al.

The PEG conjugated to the polypeptide sequence can be linear or branched. Branched PEG derivatives, "star-PEGs" and multi-armed PEGs are contemplated by the present disclosure. Specific embodiments PEGs useful in the practice of the present disclosure include a 10kDa linear PEG-aldehyde (*e.g.,* Sunbright^{®} ME-100AL, NOF America Corporation, One North Broadway, White Plains, NY 10601 USA), 10kDa linear PEG-NHS ester (*e.g.,* Sunbright^{®} ME-100CS, Sunbright^{®} ME-100AS, Sunbright^{®} ME-100GS, Sunbright^{®} ME-100HS, NOF), a 20kDa linear PEG-aldehyde *(*e.g., Sunbright^{®} ME-200AL, NOF), a 20kDa linear PEG- NHS ester (*e.g.,* Sunbright^{®} ME-200CS, Sunbright^{®} ME-200AS, Sunbright^{®} ME-200GS, Sunbright^{®} ME-200HS, NOF), a 20kDa 2-arm branched PEG-aldehyde the 20 kDA PEG-aldehyde comprising two 10kDA linear PEG molecules (*e.g.,* Sunbright^{®} GL2-200AL3, NOF), a 20kDa 2-arm branched PEG-NHS ester the 20 kDA PEG-NHS ester comprising two 10kDA linear PEG molecules (*e.g.,* Sunbright^{®} GL2-200TS, Sunbright^{®} GL200GS2, NOF), a 40kDa 2-arm branched PEG-aldehyde the 40 kDA PEG-aldehyde comprising two 20kDA linear PEG molecules (*e.g.*, Sunbright^{®} GL2-400AL3), a 40kDa 2-arm branched PEG-NHS ester the 40 kDA PEG-NHS ester comprising two 20kDA linear PEG molecules (*e.g.,* Sunbright^{®} GL2-400AL3, Sunbright^{®} GL2-400GS2, NOF), a linear 30kDa PEG-aldehyde (*e.g.,* Sunbright^{®} ME-300AL) and a linear 30kDa PEG-NHS ester.

In some embodiments, a linker can used to join the binding protein and the PEG molecule. Suitable linkers include "flexible linkers" which are generally of sufficient length to permit some movement between the modified polypeptide sequences and the linked components and molecules. The linker molecules are generally about 6-50 atoms long. The linker molecules may also be, for example, aryl acetylene, ethylene glycol oligomers containing 2-10 monomer units, diamines, diacids, amino acids, or combinations thereof. Suitable linkers can be readily selected and can be of any suitable length, such as 1 amino acid (*e.g.,* Gly), 2, 3, 4, 5, 6, 7, 8, 9, 10, 10-20, 20-30, 30-50 or more than 50 amino acids.

Examples of flexible linkers include glycine polymers (G)n, glycine-alanine polymers, alanine-serine polymers, glycine-serine polymers (for example, (GmSo)n, (GSGGS)n, (GmSoGm)n, (GmSoGmSoGm)n, (GSGGSm)n, (GSGSmG)n and (GGGSm)n, and combinations thereof, where m, n, and o are each independently selected from an integer of at least 1 to 20, *e.g.,* 1-18, 216, 3-14, 4-12, 5-10, 1, 2, 3, 4, 5, 6, 7, 8,9, or 10), and other flexible linkers. Glycine and glycine-serine polymers are relatively unstructured, and therefore may serve as a neutral tether between components. Examples of flexible linkers include, but are not limited to GGSG (SEQ ID NO:3), GGSGG (SEQ ID NO:18), GSGSG (SEQ ID NO:19), GSGGG (SEQ ID NO:20), GGGSG (SEQ ID NO:21), and GSSSG (SEQ ID NO:22). Other examples of flexible linkers are described in Section V.

Additional examples of flexible linkers include glycine polymers (G)n or glycine-serine polymers (*e.g.,* (GS)n, (GSGGS)n, (GGGS)n and (GGGGS)n, where n=1 to 50, for example, 1, 2, 3, 4, 5, 6, 7, 8, 9, 10, 10-20, 20-30, 30-50). Exemplary flexible linkers include, but are not limited to GGGS (SEQ ID NO:23), GGGGS (SEQ ID NO:1), GGSG (SEQ ID NO:3), GGSGG (SEQ ID NO:18), GSGSG (SEQ ID NO:19), GSGGG (SEQ ID NO:20), GGGSG (SEQ ID NO:21), and GSSSG (SEQ ID NO:22). A multimer (*e.g.*, 1, 2, 3, 4, 5, 6, 7, 8, 9, 10, 10-20, 20-30, or 30-50) of these linker sequences may be linked together to provide flexible linkers that may be used to conjugate two molecules. Alternative to a polypeptide linker, the linker can be a chemical linker, *e.g.,* a PEG-aldehyde linker. In some embodiments, the binding protein is acetylated at the N-terminus by enzymatic reaction with N-terminal acetyltransferase and, for example, acetyl CoA. Alternatively, or in addition to N-terminal acetylation, the binding protein can be acetylated at one or more lysine residues, e.g., by enzymatic reaction with a lysine acetyltransferase. See, for example Choudhary et al. (2009) Science 325 (5942):834-840.

In other embodiments, the binding protein can be modified to include an additional polypeptide sequence that functions as an antigenic tag, such as a FLAG sequence. FLAG sequences are recognized by biotinylated, highly specific, anti-FLAG antibodies, as described herein (see *e.g.,* Blanar et al. (1992) Science 256:1014 and LeClair, et al. (1992) PNAS-USA 89:8145). In some embodiments, the binding protein further comprises a C-terminal c-myc epitope tag.

In some embodiments, the binding protein is expressed as a fusion protein with an albumin molecule (*e.g.,* human serum albumin) which is known in the art to facilitate extended exposure *in vivo.*

In some embodiment, the binding proteins (including fusion proteins of the binding proteins) of the present disclosure are expressed as a fusion protein with one or more transition metal chelating polypeptide sequences. The incorporation of such a transition metal chelating domain facilitates purification immobilized metal affinity chromatography (IMAC) as described in Smith, et al. United States Patent No. 4,569,794 issued February 11, 1986. Examples of transition metal chelating polypeptides useful in the practice of the present disclosure are described in Smith, et al. *supra* and Dobeli, et al. United States Patent No. 5,320,663 issued May 10, 1995, the entire teachings of which are hereby incorporated by reference. Particular transition metal chelating polypeptides useful in the practice of the present disclosure are peptides comprising 3-6 contiguous histidine residues such as a six-histidine peptide (His)₆ and are frequently referred to in the art as "His-tags."

The foregoing fusion proteins may be readily produced by recombinant DNA methodology by techniques known in the art by constructing a recombinant vector comprising a nucleic acid sequence comprising a nucleic acid sequence encoding the binding protein in frame with a nucleic acid sequence encoding the fusion partner either at the N-terminus or C-terminus of the binding protein, the sequence optionally further comprising a nucleic acid sequence in frame encoding a linker or spacer polypeptide.

### VII. PHARMACEUTICAL COMPOSITION

The binding proteins of the present disclosure may be administered to a subject in a pharmaceutically acceptable dosage form. The preferred formulation depends on the intended mode of administration and therapeutic application. Pharmaceutical dosage forms of the binding proteins described herein comprise physiologically acceptable carriers that are inherently non-toxic and non-therapeutic. Examples of such carriers include ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, and PEG. Carriers for topical or gel-based forms of polypeptides include polysaccharides such as sodium carboxymethylcellulose or methylcellulose, polyvinylpyrrolidone, polyacrylates, polyoxyethylene-polyoxypropylene-block polymers, PEG, polymeric amino acids, amino acid copolymers, and lipid aggregates (such as oil droplets or liposomes).

The pharmaceutical compositions may also comprise pharmaceutically-acceptable, non-toxic carriers, excipients, stabilizers, or diluents, which are defined as vehicles commonly used to formulate pharmaceutical compositions for animal or human administration. The diluent is selected so as not to affect the biological activity of the combination. Acceptable carriers, excipients, or stabilizers are non-toxic to recipients at the dosages and concentrations employed, and include buffers such as phosphate, citrate, and other organic acids; antioxidants including ascorbic acid and methionine; preservatives (such as octadecyidimethylbenzyl ammonium chloride; hexamethonium chloride: benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine: monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

Formulations to be used for *in vivo* administration are typically sterile. Sterilization of the compositions of the present disclosure may readily accomplished by filtration through sterile filtration membranes.

Typically, compositions are prepared as injectables, either as liquid solutions or suspensions: solid forms suitable for solution in, or suspension in, liquid vehicles prior to injection can also be prepared. The preparation also can be emulsified or encapsulated in liposomes or micro particles such as polylactide, polyglycolide, or copolymer for enhanced adjuvant effect, as discussed above (Langer, Science 249: 1527, 1990 and Hanes, Advanced Drug Delivery Reviews 28: 97-119, 1997). The agents of this disclosure can be administered in the form of a depot injection or implant preparation which can be formulated in such a manner as to permit a sustained or pulsatile release of the active ingredient. The pharmaceutical compositions are generally formulated as sterile, substantially isotonic and in full compliance with all Good Manufacturing Practice (GMP) regulations of the U.S. Food and Drug Administration.

Administration of a binding protein described herein may be achieved through any of a variety of art recognized methods including but not limited to the topical, intravascular injection (including intravenous or intraarterial infusion), intradermal injection, subcutaneous injection, intramuscular injection, intraperitoneal injection, intracranial injection, intratumoral injection, intranodal injection, transdermal, transmucosal, iontophoretic delivery, intralymphatic injection (Senti and Kundig (2009) Current Opinions in Allergy and Clinical Immunology 9(6):537-543), intragastric infusion, intraprostatic injection, intravesical infusion (e.g., bladder), respiratory inhalers including nebulizers, intraocular injection, intraabdominal injection, intralesional injection, intraovarian injection, intracerebral infusion or injection, intracerebroventricular injection (ICVI), and the like. In some embodiments, administration includes the administration of the binding protein itself (*e.g.,* parenteral), as well as the administration of a recombinant vector (*e.g.,* viral or non-viral vector) to cause the *in situ* expression of the binding protein in the subject. Alternatively, a cell, such as a cell isolated from the subject, could also be recombinantly modified to express the binding protein of the present disclosure.

The dosage of the pharmaceutical compositions depends on factors including the route of administration, the disease to be treated, and physical characteristics, *e.g.,* age, weight, general health, of the subject. Typically, the amount of a binding protein contained within a single dose may be an amount that effectively prevents, delays, or treats the disease without inducing significant toxicity. A pharmaceutical composition of the disclosure may include a dosage of a binding protein described herein ranging from 0.01 to 500 mg/kg (*e.g.,* from 0.01 to 450 mg, from 0.01 to 400 mg, from 0.01 to 350 mg, from 0.01 to 300 mg, from 0.01 to 250 mg, from 0.01 to 200 mg, from 0.01 to 150 mg, from 0.01 to 100 mg, from 0.01 to 50 mg, from 0.01 to 10 mg, from 0.01 to 1 mg, from 0.1 to 500 mg/kg, from 1 to 500 mg/kg, from 5 to 500 mg/kg, from 10 to 500 mg/kg, from 50 to 500 mg/kg, from 100 to 500 mg/kg, from 150 to 500 mg/kg, from 200 to 500 mg/kg, from 250 to 500 mg/kg, from 300 to 500 mg/kg, from 350 to 500 mg/kg, from 400 to 500 mg/kg, or from 450 to 500 mg/kg) and, in a more specific embodiment, about 1 to about 100 mg/kg (*e.g.,* about 1 to about 90 mg/kg, about 1 to about 80 mg/kg, about 1 to about 70 mg/kg, about 1 to about 60 mg/kg, about 1 to about 50 mg/kg, about 1 to about 40 mg/kg, about 1 to about 30 mg/kg, about 1 to about 20 mg/kg, about 1 to about 10 mg/kg, about 10 to about 100 mg/kg, about 20 to about 100 mg/kg, about 30 to about 100 mg/kg, about 40 to about 100 mg/kg, about 50 to about 100 mg/kg, about 60 to about 100 mg/kg, about 70 to about 100 mg/kg, about 80 to about 100 mg/kg, or about 90 to about 100 mg/kg). In some embodiments, a pharmaceutical composition of the disclosure may include a dosage of a binding protein described herein ranging from 0.01 to 20 mg/kg (*e.g.,* from 0.01 to 15 mg/kg, from 0.01 to 10 mg/kg, from 0.01 to 8 mg/kg, from 0.01 to 6 mg/kg, from 0.01 to 4 mg/kg, from 0.01 to 2 mg/kg, from 0.01 to 1 mg/kg, from 0.01 to 0.1 mg/kg, from 0.01 to 0.05 mg/kg, from 0.05 to 20 mg/kg, from 0.1 to 20 mg/kg, from 1 to 20 mg/kg, from 2 to 20 mg/kg, from 4 to 20 mg/kg, from 6 to 20 mg/kg, from 8 to 20 mg/kg, from 10 to 20 mg/kg, from 15 to 20 mg/kg). The dosage may be adapted by the physician in accordance with conventional factors such as the extent of the disease and different parameters of the subject.

A pharmaceutical composition containing a binding protein described herein can be administered to a subject in need thereof, for example, one or more times (*e.g.,* 1-10 times or more) daily, weekly, monthly, biannually, annually, or as medically necessary. Dosages may be provided in either a single or multiple dosage regimens. The timing between administrations may decrease as the medical condition improves or increase as the health of the patient declines. A course of therapy may be a single dose or in multiple doses over a period of time. In some embodiments, a single dose is used. In some embodiments, two or more split doses administered over a period of 1, 2, 3, 4, 5, 6, 7, 8, 9, 10, 11, 12, 13, 14, 21, 28, 30, 60, 90, 120 or 180 days are used. Each dose administered in such split dosing protocols may be the same in each administration or may be different. Multi-day dosing protocols over time periods may be provided by the skilled artisan (*e.g.,* physician) monitoring the administration, taking into account the response of the subject to the treatment including adverse effects of the treatment and their modulation as discussed above.

### VIII. INDICATIONS

### Neoplastic Diseases

The present disclosure provides methods of use of binding proteins in the treatment of subjects suffering from a neoplastic disease by the administration of a therapeutically effective amount of a binding protein (or nucleic acid encoding a binding protein including recombinant vectors encoding binding proteins) as described herein.

The compositions and methods of the present disclosure are useful in the treatment of subject suffering from a neoplastic disease characterized by the presence neoplasms, including benign and malignant neoplasms, and neoplastic disease. Examples benign neoplasms amenable to treatment using the compositions and methods of the present disclosure include but are not limited to adenomas, fibromas, hemangiomas, and lipomas. Examples of pre-malignant neoplasms amenable to treatment using the compositions and methods of the present disclosure include but are not limited to hyperplasia, atypia, metaplasia, and dysplasia. Examples of malignant neoplasms amenable to treatment using the compositions and methods of the present disclosure include but are not limited to carcinomas (cancers arising from epithelial tissues such as the skin or tissues that line internal organs), leukemias, lymphomas, and sarcomas typically derived from bone fat, muscle, blood vessels or connective tissues). Also included in the term neoplasms are viral induced neoplasms such as warts and EBV induced disease *(i.e.,* infectious mononucleosis), scar formation, hyperproliferative vascular disease including intimal smooth muscle cell hyperplasia, restenosis, and vascular occlusion and the like.

The term "neoplastic disease" includes cancers characterized by solid tumors and non-solid tumors including but not limited to breast cancers; sarcomas (including but not limited to osteosarcomas and angiosarcomas and fibrosarcomas), leukemias, lymphomas, genitourinary cancers (including but not limited to ovarian, urethral, bladder, and prostate cancers); gastrointestinal cancers (including but not limited to colon esophageal and stomach cancers); lung cancers; myelomas: pancreatic cancers; liver cancers; kidney cancers; endocrine cancers; skin cancers; and brain or central and peripheral nervous (CNS) system tumors, malignant or benign, including gliomas and neuroblastomas, astrocytomas, myelodysplastic disorders; cervical carcinoma-in-situ: intestinal polyposes; oral leukoplakias; histiocytoses, hyperprofroliferative scars including keloid scars, hemangiomas; hyperproliferative arterial stenosis, psoriasis, inflammatory arthritis: hyperkeratoses and papulosquamous eruptions including arthritis.

The term neoplastic disease includes carcinomas. The term "carcinoma" refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. The term neoplastic disease includes adenocarcinomas. An "adenocarcinoma" refers to a carcinoma derived from glandular tissue or in which the tumor cells form recognizable glandular structures.

The term "hematopoietic neoplastic disorders" refers to neoplastic diseases involving hyperplastic/neoplastic cells of hematopoietic origin, *e.g*., arising from myeloid, lymphoid or erythroid lineages, or precursor cells thereof. Myeloid neoplasms include, but are not limited to, myeloproliferative neoplasms, myeloid and lymphoid disorders with eosinophilia, myeloproliferative/myelodysplastic neoplasms, myelodysplastic syndromes, acute myeloid leukemia and related precursor neoplasms, and acute leukemia of ambiguous lineage. Exemplary myeloid disorders amenable to treatment in accordance with the present disclosure include, but are not limited to, acute promyeloid leukemia (APML), acute myelogenous leukemia (AML) and chronic myelogenous leukemia (CML). Lymphoid neoplasms include, but are not limited to, precursor lymphoid neoplasms, mature B-cell neoplasms, mature T-cell neoplasms, Hodgkin's Lymphoma, and immunodeficiency-associated lymphoproliferative disorders. Exemplary lymphic disorders amenable to treatment in accordance with the present disclosure include, but are not limited to, acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM).

In some instances, the hematopoietic neoplastic disorder arises from poorly differentiated acute leukemias (*e.g.,* erythroblastic leukemia and acute megakaryoblastic leukemia). As used herein, the term "hematopoietic neoplastic disorders" refers malignant lymphomas including, but are not limited to, non-Hodgkins lymphoma and variants thereof, peripheral T cell lymphomas, adult T-cell leukemia/lymphoma (ATL), cutaneous T cell lymphoma (CTCL), large granular lymphocytic leukemia (LGF), Hodgkin's disease and Reed-Stemberg disease.

The determination of whether a subject is "suffering from a neoplastic disease" refers to a determination made by a physician with respect to a subject based on the available information accepted in the field for the identification of a disease, disorder or condition including but not limited to X-ray, CT-scans, conventional laboratory diagnostic tests (*e.g.,* blood count, etc.), genomic data, protein expression data, immunohistochemistry, that the subject requires or will benefit from treatment.

The determination of efficacy of the methods of the present disclosure in the treatment of cancer is generally associated with the achievement of one or more art recognized parameters such as reduction in lesions particularly reduction of metastatic lesion, reduction in metastatsis, reduction in tumor volume, improvement in ECOG score, and the like. Determining response to treatment can be assessed through the measurement of biomarker that can provide reproducible information useful in any aspect of binding protein therapy, including the existence and extent of a subject's response to such therapy and the existence and extent of untoward effects caused by such therapy. The response to treatment may be characterized by improvements in conventional measures of clinical efficacy may be employed such as Complete Response (CR), Partial Response (PR), Stable Disease (SD) and with respect to target lesions, Complete Response (CR)," Incomplete Response/Stable Disease (SD) as defined by RECIST as well as immune-related Complete Response (irCR), immune-related Partial Response (irPR), and immune-related Stable Disease (irSD) as defined Immune-Related Response Criteria (irRC) are considered by those of skill in the art as evidencing efficacy in the treatment of neoplastic disease in mammalian (*e.g*., human) subjects.

### Infectious Diseases

The present disclosure provides methods of use of binding proteins in the treatment of subjects suffering from an infectious disease by the administration of a therapeutically effective amount of a binding protein (or nucleic acid encoding an binding protein including recombinant vectors encoding binding proteins) as described herein.

In some embodiments the infection is a chronic infection, *i.e.,* an infection that is not cleared by the host immune system within a period of up to 1 week, 2 weeks, etc. In some cases, chronic infections involve integration of pathogen genetic elements into the host genome, e.g., retroviruses, lentiviruses, Hepatitis B virus, etc. In other cases, chronic infections, for example certain intracellular bacteria or protozoan pathogens, result from a pathogen cell residing within a host cell. Additionally, in some embodiments, the infection is in a latent stage, as with herpes viruses or human papilloma viruses.

Viral pathogens of interest include without limitation, retroviral, hepadna, lentiviral, etc. pathogens, *e.g.,* HIV-1: HIV-2, HTLV, FIV, SIV, etc., Hepatitis A, B, C, D, E virus, etc. In some embodiments, the methods of the invention involve diagnosis of a patient as suffering from an infection; or selection of a patient previously diagnosed as suffering from an infection; treating the patient with a regimen of variant type III interferon therapy, optionally in combination with an additional therapy; and monitoring the patient for efficacy of treatment. Monitoring may measure clinical indicia of infection, *e.g*., fever, white blood cell count, etc., and/or direct monitoring for presence of the pathogen. Treatment may be combined with other active agents. Cytokines may also be included, *e.g*., interferon γ, tumor necrosis factor α, interleukin 12, etc. Antiviral agents, *e.g.,* acyclovir, gancyclovir, etc., may also be used in treatment. Subjects suspected of having an infection, including an HCV infection, can be screened prior to therapy. Further, subjects receiving therapy may be tested in order to assay the activity and efficacy of the treatment. Significant improvements in one or more parameters is indicative of efficacy. It is well within the skill of the ordinary healthcare worker (*e.g.,* clinician) to adjust dosage regimen and dose amounts to provide for optimal benefit to the patient according to a variety of factors (*e.g.,* patient-dependent factors such as the severity of the disease and the like, the compound administered, and the like). For example, HCV infection in an individual can be detected and/or monitored by the presence of HCV RNA in blood, and/or having anti-HCV antibody in their serum. Other clinical signs and symptoms that can be useful in diagnosis and/or monitoring of therapy include assessment of liver function and assessment of liver fibrosis (*e.g.,* which may accompany chronic viral infection).

Subjects for whom the therapy described herein can be administered include naive individuals (*e.g.,* individuals who are diagnosed with an infection, but who have not been previously treated) and individuals who have failed prior treatment ("treatment failure" patients). For HCV therapy, previous treatment includes, for example, treatment with IFN- α monotherapy (*e.g.,* IFN- α and/or PEGylated IFN- α) or IFN- α combination therapy, where the combination therapy may include administration of IFN- α and an antiviral agent such as ribavirin. Treatment failure patients include non-responders (*i.e.,* individuals in whom the HCV titer was not significantly or sufficiently reduced by a previous treatment for HCV to provide a clinically significant response, *e.g.,* a previous IFN-α monotherapy, a previous IFN-α and ribavirin combination therapy, or a previous pegylated IFN-α and ribavirin combination therapy); and relapsers (*i.e.,* individuals who were previously treated for HCV (*e.g.,* who received a previous IFN-α monotherapy, a previous IFN-α and ribavirin combination therapy, or a previous pegylated IFN-α and ribavirin combination therapy), in whom the HCV titer decreased to provide a clinically significant response, but in whom the decreased HCV titer was not maintained due to a subsequent increase in HCV titer).

Other subjects for whom the therapy disclosed herein is of interest include subject who are"difficult to treat" subjects due to the nature of the HCV infection. "Difficult to treat" subjects are those who 1) have high-titer HCV infection, which is normally defined as an HCV titer of at least about 10⁵, at least about 5 x 10⁵, or at least about 10⁶ or more genome copies of HCV per milliliter of serum, 2) are infected with HCV of a genotype that is recognized in the field as being associated with treatment failure (*e.g*., HCV genotype 1, subtypes thereof (*e.g.,* 1a, 1b, etc.), and quasispecies thereof or 3) both.

In other embodiment methods are provided for treating or reducing primary or metastatic cancer in a regimen comprising contacting a subject in need of treatment with a therapeutically effective amount or an effective dose of IFN λ synthekines or IFN λ variant polypeptides. Effective doses for the treatment of cancer vary depending upon many different factors, including means of administration, target site, physiological state of the patient, whether the patient is human or an animal, other medications administered, and whether treatment is prophylactic or therapeutic. Usually, the patient is a human, but nonhuman mammals may also be treated, *e.g.,* companion animals such as dogs, cats, horses, etc., laboratory mammals such as rabbits, mice, rats, etc., and the like. Treatment dosages can be titrated to optimize safety and efficacy.

In prophylactic applications, a relatively low dosage may be administered at relatively infrequent intervals over a long period of time. Some patients continue to receive treatment for the rest of their lives. In other therapeutic applications, a relatively high dosage at relatively short intervals is sometimes required until progression of the disease is reduced or terminated, and preferably until the patient shows partial or complete amelioration of symptoms of disease. Thereafter, the patent can be administered a prophylactic regime.

In still other embodiments, methods of the present invention include treating, reducing or preventing tumor growth, tumor metastasis or tumor invasion of cancers including carcinomas, hematologic cancers, melanomas, sarcomas, gliomas, particularly cancers of epithelial origin that express IFN λR1 and IFNAR1 or IFNAR2, or IL-10Rβ and IFNAR1 or IFNAR2. In some embodiments a cancer is assessed for responsiveness to an IFN λ synthekine by determining whether the cancer expresses the cognate receptors that the synthekine activates, e.g., determing the expression of IFN λR1, and IFNAR1 or IFNAR2. Tissues known to express IFN λR1 include, for example, lung, heart, liver (hepatocytes), prostate, keratinocytes and melanocytes. Cancers responsive to IFN λ and IFN λ synthekines may include, without limitation, melanoma, fibrosarcoma, hepatocellular carcinoma, bladder carcinoma, Burkitt's lymphoma, colorectal carcinoma, glioblastoma, non-small cell lung cancer, esophageal carcinoma, and osteosarcoma, among others.

For prophylactic applications, pharmaceutical compositions or medicaments are administered to a patient susceptible to, or otherwise at risk of disease in an amount sufficient to eliminate or reduce the risk, lessen the severity, or delay the outset of the disease, including biochemical, histologic and/or behavioral symptoms of the disease, its complications and intermediate pathological phenotypes presenting during development of the disease.

### EXAMPLES

### Example 1 - VₕH Generation:

Camels were acclimated at research facility for at least 7 days before immunization. Antigen was diluted with 1×PBS (antigen total about 1 mg). The quality of the antigen was assessed by SDS-PAGE to ensure purity (*e.g.,* >80%). For the first time, 10 mL CFA (then followed 6 times using IFA) was added into mortar, then 10 mL antigen in 1×PBS was slowly added into the mortar with the pestle grinding. The antigen and CFA/IFA were ground until the component showed milky white color and appeared hard to disperse. Camels were injected with antigen emulsified in CFA subcutaneously at at least six sites on the body, injecting about 2 mL at each site (total of 10 mL per camel). A stronger immune response was generated by injecting more sites and in larger volumes. The immunization was conducted every week (7 days), for 7 times. The needle was inserted into the subcutaneous space for 10 to 15 seconds after each injection to avoid leakage of the emulsion. Alternatively, a light pull on the syringe plunger also prevented leakage. The blood sample was collected three days later after 7^{th} immunization.

After immunization, the library was constructed. Briefly, RNA was extracted from blood and transcribed to cDNA. The V_{H}H regions were obtained via two-step PCR, which fragment about 400 bp. The PCR outcomes and the vector of pMECS phagemid were digested with *Pst I* and *Not I,* subsequently, ligated to pMECS/Nb recombinant. After ligation, the products were transformed into *Escherichia coli (E. coli)* TG1 cells by electroporation. Then, the transformants were enriched in growth medium and planted on plates. Finally, the library size was estimated by counting the number of colonies.

Library biopanning was conducted to screen candidates against the antigens after library construction. Phage display technology was applied in this procedure. Positive colonies were identified by PE-ELISA.

### Example 2. Generation of anti-hIL10R VHHs

Camels were immunized with the extracellular domains of the human IL10Rα (amino acids 22-235, UniProtKB Q13651, hIL-10Rα_{ecd}) and IL10Rβ (amino acids 20-220, UniProtKB Q08334, hIL-10Rβ_{ecd}) weekly for seven weeks and PBMCs harvested on day 52. Phage display libraries were constructed and biopanning conducted as described in Example 1 above. 50 VHH sequences were obtained after selection on hIL10-R1 and 47 VHH sequences were obtained after selection on hIL10-R2. Sequences were clonotyped using germline assignment and CDR3 sequence similarity.

### Example 3. Synthesis of DNA Encoding Synthekines

Seven unique anti- hIL-10Rα_{ecd} sequences (SEQ ID Nos: 44-50) and seven unique anti- hIL-10Rβ_{ecd} sequences (SEQ ID Nos: 51-57) were selected from each cohort and DNA was synthesized consisting of one IL-10Rα VHH encoding DNA and one IL-10Rβ VHH encoding DNA separated by a linker sequence by GGGS (SEQ ID NO:23) encoding DNA. DNA was for each possible VHH combination and in both orientations for a total of 98 7 x7 x 2 = 98 VHH dimers. An Ala-Ser ("AS") linker followed by His-6 DNA (ASH6, SEQ ID NO 430) was added at the 3' end of each DNA construct. The codon optimized DNA sequences encoding these constructs are provided as SEQ ID Nos: 290-237 and the orientation of components thereof are described in Table 2 of the specification above.

### Example 4. Recombinant Production and Purification

Codon optimized DNA inserts (SEQ ID Nos: 290-237) and cloned into modified pcDNA3.4 (Genscript) for small scale expression in HEK293 cells in 24 well plates. Supernatants The cells The IL2R binding proteins were purified in substantial accordance with the following procedure. Using a Hamilton Star automated system, 96 x 4 ml of supernatants in 4 x 24-well blocks were re-arrayed into 4 x 96-well, 1 mL blocks. PhyNexus micropipette tips (Biotage, San Jose CA) holding 80 uL of Ni-Excel IMAC resin (Cytiva) are equilibrated wash buffer: PBS pH 7.4, 30 mM imidazole. PhyNexus tips were dipped and cycled through 14 cycles of 1 mL pipetting across all 4 x 96-well blocks. PhyNexus tips were washed in 2 x 1 mL blocks holding wash buffer. PhyNexus tips were eluted in 3 x 0.36 mL blocks holding elution buffer: PBS pH 7.4, 400 mM Imidazole. PhyNexus tips were regenerated in 3 x 1 mL blocks of 0.5 M sodium hydroxide.

The purified protein eluates were quantified using a Biacore^{®} T200 as in substantial accordance with the following procedure. 10 uL of the first 96 x 0.36 mL eluates were transferred to a Biacore^{®} 96-well microplate and diluted to 60 uL in HBS-EP+ buffer (10 mM Hepes pH 7.4, 150 mM NaCl, 1 mM EDTA, 0.05% Tween 20). Each of the 96 samples was injected on a CM5 series S chip previously functionalized with anti-histidine capture antibody (Cytiva): injection is performed for 18 seconds at 5 uL/min. Capture levels were recorded 60 seconds after buffer wash. A standard curve of known VHH concentrations (270, 90, 30, 10, 3.3, 1.1 µg/mL) was acquired in each of the 4 Biacore chip flow cells to eliminate cell-to-cell surface variability. The 96 captures were interpolated against the standard curve using a non-linear model including specific and unspecific, one-site binding. Concentrations in the first elution block varied from 12 to 452 µg /mL corresponding to a 4-149 µg. SDS-PAGE analysis of 5 randomly picked samples was performed to ensure molecular weight of eluates corresponded to expected values (~30 KDa).

The concentration of the proteins was normalized using the Hamilton Star automated system in substantial accordance with the following procedure. Concentration values are imported in an Excel spreadsheet where pipetting volumes were calculated to perform dilution to 50 µg/mL in 0.22 mL. The spreadsheet was imported in a Hamilton Star method dedicated to performing dilution pipetting using the first elution block and elution buffer as diluent. The final, normalized plate was sterile filtered using 0.22 µm filter plates (Corning) and the material used for the following *in vitro* assays.

### Example 5. IL10 Activity Assay:

HEK-Blue^{™} IL-10 reporter cell line (Invivogen, San Diego CA) was used for screening the IL10R1/R2 VHHs. HEK-Blue^{™} IL-10 cells were generated by stable transfection of the human embryonic kidney HEK293 cell line with the genes encoding hIL-10R α and β chains, human STAT3, and the STAT3-inducible SEAP (secreted embryonic alkaline phosphatase) reporter. Binding of IL-10 to its receptor on the surface of HEK-Blue^{™} IL-10 cells triggers JAK1/STAT3 signaling and the subsequent production of SEAP. The signal was then detected by quantifying SEAP activity in the cell culture supernatant using a QUANTI-Blue^{™} development solution (Invivogen, San Diego CA) and the absorbance values were measured spectrophotometrically at 630 nm. Because STAT3 is also implicated in the signaling of cytokines such as IFN-α/β and IL-6, HEK-Blue^{™} IL-10 cells are knockout for the expression of hIFNAR2 and hIL-6R.

### Example 6. Screening of SEQ ID NOs: 192-289:

To screen the IL10R1/R2 VHHs, HEK-Blue^{™} IL-10 cells were seeded in a 96-well plate at 50,000 cells per well and treated with either 25 nM or 100 nM protein (in triplicates) for 24 hours. Recombinant Animal-Free Human IL-10 (Shenandoah Biotechnology, Inc. Warwick, PA Catalog No. 100-83AF) was used as a positive control and unstimulated cells were used as a negative control. 24 hours post treatment, 20 µl of the cell supernatant was transferred to a flat-bottom 96 well plate and the assay was developed by adding 180 µl of the QUANTI-Blue^{™} (Invivogen) for 2 hours. The absorbance values were measured at 630 nm on the Envision^{®} (PerkinElmer, Waltham MA) multilabel plate reader. The results of this screening are presented in Table 3 of the specification.

### INFORMAL SEQUENCE LISTING

| **SEQ ID NO** | **Notes** | **Amino Acid or DNA Sequence** |
|---|---|---|
| **1** | linker | GGGGS |
| **2** | linker | GGGGGS |
| **3** | linker | GGSG |
| **4** | linker | SGGG |
| **5** | linker | GSGS |
| **6** | linker | GSGSGS |
| **7** | linker | GSGSGSGSGS |
| **8** | linker | GSGSGSGSGSGS |
| **9** | linker | GGSGGS |
| **10** | linker | GGSGGSGGS |
| **11** | linker | GGSGGSGGSGGS |
| **12** | linker | GGSGGGSG |
| **13** | linker | GGSGGGSGGGSG |
| **14** | linker | GGSGGGSGGGSGGGSG |
| **15** | linker | GGSGGGSGGGSGGGSGGGSG |
| **16** | linker | GGGGSGGGGS |
| **17** | linker | GGGGSGGGGSGGGGS |
| **18** | linker | GGSGG |
| **19** | linker | GSGSG |
| **20** | linker | GSGGG |
| **21** | linker | GGGSG |
| **22** | linker | GSSSG |
| **23** | linker | GGGS |
| **24** | Anti-gp130 V_{H}H | QVQLQESGGGSVQAGGSLRLSCTASGAIASGYIDSRWCMAWFRQAPGKEREG VAAIWPGGGLTVYADSVKGRFTISRDHAKNTLYLQMNNLKPEDTAMYYCAA GSPRMCPSLEFGFDYWGQGTQVTVSS |
| **25** | Anti-gp130 V_{H}H | QVQLQESGGGSVQAGGSLRLSCVASASTYCTYDMHWYRQAPGKGREFVSAID SDGTTRYADSVKGRFTISQGTAKNTVYLQMNSLQPEDTAMYYCKTVCVVGSR WSDYWGQGTQVTVSS |
| **26** | Anti-gp130 V_{H}H | QVQLQESGGGSVQAGGSLRLSCTAPGFTSNSCGMDWYRQAPGKEREFVSSIST DGTTGYADSVKGRFTISKDKAKDTVYLQMNSLKPEDTGMYSCKTKDGTIATM ELCDFGYWGQGTQVTVSS |
| **27** | Anti-gp130 V_{H}H | QVQLQESGGGSVQAGGSLRLSCAASGYPYSNGYMGWFRQAPGKEREGVATIY TGDGRTYYADSVKGRFTISRDNAKNTVDLQMSSLKPEDTAMYYCAARAAPLY SSGSPLTRARYNVWGQGTQVTVSS |
| **28** | Anti-gp130 V_{H}H | QVQLQESGGGSVQAGGSLTLSCAASEYAYSTCNMGWYRQAPGKERELVSAFI SDGSTYYADSVKGRFTITRDNAKNTVYLQMNSLKPEDTAIYYCSANCYRRLRN YWGQGTQVTVSS |
| **29** | Anti-gp130 V_{H}H | QVQLQESGGGLVQPGGSLRLSCTASGLTFDDSVMGWFRQAPGKGREAVSCISS SGANAFYADSVKGRFTISRDNAKNTLYLQMNSLKPEDTATYYCKRGHACAGY YPIPYDDYWGQGTQVTVSS |
| **30** | Anti-Il2Rb/Anti-CD122 V_{H}H | QVQLQESGGGSVQAGGSLRLSCAASGYEYCRIHMTWYRQGPGKEREFVSSIGS DGRKTYANSVTGRFTISRDNANHTVYLQMNSLSPEDTAMYYCKTEYLYGLGC PDGSAYWGQGTQVTVSS |
| **31** | Anti-Il2Rb/Anti-CD122 V_{H}H | QVQLQESGGGSVQAGGSLRLSCAASEYTASRYCMAWFRQAPGKEREGVAAIH PGGGTTYYADSVKGRFSISQDSADNTLYLQMNSLKPEDTAMYYCAAGSLWVP FGDRCAANYWGQGTQVTVSS |
| **32** | Anti-Il2Rb/Anti-CD122 V_{H}H | QVQLQESGGGLVQPGGSLRLSCVASGFTFSNYWIFWVRQAAGKGLEWLSTSN TGGDTTKYADSVKGRFTISRDSAKNTEYLQMNSLKPEDTAVYYCETGRCARSG GYQGTQVTVSS |
| **33** | Anti-Il2Rb/Anti-CD122 V_{H}H | QVQLQESGGGSVQVGGSLRLSCATSGDTKSIRCMGWFRQTPGKEREGIAAIDR EGFATYADSVYDRFTIAQDNAQNTLYLEMNALKPEDTAMYYCAAQNMCRVV RGAMTGVDYWGKGTQVTVSS |
| **34** | Anti-Il2Rb/Anti-CD122 V_{H}H | QVQLQESGGGSVQVGGSLKLSCAASGYTYSSYCMGWFRQAPGKEREGVAAI DSDGSTSYADSVKGRFTISQDDAKNTLYLQMNSLKPEDTAMYYCAASYEVVD CYPSGYGQDYWGKGTQVTVSS |
| **35** | Anti-Il2Rb/Anti-CD122 V_{H}H | QVQLQESGGGSVQAGGSLRLSCVGSGYTYDTSDMSWYRQAPGKEREFVSDID SGDWAAYADAVKGRFTISRDNAKKTVYLQMNSLEPEDTAMYYCKASYWKW GKLNNFWGPGTQVTVSS |
| **36** | Anti-Il2Rb/Anti-CD122 V_{H}H | QVQLQESGGGLVQPGGSLKLSCAASGFRFSNYGMSWVRQAPGEGLEWVSYIN GDGSRTHYADSVKGRFTISRDNAKNTLYLQLNSLKTEDTAMYYCEKGLSRDG WSLSAASRGQGTQVTVSS |
| **37** | Anti-Il2Rb/Anti-CD122 V_{H}H | QVQLQESGGGSVQTGGSLRLSCAVSGYTTYSFNYMGWFRQAPGKEREGVAVI YTGGGSTLYADSVKGRFTISQDNAKNTVYLQMNSLKPEDTAMYYCAADDQRF ASPLYAYFGYWGQGTQVTVSS |
| **38** | Anti-IL2Rgamma/Ant i-CD132 | QVQLQESGGGLVQPGGSLRLSCTASGFSFSSYPMTWARQAPGKGLEWVSTIAS DGGSTAYAASVEGRFTISRDNAKSTLYLQLNSLKTEDTAMYYCTKGYGDGTP APGQGTQVTVSS |
| **39** | Anti-IL2Rgamma/Ant i-CD132 | QVQLQESGGGLVQPGGSLRLSCAASGFTFSSAHMSWVRQAPGKGREWIASIYS GGGTFYADSVKGRFTISRDNAKNTLYLQLNSLKAEDTAMYYCATNRLHYYSD DDSLRGQGTQVTVSS |
| **40** | Anti-IL2Rgamma/Ant i-CD132 | QVQLQESGGGSVQAGGSLRLSCTASGFTFDDREMNWYRQAPGNECELVSTISS DGSTYYADSVKGRFTISQDNAKNTVYLQMDSVKPEDTAVYYCAADFMIAIQA PGAGCWGQGTQVTVSS |
| **41** | Anti-IL2Rgamma/Ant i-CD132 | QVQLQESGGGSVQAGGSLRLSCVASGYTSCMGWFRQAPGKEREAVATIYTRG RSIYYADSVKGRFTISQDNAKNTLYLQMNSLKPEDIAMYSCAAGGYSWSAGCE FNYWGQGTQVTVSS |
| **42** | Anti-IL2Rgamma/Ant i-CD132 | QVQLQESGGGSVQAGGSLRLSCTASGFTFDDSDMGWYRQAPGNECELVSTISS DGSTYYADSVKGRFTISQDNAKNTVYLQMNSLKPEDTAVYYCAAEPRGYYSN YGGRRECNYWGQGTQVTVSS |
| **43** | Anti-IL2Rgamma/Ant i-CD132 | QVQLQESGGGSVQAGGSLRLSCVASGYTFSSYCMGWFRQAPGKEREGVAALG GGSTYYADSVKGRFTISQDNAKNTLYLQMNSLKPEDTAMYYCAAAWVACLE FGGSWYDLARYKHWGQGTQVTVSS |
| **44** | Anti-IL10Rα | QVQLQESGGGSVQAGGSLRLSCAASRYLYSIDYMAWFRQSPGKEREPVAVIYT ASGATFYPDSVKGRFTISQDNAKMTVYLQMNSLKSEDTAMYYCAAVRKTDSY LFDAQSFTYWGQGTQVTVSS |
| **45** | Anti-IL10Rα | QVQLQESGGGSVQAGGSLRLSCAASRFTYSSYCMGWFRQAPGKEREGVASIDS DGSTSYTDSVKGRFTISKDNAKNTLYLQMNSLKPEDTAMYYCALDLMSTVVP GFCGFLLSAGMDYWGKGTQVTVSS |
| **46** | Anti-IL 10Rα | QVQLQESGGGSVQAGGSLRLSCAASGYTYSMYCMGWFRQAPGKEREGVAQI NSDGSTSYADSVKGRFTISKDNAKNTLYLQMNSLKPEDTAMYYCAADSRVYG GSWYERLCGPYTYEYNYWGQGTQVTVSS |
| **47** | Anti-IL 10Rα | QVQLQESGGGSVQAGGSLRLSCAVSGYAYSTYCMGWFRQAPGKEREGVAAID SGGSTSYADSVKGRFTISKDNAKNTLYLRMNSLKPEDTAMYYCAAVPPPPDGG SCLFLGPEIKVSKADFRYWGQGTQVTVSS |
| **48** | Anti-IL 10Rα | QVQLQESGGGSVQAGGSLRLSCTVSGYTYSSNCMGWFRQAPGKEREGVATIY TGGGNTYYADSVKGRFTISQDNAKNTVYLQMNNLKPEDTAMYYCAAEPLSRV YGGSCPTPTFDYWGQGTQVTVSS |
| **49** | Anti-IL 10Rα | QVQLQESGGGSVQAGGSLRLSCGASGYTYSSYCMGWFRQVPGKEREGVAVID SDGSTSYADSVKGRFTISKDNGKNTLYLQMNSLKPEDTAMYYCAADLGHYRP PCGVLYLGMDYWGKGTQVTVSS |
| **50** | Anti-IL 10Rα | QVQLQESGGGSVQAGGSLRLSCAASGYSNCSYDMTWYRQAPGKEREFVSAIH SDGSTRYADSVKGRFFISQDNAKNTVYLQMNSLKPEDTAMYYCKTDPLHCRA HGGSWYSVRANYWGQGTQVTVSS |
| **51** | Anti-IL10Rβ | QVQLQESGGGSVQAGGSLRLSCAASGYTYSSGCMGWFRQAPGKEREAVAAIN SDGSTSYADSVKGRFTISKDNAKNTLYLQMNSLKPEDTAMYYCAAEPYCSGG YPRWSVAEFGYWGQGTQVTVSS |
| **52** | Anti-IL10Rβ | QVQLQESGGGSVQAGGSLRLSCAASGYTYSSYCMGWFRQAPGKEREGVAHID SDGSTSYADSVKGRFTISKDNAKNTLYLQMNSLKPEDTAMYYCAADPIPGPGY CDGGPNKYWGQGTQVTVSS |
| **53** | Anti-IL10Rβ | QVQLQESGGGSVQAGGSLRLSCAASRYTYNSYCMGWFRQAPGKEREGVATID SDGMTRYADSVKGRFTISKDNAKNTLYLQMNSLKPEDTAMYYCAADADCTIA AMTTNPLGQGTQVTVSS |
| **54** | Anti-IL10Rβ | QVQLQESGGGSVQAGGSLRLSCTVSRYTASVNYMGWFRQAPGKEREGVATIF TGAGTTYYANSVKGRFTISRDNAKNTAYLQMNSLKPEDTAIYYCAVDFRGGLL YRPAYEYTYRGQGTQVTVSS |
| **55** | Anti-IL10Rβ | QVQLQESGGGSVEAGGSLRLSCAASGYTHSSYCMGWFRQAPGKEREGVAAID VDGSTTYADSVKGRFTISKDNAKNTLYLQMNSLKPEDTGMYYCAAEFADCSS NYFLPPGAVRYWGQGTQVTVSS |
| **56** | Anti-IL10Rβ | QVQLQESGGGSVQAGGSLRLSCAASGYSYSSYCMGWFRQAPGKEREGVATID SDGMTRYADSVKGRFTISKDNAKNTLYLQMNSLKPEDTAMYYCAAPLYDCDS GAVGRNPPYWGQGTQVTVSS |
| **57** | Anti-IL10Rβ | QVQLQESGGGSVQTGGSLRLSCAASGYTYLRGCMGWFRQAPGKEREGVAVM DVVGDRRSYIDSVKGRFTISRDNAANSVYLQMDNLKPEDTAMYYCTAGPNCV GWRSGLDYWGQGTQVTVSS |
| **58** | Anti-IL12Rβ2 | QVQLQESGGGSVQAGGSLRLSCAASGFTVTRYCMGWLRQAPGKQREGVAIIE RDGRTGYADSVKGRFTISKDNAKNTLYLQMNSLKPEDTAMYYCGAIEGSCRP DFGYRGQGTQVTVSS |
| **59** | Anti-IL12Rβ2 | QVQLQESGGGSVQAGGSLRLSCTASGLTFDDVEMAWYRQGPGDDYDLVSSIN TDSRVYYVDSVKDRFTISRDNAKNTLYLQMNNLKPEDTAVYYCAADPWGGD LRGYPNYWGQGTQVTVSS |
| **60** | Anti-IL12Rβ2 | QVQLQESGGGLVQAGGSLRLSCQASGYTYGLFCMGWFRQVSGKKREGVAVV DSPGGRHVADSLKGRFTISKDNANNILYLDMTNLKSEDTATYYCAADPEKYCF LFSDAGYQYWGQGTQVTVSS |
| **61** | Anti-IL 12Rβ2 | QVQLQESGGGSVQAGGSLRLSCAASGVTYSRYCMGWFRQAPGLERERVAHIY SRGIITYYTDSVKGRFTISQDSAKKTVYLQMNSLKPEDTAMYYCAATRETYGG SGDCGYESVYNYWAQGTQVTVSS |
| **62** | Anti-IL 12Rβ2 | QVQLQESGGGLVQPGGSLKLSCAASGFTFSTYAMSWVRQAPGKEPEWISRISS GGGNTYYADAVKGRFAISRDNAKNTLYLQLNSLKTEDTAIYVCTMDDYYGGS WHPISRGHGTQVTVSS |
| **63** | Anti-IL12Rβ2 | QVQLQESGGGSVQAGGSLRLSCSASGFTVDDFAMGWYRQAPGNECELVSTISS GGSTYYADSVKGRFTISQDSAKNTVYLQMNSLKPEDTAVYYCAPSSVGCPLGY WGQGTQVTVSS |
| **64** | Anti-IL23R | QVQLQESGGGSVQAGGSLRLSCAASGFTVTRYCMGWLRQAPGKQREGVAIIE RDGRTGYADSVKGRFTISKDNAKNTLYLQMNSLKPEDTAMYYCGAIEGSCRP DFGYRGQGTQVTVSS |
| **65** | Anti-IL23R | QVQLQESGGGSVQAGGSLRLSCTASGLTFDDVEMAWYRQGPGDDYDLVSSIN TDSRVYYVDSVKDRFTISRDNAKNTLYLQMNNLKPEDTAVYYCAADPWGGD LRGYPNYWGQGTQVTVSS |
| **66** | Anti-IL23R | QVQLQESGGGLVQAGGSLRLSCQASGYTYGLFCMGWFRQVSGKKREGVAVV DSPGGRHVADSLKGRFTISKDNANNILYLDMTNLKSEDTATYYCAADPEKYCF LFSDAGYQYWGQGTQVTVSS |
| **67** | Anti-IL23R | QVQLQESGGGSVQAGGSLRLSCAASGVTYSRYCMGWFRQAPGLERERVAHIY SRGIITYYTDSVKGRFTISQDSAKKTVYLQMNSLKPEDTAMYYCAATRETYGG SGDCGYESVYNYWAQGTQVTVSS |
| **68** | Anti-IL23R | QVQLQESGGGLVQPGGSLKLSCAASGFTFSTYAMSWVRQAPGKEPEWISRISS GGGNTYYADAVKGRFAISRDNAKNTLYLQLNSLKTEDTAIYVCTMDDYYGGS WHPISRGHGTQVTVSS |
| **69** | Anti-IL23R | QVQLQESGGGSVQAGGSLRLSCSASGFTVDDFAMGWYRQAPGNECELVSTISS GGSTYYADSVKGRFTISQDSAKNTVYLQMNSLKPEDTAVYYCAPSSVGCPLGY WGQGTQVTVSS |
| **70** | Anti-IL27Ralpha | QVQLQESGGGLVQPGESLRLSCTASGFTFSNYAMSWVRQAPGKGLEWVSGIN VAYGITSYADSVKGRFTISRDNTKNTLYLQLNSLKTEDTAIYYCVKHSGTTIPR GFISYTKRGQGTQVTVSS |
| **71** | Anti-IL27Ralpha | QVQLQESGGGSVQVGGSLRLSCAASGFTFSSYPMSWVRQAPGKGLEWISTISA GGDTTLYADSVKGRFTSSRDNAKNTLYLQLNSLKTEDTAIYYCAKRIDCNSGY CYRRNYWGQGTQVTVSS |
| **72** | Anti-IL27Ralpha | QVQLQESGGGSVQAGGSLRLSCRASGSTYSNYCLGWFRQITGKEREGVAVIN WVGGMLYFADSVKGRFTVSQDQAKNTLYLQMNSLKPEDTAMYYCAAESVSS FSCGGWLTRPDRVPYWGQGTQVTVSS |
| **73** | Anti-IL27Ralpha | QVQLQESGGGSVQAGGSLRLSCVASGYVSCDYFLPSWYRQAPGKEREFVSIID GTGSTSYAASVKGRFTASQDKGKNIAYLQMNSLKPEDTAMYYCKASCVRGRG ISEYWGQGTQVTVSS |
| **74** | Anti-IL27Ralpha | QVQLQESGGGSVQSGGSLRLSCAASGFTYSTSNSWMAWFRQAPGKEREGVAA IYTVGGSIFYADSVRGRFTISQDATKNMFYLQMNTLKPEDTAMYYCAAASGRL RGKWFWPYEYNYWGQGTQVTVSS |
| **75** | Anti-IL27Ralpha | QVQLQESGGGLVQPGGSLRLSCAASGFTFSHSGMSWVRQAPGKGLEWVSTINS GGASTYYTDSVKGRFTISRDNAKNMLYLQLNSLKTEDTAMYYCAKGGSGYGD ASRMTSPGSQGTQVTVSS |
| **76** | Anti-IL28Ralpha | QVQLQESGGGSVQSGGSLRLSCAASGFTYSSYCMGWFRQAPGKEREGVAAIDS DGSTSYADSVKGRFTISKDNAKNTLYLQMNSLRPEDTAMYYCAADGEYNDYV CWSTGLRYRGQGTQVTVSS |
| **77** | Anti-IL28Ralpha | QVQLQESGGGLVQPGGSLRLSCVASGFTFSDYAMSWVRQAPGMGLERVSAIG RDGSTFYPDSVKGRFTISRDNAKNTLYLQLNSLKTEDTAMYYCAKEEPGSSSR GQGTQVTVSS |
| **78** | Anti-IL28Ralpha | QVQLQESGGGSVQAGGSLRLSCAVSRYTISRSDCMGWFRQAPGKEREGVARIG SDGTTSYADSVKERFTISKDNAKNILYLQMNSLKPEDTARYYCAATALLLGRG SACHKEVSVFSWWGQGTQVTVSS |
| **79** | Anti-IL28Ralpha | QVQLQESGGGLVQPGGSLRLSCAASGFTFSNYGMSWVRQAPGKGLEWVSGIN SGGDDTFYTDSVKGRFTISRDNAKNTLYLQMNSLKTEDTAMYYCAMGASGMI PRGQGTQITVSS |
| **80** | Anti-IL28Ralpha | QVQLQESGGGSVQLGGSLRLSCLVSGSTDNIKYMGWFRQAPGKEREGVAAVY TSGGAVVYADSVKGRFTISQDDAKNTMYLQMNSLKPEDTAMYYCAASRAPAP PRLLLQRALVEYWGQGTQVTVSS |
| **81** | Anti-IL28Ralpha | QVQLQESGGGLVQPGGSLRLSCAASGFTFSNATMSWVRQAPGKEIEWVSAISN SRGTKYYAAFVKGRFTISRDNAKNTLYLQLNNLKTEDTAMYYCTKDWKTSYS DYDLSDGQGTQVTVSS |
| **82** | Anti-IL28Ralpha | QVQLQESGGGSVQAGGSLRLSCASSGYISSSYCMAWFRQAPGKEREGAAGVT RDGKTYYGDSVKGRFAISRDNAKNTLYLQMNSLKPEDTAMYYCAAGPPPCITS MPAGGDYGYRYWGQGTQVTVSS |
| **83** | Anti-mouseGp130 | QVQLQESGGALVQPGGSLRLSCAASGFTFSYYAMKWVRQAPGKGLEWVSSIS GGGGATYYADSVKGRFTISRDNTNDTLYLQMNSLKTEDTAVYYCAAQNLDYR GQGTQVTVSS |
| **84** | Anti-mouseGp130 | QVQLQESGGGLVQPGGSLRLSCTASGFTFNSAHLKWERQPPGKGLEWVSFITN GGASTGYADSVKGRFTISRDDAKNTLYLQMNNLKTEDTAVYYCATGGLRGQG TQVTVSS |
| **85** | Anti-mouseGp130 | QVQLQESGGGLVQPGGSLRLSCAASGFTLSTYWMYWVRQAPGKGPEWVSAV SRGGFNTYYADSVKGRFTISRDNAKNTVYLQMNSLKPEDTAVYYCMSSVSFY GWPPDRVPSPTGQGTQVTVSS |
| **86** | Anti-mouseGp130 | QVQLQESGGGSVQPGGSLRLSCAASGFTFSTYDMSWVRQAPGKGLEWVSTIN YSGSSTYYVDSVLGRFTIARDNAKNTLYLQMNNLQTEDTAVYYCASVKERRS NGHPIVFGDRGQGTQVTVSS |
| **87** | Anti-mouseGp130 | QVQLQESGGGLVQPGGSLRLSCAASGFTFRNYAMSWVRQAPGKGLEWVSAIN SGGGSTYYADSVKGRFTISRDNAKNTLYLQMNSLKPEDTAMYYCAKHVTGDY DPSLRYEYNYWSQGTQVTVSS |
| **88** | Anti-mouseGp130 | QVQLQESGGGSVQAGGSLRLSCVISGFTYRQTFMGWFRQVVGKEREGVAAIST GGGSTIYADSVKGRFTISQDSSKDTVYLEMNGLKLEDTGMYYCAASTVITSESI NRNLYQYWGQGTQVTVSS |
| **89** | Anti-mouseGp130 | QVQLQESGGGSVQAGGFLRLSCAFSGYTGCMGWFRQGPGQEREGVASINDGG SLTYADSVKGRFTISKDNAKKTLDLQMNTLKPEDTAMYYCAASLSYCLNPTLR VDGYNYWGQGTQVTVSS |
| **90** | Anti-IL2Rbeta/anti-CD122 (mouse) | QVQLQESGGGLVQPGGSLRLSCAASGFTFSLYDMSWVRQAPGKGLEWVSGIN SGGYSTYYAASAKGRFTISRDNAKNTLYLQLSSVKTEDTAMYYCAQRGLTSPY VIPNIRLQGTQVTVSS |
| **91** | Anti-IL2Rbeta/anti-CD122 (mouse) | QVQLQESGGGLVQPGGSLRLSCAASEFTFSNNWMHWVRQAPGKGFEWVSSIH SGMAITHYRGSVKGRFTISSDIAKNIVSLQMNSLKAEDTAVYYCVGEGNWGQG TQVTVSS |
| **92** | Anti-IL2Rgamma/Ant i-CD132 (mouse) | QVQLQESGGGSVLAGGSLRLSCVASGYGYNYIGWFRQTPGKEREGVAVIYTG GGDTYYADSVKGRFTASRDNAKSTLYLQMNSLEPEDTAMYYGVARYCVGSV YACLRGGHDEYAHWGQGTQVTVSS |
| **93** | Anti-IL2Rgamma/Ant i-CD132 (mouse) | QVQLQESGGGSVQPGGSLRLSCAASGSTYANYLMGWFRQAPGKEREGVAAIY SGGGSTYYADSVKGRFTISQDNAKNTLYLQMNSLKPEDTAMYYCAAASAVKG DKGDIVVVVTGTQRMEYDYWGHGTQVTVSS |
| **94** | Anti-IL2Rgamma/Ant i-CD132 (mouse) | QVQLQESGGGSVQAGASLRLSCSVSGFTFDESVMSWLRQGPGNECDAVAIISS DDNTYYDDSVKGRFTISEDNAKNMVYLQMNSLKPEDTAVYYCAARRRRPVY DSDYELRPRPLCGDFGVWGQGTQVTVSS |
| **95** | Anti-IL2Rgamma/Ant i-CD132 (mouse) | QVQLQESGGGSVQAGGSLRLSCIGSGLPFDEDDMGWYRQAPGNECELVSSISS DGTAYYADSVKGRFTISRDNAKNTVLLQMNSLKPEDTAVYYCAAGVHRQFGG SSSCGDAFYGMDYWGKGTQVTVSS |
| **96** | Anti-IL2Rgamma/ Anti-CD132 (mouse) | QVQLQESGGGSVQAGGSLRLSCVASGDVYGRNSMAWFRQAPGKEREGVAVG YSVVTTTYYADSVKGRFTISEDNDKNTVYLEMNSLKPEDTAMYYCAADGNL WRGLRPSEYTYWGQGTQVTVSS |
| **97** | Anti-IL2Rgamma/ Anti-CD132 (mouse) | QVQLQESGGGSVQAGGSLRLSCTASGFTFDDFDMGWYRQAPGNECELVSTISD DGSTYYADSVKGRSSISRDNAKNTVYLQMNRLKPEDTGVYYCAAEGALGSKM NCGWVGNFGYWGQGTQVTVSS |
| **98** | Anti-IL2Rgamma/ Anti-CD132 (mouse) | QVQLQESGGGSVQAGGSLRLSCATSGFPYSRYCMGWFRQAPGKEREGVAAIEP DGSTSYADSVKGRFTISQDNAVNTLYLQMNNLKPEDTAMYYCAADERCFYLK DYDLRRPAQYRYWGQGTQVTVSS |
| **99** | Anti-IL10Rbeta (mouse) | QVQLQESGGGSVQAGGSLRLSCAASGYTYNRRFMGWFRQAPGKEREGLAIIYT PNSSTFYADSVTGRFTISQDSARNTVYLQMNSLKPEDTAMYYCAAARIASMTE LSVRDMDYWGKGTQVTVSS |
| **100** | Anti-IL10Rbeta (mouse) | QVQLQESGGGSVQAGGSLRLSCTASRYIALNACMAWIRQAPGSEREVVATIVT DGSRTYYADSVKGRFTISQDNAKNTMYLQMNGLKPEDTAMYYCAADRRCPV SRAPYEYELRYWGQGTQVTVSS |
| **101** | Anti-IL10Rbeta (mouse) | QVQLQESGGGSVQAGGSLRLSCVASGDTYSRKYIAWVRQVPGKEREGVAVM YTPGSATYYTDTVMGRFTISQDNAKNTVYLQMNSLKPEDTAMYFCAAKASGS MFNFRDYTYWGQGTQVTVSS |
| **102** | Anti-IL10Rbeta (mouse) | QVQLQESGGGSVQAGGALRLSCTASGYTASSICMGWFRQAPGKERERVAVITT AASGTYYADSVNGRFSISQNNAKNTVYLQMNSLKPDDTAMYYCAATRRGGD CLDPLQTPAYNTWGQGTQVTVSS |
| **103** | Anti-IL10Rbeta (mouse) | QVQLQESGGGSVQAGGSLRLSCATSGYASCSRAMRWYRQAPGKEREFVAYID GVGSTGYADSVKGRFTISQDNAKYTAYLQMNSLKPEDTAMYYCNRGCRADG SNSLDNYWGQGTQVTVSS |
| **104** | Anti-IL10Rbeta (mouse) | QVQLQESGGGSVQAGGSLRLSCAASGYTYNGKCMAWFRQAPGKEREVVAGI YTGGSSTYYADSVKGRFTISQDNAKNTVYLQMDSLKPEDTAMYYCATSRSCS DLRRRSIAYWGQGTQVTVSS |
| **105** | Anti-IL12Rbetal (mouse) | QVQLQESGGGSVQAGGSLRLSCTASGYTYSSAFMAWFRQAPGKEREGVAAIY TRDGGTVYADSVKGRFTISQDNAKNTLYLQMNSLKAEDTAMYYCAAKIPQPG RASLLDSQTYDYWGQGTQVTVSS |
| **106** | Anti-IL12Rbetal (mouse) | QVQLQESGGGSVQAGGSLRLSCAVSGYDYCGYDVRWYRQAPGKEREFVSGID SDGSTSYADSVKGRFTISQDNAENTSYLHMFSLKPEDTAMYYCKTESPAGESA WCRNFRGMDYWGKGTQVTVSS |
| **107** | Anti-IL12Rbetal (mouse) | QVQLQESGGGSVQAGGSLRLSCVASGYSYCGYDMMWYRQAPGKEREFVALIT SDYSIRYEDSVEGRFSISRDNAKNTGYLLMSNLTPADTAIYYCKTSTAARESSW CRSRYRVASWGQGTQVTVSS |
| **108** | Anti-IL12Rbetal (mouse) | QVQLQESGGGSVQAGGSLRLSCAASRYTYTNNFMAWFRQAPGKEREGVAAIY TGDGYAYYFDSVKGRFTISQDNDKNMLYLQMNSLKPEDTAMYYCAAMERRS GRRRMTENAEYKYWGQGTQVTVSS |
| **109** | Anti-IL12Rbeta1 (mouse) | QVQLQESGGGSVQAGETLRLSCTVSGFTIDDSEMGWYRQAPGHECELVASGSS DDDTYYVDSVKGRFTISLDNAKNMVYLQMNSLKPEDTAVYYCATGPTYPPKD GDCAHWGQGTQVTVSS |
| **110** | Anti-IL12Rbeta1 (mouse) | QVQLQESGGGSVQAGGSLRLSCTASGYTYSSAFMAWFRQAPGKEREGVAAIY TRDGSPVYADSLKGRFTISQDNAKNTLHLQMNSLKPEDTAMYYCAAKIPEPGR ISLLDSQTYDYWGHGTQVTVSS |
| **111** | Anti-IL12Rbeta1 (mouse) | QVQLQESGGGSVQAGGSLRLSCAVSGYDYCGYDVRWYRRAPGKEREFVSGID SDGSTSYADSVKGRFTISQDNAENTSYLHMFSLKPEDTAMYYCKTESPAGESA WCRNFRGMDYWGKGTQVTVSS |
| **112** | Anti-IL12Rbeta2 (mouse) | QVQLQESGGGSVQAGGSLRLSCAASGYTYSNRHMGWFRQAPGKEREGVAAIY TGGGSTYYADSVKDRFTISQDNAKNTLYLQMNSLTPEDTAMYYCAADLTRWY SGGWRDPRGYKYWGQGTQVTVS (303) |
| **113** | Anti-IL12Rbeta2 (mouse) | QVQLQESGGGSVQAGGSLRLSCAASGVTYGSYYMAAWFRQAPGKEREGVASI YGGSDSTYYADSVLGRFTISQDNGKNTLYLQMNSLKPDDTAMYYCAAAPPGK WFLKRLEGHNYSYWGQGTQVTVSS |
| **114** | Anti-IL12Rbeta2 (mouse) | QVQLQESGGGSVQVGGSLRLSCAASGFTYSSSCLGWFRQAPGKEREGVATIYP AGGNIFYADSVKGRFTISQDNAKNTVYLQMDSLKPEDTAMYYCAARGGQTW GSGGNRCSLWLPAYNYWGQGTQVTVSS |
| **115** | Anti-IL12Rbeta2 (mouse) | QVQLQESGGGSVQVGGSLRLSCAVSGKLYGGAWFRQAQGKGREGVAAIWIGT GTTFYADSVKGRFTISRDNAKNTVYLQMDGLKPEDTALYYCAADDRPGYRDP LAPVSYNHWGQGTQVTVSS |
| **116** | Anti-IL12Rbeta2 (mouse) | QVQLQESGGGSVQAGGSLRLSCAASGITYRGVWMGWFRQAPGKEREGVATIY TGSGHTYYADSVKGRFTISQDNAKNTVYLQMNSLKPEDTAMYYCAARTVGGT FYTLAADSFNTWGQGTQVTVSS |
| **117** | Anti-IL12Rbeta2 (mouse) | QVQLQESGGGSVQDGGSLRLSCAASGDIYARNCMGWFRQAPGKEREKIAVAD TGGRSPYYADSVKGRFTISRDNAKNTVDLQMNSLKPEDTAVYYCAAGPLVPV VNTAARCVYEYWGQGTQVTVSS |
| **118** | Anti-il23R mouse | QVQLQESGGGSVQAGGSLRLSCAASGYTYSSCTMGWYRQAPGKERELVSMLI SDGSTFYADSVKGRFTFSQEYAKNTVYLQMNSLKPEDTAMYYCGCATLGSRT VWGQGTQVTVSS |
| **119** | Anti-il23R mouse | QVQLQESGGGSVQAGGSLTLSCTAPGFTFRLAAMRWVRQAPGKGLEWVSGID SRGSTIYADSVKGRFTISKDNAKNTLYLQLNSLKTEDTAMYYCAQGVYGDTYS GSQGTQVTVSS |
| **120** | Anti-il23R mouse | QVQLQESGGGSVQAGGSLRLSCTASVNTYCEYNMSWYRQAPGKEREFVSGVD SDGSTRYSESVKGRFTISQDNAKNTMYLQMNGLKPEDTAMYYCKTYVCTFCS GNSCYYEYKYYYEGQGTQVTVSS |
| **121** | Anti-il23R mouse | QVQLQESGGGSVQAGGSLRLSCAASGYTYSNNCMGWFRQAPGKDRERIANIY TGGGRTTYADSVKGRFTISQDSAKSTVYLQMNSLKPEDTAMYYCAAGSCGSA RSEYSYWGQGTQVTVSS |
| **122** | Anti-il23R mouse | QVQLQESGGGSVQAGGSLRLSCAASGYTFCMAWFRQAPGKEREGVARFYTRD GYTYYSDSVKGRFTISQNNAKNTLYLQMNSLKSEDTAMYYCAADLARCSSNK NDFRYWGQGTQVTVSS |
| **123** | Anti-il23R mouse | QVQLQESGGGSVQAGGSLRLSCAASGYTSGNYWMGWFRQAPGKEREGVATL WTGGASTFYGDSVKGRFTISRDNFKNTLYLQMNSLKVEDTAMYYCAADPALR LGANILRPAEYKYWGQGTQVTVSS |
| **124** | Anti-il23R mouse | QVQLQESGGGLVQPGGSLRLSCAASGFTFSRSAMTWVRQAPGKGLDWVSGID SGGTTVYADSVKGRFTISRDSAKNTLYLQMNSLKTEDTAVYYCAIGLPWGNT WRTRGQGTQVTVSS |
| **125** | Anti-il27R (mouse) | QVQLQESGGGSVQAGGSLRLSCAVSGDSTYSMGWFRQPPGKEREGVAAITKDI TIHADSVKGRFTISKDNAKNTLYLQMNSLKPEDTAMYYCAAHRPYGPPLNPR WYTYWGQGTQVTVSS |
| **126** | Anti-il27R (mouse) | QVQLQESGGGSVQAGGSLRLSCTASGYTSSRYCMGWFRQTPGKKREGVAAIY TGGGTTFYHGSVKGRFTISQDNTTNTVYLQMHNLKPEDTAMYYCAAGPVTRA CDEYNYWGQGTQVTVSS |
| **127** | Anti-il27R (mouse) | QVQLQESGGGSVQAGGSLRLSCAASGYSINRMGWFRQAPGKEREGVAAISIGG GQTYYADSVKGRFTISQDNAKNTVDLQMNSLKPEDTAMYYCAAGLVYGEAW LDSRHYNKWGQGTQVTVSS |
| **128** | Anti-il27R (mouse) | QVQLQESGGGSVQAGGSLRLSCAGSGYSLSNYCMGWFRQAPGQGREGVASLR FVSGATFYADSVKGRFTIAQDNAKNTLYLQMNSLKPEDTAMYYCGIKSRGICG GRLVDVDFGNWGQGTQVTVSS |
| **129** | Anti-i127R (mouse) | QVQLQESGGGSVQAGGSLRLSCAASKNSNFMGWFRQAPGKEREGVAAMMTK NNNTYYADSVKGRFTISHDNAKNTVYLQMDSLKPEDTAVYYCAAVYRTRRLR VLEAANFDYWGQGTQVTVSS |
| **130** | Anti-i127R (mouse) | QVQLQESGGGSVQAGGSLRLSCAASGYTYSSYCMAWFRQAPGKEREGVAAID SDGSTSYADSVKGRFTISKDNAKNTLYLQMNSLKPEDTAMYYCAAASGRCLG PGIRSLIWGQGTQVTVSS |
| **131** | IL12Rβ1VHH-GGGS-IL12Rβ2VHH | QVQLQESGGGSVQAGGSLRLSCTASGYTYSSAFMAWFRQAPGKEREGVAAIY TRDGGTVYADSVKGRFTISQDNAKNTLYLQMNSLKAEDTAMYYCAAKIPQPG RASLLDSQTYDYWGQGTQVTVSSGGGSQVQLQESGGGSVQAGGSLRLSCAAS GYTYSNRHMGWFRQAPGKEREGVAAIYTGGGSTYYADSVKDRFTISQDNAKN TLYLQMNSLTPEDTAMYYCAADLTRWYSGGWRDPRGYKYWGQGTQVTVS (SEQ ID NO:131) |
| **132** | IL12Rβ1VHH-GGGS-IL12Rβ2VHH | QVQLQESGGGSVQAGGSLRLSCTASGYTYSSAFMAWFRQAPGKEREGVAAIY TRDGGTVYADSVKGRFTISQDNAKNTLYLQMNSLKAEDTAMYYCAAKIPQPG RASLLDSQTYDYWGQGTQVTVSSGGGSQVQLQESGGGSVQAGGSLRLSCAAS GVTYGSYYMAAWFRQAPGKEREGVASIYGGSDSTYYADSVLGRFTISQDNGK NTLYLQMNSLKPDDTAMYYCAAAPPGKWFLKRLEGHNYSYWGQGTQVTVSS (SEQ ID NO:132) |
| **133** | IL12Rβ1VHH-GGGS-IL12Rβ2VHH | QVQLQESGGGSVQAGGSLRLSCTASGYTYSSAFMAWFRQAPGKEREGVAAIY TRDGGTVYADSVKGRFTISQDNAKNTLYLQMNSLKAEDTAMYYCAAKIPQPG RASLLDSQTYDYWGQGTQVTVSSGGGSQVQLQESGGGSVQVGGSLRLSCAAS GFTYSSSCLGWFRQAPGKEREGVATIYPAGGNIFYADSVKGRFTISQDNAKNTV YLQMDSLKPEDTAMYYCAARGGQTWGSGGNRCSLWLPAYNYWGQGTQVTV SS (SEQ ID NO:133) |
| **134** | IL12Rβ1VHH-GGGS-IL12Rβ2VHH | QVQLQESGGGSVQAGGSLRLSCTASGYTYSSAFMAWFRQAPGKEREGVAAIY TRDGGTVYADSVKGRFTISQDNAKNTLYLQMNSLKAEDTAMYYCAAKIPQPG RASLLDSQTYDYWGQGTQVTVSSGGGSQVQLQESGGGSVQVGGSLRLSCAVS GKLYGGAWFRQAQGKGREGVAAIWIGTGTTFYADSVKGRFTISRDNAKNTVY LQMDGLKPEDTALYYCAADDRPGYRDPLAPVSYNHWGQGTQVTVSS (SEQ ID NO:134) |
| **135** | IL12Rβ1VHH-GGGS-IL12Rβ2VHH | QVQLQESGGGSVQAGGSLRLSCTASGYTYSSAFMAWFRQAPGKEREGVAAIY TRDGGTVYADSVKGRFTISQDNAKNTLYLQMNSLKAEDTAMYYCAAKIPQPG RASLLDSQTYDYWGQGTQVTVSSGGGSQVQLQESGGGSVQAGGSLRLSCAAS GITYRGVWMGWFRQAPGKEREGVATIYTGSGHTYYADSVKGRFTISQDNAKN TVYLQMNSLKPEDTAMYYCAARTVGGTFYTLAADSFNTWGQGTQVTVSS (SEQ ID NO:135) |
| **136** | IL12Rβ1VHH-GGGS-IL12Rβ2VHH | QVQLQESGGGSVQAGGSLRLSCTASGYTYSSAFMAWFRQAPGKEREGVAAIY TRDGGTVYADSVKGRFTISQDNAKNTLYLQMNSLKAEDTAMYYCAAKIPQPG RASLLDSQTYDYWGQGTQVTVSSGGGSQVQLQESGGGSVQDGGSLRLSCAAS GDIYARNCMGWFRQAPGKEREKIAVADTGGRSPYYADSVKGRFTISRDNAKN TVDLQMNSLKPEDTAVYYCAAGPLVPVVNTAARCVYEYWGQGTQVTVSS (SEQ ID NO:136) |
| **137** | IL12Rβ1VHH-GGGS-IL12Rβ2VHH | QVQLQESGGGSVQAGGSLRLSCAVSGYDCGYDVRWYRQAPGKEREFVSGID SDGSTSYADSVKGRFTISQDNAENTSYLHMFSLKPEDTAMYYCKTESPAGESA WCRNFRGMDYWGKGTQVTVSSGGGSQVQLQESGGGSVQAGGSLRLSCAASG YTYSNRHMGWFRQAPGKEREGVAAIYTGGGSTYYADSVKDRFTISQDNAKNT LYLQMNSLTPEDTAMYYCAADLTRWYSGGWRDPRGYKYWGQGTQVTVS (SEQ ID NO:137) |
| **138** | IL12Rβ1VHH-GGGS-IL12Rβ2VHH | QVQLQESGGGSVQAGGSLRLSCAVSGYDYCGYDVRWYRQAPGKEREFVSGID SDGSTSYADSVKGRFTISQDNAENTSYLHMFSLKPEDTAMYYCKTESPAGESA WCRNFRGMDYWGKGTQVTVSSGGGSQVQLQESGGGSVQAGGSLRLSCAASG VTYGSYYMAAWFRQAPGKEREGVASIYGGSDSTYYADSVLGRFTISQDNGKN TLYLQMNSLKPDDTAMYYCAAAPPGKWFLKRLEGHNYSYWGQGTQVTVSS (SEQ ID NO:138) |
| **139** | IL12Rβ1VHH-GGGS-IL12Rβ2VHH | QVQLQESGGGSVQAGGSLRLSCAVSGYDYCGYDVRWYRQAPGKEREFVSGID SDGSTSYADSVKGRFTISQDNAENTSYLHMFSLKPEDTAMYYCKTESPAGESA WCRNFRGMDYWGKGTQVTVSSGGGSQVQLQESGGGSVQVGGSLRLSCAASG FTYSSSCLGWFRQAPGKEREGVATIYPAGGNIFYADSVKGRFTISQDNAKNTVY LQMDSLKPEDTAMYYCAARGGQTWGSGGNRCSLWLPAYNYWGQGTQVTVS S (SEQ ID NO:139) |
| **140** | IL12Rβ1VHH-GGGS-IL12Rβ2VHH | QVQLQESGGGSVQAGGSLRLSCAVSGYDYCGYDVRWYRQAPGKEREFVSGID SDGSTSYADSVKGRFTISQDNAENTSYLHMFSLKPEDTAMYYCKTESPAGESA WCRNFRGMDYWGKGTQVTVSSGGGSQVQLQESGGGSVQVGGSLRLSCAVSG KLYGGAWFRQAQGKGREGVAAIWIGTGTTFYADSVKGRFTISRDNAKNTVYL QMDGLKPEDTALYYCAADDRPGYRDPLAPVSYNHWGQGTQVTVSS (SEQ ID NO:140) |
| **141** | IL12Rβ1VHH-GGGS-IL12Rβ2VHH | QVQLQESGGGSVQAGGSLRLSCAVSGYDYCGYDVRWYRQAPGKEREFVSGID SDGSTSYADSVKGRFTISQDNAENTSYLHMFSLKPEDTAMYYCKTESPAGESA WCRNFRGMDYWGKGTQVTVSSGGGSQVQLQESGGGSVQAGGSLRLSCAASG ITYRGVWMGWFRQAPGKEREGVATIYTGSGHTYYADSVKGRFTISQDNAKNT VYLQMNSLKPEDTAMYYCAARTVGGTFYTLAADSFNTWGQGTQVTVSS (SEQ ID NO:141) |
| **142** | IL12Rβ1VHH-GGGS-IL12Rβ2VHH | QVQLQESGGGSVQAGGSLRLSCAVSGYDYCGYDVRWYRQAPGKEREFVSGID SDGSTSYADSVKGRFTISQDNAENTSYLHMFSLKPEDTAMYYCKTESPAGESA WCRNFRGMDYWGKGTQVTVSSGGGSQVQLQESGGGSVQDGGSLRLSCAASG DIYARNCMGWFRQAPGKEREKIAVADTGGRSPYYADSVKGRFTISRDNAKNT VDLQMNSLKPEDTAVYYCAAGPLVPVVNTAARCVYEYWGQGTQVTVSS (SEQ ID NO:142) |
| **143** | IL12Rβ1VHH-GGGS-IL12Rβ2VHH | QVQLQESGGGSVQAGGSLRLSCVASGYSYCGYDMMWYRQAPGKEREFVALIT SDYSIRYEDSVEGRFSISRDNAKNTGYLLMSNLTPADTAIYYCKTSTAARESSW CRSRYRVASWGQGTQVTVSSGGGSQVQLQESGGGSVQAGGSLRLSCAASGYT YSNRHMGWFRQAPGKEREGVAAIYTGGGSTYYADSVKDRFTISQDNAKNTLY LQMNSLTPEDTAMYYCAADLTRWYSGGWRDPRGYKYWGQGTQVTVS (sEQ ID NO:143) |
| **144** | IL12Rβ1VHH -GGGS-IL12Rβ2VHH | QVQLQESGGGSVQAGGSLRLSCVASGYSYCGYDMMWYRQAPGKEREFVAL ITSDYSIRYEDSVEGRFSISRDNAKNTGYLLMSNLTPADTAIYYCKTSTAARES SWCRSRYRVASWGQGTQVTVSSGGGSQVQLQESGGGSVQAGGSLRLSCAA SGVTYGSYYMAAWFRQAPGKEREGVASIYGGSDSTYYADSVLGRFTISQDN GKNTLYLQMNSLKPDDTAMYYCAAAPPGKWFLKRLEGHNYSYWGQGTQV TVSS (SEQ ID NO:144) |
| **145** | IL12Rβ1VHH -GGGS-IL12Rβ2VHH | QVQLQESGGGSVQAGGSLRLSCVASGYSYCGYDMMWYRQAPGKEREFVAL ITSDYSIRYEDSVEGRFSISRDNAKNTGYLLMSNLTPADTAIYYCKTSTAARES SWCRSRYRVASWGQGTQVTVSSGGGSQVQLQESGGGSVQVGGSLRLSCAA SGFTYSSSCLGWFRQAPGKEREGVATIYPAGGNIFYADSVKGRFTISQDNAKN TVYLQMDSLKPEDTAMYYCAARGGQTWGSGGNRCSLWLPAYNYWGQGTQ VTVSS (SEQ ID NO:145) |
| **146** | IL12Rβ1VHH -GGGS-IL12Rβ2VHH | QVQLQESGGGSVQAGGSLRLSCVASGYSYCGYDMMWYRQAPGKEREFVAL ITSDYSIRYEDSVEGRFSISRDNAKNTGYLLMSNLTPADTAIYYCKTSTAARES SWCRSRYRVASWGQGTQVTVSSGGGSQVQLQESGGGSVQVGGSLRLSCAV SGKLYGGAWFRQAQGKGREGVAAIWIGTGTTFYADSVKGRFTISRDNAKNT VYLQMDGLKPEDTALYYCAADDRPGYRDPLAPVSYNHWGQGTQVTVSS (SEQ ID NO:149) |
| **147** | IL12Rβ1VHH -GGGS-IL12Rβ2VHH | QVQLQESGGGSVQAGGSLRLSCVASGYSYCGYDMMWYRQAPGKEREFVAL ITSDYSIRYEDSVEGRFSISRDNAKNTGYLLMSNLTPADTAIYYCKTSTAARES SWCRSRYRVASWGQGTQVTVSSGGGSQVQLQESGGGSVQAGGSLRLSCAA SGITYRGVWMGWFRQAPGKEREGVATIYTGSGHTYYADSVKGRFTISQDNA KNTVYLQMNSLKPEDTAMYYCAARTVGGTFYTLAADSFNTWGQGTQVTVS S (SEQ ID NO:147) |
| **148** | IL12Rβ1VHH -GGGS-IL12Rβ2VHH | QVQLQESGGGSVQAGGSLRLSCVASGYSYCGYDMMWYRQAPGKEREFVAL ITSDYSIRYEDSVEGRFSISRDNAKNTGYLLMSNLTPADTAIYYCKTSTAARES SWCRSRYRVASWGQGTQVTVSSGGGSQVQLQESGGGSVQDGGSLRLSCAA SGDIYARNCMGWFRQAPGKEREKIAVADTGGRSPYYADSVKGRFTISRDNA KNTVDLQMNSLKPEDTAVYYCAAGPLVPVVNTAARCVYEYWGQGTQVTVS S (SEQ ID NO:148) |
| **149** | IL12Rβ1VHH -GGGS-IL12Rβ2VHH | QVQLQESGGGSVQAGGSLRLSCAASRYTYTNNFMAWFRQAPGKEREGVAAI YTGDGYAYYFDSVKGRFTISQDNDKNMLYLQMNSLKPEDTAMYYCAAMER RSGRRRMTENAEYKYWGQGTQVTVSSGGGSQVQLQESGGGSVQAGGSLRL SCAASGYTYSNRHMGWFRQAPGKEREGVAAIYTGGGSTYYADSVKDRFTIS QDNAKNTLYLQMNSLTPEDTAMYYCAADLTRWYSGGWRDPRGYKYWGQ GTQVTVS (SEQ ID NO:149) |
| **150** | IL12Rβ1VHH -GGGS-IL12Rβ2VHH | QVQLQESGGGSVQAGGSLRLSCAASRYTYTNNFMAWFRQAPGKEREGVAAI YTGDGYAYYFDSVKGRFTISQDNDKNMLYLQMNSLKPEDTAMYYCAAMER RSGRRRMTENAEYKYWGQGTQVTVSSGGGSQVQLQESGGGSVQAGGSLRL SCAASGVTYGSYYMAAWFRQAPGKEREGVASIYGGSDSTYYADSVLGRFTIS QDNGKNTLYLQMNSLKPDDTAMYYCAAAPPGKWFLKRLEGHNYSYWGQG TQVTVSS (SEQ ID NO:150) |
| **151** | IL12Rβ1VHH -GGGS-IL12Rβ2VHH | QVQLQESGGGSVQAGGSLRLSCAASRYTYTNNFMAWFRQAPGKEREGVAAI YTGDGYAYYFDSVKGRFTISQDNDKNMLYLQMNSLKPEDTAMYYCAAMER RSGRRRMTENAEYKYWGQGTQVTVSSGGGSQVQLQESGGGSVQVGGSLRL SCAASGFTYSSSCLGWFRQAPGKEREGVATIYPAGGNIFYADSVKGRFTISQD NAKNTVYLQMDSLKPEDTAMYYCAARGGQTWGSGGNRCSLWLPAYNYWG QGTQVTVSS (SEQ ID NO:151) |
| **152** | IL12Rβ1VHH -GGGS-IL12Rβ2VHH | QVQLQESGGGSVQAGGSLRLSCAASRYTYTNNFMAWFRQAPGKEREGVAAI YTGDGYAYYFDSVKGRFTISQDNDKNMLYLQMNSLKPEDTAMYYCAAMER RSGRRRMTENAEYKYWGQGTQVTVSSGGGSQVQLQESGGGSVQVGGSLRL SCAVSGKLYGGAWFRQAQGKGREGVAAIWIGTGTTFYADSVKGRFTISRDN AKNTVYLQMDGLKPEDTALYYCAADDRPGYRDPLAPVSYNHWGQGTQVT VSS (SEQ ID NO:152) |
| **153** | IL12Rβ1VHH -GGGS-IL12Rβ2VHH | QVQLQESGGGSVQAGGSLRLSCAASRYTYTNNFMAWFRQAPGKEREGVAAI YTGDGYAYYFDSVKGRFTISQDNDKNMLYLQMNSLKPEDTAMYYCAAMER RSGRRRMTENAEYKYWGQGTQVTVSSGGGSQVQLQESGGGSVQAGGSLRL SCAASGITYRGVWMGWFRQAPGKEREGVATIYTGSGHTYYADSVKGRFTIS QDNAKNTVYLQMNSLKPEDTAMYYCAARTVGGTFYTLAADSFNTWGQGT QVTVSS (SEQ ID NO:153) |
| **154** | IL12Rβ1VHH -GGGS-IL12Rβ2VHH | QVQLQESGGGSVQAGGSLRLSCAASRYTYTNNFMAWFRQAPGKEREGVAAI YTGDGYAYYFDSVKGRFTISQDNDKNMLYLQMNSLKPEDTAMYYCAAMER RSGRRRMTENAEYKYWGQGTQVTVSSGGGSQVQLQESGGGSVQDGGSLRL SCAASGDIYARNCMGWFRQAPGKEREKIAVADTGGRSPYYADSVKGRFTISR DNAKNTVDLQMNSLKPEDTAVYYCAAGPLVPVVNTAARCVYEYWGQGTQ VTVSS (SEQ ID NO:154) |
| **155** | IL12Rβ1VHH -GGGS-IL12Rβ2VHH | QVQLQESGGGSVQAGETLRLSCTVSGFTIDDSEMGWYRQAPGHECELVASG SSDDDTYYVDSVKGRFTISLDNAKNMVYLQMNSLKPEDTAVYYCATGPTYP PKDGDCAHWGQGTQVTVSSGGGSQVQLQESGGGSVQAGGSLRLSCAASGY TYSNRHMGWFRQAPGKEREGVAAIYTGGGSTYYADSVKDRFTISQDNAKNT LYLQMNSLTPEDTAMYYCAADLTRWYSGGWRDPRGYKYWGQGTQVTVS (SEQ ID NO:155) |
| **156** | IL12Rβ1VHH -GGGS-IL12Rβ2VHH | QVQLQESGGGSVQAGETLRLSCTVSGFTIDDSEMGWYRQAPGHECELVASG SSDDDTYYVDSVKGRFTISLDNAKNMVYLQMNSLKPEDTAVYYCATGPTYP PKDGDCAHWGQGTQVTVSSGGGSQVQLQESGGGSVQAGGSLRLSCAASGV TYGSYYMAAWFRQAPGKEREGVASIYGGSDSTYYADSVLGRFTISQDNGKN TLYLQMNSLKPDDTAMYYCAAAPPGKWFLKRLEGHNYSYWGQGTQVTVSS (SEQ ID NO:156) |
| **157** | IL12Rβ1VHH -GGGS-IL12Rβ2VHH | QVQLQESGGGSVQAGETLRLSCTVSGFTIDDSEMGWYRQAPGHECELVASG SSDDDTYYVDSVKGRFTISLDNAKNMVYLQMNSLKPEDTAVYYCATGPTYP PKDGDCAHWGQGTQVTVSSGGGSQVQLQESGGGSVQVGGSLRLSCAASGF TYSSSCLGWFRQAPGKEREGVATIYPAGGNIFYADSVKGRFTISQDNAKNTV YLQMDSLKPEDTAMYYCAARGGQTWGSGGNRCSLWLPAYNYWGQGTQVT VSS (SEQ ID NO:157) |
| **158** | IL12Rβ1VHH -GGGS-IL12Rβ2VHH | QVQLQESGGGSVQAGETLRLSCTVSGFTIDDSEMGWYRQAPGHECELVASG SSDDDTYYVDSVKGRFTISLDNAKNMVYLQMNSLKPEDTAVYYCATGPTYP PKDGDCAHWGQGTQVTVSSGGGSQVQLQESGGGSVQVGGSLRLSCAVSGK LYGGAWFRQAQGKGREGVAAIWIGTGTTFYADSVKGRFTISRDNAKNTVYL QMDGLKPEDTALYYCAADDRPGYRDPLAPVSYNHWGQGTQVTVSS (SEQ ID NO:158) |
| **159** | IL12Rβ1VHH -GGGS-IL12Rβ2VHH | QVQLQESGGGSVQAGETLRLSCTVSGFTIDDSEMGWYRQAPGHECELVASG SSDDDTYYVDSVKGRFTISLDNAKNMVYLQMNSLKPEDTAVYYCATGPTYP PKDGDCAHWGQGTQVTVSSGGGSQVQLQESGGGSVQAGGSLRLSCAASGIT YRGVWMGWFRQAPGKEREGVATIYTGSGHTYYADSVKGRFTISQDNAKNT VYLQMNSLKPEDTAMYYCAARTVGGTFYTLAADSFNTWGQGTQVTVSS (SEQ ID NO:159) |
| **160** | IL12Rβ1VHH -GGGS-IL12Rβ2VHH | QVQLQESGGGSVQAGETLRLSCTVSGFTIDDSEMGWYRQAPGHECELVASG SSDDDTYYVDSVKGRFTISLDNAKNMVYLQMNSLKPEDTAVYYCATGPTYP PKDGDCAHWGQGTQVTVSSGGGSQVQLQESGGGSVQDGGSLRLSCAASGD IYARNCMGWFRQAPGKEREKIAVADTGGRSPYYADSVKGRFTISRDNAKNT VDLQMNSLKPEDTAVYYCAAGPLVPVVNTAARCVYEYWGQGTQVTVSS (SEQ ID NO:160) |
| **161** | IL12Rβ1VHH -GGGS-IL12Rβ2VHH | QVQLQESGGGSVQAGGSLRLSCAASGYTYSNRHMGWFRQAPGKEREGVAAI YTGGGSTYYADSVKDRFTISQDNAKNTLYLQMNSLTPEDTAMYYCAADLTR WYSGGWRDPRGYKYWGQGTQVTVSGGGSQVQLQESGGGSVQAGGSLRLS CTASGYTYSSAFMAWFRQAPGKEREGVAAIYTRDGGTVYADSVKGRFTISQ DNAKNTLYLQMNSLKAEDTAMYYCAAKIPQPGRASLLDSQTYDYWGQGTQ VTVSS (SEQ ID NO:161) |
| **162** | IL12Rβ1VHH -GGGS-IL12Rβ2VHH | QVQLQESGGGSVQAGGSLRLSCAASGYTYSNRHMGWFRQAPGKEREGVAAI YTGGGSTYYADSVKDRFTISQDNAKNTLYLQMNSLTPEDTAMYYCAADLTR WYSGGWRDPRGYKYWGQGTQVTVSGGGSQVQLQESGGGSVQAGGSLRLS CAVSGYDYCGYDVRWYRQAPGKEREFVSGIDSDGSTSYADSVKGRFTISQD NAENTSYLHMFSLKPEDTAMYYCKTESPAGESAWCRNFRGMDYWGKGTQV TVSS (SEQ ID NO:162) |
| **163** | IL12Rβ1VHH -GGGS-IL12Rβ2VHH | QVQLQESGGGSVQAGGSLRLSCAASGYTYSNRHMGWFRQAPGKEREGVAAI YTGGGSTYYADSVKDRFTISQDNAKNTLYLQMNSLTPEDTAMYYCAADLTR WYSGGWRDPRGYKYWGQGTQVTVSGGGSQVQLQESGGGSVQAGGSLRLS CVASGYSYCGYDMMWYRQAPGKEREFVALITSDYSIRYEDSVEGRFSISRDN AKNTGYLLMSNLTPADTAIYYCKTSTAARESSWCRSRYRVASWGQGTQVTV SS (SEQ ID NO:163) |
| **164** | IL12Rβ1VHH -GGGS-IL12Rβ2VHH | QVQLQESGGGSVQAGGSLRLSCAASGYTYSNRHMGWFRQAPGKEREGVAAI YTGGGSTYYADSVKDRFTISQDNAKNTLYLQMNSLTPEDTAMYYCAADLTR WYSGGWRDPRGYKYWGQGTQVTVSGGGSQVQLQESGGGSVQAGGSLRLS CAASRYTYTNNFMAWFRQAPGKEREGVAAIYTGDGYAYYFDSVKGRFTISQ DNDKNMLYLQMNSLKPEDTAMYYCAAMERRSGRRRMTENAEYKYWGQG TQVTVSS (SEQ ID NO:164) |
| **165** | IL12Rβ1VHH -GGGS-IL12Rβ2VHH | QVQLQESGGGSVQAGGSLRLSCAASGYTYSNRHMGWFRQAPGKEREGVAAI YTGGGSTYYADSVKDRFTISQDNAKNTLYLQMNSLTPEDTAMYYCAADLTR WYSGGWRDPRGYKYWGQGTQVTVSGGGSQVQLQESGGGSVQAGETLRLS CTVSGFTIDDSEMGWYRQAPGHECELVASGSSDDDTYYVDSVKGRFTISLDN AKNMVYLQMNSLKPEDTAVYYCATGPTYPPKDGDCAHWGQGTQVTVSS (SEQ ID NO:165) |
| **166** | IL12Rβ1VHH -GGGS-IL12Rβ2VHH | QVQLQESGGGSVQAGGSLRLSCAASGVTYGSYYMAAWFRQAPGKEREGVA SIYGGSDSTYYADSVLGRFTISQDNGKNTLYLQMNSLKPDDTAMYYCAAAPP GKWFLKRLEGHNYSYWGQGTQVTVSSGGGSQVQLQESGGGSVQAGGSLRL SCTASGYTYSSAFMAWFRQAPGKEREGVAAIYTRDGGTVYADSVKGRFTISQ DNAKNTLYLQMNSLKAEDTAMYYCAAKIPQPGRASLLDSQTYDYWGQGTQ VTVSS (SEQ ID NO:166) |
| **167** | IL12Rβ1VHH -GGGS-IL12Rβ2VHH | QVQLQESGGGSVQAGGSLRLSCAASGVTYGSYYMAAWFRQAPGKEREGVA SIYGGSDSTYYADSVLGRFTISQDNGKNTLYLQMNSLKPDDTAMYYCAAAPP GKWFLKRLEGHNYSYWGQGTQVTVSSGGGSQVQLQESGGGSVQAGGSLRL SCAVSGYDYCGYDVRWYRQAPGKEREFVSGIDSDGSTSYADSVKGRFTISQD NAENTSYLHMFSLKPEDTAMYYCKTESPAGESAWCRNFRGMDYWGKGTQV TVSS (SEQ ID NO:167) |
| **168** | IL12Rβ1VHH -GGGS-IL12Rβ2VHH | QVQLQESGGGSVQAGGSLRLSCAASGVTYGSYYMAAWFRQAPGKEREGVA SIYGGSDSTYYADSVLGRFTISQDNGKNTLYLQMNSLKPDDTAMYYCAAAPP GKWFLKRLEGHNYSYWGQGTQVTVSSGGGSQVQLQESGGGSVQAGGSLRL SCVASGYSYCGYDMMWYRQAPGKEREFVALITSDYSIRYEDSVEGRFSISRD NAKNTGYLLMSNLTPADTAIYYCKTSTAARESSWCRSRYRVASWGQGTQVT VSS (SEQ ID NO:168) |
| **169** | IL12Rβ1VHH -GGGS-IL12Rβ2VHH | QVQLQESGGGSVQAGGSLRLSCAASGVTYGSYYMAAWFRQAPGKEREGVA SIYGGSDSTYYADSVLGRFTISQDNGKNTLYLQMNSLKPDDTAMYYCAAAPP GKWFLKRLEGHNYSYWGQGTQVTVSSGGGSQVQLQESGGGSVQAGGSLRL SCAASRYTYTNNFMAWFRQAPGKEREGVAAIYTGDGYAYYFDSVKGRFTIS QDNDKNMLYLQMNSLKPEDTAMYYCAAMERRSGRRRMTENAEYKYWGQ GTQVTVSS (SEQ ID NO:169) |
| **170** | IL12Rβ1VHH -GGGS-IL12Rβ2VHH | QVQLQESGGGSVQAGGSLRLSCAASGVTYGSYYMAAWFRQAPGKEREGVA SIYGGSDSTYYADSVLGRFTISQDNGKNTLYLQMNSLKPDDTAMYYCAAAPP GKWFLKRLEGHNYSYWGQGTQVTVSSGGGSQVQLQESGGGSVQAGETLRL SCTVSGFTIDDSEMGWYRQAPGHECELVASGSSDDDTYYVDSVKGRFTISLD NAKNMVYLQMNSLKPEDTAVYYCATGPTYPPKDGDCAHWGQGTQVTVSS (SEQ ID NO:170) |
| **171** | IL12Rβ1VHH -GGGS-IL12Rβ2VHH | QVQLQESGGGSVQVGGSLRLSCAASGFTYSSSCLGWFRQAPGKEREGVATIY PAGGNIFYADSVKGRFTISQDNAKNTVYLQMDSLKPEDTAMYYCAARGGQT WGSGGNRCSLWLPAYNYWGQGTQVTVSSGGGSQVQLQESGGGSVQAGGS LRLSCTASGYTYSSAFMAWFRQAPGKEREGVAAIYTRDGGTVYADSVKGRF TISQDNAKNTLYLQMNSLKAEDTAMYYCAAKIPQPGRASLLDSQTYDYWGQ GTQVTVSS (SEQ ID NO:171) |
| **172** | IL12Rβ1VHH -GGGS-IL12Rβ2VHH | QVQLQESGGGSVQVGGSLRLSCAASGFTYSSSCLGWFRQAPGKEREGVATIY PAGGNIFYADSVKGRFTISQDNAKNTVYLQMDSLKPEDTAMYYCAARGGQT WGSGGNRCSLWLPAYNYWGQGTQVTVSSGGGSQVQLQESGGGSVQAGGS LRLSCAVSGYDYCGYDVRWYRQAPGKEREFVSGIDSDGSTSYADSVKGRFTI SQDNAENTSYLHMFSLKPEDTAMYYCKTESPAGESAWCRNFRGMDYWGKG TQVTVSS (SEQ ID NO:172) |
| **173** | IL12Rβ1VHH -GGGS-IL12Rβ2VHH | QVQLQESGGGSVQVGGSLRLSCAASGFTYSSSCLGWFRQAPGKEREGVATIY PAGGNIFYADSVKGRFTISQDNAKNTVYLQMDSLKPEDTAMYYCAARGGQT WGSGGNRCSLWLPAYNYWGQGTQVTVSSGGGSQVQLQESGGGSVQAGGS LRLSCVASGYSYCGYDMMWYRQAPGKEREFVALITSDYSIRYEDSVEGRFSI SRDNAKNTGYLLMSNLTPADTAIYYCKTSTAARESSWCRSRYRVASWGQGT QVTVSS (SEQ ID NO:173) |
| **174** | IL12Rβ1VHH -GGGS-IL12Rβ2VHH | QVQLQESGGGSVQVGGSLRLSCAASGFTYSSSCLGWFRQAPGKEREGVATIY PAGGNIFYADSVKGRFTISQDNAKNTVYLQMDSLKPEDTAMYYCAARGGQT WGSGGNRCSLWLPAYNYWGQGTQVTVSSGGGSQVQLQESGGGSVQAGGS LRLSCAASRYTYTNNFMAWFRQAPGKEREGVAAIYTGDGYAYYFDSVKGRF TISQDNDKNMLYLQMNSLKPEDTAMYYCAAMERRSGRRRMTENAEYKYW GQGTQVTVSS (SEQ ID NO:174) |
| **175** | IL12Rβ1VHH -GGGS-IL12Rβ2VHH | QVQLQESGGGSVQVGGSLRLSCAASGFTYSSSCLGWFRQAPGKEREGVATIY PAGGNIFYADSVKGRFTISQDNAKNTVYLQMDSLKPEDTAMYYCAARGGQT WGSGGNRCSLWLPAYNYWGQGTQVTVSSGGGSQVQLQESGGGSVQAGET LRLSCTVSGFTIDDSEMGWYRQAPGHECELVASGSSDDDTYYVDSVKGRFTI SLDNAKNMVYLQMNSLKPEDTAVYYCATGPTYPPKDGDCAHWGQGTQVT VSS (SEQ ID NO:175) |
| **176** | IL12Rβ1VHH -GGGS-IL12Rβ2VHH | QVQLQESGGGSVQVGGSLRLSCAVSGKLYGGAWFRQAQGKGREGVAAIWI GTGTTFYADSVKGRFTISRDNAKNTVYLQMDGLKPEDTALYYCAADDRPGY RDPLAPVSYNHWGQGTQVTVSSGGGSQVQLQESGGGSVQAGGSLRLSCTAS GYTYSSAFMAWFRQAPGKEREGVAAIYTRDGGTVYADSVKGRFTISQDNAK NTLYLQMNSLKAEDTAMYYCAAKIPQPGRASLLDSQTYDYWGQGTQVTVS S (SEQ ID NO:176) |
| **177** | IL12Rβ1VHH -GGGS-IL12Rβ2VHH | QVQLQESGGGSVQVGGSLRLSCAVSGKLYGGAWFRQAQGKGREGVAAIWI GTGTTFYADSVKGRFTISRDNAKNTVYLQMDGLKPEDTALYYCAADDRPGY RDPLAPVSYNHWGQGTQVTVSSGGGSQVQLQESGGGSVQAGGSLRLSCAVS GYDYCGYDVRWYRQAPGKEREFVSGIDSDGSTSYADSVKGRFTISQDNAEN TSYLHMFSLKPEDTAMYYCKTESPAGESAWCRNFRGMDYWGKGTQVTVSS (SEQ ID NO:177) |
| **178** | IL12Rβ1VHH -GGGS-IL12Rβ2VHH | QVQLQESGGGSVQVGGSLRLSCAVSGKLYGGAWFRQAQGKGREGVAAIWI GTGTTFYADSVKGRFTISRDNAKNTVYLQMDGLKPEDTALYYCAADDRPGY RDPLAPVSYNHWGQGTQVTVSSGGGSQVQLQESGGGSVQAGGSLRLSCVAS GYSYCGYDMMWYRQAPGKEREFVALITSDYSIRYEDSVEGRFSISRDNAKNT GYLLMSNLTPADTAIYYCKTSTAARESSWCRSRYRVASWGQGTQVTVSS (SEQ ID NO:178) |
| **179** | IL12Rβ1VHH -GGGS-IL12Rβ2VHH | QVQLQESGGGSVQVGGSLRLSCAVSGKLYGGAWFRQAQGKREGVAAIWI GTGTTFYADSVKGRFTISRDNAKNTVYLQMDGLKPEDTALYYCAADDRPGY RDPLAPVSYNHWGQGTQVTVSSGGGSQVQLQESGGGSVQAGGSLRLSCAAS RYTYTNNFMAWFRQAPGKEREGVAAIYTGDGYAYYFDSVKGRFTISQDNDK NMLYLQMNSLKPEDTAMYYCAAMERRSGRRRMTENAEYKYWGQGTQVTV SS (SEQ ID NO:179) |
| **180** | IL12Rβ1VHH -GGGS-IL12Rβ2VHH | QVQLQESGGGSVQVGGSLRLSCAVSGKLYGGAWFRQAQGKGREGVAAIWI GTGTTFYADSVKGRFTISRDNAKNTVYLQMDGLKPEDTALYYCAADDRPGY RDPLAPVSYNHWGQGTQVTVSSGGGSQVQLQESGGGSVQAGETLRLSCTVS GFTIDDSEMGWYRQAPGHECELVASGSSDDDTYYVDSVKGRFTISLDNAKN MVYLQMNSLKPEDTAVYYCATGPTYPPKDGDCAHWGQGTQVTVSS (SEQ ID NO:180) |
| **181** | IL12Rβ1VHH -GGGS-IL12Rβ2VHH | QVQLQESGGGSVQAGGSLRLSCAASGITYRGVWMGWFRQAPGKEREGVATI YTGSGHTYYADSVKGRFTISQDNAKNTVYLQMNSLKPEDTAMYYCAARTV GGTFYTLAADSFNTWGQGTQVTVSSGGGSQVQLQESGGGSVQAGGSLRLSC TASGYTYSSAFMAWFRQAPGKEREGVAAIYTRDGGTVYADSVKGRFTISQD NAKNTLYLQMNSLKAEDTAMYYCAAKIPQPGRASLLDSQTYDYWGQGTQV TVSS (SEQ ID NO:181) |
| **182** | IL12Rβ1VHH -GGGS-IL12Rβ2VHH | QVQLQESGGGSVQAGGSLRLSCAASGITYRGVWMGWFRQAPGKEREGVATI YTGSGHTYYADSVKGRFTISQDNAKNTVYLQMNSLKPEDTAMYYCAARTV GGTFYTLAADSFNTWGQGTQVTVSSGGGSQVQLQESGGGSVQAGGSLRLSC AVSGYDYCGYDVRWYRQAPGKEREFVSGIDSDGSTSYADSVKGRFTISQDN AENTSYLHMFSLKPEDTAMYYCKTESPAGESAWCRNFRGMDYWGKGTQVT VSS (SEQ ID NO:182) |
| **183** | IL12Rβ1VHH -GGGS-IL12Rβ2VHH | QVQLQESGGGSVQAGGSLRLSCAASGITYRGVWMGWFRQAPGKEREGVATI YTGSGHTYYADSVKGRFTISQDNAKNTVYLQMNSLKPEDTAMYYCAARTV GGTFYTLAADSFNTWGQGTQVTVSSGGGSQVQLQESGGGSVQAGGSLRLSC VASGYSYCGYDMMWYRQAPGKEREFVALITSDYSIRYEDSVEGRFSISRDNA KNTGYLLMSNLTPADTAIYYCKTSTAARESSWCRSRYRVASWGQGTQVTVS S (SEQ ID NO:183) |
| **184** | IL12Rβ1VHH -GGGS-IL12Rβ2VHH | QVQLQESGGGSVQAGGSLRLSCAASGITYRGVWMGWFRQAPGKEREGVATI YTGSGHTYYADSVKGRFTISQDNAKNTVYLQMNSLKPEDTAMYYCAARTV GGTFYTLAADSFNTWGQGTQVTVSSGGGSQVQLQESGGGSVQAGGSLRLSC AASRYTYTNNFMAWFRQAPGKEREGVAAIYTGDGYAYYFDSVKGRFTISQD NDKNMLYLQMNSLKPEDTAMYYCAAMERRSGRRRMTENAEYKYWGQGT QVTVSS (SEQ ID NO:184) |
| **185** | IL12Rβ1VHH -GGGS-IL12Rβ2VHH | QVQLQESGGGSVQAGGSLRLSCAASGITYRGVWMGWFRQAPGKEREGVATI YTGSGHTYYADSVKGRFTISQDNAKNTVYLQMNSLKPEDTAMYYCAARTV GGTFYTLAADSFNTWGQGTQVTVSSGGGSQVQLQESGGGSVQAGETLRLSC TVSGFTIDDSEMGWYRQAPGHECELVASGSSDDDTYYVDSVKGRFTISLDNA KNMVYLQMNSLKPEDTAVYYCATGPTYPPKDGDCAHWGQGTQVTVSS (SEQ ID NO:185) |
| **186** | IL12Rβ1VHH -GGGS-IL12Rβ2VHH | QVQLQESGGGSVQDGGSLRLSCAASGDIYARNCMGWFRQAPGKEREKIAVA DTGGRSPYYADSVKGRFTISRDNAKNTVDLQMNSLKPEDTAVYYCAAGPLV PVVNTAARCVYEYWGQGTQVTVSSGGGSQVQLQESGGGSVQAGGSLRLSC TASGYTYSSAFMAWFRQAPGKEREGVAAIYTRDGGTVYADSVKGRFTISQD NAKNTLYLQMNSLKAEDTAMYYCAAKIPQPGRASLLDSQTYDYWGQGTQV TVSS (SEQ ID NO:186) |
| **187** | IL12Rβ1VHH -GGGS-IL12Rβ2VHH | QVQLQESGGGSVQDGGSLRLSCAASGDIYARNCMGWFRQAPGKEREKIAVA DTGGRSPYYADSVKGRFTISRDNAKNTVDLQMNSLKPEDTAVYYCAAGPLV PVVNTAARCVYEYWGQGTQVTVSSGGGSQVQLQESGGGSVQAGGSLRLSC AVSGYDYCGYDVRWYRQAPGKEREFVSGIDSDGSTSYADSVKGRFTISQDN AENTSYLHMFSLKPEDTAMYYCKTESPAGESAWCRNFRGMDYWGKGTQVT VSS (SEQ ID NO:187) |
| **188** | IL12Rβ1VHH -GGGS-IL12Rβ2VHH | QVQLQESGGGSVQDGGSLRLSCAASGDIYARNCMGWFRQAPGKEREKIAVA DTGGRSPYYADSVKGRFTISRDNAKNTVDLQMNSLKPEDTAVYYCAAGPLV PVVNTAARCVYEYWGQGTQVTVSSGGGSQVQLQESGGGSVQAGGSLRLSC VASGYSYCGYDMMWYRQAPGKEREFVALITSDYSIRYEDSVEGRFSISRDNA KNTGYLLMSNLTPADTAIYYCKTSTAARESSWCRSRYRVASWGQGTQVTVS S (SEQ ID NO:188) |
| **189** | IL12Rβ1VHH -GGGS-IL12Rβ2VHH | QVQLQESGGGSVQDGGSLRLSCAASGDIYARNCMGWFRQAPGKEREKIAVA DTGGRSPYYADSVKGRFTISRDNAKNTVDLQMNSLKPEDTAVYYCAAGPLV PVVNTAARCVYEYWGQGTQVTVSSGGGSQVQLQESGGGSVQAGGSLRLSC AASRYTYTNNFMAWFRQAPGKEREGVAAIYTGDGYAYYFDSVKGRFTISQD NDKNMLYLQMNSLKPEDTAMYYCAAMERRSGRRRMTENAEYKYWGQGT QVTVSS (SEQ ID NO:189) |
| **190** | IL12Rβ1VHH -GGGS-IL12Rβ2VHH | QVQLQESGGGSVQDGGSLRLSCAASGDIYARNCMGWFRQAPGKEREKIAVA DTGGRSPYYADSVKGRFTISRDNAKNTVDLQMNSLKPEDTAVYYCAAGPLV PVVNTAARCVYEYWGQGTQVTVSSGGGSQVQLQESGGGSVQAGETLRLSC TVSGFTIDDSEMGWYRQAPGHECELVASGSSDDDTYYVDSVKGRFTISLDNA KNMVYLQMNSLKPEDTAVYYCATGPTYPPKDGDCAHWGQGTQVTVSS (SEQ ID NO:190) |
| **191** | linker | GSGSGSGS |
| **192** | IL10RαVHH-GGGS-IL10RβVHH-ASH6 | QVQLQESGGGSVQAGGSLRLSCAASRYLYSIDYMAWFRQSPGKEREPVAVIY TASGATFYPDSVKGRFTISQDNAKMTVYLQMNSLKSEDTAMYYCAAVRKTD SYLFDAQSFTYWGQGTQVTVSSGGGSQVQLQESGGGSVQAGGSLRLSCAASG YTYSSGCMGWFRQAPGKEREAVAAINSDGSTSYADSVKGRFTISKDNAKNTL YLQMNSLKPEDTAMYYCAAEPYCSGGYPRWSVAEFGYWGQGTQVTVSSASH HHHHH |
| **193** | IL10RαVHH-GGGS-IL10RβVHH-ASH6 | QVQLQESGGGSVQAGGSLRLSCAASRYLYSIDYMAWFRQSPGKEREPVAVIY TASGATFYPDSVKGRFTISQDNAKMTVYLQMNSLKSEDTAMYYCAAVRKTD SYLFDAQSFTYWGQGTQVTVSSGGGSQVQLQESGGGSVQAGGSLRLSCAASG YTYSSYCMGWFRQAPGKEREGVAHIDSDGSTSYADSVKGRFTISKDNAKNTL YLQMNSLKPEDTAMYYCAADPIPGPGYCDGGPNKYWGQGTQVTVSSASHHH HHH |
| **194** | IL10RαVHH-GGGS-IL10RβVHH-ASH6 | QVQLQESGGGSVQAGGSLRLSCAASRYLYSIDYMAWFRQSPGKEREPVAVIY TASGATFYPDSVKGRFTISQDNAKMTVYLQMNSLKSEDTAMYYCAAVRKTD SYLFDAQSFTYWGQGTQVTVSSGGGSQVQLQESGGGSVQAGGSLRLSCAASR YTYNSYCMGWFRQAPGKEREGVATIDSDGMTRYADSVKGRFTISKDNAKNT LYLQMNSLKPEDTAMYYCAADADCTIAAMTTNPLGQGTQVTVSSASHHHHH H |
| **195** | IL10RαVHH-GGGS-IL10RβVHH-ASH6 | QVQLQESGGGSVQAGGSLRLSCAASRYLYSIDYMAWFRQSPGKEREPVAVIY TASGATFYPDSVKGRFTISQDNAKMTVYLQMNSLKSEDTAMYYCAAVRKTD SYLFDAQSFTYWGQGTQVTVSSGGGSQVQLQESGGGSVQAGGSLRLSCTVSR YTASVNYMGWFRQAPGKEREGVATIFTGAGTTYYANSVKGRFTISRDNAKNT AYLQMNSLKPEDTAIYYCAVDFRGGLLYRPAYEYTYRGQGTQVTVSSASHHH HHH |
| **196** | IL10RαVHH-GGGS-IL10RβVHH-ASH6 | QVQLQESGGGSVQAGGSLRLSCAASRYLYSIDYMAWFRQSPGKEREPVAVIY TASGATFYPDSVKGRFTISQDNAKMTVYLQMNSLKSEDTAMYYCAAVRKTD SYLFDAQSFTYWGQGTQVTVSSGGGSQVQLQESGGGSVEAGGSLRLSCAASG YTHSSYCMGWFRQAPGKEREGVAAIDVDGSTTYADSVKGRFTISKDNAKNTL YLQMNSLKPEDTGMYYCAAEFADCSSNYFLPPGAVRYWGQGTQVTVSSASH HHHHH |
| **197** | IL10RαVHH-GGGS-IL10RβVHH-ASH6 | QVQLQESGGGSVQAGGSLRLSCAASRYLYSIDYMAWFRQSPGKEREPVAVIY TASGATFYPDSVKGRFTISQDNAKMTVYLQMNSLKSEDTAMYYCAAVRKTD SYLFDAQSFTYWGQGTQVTVSSGGGSQVQLQESGGGSVQAGGSLRLSCAASG YSYSSYCMGWFRQAPGKEREGVATIDSDGMTRYADSVKGRFTISKDNAKNTL YLQMNSLKPEDTAMYYCAAPLYDCDSGAVGRNPPYWGQGTQVTVSSASHH HHHH |
| **198** | IL10RαVHH-GGGS-IL10RβVHH-ASH6 | QVQLQESGGGSVQAGGSLRLSCAASRYLYSIDYMAWFRQSPGKEREPVAVIY TASGATFYPDSVKGRFTISQDNAKMTVYLQMNSLKSEDTAMYYCAAVRKTD SYLFDAQSFTYWGQGTQVTVSSGGGSQVQLQESGGGSVQTGGSLRLSCAASG YTYLRGCMGWFRQAPGKEREGVAVMDVVGDRRSYIDSVKGRFTISRDNAAN SVYLQMDNLKPEDTAMYYCTAGPNCVGWRSGLDYWGQGTQVTVSSASHHH HHH |
| **199** | IL10RαVHH-GGGS-IL10RβVHH-ASH6 | QVQLQESGGGSVQAGGSLRLSCAASRFTYSSYCMGWFRQAPGKEREGVASID SDGSTSYTDSVKGRFTISKDNAKNTLYLQMNSLKPEDTAMYYCALDLMSTVV PGFCGFLLSAGMDYWGKGTQVTVSSGGGSQVQLQESGGGSVQAGGSLRLSC AASGYTYSSGCMGWFRQAPGKEREAVAAINSDGSTSYADSVKGRFTISKDNA KNTLYLQMNSLKPEDTAMYYCAAEPYCSGGYPRWSVAEFGYWGQGTQVTV SSASHHHHHH |
| **200** | IL10RαVHH-GGGS-IL10RβVHH-ASH6 | QVQLQESGGGSVQAGGSLRLSCAASRFTYSSYCMGWFRQAPGKEREGVASID SDGSTSYTDSVKGRFTISKDNAKNTLYLQMNSLKPEDTAMYYCALDLMSTVV PGFCGFLLSAGMDYWGKGTQVTVSSGGGSQVQLQESGGGSVQAGGSLRLSC AASGYTYSSYCMGWFRQAPGKEREGVAHIDSDGSTSYADSVKGRFTISKDNA KNTLYLQMNSLKPEDTAMYYCAADPIPGPGYCDGGPNKYWGQGTQVTVSSA SHHHHHH |
| **201** | IL10RαVHH-GGGS-IL10RβVHH-ASH6 | QVQLQESGGGSVQAGGSLRLSCAASRFTYSSYCMGWFRQAPGKEREGVASID SDGSTSYTDSVKGRFTISKDNAKNTLYLQMNSLKPEDTAMYYCALDLMSTVV PGFCGFLLSAGMDYWGKGTQVTVSSGGGSQVQLQESGGGSVQAGGSLRLSC AASRYTYNSYCMGWFRQAPGKEREGVATIDSDGMTRYADSVKGRFTISKDN AKNTLYLQMNSLKPEDTAMYYCAADADCTIAAMTTNPLGQGTQVTVSSASH HHHHH |
| **202** | IL10RαVHH-GGGS-IL10RβVHH-ASH6 | QVQLQESGGGSVQAGGSLRLSCAASRFTYSSYCMGWFRQAPGKEREGVASID SDGSTSYTDSVKGRFTISKDNAKNTLYLQMNSLKPEDTAMYYCALDLMSTVV PGFCGFLLSAGMDYWGKGTQVTVSSGGGSQVQLQESGGGSVQAGGSLRLSC TVSRYTASVNYMGWFRQAPGKEREGVATIFTGAGTTYYANSVKGRFTISRDN AKNTAYLQMNSLKPEDTAIYYCAVDFRGGLLYRPAYEYTYRGQGTQVTVSSA SHHHHHH |
| **203** | IL10RαVHH-GGGS-IL10RβVHH-ASH6 | QVQLQESGGGSVQAGGSLRLSCAASRFTYSSYCMGWFRQAPGKEREGVASID SDGSTSYTDSVKGRFTISKDNAKNTLYLQMNSLKPEDTAMYYCALDLMSTVV PGFCGFLLSAGMDYWGKGTQVTVSSGGGSQVQLQESGGGSVEAGGSLRLSC AASGYTHSSYCMGWFRQAPGKEREGVAAIDVDGSTTYADSVKGRFTISKDNA KNTLYLQMNSLKPEDTGMYYCAAEFADCSSNYFLPPGAVRYWGQGTQVTVS SASHHHHHH |
| **204** | IL10RαVHH-GGGS-IL10RβVHH-ASH6 | QVQLQESGGGSVQAGGSLRLSCAASRFTYSSYCMGWFRQAPGKEREGVASID SDGSTSYTDSVKGRFTISKDNAKNTLYLQMNSLKPEDTAMYYCALDLMSTVV PGFCGFLLSAGMDYWGKGTQVTVSSGGGSQVQLQESGGGSVQAGGSLRLSC AASGYSYSSYCMGWFRQAPGKEREGVATIDSDGMTRYADSVKGRFTISKDNA KNTLYLQMNSLKPEDTAMYYCAAPLYDCDSGAVGRNPPYWGQGTQVTVSSA SHHHHHH |
| **205** | IL10RαVHH-GGGS-IL10RβVHH-ASH6 | QVQLQESGGGSVQAGGSLRLSCAASRFTYSSYCMGWFRQAPGKEREGVASID SDGSTSYTDSVKGRFTISKDNAKNTLYLQMNSLKPEDTAMYYCALDLMSTVV PGFCGFLLSAGMDYWGKGTQVTVSSGGGSQVQLQESGGGSVQTGGSLRLSC AASGYTYLRGCMGWFRQAPGKEREGVAVMDVVGDRRSYIDSVKGRFTISRD NAANSVYLQMDNLKPEDTAMYYCTAGPNCVGWRSGLDYWGQGTQVTVSSA SHHHHHH |
| **206** | IL10RαVHH-GGGS-IL10RβVHH-ASH6 | QVQLQESGGGSVQAGGSLRLSCAASGYTYSMYCMGWFRQAPGKEREGVAQI NSDGSTSYADSVKGRFTISKDNAKNTLYLQMNSLKPEDTAMYYCAADSRVY GGSWYERLCGPYTYEYNYWGQGTQVTVSSGGGSQVQLQESGGGSVQAGGSL RLSCAASGYTYSSGCMGWFRQAPGKEREAVAAINSDGSTSYADSVKGRFTISK DNAKNTLYLQMNSLKPEDTAMYYCAAEPYCSGGYPRWSVAEFGYWGQGTQ VTVSSASHHHHHH |
| **207** | IL10RαVHH-GGGS-IL10RβVHH-ASH6 | QVQLQESGGGSVQAGGSLRLSCAASGYTYSMYCMGWFRQAPGKEREGVAQI NSDGSTSYADSVKGRFTISKDNAKNTLYLQMNSLKPEDTAMYYCAADSRVY GGSWYERLCGPYTYEYNYWGQGTQVTVSSGGGSQVQLQESGGGSVQAGGSL RLSCAASGYTYSSYCMGWFRQAPGKEREGVAHIDSDGSTSYADSVKGRFTISK DNAKNTLYLQMNSLKPEDTAMYYCAADPIPGPGYCDGGPNKYWGQGTQVT VSSASHHHHHH |
| **208** | IL10RαVHH-GGGS-IL10RβVHH-ASH6 | QVQLQESGGGSVQAGGSLRLSCAASGYTYSMYCMGWFRQAPGKEREGVAQI NSDGSTSYADSVKGRFTISKDNAKNTLYLQMNSLKPEDTAMYYCAADSRVY GGSWYERLCGPYTYEYNYWGQGTQVTVSSGGGSQVQLQESGGGSVQAGGSL RLSCAASRYTYNSYCMGWFRQAPGKEREGVATIDSDGMTRYADSVKGRFTIS KDNAKNTLYLQMNSLKPEDTAMYYCAADADCTIAAMTTNPLGQGTQVTVSS ASHHHHHH |
| **209** | IL10RαVHH-GGGS-IL10RβVHH-ASH6 | QVQLQESGGGSVQAGGSLRLSCAASGYTYSMYCMGWFRQAPGKEREGVAQI NSDGSTSYADSVKGRFTISKDNAKNTLYLQMNSLKPEDTAMYYCAADSRVY GGSWYERLCGPYTYEYNYWGQGTQVTVSSGGGSQVQLQESGGGSVQAGGSL RLSCTVSRYTASVNYMGWFRQAPGKEREGVATIFTGAGTTYYANSVKGRFTI SRDNAKNTAYLQMNSLKPEDTAIYYCAVDFRGGLLYRPAYEYTYRGQGTQV TVSSASHHHHHH |
| **210** | IL10RαVHH-GGGS-IL10RβVHH-ASH6 | QVQLQESGGGSVQAGGSLRLSCAASGYTYSMYCMGWFRQAPGKEREGVAQI NSDGSTSYADSVKGRFTISKDNAKNTLYLQMNSLKPEDTAMYYCAADSRVY GGSWYERLCGPYTYEYNYWGQGTQVTVSSGGGSQVQLQESGGGSVEAGGSL RLSCAASGYTHSSYCMGWFRQAPGKEREGVAAIDVDGSTTYADSVKGRFTIS KDNAKNTLYLQMNSLKPEDTGMYYCAAEFADCSSNYFLPPGAVRYWGQGTQ VTVSSASHHHHHH |
| **211** | IL10RαVHH-GGGS-IL10RβVHH-ASH6 | QVQLQESGGGSVQAGGSLRLSCAASGYTYSMYCMGWFRQAPGKEREGVAQI NSDGSTSYADSVKGRFTISKDNAKNTLYLQMNSLKPEDTAMYYCAADSRVY GGSWYERLCGPYTYEYNYWGQGTQVTVSSGGGSQVQLQESGGGSVQAGGSL RLSCAASGYSYSSYCMGWFRQAPGKEREGVATIDSDGMTRYADSVKGRFTIS KDNAKNTLYLQMNSLKPEDTAMYYCAAPLYDCDSGAVGRNPPYWGQGTQV TVSSASHHHHHH |
| **212** | IL10RαVHH-GGGS-IL10RβVHH-ASH6 | QVQLQESGGGSVQAGGSLRLSCAASGYTYSMYCMGWFRQAPGKEREGVAQI NSDGSTSYADSVKGRFTISKDNAKNTLYLQMNSLKPEDTAMYYCAADSRVY GGSWYERLCGPYTYEYNYWGQGTQVTVSSGGGSQVQLQESGGGSVQTGGSL RLSCAASGYTYLRGCMGWFRQAPGKEREGVAVMDVVGDRRSYIDSVKGRFT ISRDNAANSVYLQMDNLKPEDTAMYYCTAGPNCVGWRSGLDYWGQGTQVT VSSASHHHHHH |
| **213** | IL10RαVHH-GGGS-IL10RβVHH-ASH6 | QVQLQESGGGSVQAGGSLRLSCAVSGYAYSTYCMGWFRQAPGKEREGVAAI DSGGSTSYADSVKGRFTISKDNAKNTLYLRMNSLKPEDTAMYYCAAVPPPPD GGSCLFLGPEIKVSKADFRYWGQGTQVTVSSGGGSQVQLQESGGGSVQAGGS LRLSCAASGYTYSSGCMGWFRQAPGKEREAVAAINSDGSTSYADSVKGRFTIS KDNAKNTLYLQMNSLKPEDTAMYYCAAEPYCSGGYPRWSVAEFGYWGQGT QVTVSSASHHHHHH |
| **214** | IL10RαVHH-GGGS-IL10RβVHH-ASH6 | QVQLQESGGGSVQAGGSLRLSCAVSGYAYSTYCMGWFRQAPGKEREGVAAI DSGGSTSYADSVKGRFTISKDNAKNTLYLRMNSLKPEDTAMYYCAAVPPPPD GGSCLFLGPEIKVSKADFRYWGQGTQVTVSSGGGSQVQLQESGGGSVQAGGS LRLSCAASGYTYSSYCMGWFRQAPGKEREGVAHIDSDGSTSYADSVKGRFTIS KDNAKNTLYLQMNSLKPEDTAMYYCAADPIPGPGYCDGGPNKYWGQGTQV TVSSASHHHHHH |
| **215** | IL10RαVHH-GGGS-IL10RβVHH-ASH6 | QVQLQESGGGSVQAGGSLRLSCAVSGYAYSTYCMGWFRQAPGKEREGVAAI DSGGSTSYADSVKGRFTISKDNAKNTLYLRMNSLKPEDTAMYYCAAVPPPPD GGSCLFLGPEIKVSKADFRYWGQGTQVTVSSGGGSQVQLQESGGGSVQAGGS LRLSCAASRYTYNSYCMGWFRQAPGKEREGVATIDSDGMTRYADSVKGRFTI SKDNAKNTLYLQMNSLKPEDTAMYYCAADADCTIAAMTTNPLGQGTQVTVS SASHHHHHH |
| **216** | IL10RαVHH-GGGS-IL10RβVHH-ASH6 | QVQLQESGGGSVQAGGSLRLSCAVSGYAYSTYCMGWFRQAPGKEREGVAAI DSGGSTSYADSVKGRFTISKDNAKNTLYLRMNSLKPEDTAMYYCAAVPPPPD GGSCLFLGPEIKVSKADFRYWGQGTQVTVSSGGGSQVQLQESGGGSVQAGGS LRLSCTVSRYTASVNYMGWFRQAPGKEREGVATIFTGAGTTYYANSVKGRFT ISRDNAKNTAYLQMNSLKPEDTAIYYCAVDFRGGLLYRPAYEYTYRGQGTQV TVSSASHHHHHH |
| **217** | IL10RαVHH-GGGS-IL10RβVHH-ASH6 | QVQLQESGGGSVQAGGSLRLSCAVSGYAYSTYCMGWFRQAPGKEREGVAAI DSGGSTSYADSVKGRFTISKDNAKNTLYLRMNSLKPEDTAMYYCAAVPPPPD GGSCLFLGPEIKVSKADFRYWGQGTQVTVSSGGGSQVQLQESGGGSVEAGGS LRLSCAASGYTHSSYCMGWFRQAPGKEREGVAAIDVDGSTTYADSVKGRFTI SKDNAKNTLYLQMNSLKPEDTGMYYCAAEFADCSSNYFLPPGAVRYWGQGT QVTVSSASHHHHHH |
| **218** | IL10RαVHH-GGGS-IL10RβVHH-ASH6 | QVQLQESGGGSVQAGGSLRLSCAVSGYAYSTYCMGWFRQAPGKEREGVAAI DSGGSTSYADSVKGRFTISKDNAKNTLYLRMNSLKPEDTAMYYCAAVPPPPD GGSCLFLGPEIKVSKADFRYWGQGTQVTVSSGGGSQVQLQESGGGSVQAGGS LRLSCAASGYSYSSYCMGWFRQAPGKEREGVATIDSDGMTRYADSVKGRFTI SKDNAKNTLYLQMNSLKPEDTAMYYCAAPLYDCDSGAVGRNPPYWGQGTQ VTVSSASHHHHHH |
| **219** | IL10RαVHH-GGGS-IL10RβVHH-ASH6 | QVQLQESGGGSVQAGGSLRLSCAVSGYAYSTYCMGWFRQAPGKEREGVAAI DSGGSTSYADSVKGRFTISKDNAKNTLYLRMNSLKPEDTAMYYCAAVPPPPD GGSCLFLGPEIKVSKADFRYWGQGTQVTVSSGGGSQVQLQESGGGSVQTGGS LRLSCAASGYTYLRGCMGWFRQAPGKEREGVAVMDVVGDRRSYIDSVKGRF TISRDNAANSVYLQMDNLKPEDTAMYYCTAGPNCVGWRSGLDYWGQGTQV TVSSASHHHHHH |
| **220** | IL10RαVHH-GGGS-IL10RβVHH-ASH6 | QVQLQESGGGSVQAGGSLRLSCTVSGYTYSSNCMGWFRQAPGKEREGVATIY TGGGNTYYADSVKGRFTISQDNAKNTVYLQMNNLKPEDTAMYYCAAEPLSR VYGGSCPTPTFDYWGQGTQVTVSSGGGSQVQLQESGGGSVQAGGSLRLSCAA SGYTYSSGCMGWFRQAPGKEREAVAAINSDGSTSYADSVKGRFTISKDNAKN TLYLQMNSLKPEDTAMYYCAAEPYCSGGYPRWSVAEFGYWGQGTQVTVSSA SHHHHHH |
| **221** | IL10RαVHH-GGGS-IL10RβVHH-ASH6 | QVQLQESGGGSVQAGGSLRLSCTVSGYTYSSNCMGWFRQAPGKEREGVATIY TGGGNTYYADSVKGRFTISQDNAKNTVYLQMNNLKPEDTAMYYCAAEPLSR VYGGSCPTPTFDYWGQGTQVTVSSGGGSQVQLQESGGGSVQAGGSLRLSCAA SGYTYSSYCMGWFRQAPGKEREGVAHIDSDGSTSYADSVKGRFTISKDNAKN TLYLQMNSLKPEDTAMYYCAADPIPGPGYCDGGPNKYWGQGTQVTVSSASH HHHHH |
| **222** | IL10RαVHH-GGGS-IL10RβVHH-ASH6 | QVQLQESGGGSVQAGGSLRLSCTVSGYTYSSNCMGWFRQAPGKEREGVATIY TGGGNTYYADSVKGRFTISQDNAKNTVYLQMNNLKPEDTAMYYCAAEPLSR VYGGSCPTPTFDYWGQGTQVTVSSGGGSQVQLQESGGGSVQAGGSLRLSCAA SRYTYNSYCMGWFRQAPGKEREGVATIDSDGMTRYADSVKGRFTISKDNAK NTLYLQMNSLKPEDTAMYYCAADADCTIAAMTTNPLGQGTQVTVSSASHHH HHH |
| **223** | IL10RαVHH-GGGS-IL10RβVHH-ASH6 | QVQLQESGGGSVQAGGSLRLSCTVSGYTYSSNCMGWFRQAPGKEREGVATIY TGGGNTYYADSVKGRFTISQDNAKNTVYLQMNNLKPEDTAMYYCAAEPLSR VYGGSCPTPTFDYWGQGTQVTVSSGGGSQVQLQESGGGSVQAGGSLRLSCTV SRYTASVNYMGWFRQAPGKEREGVATIFTGAGTTYYANSVKGRFTISRDNAK NTAYLQMNSLKPEDTAIYYCAVDFRGGLLYRPAYEYTYRGQGTQVTVSSASH HHHHH |
| **224** | IL10RαVHH-GGGS-IL10RβVHH-ASH6 | QVQLQESGGGSVQAGGSLRLSCTVSGYTYSSNCMGWFRQAPGKEREGVATIY TGGGNTYYADSVKGRFTISQDNAKNTVYLQMNNLKPEDTAMYYCAAEPLSR VYGGSCPTPTFDYWGQGTQVTVSSGGGSQVQLQESGGGSVEAGGSLRLSCAA SGYTHSSYCMGWFRQAPGKEREGVAAIDVDGSTTYADSVKGRFTISKDNAKN TLYLQMNSLKPEDTGMYYCAAEFADCSSNYFLPPGAVRYWGQGTQVTVSSA SHHHHHH |
| **225** | IL10RαVHH-GGGS-IL10RβVHH-ASH6 | QVQLQESGGGSVQAGGSLRLSCTVSGYTYSSNCMGWFRQAPGKEREGVATIY TGGGNTYYADSVKGRFTISQDNAKNTVYLQMNNLKPEDTAMYYCAAEPLSR VYGGSCPTPTFDYWGQGTQVTVSSGGGSQVQLQESGGGSVQAGGSLRLSCAA SGYSYSSYCMGWFRQAPGKEREGVATIDSDGMTRYADSVKGRFTISKDNAKN TLYLQMNSLKPEDTAMYYCAAPLYDCDSGAVGRNPPYWGQGTQVTVSSASH HHHHH |
| **226** | IL10RαVHH-GGGS-IL10RβVHH-ASH6 | QVQLQESGGGSVQAGGSLRLSCTVSGYTYSSNCMGWFRQAPGKEREGVATIY TGGGNTYYADSVKGRFTISQDNAKNTVYLQMNNLKPEDTAMYYCAAEPLSR VYGGSCPTPTFDYWGQGTQVTVSSGGGSQVQLQESGGGSVQTGGSLRLSCAA SGYTYLRGCMGWFRQAPGKEREGVAVMDVVGDRRSYIDSVKGRFTISRDNA ANSVYLQMDNLKPEDTAMYYCTAGPNCVGWRSGLDYWGQGTQVTVSSASH HHHHH |
| **227** | IL10RαVHH-GGGS-IL10RβVHH-ASH6 | QVQLQESGGGSVQAGGSLRLSCGASGYTYSSYCMGWFRQVPGKEREGVAVI DSDGSTSYADSVKGRFTISKDNGKNTLYLQMNSLKPEDTAMYYCAADLGHY RPPCGVLYLGMDYWGKGTQVTVSSGGGSQVQLQESGGGSVQAGGSLRLSCA ASGYTYSSGCMGWFRQAPGKEREAVAAINSDGSTSYADSVKGRFTISKDNAK NTLYLQMNSLKPEDTAMYYCAAEPYCSGGYPRWSVAEFGYWGQGTQVTVSS ASHHHHHH |
| **228** | IL10RαVHH-GGGS-IL10RβVHH-ASH6 | QVQLQESGGGSVQAGGSLRLSCGASGYTYSSYCMGWFRQVPGKEREGVAVI DSDGSTSYADSVKGRFTISKDNGKNTLYLQMNSLKPEDTAMYYCAADLGHY RPPCGVLYLGMDYWGKGTQVTVSSGGGSQVQLQESGGGSVQAGGSLRLSCA ASGYTYSSYCMGWFRQAPGKEREGVAHIDSDGSTSYADSVKGRFTISKDNAK NTLYLQMNSLKPEDTAMYYCAADPIPGPGYCDGGPNKYWGQGTQVTVSSAS HHHHHH |
| **229** | IL10RαVHH-GGGS-IL10RβVHH-ASH6 | QVQLQESGGGSVQAGGSLRLSCGASGYTYSSYCMGWFRQVPGKEREGVAVI DSDGSTSYADSVKGRFTISKDNGKNTLYLQMNSLKPEDTAMYYCAADLGHY RPPCGVLYLGMDYWGKGTQVTVSSGGGSQVQLQESGGGSVQAGGSLRLSCA ASRYTYNSYCMGWFRQAPGKEREGVATIDSDGMTRYADSVKGRFTISKDNA KNTLYLQMNSLKPEDTAMYYCAADADCTIAAMTTNPLGQGTQVTVSSASHH HHHH |
| **230** | IL10RαVHH-GGGS-IL10RβVHH-ASH6 | QVQLQESGGGSVQAGGSLRLSCGASGYTYSSYCMGWFRQVPGKEREGVAVI DSDGSTSYADSVKGRFTISKDNGKNTLYLQMNSLKPEDTAMYYCAADLGHY RPPCGVLYLGMDYWGKGTQVTVSSGGGSQVQLQESGGGSVQAGGSLRLSCT VSRYTASVNYMGWFRQAPGKEREGVATIFTGAGTTYYANSVKGRFTISRDNA KNTAYLQMNSLKPEDTAIYYCAVDFRGGLLYRPAYEYTYRGQGTQVTVSSAS HHHHHH |
| **231** | IL10RαVHH-GGGS-IL10RβVHH-ASH6 | QVQLQESGGGSVQAGGSLRLSCGASGYTYSSYCMGWFRQVPGKEREGVAVI DSDGSTSYADSVKGRFTISKDNGKNTLYLQMNSLKPEDTAMYYCAADLGHY RPPCGVLYLGMDYWGKGTQVTVSSGGGSQVQLQESGGGSVEAGGSLRLSCA ASGYTHSSYCMGWFRQAPGKEREGVAAIDVDGSTTYADSVKGRFTISKDNAK NTLYLQMNSLKPEDTGMYYCAAEFADCSSNYFLPPGAVRYWGQGTQVTVSS ASHHHHHH |
| **232** | IL10RαVHH-GGGS-IL10RβVHH-ASH6 | QVQLQESGGGSVQAGGSLRLSCGASGYTYSSYCMGWFRQVPGKEREGVAVI DSDGSTSYADSVKGRFTISKDNGKNTLYLQMNSLKPEDTAMYYCAADLGHY RPPCGVLYLGMDYWGKGTQVTVSSGGGSQVQLQESGGGSVQAGGSLRLSCA ASGYSYSSYCMGWFRQAPGKEREGVATIDSDGMTRYADSVKGRFTISKDNAK NTLYLQMNSLKPEDTAMYYCAAPLYDCDSGAVGRNPPYWGQGTQVTVSSAS HHHHHH |
| **233** | IL10RαVHH-GGGS-IL10RβVHH-ASH6 | QVQLQESGGGSVQAGGSLRLSCGASGYTYSSYCMGWFRQVPGKEREGVAVI DSDGSTSYADSVKGRFTISKDNGKNTLYLQMNSLKPEDTAMYYCAADLGHY RPPCGVLYLGMDYWGKGTQVTVSSGGGSQVQLQESGGGSVQTGGSLRLSCA ASGYTYLRGCMGWFRQAPGKEREGVAVMDVVGDRRSYIDSVKGRFTISRDN AANSVYLQMDNLKPEDTAMYYCTAGPNCVGWRSGLDYWGQGTQVTVSSAS HHHHHH |
| **234** | IL10RαVHH-GGGS-IL10RβVHH-ASH6 | QVQLQESGGGSVQAGGSLRLSCAASGYSNCSYDMTWYRQAPGKEREFVSAIH SDGSTRYADSVKGRFFISQDNAKNTVYLQMNSLKPEDTAMYYCKTDPLHCRA HGGSWYSVRANYWGQGTQVTVSSGGGSQVQLQESGGGSVQAGGSLRLSCA ASGYTYSSGCMGWFRQAPGKEREAVAAINSDGSTSYADSVKGRFTISKDNAK NTLYLQMNSLKPEDTAMYYCAAEPYCSGGYPRWSVAEFGYWGQGTQVTVSS ASHHHHHH |
| **235** | IL10RαVHH-GGGS-IL10RβVHH-ASH6 | QVQLQESGGGSVQAGGSLRLSCAASGYSNCSYDMTWYRQAPGKEREFVSAIH SDGSTRYADSVKGRFFISQDNAKNTVYLQMNSLKPEDTAMYYCKTDPLHCRA HGGSWYSVRANYWGQGTQVTVSSGGGSQVQLQESGGGSVQAGGSLRLSCA ASGYTYSSYCMGWFRQAPGKEREGVAHIDSDGSTSYADSVKGRFTISKDNAK NTLYLQMNSLKPEDTAMYYCAADPIPGPGYCDGGPNKYWGQGTQVTVSSAS HHHHHH |
| **236** | IL10RαVHH-GGGS-IL10RβVHH-ASH6 | QVQLQESGGGSVQAGGSLRLSCAASGYSNCSYDMTWYRQAPGKEREFVSAIH SDGSTRYADSVKGRFFISQDNAKNTVYLQMNSLKPEDTAMYYCKTDPLHCRA HGGSWYSVRANYWGQGTQVTVSSGGGSQVQLQESGGGSVQAGGSLRLSCA ASRYTYNSYCMGWFRQAPGKEREGVATIDSDGMTRYADSVKGRFTISKDNA KNTLYLQMNSLKPEDTAMYYCAADADCTIAAMTTNPLGQGTQVTVSSASHH HHHH |
| **237** | IL10RαVHH-GGGS-IL10RβVHH-ASH6 | QVQLQESGGGSVQAGGSLRLSCAASGYSNCSYDMTWYRQAPGKEREFVSAIH SDGSTRYADSVKGRFFISQDNAKNTVYLQMNSLKPEDTAMYYCKTDPLHCRA HGGSWYSVRANYWGQGTQVTVSSGGGSQVQLQESGGGSVQAGGSLRLSCTV SRYTASVNYMGWFRQAPGKEREGVATIFTGAGTTYYANSVKGRFTISRDNAK NTAYLQMNSLKPEDTAIYYCAVDFRGGLLYRPAYEYTYRGQGTQVTVSSASH HHHHH |
| **238** | IL10RαVHH-GGGS-IL10RβVHH-ASH6 | QVQLQESGGGSVQAGGSLRLSCAASGYSNCSYDMTWYRQAPGKEREFVSAIH SDGSTRYADSVKGRFFISQDNAKNTVYLQMNSLKPEDTAMYYCKTDPLHCRA HGGSWYSVRANYWGQGTQVTVSSGGGSQVQLQESGGGSVEAGGSLRLSCAA SGYTHSSYCMGWFRQAPGKEREGVAAIDVDGSTTYADSVKGRFTISKDNAKN TLYLQMNSLKPEDTGMYYCAAEFADCSSNYFLPPGAVRYWGQGTQVTVSSA SHHHHHH |
| **239** | IL10RαVHH-GGGS-IL10RβVHH-ASH6 | QVQLQESGGGSVQAGGSLRLSCAASGYSNCSYDMTWYRQAPGKEREFVSAIH SDGSTRYADSVKGRFFISQDNAKNTVYLQMNSLKPEDTAMYYCKTDPLHCRA HGGSWYSVRANYWGQGTQVTVSSGGGSQVQLQESGGGSVQAGGSLRLSCA ASGYSYSSYCMGWFRQAPGKEREGVATIDSDGMTRYADSVKGRFTISKDNAK NTLYLQMNSLKPEDTAMYYCAAPLYDCDSGAVGRNPPYWGQGTQVTVSSAS HHHHHH |
| **240** | IL10RαVHH-GGGS-IL10RβVHH-ASH6 | QVQLQESGGGSVQAGGSLRLSCAASGYSNCSYDMTWYRQAPGKEREFVSAIH SDGSTRYADSVKGRFFISQDNAKNTVYLQMNSLKPEDTAMYYCKTDPLHCRA HGGSWYSVRANYWGQGTQVTVSSGGGSQVQLQESGGGSVQTGGSLRLSCAA SGYTYLRGCMGWFRQAPGKEREGVAVMDVVGDRRSYIDSVKGRFTISRDNA ANSVYLQMDNLKPEDTAMYYCTAGPNCVGWRSGLDYWGQGTQVTVSSASH HHHHH |
| **241** | IL10RβVHH-GGGS-IL10RαVHH-ASH6 | QVQLQESGGGSVQAGGSLRLSCAASGYTYSSGCMGWFRQAPGKEREAVAAI NSDGSTSYADSVKGRFTISKDNAKNTLYLQMNSLKPEDTAMYYCAAEPYCSG GYPRWSVAEFGYWGQGTQVTVSSGGGSQVQLQESGGGSVQAGGSLRLSCAA SRYLYSIDYMAWFRQSPGKEREPVAVIYTASGATFYPDSVKGRFTISQDNAKM TVYLQMNSLKSEDTAMYYCAAVRKTDSYLFDAQSFTYWGQGTQVTVSSASH HHHHH |
| **242** | IL10RβVHH-GGGS-IL10RαVHH-ASH6 | QVQLQESGGGSVQAGGSLRLSCAASGYTYSSGCMGWFRQAPGKEREAVAAI NSDGSTSYADSVKGRFTISKDNAKNTLYLQMNSLKPEDTAMYYCAAEPYCSG GYPRWSVAEFGYWGQGTQVTVSSGGGSQVQLQESGGGSVQAGGSLRLSCAA SRFTYSSYCMGWFRQAPGKEREGVASIDSDGSTSYTDSVKGRFTISKDNAKNT LYLQMNSLKPEDTAMYYCALDLMSTVVPGFCGFLLSAGMDYWGKGTQVTV SSASHHHHHH |
| **243** | IL10RβVHH-GGGS-IL10RαVHH-ASH6 | QVQLQESGGGSVQAGGSLRLSCAASGYTYSSGCMGWFRQAPGKEREAVAAI NSDGSTSYADSVKGRFTISKDNAKNTLYLQMNSLKPEDTAMYYCAAEPYCSG GYPRWSVAEFGYWGQGTQVTVSSGGGSQVQLQESGGGSVQAGGSLRLSCAA SGYTYSMYCMGWFRQAPGKEREGVAQINSDGSTSYADSVKGRFTISKDNAKN TLYLQMNSLKPEDTAMYYCAADSRVYGGSWYERLCGPYTYEYNYWGQGTQ VTVSSASHHHHHH |
| **244** | IL10RβVHH-GGGS-IL10RαVHH-ASH6 | QVQLQESGGGSVQAGGSLRLSCAASGYTYSSGCMGWFRQAPGKEREAVAAI NSDGSTSYADSVKGRFTISKDNAKNTLYLQMNSLKPEDTAMYYCAAEPYCSG GYPRWSVAEFGYWGQGTQVTVSSGGGSQVQLQESGGGSVQAGGSLRLSCAV SGYAYSTYCMGWFRQAPGKEREGVAAIDSGGSTSYADSVKGRFTISKDNAKN TLYLRMNSLKPEDTAMYYCAAVPPPPDGGSCLFLGPEIKVSKADFRYWGQGT QVTVSSASHHHHHH |
| **245** | IL10RβVHH-GGGS-IL10RαVHH-ASH6 | QVQLQESGGGSVQAGGSLRLSCAASGYTYSSGCMGWFRQAPGKEREAVAAI NSDGSTSYADSVKGRFTISKDNAKNTLYLQMNSLKPEDTAMYYCAAEPYCSG GYPRWSVAEFGYWGQGTQVTVSSGGGSQVQLQESGGGSVQAGGSLRLSCTV SGYTYSSNCMGWFRQAPGKEREGVATIYTGGGNTYYADSVKGRFTISQDNAK NTVYLQMNNLKPEDTAMYYCAAEPLSRVYGGSCPTPTFDYWGQGTQVTVSS ASHHHHHH |
| **246** | IL10RβVHH-GGGS-IL10RαVHH-ASH6 | QVQLQESGGGSVQAGGSLRLSCAASGYTYSSGCMGWFRQAPGKEREAVAAI NSDGSTSYADSVKGRFTISKDNAKNTLYLQMNSLKPEDTAMYYCAAEPYCSG GYPRWSVAEFGYWGQGTQVTVSSGGGSQVQLQESGGGSVQAGGSLRLSCGA SGYTYSSYCMGWFRQVPGKEREGVAVIDSDGSTSYADSVKGRFTISKDNGKN TLYLQMNSLKPEDTAMYYCAADLGHYRPPCGVLYLGMDYWGKGTQVTVSS ASHHHHHH |
| **247** | IL10RβVHH-GGGS-IL10RαVHH-ASH6 | QVQLQESGGGSVQAGGSLRLSCAASGYTYSSGCMGWFRQAPGKEREAVAAI NSDGSTSYADSVKGRFTISKDNAKNTLYLQMNSLKPEDTAMYYCAAEPYCSG GYPRWSVAEFGYWGQGTQVTVSSGGGSQVQLQESGGGSVQAGGSLRLSCAA SGYSNCSYDMTWYRQAPGKEREFVSAIHSDGSTRYADSVKGRFFISQDNAKN TVYLQMNSLKPEDTAMYYCKTDPLHCRAHGGSWYSVRANYWGQGTQVTVS SASHHHHHH |
| **248** | IL10RβVHH-GGGS-IL10RαVHH-ASH6 | QVQLQESGGGSVQAGGSLRLSCAASGYTYSSYCMGWFRQAPGKEREGVAHI DSDGSTSYADSVKGRFTISKDNAKNTLYLQMNSLKPEDTAMYYCAADPIPGP GYCDGGPNKYWGQGTQVTVSSGGGSQVQLQESGGGSVQAGGSLRLSCAASR YLYSIDYMAWFRQSPGKEREPVAVIYTASGATFYPDSVKGRFTISQDNAKMTV YLQMNSLKSEDTAMYYCAAVRKTDSYLFDAQSFTYWGQGTQVTVSSASHHH HHH |
| **249** | IL10RβVHH-GGGS-IL10RαVHH-ASH6 | QVQLQESGGGSVQAGGSLRLSCAASGYTYSSYCMGWFRQAPGKEREGVAHI DSDGSTSYADSVKGRFTISKDNAKNTLYLQMNSLKPEDTAMYYCAADPIPGP GYCDGGPNKYWGQGTQVTVSSGGGSQVQLQESGGGSVQAGGSLRLSCAASR FTYSSYCMGWFRQAPGKEREGVASIDSDGSTSYTDSVKGRFTISKDNAKNTLY LQMNSLKPEDTAMYYCALDLMSTVVPGFCGFLLSAGMDYWGKGTQVTVSSA SHHHHHH |
| **250** | IL10RβVHH-GGGS-IL10RαVHH-ASH6 | QVQLQESGGGSVQAGGSLRLSCAASGYTYSSYCMGWFRQAPGKEREGVAHI DSDGSTSYADSVKGRFTISKDNAKNTLYLQMNSLKPEDTAMYYCAADPIPGP GYCDGGPNKYWGQGTQVTVSSGGGSQVQLQESGGGSVQAGGSLRLSCAASG YTYSMYCMGWFRQAPGKEREGVAQINSDGSTSYADSVKGRFTISKDNAKNTL YLQMNSLKPEDTAMYYCAADSRVYGGSWYERLCGPYTYEYNYWGQGTQVT VSSASHHHHHH |
| **251** | IL10RβVHH-GGGS-IL10RαVHH-ASH6 | QVQLQESGGGSVQAGGSLRLSCAASGYTYSSYCMGWFRQAPGKEREGVAHI DSDGSTSYADSVKGRFTISKDNAKNTLYLQMNSLKPEDTAMYYCAADPIPGP GYCDGGPNKYWGQGTQVTVSSGGGSQVQLQESGGGSVQAGGSLRLSCAVSG YAYSTYCMGWFRQAPGKEREGVAAIDSGGSTSYADSVKGRFTISKDNAKNTL YLRMNSLKPEDTAMYYCAAVPPPPDGGSCLFLGPEIKVSKADFRYWGQGTQV TVSSASHHHHHH |
| **252** | IL10RβVHH-GGGS-IL10RαVHH-ASH6 | QVQLQESGGGSVQAGGSLRLSCAASGYTYSSYCMGWFRQAPGKEREGVAHI DSDGSTSYADSVKGRFTISKDNAKNTLYLQMNSLKPEDTAMYYCAADPIPGP GYCDGGPNKYWGQGTQVTVSSGGGSQVQLQESGGGSVQAGGSLRLSCTVSG YTYSSNCMGWFRQAPGKEREGVATIYTGGGNTYYADSVKGRFTISQDNAKNT VYLQMNNLKPEDTAMYYCAAEPLSRVYGGSCPTPTFDYWGQGTQVTVSSAS HHHHHH |
| **253** | IL10RβVHH-GGGS-IL10RαVHH-ASH6 | QVQLQESGGGSVQAGGSLRLSCAASGYTYSSYCMGWFRQAPGKEREGVAHI DSDGSTSYADSVKGRFTISKDNAKNTLYLQMNSLKPEDTAMYYCAADPIPGP GYCDGGPNKYWGQGTQVTVSSGGGSQVQLQESGGGSVQAGGSLRLSCGASG YTYSSYCMGWFRQVPGKEREGVAVIDSDGSTSYADSVKGRFTISKDNGKNTL YLQMNSLKPEDTAMYYCAADLGHYRPPCGVLYLGMDYWGKGTQVTVSSAS HHHHHH |
| **254** | IL10RβVHH-GGGS-IL10RαVHH-ASH6 | QVQLQESGGGSVQAGGSLRLSCAASGYTYSSYCMGWFRQAPGKEREGVAHI DSDGSTSYADSVKGRFTISKDNAKNTLYLQMNSLKPEDTAMYYCAADPIPGP GYCDGGPNKYWGQGTQVTVSSGGGSQVQLQESGGGSVQAGGSLRLSCAASG YSNCSYDMTWYRQAPGKEREFVSAIHSDGSTRYADSVKGRFFISQDNAKNTV YLQMNSLKPEDTAMYYCKTDPLHCRAHGGSWYSVRANYWGQGTQVTVSSA SHHHHHH |
| **255** | IL10RβVHH-GGGS-IL10RαVHH-ASH6 | QVQLQESGGGSVQAGGSLRLSCAASRYTYNSYCMGWFRQAPGKEREGVATI DSDGMTRYADSVKGRFTISKDNAKNTLYLQMNSLKPEDTAMYYCAADADCT IAAMTTNPLGQGTQVTVSSGGGSQVQLQESGGGSVQAGGSLRLSCAASRYLY SIDYMAWFRQSPGKEREPVAVIYTASGATFYPDSVKGRFTISQDNAKMTVYLQ MNSLKSEDTAMYYCAAVRKTDSYLFDAQSFTYWGQGTQVTVSSASHHHHHH |
| **256** | IL10RβVHH-GGGS-IL10RαVHH-ASH6 | QVQLQESGGGSVQAGGSLRLSCAASRYTYNSYCMGWFRQAPGKEREGVATI DSDGMTRYADSVKGRFTISKDNAKNTLYLQMNSLKPEDTAMYYCAADADCT IAAMTTNPLGQGTQVTVSSGGGSQVQLQESGGGSVQAGGSLRLSCAASRFTY SSYCMGWFRQAPGKEREGVASIDSDGSTSYTDSVKGRFTISKDNAKNTLYLQ MNSLKPEDTAMYYCALDLMSTVVPGFCGFLLSAGMDYWGKGTQVTVSSASH HHHHH |
| **257** | IL10RβVHH-GGGS-IL10RαVHH-ASH6 | QVQLQESGGGSVQAGGSLRLSCAASRYTYNSYCMGWFRQAPGKEREGVATI DSDGMTRYADSVKGRFTISKDNAKNTLYLQMNSLKPEDTAMYYCAADADCT IAAMTTNPLGQGTQVTVSSGGGSQVQLQESGGGSVQAGGSLRLSCAASGYTY SMYCMGWFRQAPGKEREGVAQINSDGSTSYADSVKGRFTISKDNAKNTLYLQ MNSLKPEDTAMYYCAADSRVYGGSWYERLCGPYTYEYNYWGQGTQVTVSS ASHHHHHH |
| **258** | IL10RβVHH-GGGS-IL10RαVHH-ASH6 | QVQLQESGGGSVQAGGSLRLSCAASRYTYNSYCMGWFRQAPGKEREGVATI DSDGMTRYADSVKGRFTISKDNAKNTLYLQMNSLKPEDTAMYYCAADADCT IAAMTTNPLGQGTQVTVSSGGGSQVQLQESGGGSVQAGGSLRLSCAVSGYAY STYCMGWFRQAPGKEREGVAAIDSGGSTSYADSVKGRFTISKDNAKNTLYLR MNSLKPEDTAMYYCAAVPPPPDGGSCLFLGPEIKVSKADFRYWGQGTQVTVS SASHHHHHH |
| **259** | IL10RβVHH-GGGS-IL10RαVHH-ASH6 | QVQLQESGGGSVQAGGSLRLSCAASRYTYNSYCMGWFRQAPGKEREGVATI DSDGMTRYADSVKGRFTISKDNAKNTLYLQMNSLKPEDTAMYYCAADADCT IAAMTTNPLGQGTQVTVSSGGGSQVQLQESGGGSVQAGGSLRLSCTVSGYTY SSNCMGWFRQAPGKEREGVATIYTGGGNTYYADSVKGRFTISQDNAKNTVYL QMNNLKPEDTAMYYCAAEPLSRVYGGSCPTPTFDYWGQGTQVTVSSASHHH HHH |
| **260** | IL10RβVHH-GGGS-IL10RαVHH-ASH6 | QVQLQESGGGSVQAGGSLRLSCAASRYTYNSYCMGWFRQAPGKEREGVATI DSDGMTRYADSVKGRFTISKDNAKNTLYLQMNSLKPEDTAMYYCAADADCT IAAMTTNPLGQGTQVTVSSGGGSQVQLQESGGGSVQAGGSLRLSCGASGYTY SSYCMGWFRQVPGKEREGVAVIDSDGSTSYADSVKGRFTISKDNGKNTLYLQ MNSLKPEDTAMYYCAADLGHYRPPCGVLYLGMDYWGKGTQVTVSSASHHH HHH |
| **261** | IL10RβVHH-GGGS-IL10RαVHH-ASH6 | QVQLQESGGGSVQAGGSLRLSCAASRYTYNSYCMGWFRQAPGKEREGVATI DSDGMTRYADSVKGRFTISKDNAKNTLYLQMNSLKPEDTAMYYCAADADCT IAAMTTNPLGQGTQVTVSSGGGSQVQLQESGGGSVQAGGSLRLSCAASGYSN CSYDMTWYRQAPGKEREFVSAIHSDGSTRYADSVKGRFFISQDNAKNTVYLQ MNSLKPEDTAMYYCKTDPLHCRAHGGSWYSVRANYWGQGTQVTVSSASHH HHHH |
| **262** | IL10RβVHH-GGGS-IL10RαVHH-ASH6 | QVQLQESGGGSVQAGGSLRLSCTVSRYTASVNYMGWFRQAPGKEREGVATIF TGAGTTYYANSVKGRFTISRDNAKNTAYLQMNSLKPEDTAIYYCAVDFRGGL LYRPAYEYTYRGQGTQVTVSSGGGSQVQLQESGGGSVQAGGSLRLSCAASRY LYSIDYMAWFRQSPGKEREPVAVIYTASGATFYPDSVKGRFTISQDNAKMTVY LQMNSLKSEDTAMYYCAAVRKTDSYLFDAQSFTYWGQGTQVTVSSASHHHH HH |
| **263** | IL10RβVHH-GGGS-IL10RαVHH-ASH6 | QVQLQESGGGSVQAGGSLRLSCTVSRYTASVNYMGWFRQAPGKEREGVATIF TGAGTTYYANSVKGRFTISRDNAKNTAYLQMNSLKPEDTAIYYCAVDFRGGL LYRPAYEYTYRGQGTQVTVSSGGGSQVQLQESGGGSVQAGGSLRLSCAASRF TYSSYCMGWFRQAPGKEREGVASIDSDGSTSYTDSVKGRFTISKDNAKNTLYL QMNSLKPEDTAMYYCALDLMSTVVPGFCGFLLSAGMDYWGKGTQVTVSSAS HHHHHH |
| **264** | IL10RβVHH-GGGS-IL10RαVHH-ASH6 | QVQLQESGGGSVQAGGSLRLSCTVSRYTASVNYMGWFRQAPGKEREGVATIF TGAGTTYYANSVKGRFTISRDNAKNTAYLQMNSLKPEDTAIYYCAVDFRGGL LYRPAYEYTYRGQGTQVTVSSGGGSQVQLQESGGGSVQAGGSLRLSCAASGY TYSMYCMGWFRQAPGKEREGVAQINSDGSTSYADSVKGRFTISKDNAKNTLY LQMNSLKPEDTAMYYCAADSRVYGGSWYERLCGPYTYEYNYWGQGTQVTV SSASHHHHHH |
| **265** | IL10RβVHH-GGGS-IL10RαVHH-ASH6 | QVQLQESGGGSVQAGGSLRLSCTVSRYTASVNYMGWFRQAPGKEREGVATIF TGAGTTYYANSVKGRFTISRDNAKNTAYLQMNSLKPEDTAIYYCAVDFRGGL LYRPAYEYTYRGQGTQVTVSSGGGSQVQLQESGGGSVQAGGSLRLSCAVSGY AYSTYCMGWFRQAPGKEREGVAAIDSGGSTSYADSVKGRFTISKDNAKNTLY LRMNSLKPEDTAMYYCAAVPPPPDGGSCLFLGPEIKVSKADFRYWGQGTQVT VSSASHHHHHH |
| **266** | IL10RβVHH-GGGS-IL10RαVHH-ASH6 | QVQLQESGGGSVQAGGSLRLSCTVSRYTASVNYMGWFRQAPGKEREGVATIF TGAGTTYYANSVKGRFTISRDNAKNTAYLQMNSLKPEDTAIYYCAVDFRGGL LYRPAYEYTYRGQGTQVTVSSGGGSQVQLQESGGGSVQAGGSLRLSCTVSGY TYSSNCMGWFRQAPGKEREGVATIYTGGGNTYYADSVKGRFTISQDNAKNTV YLQMNNLKPEDTAMYYCAAEPLSRVYGGSCPTPTFDYWGQGTQVTVSSASH HHHHH |
| **267** | IL10RβVHH-GGGS-IL10RαVHH-ASH6 | QVQLQESGGGSVQAGGSLRLSCTVSRYTASVNYMGWFRQAPGKEREGVATIF TGAGTTYYANSVKGRFTISRDNAKNTAYLQMNSLKPEDTAIYYCAVDFRGGL LYRPAYEYTYRGQGTQVTVSSGGGSQVQLQESGGGSVQAGGSLRLSCGASGY TYSSYCMGWFRQVPGKEREGVAVIDSDGSTSYADSVKGRFTISKDNGKNTLY LQMNSLKPEDTAMYYCAADLGHYRPPCGVLYLGMDYWGKGTQVTVSSASH HHHHH |
| **268** | IL10RβVHH-GGGS-IL10RαVHH-ASH6 | QVQLQESGGGSVQAGGSLRLSCTVSRYTASVNYMGWFRQAPGKEREGVATIF TGAGTTYYANSVKGRFTISRDNAKNTAYLQMNSLKPEDTAIYYCAVDFRGGL LYRPAYEYTYRGQGTQVTVSSGGGSQVQLQESGGGSVQAGGSLRLSCAASGY SNCSYDMTWYRQAPGKEREFVSAIHSDGSTRYADSVKGRFFISQDNAKNTVY LQMNSLKPEDTAMYYCKTDPLHCRAHGGSWYSVRANYWGQGTQVTVSSAS HHHHHH |
| **269** | IL10RβVHH-GGGS-IL10RαVHH-ASH6 | QVQLQESGGGSVEAGGSLRLSCAASGYTHSSYCMGWFRQAPGKEREGVAAID VDGSTTYADSVKGRFTISKDNAKNTLYLQMNSLKPEDTGMYYCAAEFADCSS NYFLPPGAVRYWGQGTQVTVSSGGGSQVQLQESGGGSVQAGGSLRLSCAAS RYLYSIDYMAWFRQSPGKEREPVAVIYTASGATFYPDSVKGRFTISQDNAKMT VYLQMNSLKSEDTAMYYCAAVRKTDSYLFDAQSFTYWGQGTQVTVSSASHH HHHH |
| **270** | IL10RβVHH-GGGS-IL10RαVHH-ASH6 | QVQLQESGGGSVEAGGSLRLSCAASGYTHSSYCMGWFRQAPGKEREGVAAID VDGSTTYADSVKGRFTISKDNAKNTLYLQMNSLKPEDTGMYYCAAEFADCSS NYFLPPGAVRYWGQGTQVTVSSGGGSQVQLQESGGGSVQAGGSLRLSCAAS RFTYSSYCMGWFRQAPGKEREGVASIDSDGSTSYTDSVKGRFTISKDNAKNTL YLQMNSLKPEDTAMYYCALDLMSTVVPGFCGFLLSAGMDYWGKGTQVTVSS ASHHHHHH |
| **271** | IL10RβVHH-GGGS-IL10RαVHH-ASH6 | QVQLQESGGGSVEAGGSLRLSCAASGYTHSSYCMGWFRQAPGKEREGVAAID VDGSTTYADSVKGRFTISKDNAKNTLYLQMNSLKPEDTGMYYCAAEFADCSS NYFLPPGAVRYWGQGTQVTVSSGGGSQVQLQESGGGSVQAGGSLRLSCAAS GYTYSMYCMGWFRQAPGKEREGVAQINSDGSTSYADSVKGRFTISKDNAKN TLYLQMNSLKPEDTAMYYCAADSRVYGGSWYERLCGPYTYEYNYWGQGTQ VTVSSASHHHHHH |
| **272** | IL10RβVHH-GGGS-IL10RαVHH-ASH6 | QVQLQESGGGSVEAGGSLRLSCAASGYTHSSYCMGWFRQAPGKEREGVAAID VDGSTTYADSVKGRFTISKDNAKNTLYLQMNSLKPEDTGMYYCAAEFADCSS NYFLPPGAVRYWGQGTQVTVSSGGGSQVQLQESGGGSVQAGGSLRLSCAVS GYAYSTYCMGWFRQAPGKEREGVAAIDSGGSTSYADSVKGRFTISKDNAKNT LYLRMNSLKPEDTAMYYCAAVPPPPDGGSCLFLGPEIKVSKADFRYWGQGTQ VTVSSASHHHHHH |
| **273** | IL10RβVHH-GGGS-IL10RαVHH-ASH6 | QVQLQESGGGSVEAGGSLRLSCAASGYTHSSYCMGWFRQAPGKEREGVAAID VDGSTTYADSVKGRFTISKDNAKNTLYLQMNSLKPEDTGMYYCAAEFADCSS NYFLPPGAVRYWGQGTQVTVSSGGGSQVQLQESGGGSVQAGGSLRLSCTVSG YTYSSNCMGWFRQAPGKEREGVATIYTGGGNTYYADSVKGRFTISQDNAKNT VYLQMNNLKPEDTAMYYCAAEPLSRVYGGSCPTPTFDYWGQGTQVTVSSAS HHHHHH |
| **274** | IL10RβVHH-GGGS-IL10RαVHH-ASH6 | QVQLQESGGGSVEAGGSLRLSCAASGYTHSSYCMGWFRQAPGKEREGVAAID VDGSTTYADSVKGRFTISKDNAKNTLYLQMNSLKPEDTGMYYCAAEFADCSS NYFLPPGAVRYWGQGTQVTVSSGGGSQVQLQESGGGSVQAGGSLRLSCGAS GYTYSSYCMGWFRQVPGKEREGVAVIDSDGSTSYADSVKGRFTISKDNGKNT LYLQMNSLKPEDTAMYYCAADLGHYRPPCGVLYLGMDYWGKGTQVTVSSA SHHHHHH |
| **275** | IL10RβVHH-GGGS-IL10RαVHH-ASH6 | QVQLQESGGGSVEAGGSLRLSCAASGYTHSSYCMGWFRQAPGKEREGVAAID VDGSTTYADSVKGRFTISKDNAKNTLYLQMNSLKPEDTGMYYCAAEFADCSS NYFLPPGAVRYWGQGTQVTVSSGGGSQVQLQESGGGSVQAGGSLRLSCAAS GYSNCSYDMTWYRQAPGKEREFVSAIHSDGSTRYADSVKGRFFISQDNAKNT VYLQMNSLKPEDTAMYYCKTDPLHCRAHGGSWYSVRANYWGQGTQVTVSS ASHHHHHH |
| **276** | IL10RβVHH-GGGS-IL10RαVHH-ASH6 | QVQLQESGGGSVQAGGSLRLSCAASGYSYSSYCMGWFRQAPGKEREGVATID SDGMTRYADSVKGRFTISKDNAKNTLYLQMNSLKPEDTAMYYCAAPLYDCD SGAVGRNPPYWGQGTQVTVSSGGGSQVQLQESGGGSVQAGGSLRLSCAASR YLYSIDYMAWFRQSPGKEREPVAVIYTASGATFYPDSVKGRFTISQDNAKMTV YLQMNSLKSEDTAMYYCAAVRKTDSYLFDAQSFTYWGQGTQVTVSSASHHH HHH |
| **277** | IL10RβVHH-GGGS-IL10RαVHH-ASH6 | QVQLQESGGGSVQAGGSLRLSCAASGYSYSSYCMGWFRQAPGKEREGVATID SDGMTRYADSVKGRFTISKDNAKNTLYLQMNSLKPEDTAMYYCAAPLYDCD SGAVGRNPPYWGQGTQVTVSSGGGSQVQLQESGGGSVQAGGSLRLSCAASRF TYSSYCMGWFRQAPGKEREGVASIDSDGSTSYTDSVKGRFTISKDNAKNTLYL QMNSLKPEDTAMYYCALDLMSTVVPGFCGFLLSAGMDYWGKGTQVTVSSAS HHHHHH |
| **278** | IL10RβVHH-GGGS-IL10RαVHH-ASH6 | QVQLQESGGGSVQAGGSLRLSCAASGYSYSSYCMGWFRQAPGKEREGVATID SDGMTRYADSVKGRFTISKDNAKNTLYLQMNSLKPEDTAMYYCAAPLYDCD SGAVGRNPPYWGQGTQVTVSSGGGSQVQLQESGGGSVQAGGSLRLSCAASG YTYSMYCMGWFRQAPGKEREGVAQINSDGSTSYADSVKGRFTISKDNAKNTL YLQMNSLKPEDTAMYYCAADSRVYGGSWYERLCGPYTYEYNYWGQGTQVT VSSASHHHHHH |
| **279** | IL10RβVHH-GGGS-IL10RαVHH-ASH6 | QVQLQESGGGSVQAGGSLRLSCAASGYSYSSYCMGWFRQAPGKEREGVATID SDGMTRYADSVKGRFTISKDNAKNTLYLQMNSLKPEDTAMYYCAAPLYDCD SGAVGRNPPYWGQGTQVTVSSGGGSQVQLQESGGGSVQAGGSLRLSCAVSG YAYSTYCMGWFRQAPGKEREGVAAIDSGGSTSYADSVKGRFTISKDNAKNTL YLRMNSLKPEDTAMYYCAAVPPPPDGGSCLFLGPEIKVSKADFRYWGQGTQV TVSSASHHHHHH |
| **280** | IL10RβVHH-GGGS-IL10RαVHH-ASH6 | QVQLQESGGGSVQAGGSLRLSCAASGYSYSSSYCMGWFRQAPGKEREGVATID SDGMTRYADSVKGRFTISKDNAKNTLYLQMNSLKPEDTAMYYCAAPLYDCD SGAVGRNPPYWGQGTQVTVSSGGGSQVQLQESGGGSVQAGGSLRLSCTVSG YTYSSNCMGWFRQAPGKEREGVATIYTGGGNTYYADSVKGRFTISQDNAKNT VYLQMNNLKPEDTAMYYCAAEPLSRVYGGSCPTPTFDYWGQGTQVTVSSAS HHHHHH |
| **281** | IL10RβVHH-GGGS-IL10RαVHH-ASH6 | QVQLQESGGGSVQAGGSLRLSCAASGYSYSSYCMGWFRQAPGKEREGVATID SDGMTRYADSVKGRFTISKDNAKNTLYLQMNSLKPEDTAMYYCAAPLYDCD SGAVGRNPPYWGQGTQVTVSSGGGSQVQLQESGGGSVQAGGSLRLSCGASG YTYSSYCMGWFRQVPGKEREGVAVIDSDGSTSYADSVKGRFTISKDNGKNTL YLQMNSLKPEDTAMYYCAADLGHYRPPCGVLYLGMDYWGKGTQVTVSSAS HHHHHH |
| **282** | IL10RβVHH-GGGS-IL10RαVHH-ASH6 | QVQLQESGGGSVQAGGSLRLSCAASGYSYSSYCMGWFRQAPGKEREGVATID SDGMTRYADSVKGRFTISKDNAKNTLYLQMNSLKPEDTAMYYCAAPLYDCD SGAVGRNPPYWGQGTQVTVSSGGGSQVQLQESGGGSVQAGGSLRLSCAASG YSNCSYDMTWYRQAPGKEREFVSAIHSDGSTRYADSVKGRFFISQDNAKNTV YLQMNSLKPEDTAMYYCKTDPLHCRAHGGSWYSVRANYWGQGTQVTVSSA SHHHHHH |
| **283** | IL10RβVHH-GGGS-IL10RαVHH-ASH6 | QVQLQESGGGSVQTGGSLRLSCAASGYTYLRGCMGWFRQAPGKEREGVAVM DVVGDRRSYIDSVKGRFTISRDNAANSVYLQMDNLKPEDTAMYYCTAGPNC VGWRSGLDYWGQGTQVTVSSGGGSQVQLQESGGGSVQAGGSLRLSCAASRY LYSIDYMAWFRQSPGKEREPVAVIYTASGATFYPDSVKGRFTISQDNAKMTVY LQMNSLKSEDTAMYYCAAVRKTDSYLFDAQSFTYWGQGTQVTVSSASHHHH HH |
| **284** | IL10RβVHH-GGGS-IL10RαVHH-ASH6 | QVQLQESGGGSVQTGGSLRLSCAASGYTYLRGCMGWFRQAPGKEREGVAVM DVVGDRRSYIDSVKGRFTISRDNAANSVYLQMDNLKPEDTAMYYCTAGPNC VGWRSGLDYWGQGTQVTVSSGGGSQVQLQESGGGSVQAGGSLRLSCAASRF TYSSYCMGWFRQAPGKEREGVASIDSDGSTSYTDSVKGRFTISKDNAKNTLYL QMNSLKPEDTAMYYCALDLMSTVVPGFCGFLLSAGMDYWGKGTQVTVSSAS HHHHHH |
| **285** | IL10RβVHH-GGGS-IL10RαVHH-ASH6 | QVQLQESGGGSVQTGGSLRLSCAASGYTYLRGCMGWFRQAPGKEREGVAVM DVVGDRRSYIDSVKGRFTISRDNAANSVYLQMDNLKPEDTAMYYCTAGPNC VGWRSGLDYWGQGTQVTVSSGGGSQVQLQESGGGSVQAGGSLRLSCAASGY TYSMYCMGWFRQAPGKEREGVAQINSDGSTSYADSVKGRFTISKDNAKNTLY LQMNSLKPEDTAMYYCAADSRVYGGSWYERLCGPYTYEYNYWGQGTQVTV SSASHHHHHH |
| **286** | IL10RβVHH-GGGS-IL10RαVHH-ASH6 | QVQLQESGGGSVQTGGSLRLSCAASGYTYLRGCMGWFRQAPGKEREGVAVM DVVGDRRSYIDSVKGRFTISRDNAANSVYLQMDNLKPEDTAMYYCTAGPNC VGWRSGLDYWGQGTQVTVSSGGGSQVQLQESGGGSVQAGGSLRLSCAVSGY AYSTYCMGWFRQAPGKEREGVAAIDSGGSTSYADSVKGRFTISKDNAKNTLY LRMNSLKPEDTAMYYCAAVPPPPDGGSCLFLGPEIKVSKADFRYWGQGTQVT VSSASHHHHHH |
| **287** | IL10RβVHH-GGGS-IL10RαVHH-ASH6 | QVQLQESGGGSVQTGGSLRLSCAASGYTYLRGCMGWFRQAPGKEREGVAVM DVVGDRRSYIDSVKGRFTISRDNAANSVYLQMDNLKPEDTAMYYCTAGPNC VGWRSGLDYWGQGTQVTVSSGGGSQVQLQESGGGSVQAGGSLRLSCTVSGY TYSSNCMGWFRQAPGKEREGVATIYTGGGNTYYADSVKGRFTISQDNAKNTV YLQMNNLKPEDTAMYYCAAEPLSRVYGGSCPTPTFDYWGQGTQVTVSSASH HHHHH |
| **288** | IL10RβVHH-GGGS-IL10RαVHH-ASH6 | QVQLQESGGGSVQTGGSLRLSCAASGYTYLRGCMGWFRQAPGKEREGVAVM DVVGDRRSYIDSVKGRFTISRDNAANSVYLQMDNLKPEDTAMYYCTAGPNC VGWRSGLDYWGQGTQVTVSSGGGSQVQLQESGGGSVQAGGSLRLSCGASGY TYSSYCMGWFRQVPGKEREGVAVIDSDGSTSYADSVKGRFTISKDNGKNTLY LQMNSLKPEDTAMYYCAADLGHYRPPCGVLYLGMDYWGKGTQVTVSSASH HHHHH |
| **289** | IL10RβVHH-GGGS-IL10RαVHH-ASH6 | QVQLQESGGGSVQTGGSLRLSCAASGYTYLRGCMGWFRQAPGKEREGVAVM DVVGDRRSYIDSVKGRFTISRDNAANSVYLQMDNLKPEDTAMYYCTAGPNC VGWRSGLDYWGQGTQVTVSSGGGSQVQLQESGGGSVQAGGSLRLSCAASGY SNCSYDMTWYRQAPGKEREFVSAIHSDGSTRYADSVKGRFFISQDNAKNTVY LQMNSLKPEDTAMYYCKTDPLHCRAHGGSWYSVRANYWGQGTQVTVSSAS HHHHHH |
| **290** | DNA Sequence Encoding SEQ ID NO: 192 | CAAGTGCAGCTGCAAGAGAGCGGAGGAGGAAGCGTGCAAGCCGGAGGCT CTCTGAGACTGAGCTGTGCCGCCTCTAGGTATCTGTACAGCATCGACTAC ATGGCTTGGTTCAGACAGAGCCCCGGCAAGGAGAGGGAGCCAGTGGCTG TCATCTACACTGCCTCCGGCGCCACATTCTATCCAGATAGCGTGAAGGGA AGGTTCACTATCAGCCAAGATAACGCCAAGATGACAGTGTATCTGCAGAT GAACTCTCTGAAGAGCGAGGACACTGCCATGTACTACTGTGCCGCCGTGA GGAAGACAGATAGCTACCTCTTCGACGCCCAGAGCTTCACATACTGGGGC CAAGGCACACAAGTGACAGTCTCGAGCGGCGGAGGATCCCAAGTGCAGC TGCAAGAGAGCGGCGGAGGAAGCGTGCAAGCTGGAGGCTCTCTGAGGCT GAGCTGTGCTGCCAGCGGCTACACTTATAGCAGCGGCTGTATGGGCTGGT TCAGACAAGCCCCCGGCAAGGAAAGGGAAGCCGTGGCCGCCATCAATTC CGATGGCAGCACAAGCTACGCCGACAGCGTGAAGGGAAGGTTCACAATC AGCAAGGACAACGCCAAGAACACACTCTATCTGCAGATGAACTCTCTGAA GCCAGAGGACACAGCCATGTACTACTGCGCCGCTGAGCCTTACTGTAGCG GCGGCTACCCAAGATGGAGCGTCGCTGAGTTCGGCTACTGGGGCCAAGGC ACACAAGTGACTGTCTCGTCTGCTAGCCACCATCACCATCACCAC |
| **291** | DNA Sequence Encoding SEQ ID NO: 193 | CAAGTGCAGCTGCAAGAGAGCGGAGGAGGAAGCGTGCAAGCCGGAGGCT CTCTGAGACTGAGCTGTGCCGCCTCTAGGTATCTGTACAGCATCGACTAC ATGGCTTGGTTCAGACAGAGCCCCGGCAAGGAGAGGGAGCCAGTGGCTG TCATCTACACTGCCTCCGGCGCCACATTCTATCCAGATAGCGTGAAGGGA AGGTTCACTATCAGCCAAGATAACGCCAAGATGACAGTGTATCTGCAGAT GAACTCTCTGAAGAGCGAGGACACTGCCATGTACTACTGTGCCGCCGTGA GGAAGACAGATAGCTACCTCTTCGACGCCCAGAGCTTCACATACTGGGGC CAAGGCACACAAGTGACAGTCTCGAGCGGCGGAGGATCCCAAGTGCAGC TGCAAGAGAGCGGAGGAGGCAGCGTCCAAGCCGGAGGCTCTCTGAGGCT GAGCTGTGCTGCCAGCGGCTACACTTACAGCAGCTACTGCATGGGCTGGT TCAGACAAGCCCCCGGCAAGGAGAGAGAGGGCGTGGCTCACATCGACAG CGACGGCTCCACAAGCTACGCCGATAGCGTGAAGGGAAGGTTCACAATCT CCAAGGACAACGCCAAGAACACTCTGTACCTCCAGATGAACTCTCTGAAG CCAGAGGACACTGCCATGTACTACTGTGCCGCCGATCCAATTCCCGGCCC CGGCTACTGCGATGGCGGCCCTAACAAGTACTGGGGCCAAGGCACACAA GTGACTGTCTCGTCTGCTAGCCACCATCACCATCACCAC |
| **292** | DNA Sequence Encoding SEQ ID NO: 194 | CAAGTGCAGCTGCAAGAGAGCGGAGGAGGAAGCGTGCAAGCCGGAGGCT CTCTGAGACTGAGCTGTGCCGCCTCTAGGTATCTGTACAGCATCGACTAC ATGGCTTGGTTCAGACAGAGCCCCGGCAAGGAGAGGGAGCCAGTGGCTG TCATCTACACTGCCTCCGGCGCCACATTCTATCCAGATAGCGTGAAGGGA AGGTTCACTATCAGCCAAGATAACGCCAAGATGACAGTGTATCTGCAGAT GAACTCTCTGAAGAGCGAGGACACTGCCATGTACTACTGTGCCGCCGTGA GGAAGACAGATAGCTACCTCTTCGACGCCCAGAGCTTCACATACTGGGGC CAAGGCACACAAGTGACAGTCTCGAGCGGCGGAGGATCCCAAGTGCAGC TGCAAGAGTCCGGAGGAGGAAGCGTGCAAGCCGGCGGATCTCTGAGACT GAGCTGTGCCGCCTCTAGGTACACTTACAACAGCTACTGCATGGGCTGGT TCAGACAAGCCCCCGGCAAGGAAAGAGAGGGCGTGGCCACTATCGATAG CGACGGCATGACTAGGTACGCTGATAGCGTCAAGGGAAGGTTCACAATCT CCAAGGACAATGCTAAGAACACTCTGTACCTCCAGATGAACTCTCTGAAG CCAGAGGACACAGCCATGTACTACTGCGCTGCCGATGCCGACTGCACTAT CGCCGCCATGACTACTAATCCTCTGGGCCAAGGCACACAAGTGACTGTCT CGTCTGCTAGCCACCATCACCATCACCAC |
| **293** | DNA Sequence Encoding SEQ ID NO: 195 | CAAGTGCAGCTGCAAGAGAGCGGAGGAGGAAGCGTGCAAGCCGGAGGCT CTCTGAGACTGAGCTGTGCCGCCTCTAGGTATCTGTACAGCATCGACTAC ATGGCTTGGTTCAGACAGAGCCCCGGCAAGGAGAGGGAGCCAGTGGCTG TCATCTACACTGCCTCCGGCGCCACATTCTATCCAGATAGCGTGAAGGGA AGGTTCACTATCAGCCAAGATAACGCCAAGATGACAGTGTATCTGCAGAT GAACTCTCTGAAGAGCGAGGACACTGCCATGTACTACTGTGCCGCCGTGA GGAAGACAGATAGCTACCTCTTCGACGCCCAGAGCTTCACATACTGGGGC CAAGGCACACAAGTGACAGTCTCGAGCGGCGGAGGATCCCAAGTGCAGC TGCAAGAGTCCGGCGGAGGCAGCGTCCAAGCCGGAGGATCTCTGAGGCT GAGCTGTACAGTGAGCAGATACACTGCCAGCGTGAACTACATGGGCTGGT TCAGACAAGCCCCCGGCAAAGAGAGAGAGGGCGTGGCCACAATCTTCAC TGGCGCCGGCACAACATACTACGCCAACTCCGTCAAGGGAAGGTTCACAA TCTCTAGGGACAACGCCAAGAACACTGCCTATCTGCAGATGAACTCCCTC AAGCCAGAGGACACTGCCATCTACTACTGCGCCGTGGATTTCAGAGGCGG ACTGCTGTATAGGCCAGCCTACGAGTACACTTATAGGGGCCAAGGCACAC AAGTGACAGTCTCGTCTGCTAGCCACCATCACCATCACCAC |
| **294** | DNA Sequence Encoding SEQ ID NO: 196 | CAAGTGCAGCTGCAAGAGAGCGGAGGAGGAAGCGTGCAAGCCGGAGGCT CTCTGAGACTGAGCTGTGCCGCCTCTAGGTATCTGTACAGCATCGACTAC ATGGCTTGGTTCAGACAGAGCCCCGGCAAGGAGAGGGAGCCAGTGGCTG TCATCTACACTGCCTCCGGCGCCACATTCTATCCAGATAGCGTGAAGGGA AGGTTCACTATCAGCCAAGATAACGCCAAGATGACAGTGTATCTGCAGAT GAACTCTCTGAAGAGCGAGGACACTGCCATGTACTACTGTGCCGCCGTGA GGAAGACAGATAGCTACCTCTTCGACGCCCAGAGCTTCACATACTGGGGC CAAGGCACACAAGTGACAGTCTCGAGCGGCGGAGGATCCCAAGTGCAGC TGCAAGAGAGCGGAGGAGGCAGCGTCGAAGCTGGAGGATCTCTGAGGCT GAGCTGTGCTGCCAGCGGCTACACTCACAGCAGCTACTGTATGGGCTGGT TCAGACAAGCCCCCGGCAAGGAGAGGGAAGGCGTGGCTGCCATCGACGT GGATGGCAGCACTACTTACGCCGACAGCGTGAAGGGAAGGTTCACTATCA GCAAGGACAACGCCAAGAACACACTCTATCTGCAGATGAACAGCCTCAA GCCAGAGGACACTGGCATGTACTACTGCGCCGCCGAGTTCGCCGATTGCA GCAGCAACTACTTTCTGCCTCCCGGCGCCGTCAGATATTGGGGCCAAGGC ACTCAAGTGACAGTCTCGTCTGCTAGCCACCATCACCATCACCAC |
| **295** | DNA Sequence Encoding SEQ ID NO: 197 | CAAGTGCAGCTGCAAGAGAGCGGAGGAGGAAGCGTGCAAGCCGGAGGCT CTCTGAGACTGAGCTGTGCCGCCTCTAGGTATCTGTACAGCATCGACTAC ATGGCTTGGTTCAGACAGAGCCCCGGCAAGGAGAGGGAGCCAGTGGCTG TCATCTACACTGCCTCCGGCGCCACATTCTATCCAGATAGCGTGAAGGGA AGGTTCACTATCAGCCAAGATAACGCCAAGATGACAGTGTATCTGCAGAT GAACTCTCTGAAGAGCGAGGACACTGCCATGTACTACTGTGCCGCCGTGA GGAAGACAGATAGCTACCTCTTCGACGCCCAGAGCTTCACATACTGGGGC CAAGGCACACAAGTGACAGTCTCGAGCGGCGGAGGATCCCAAGTGCAGC TGCAAGAGAGCGGAGGAGGAAGCGTCCAAGCCGGAGGATCTCTGAGACT GAGCTGCGCCGCTAGTGGCTACTCCTACAGCAGCTACTGCATGGGCTGGT TTAGGCAAGCCCCCGGCAAGGAGAGAGAAGGCGTGGCCACTATCGACAG CGACGGCATGACAAGGTACGCCGACAGCGTGAAGGGAAGGTTCACAATC AGCAAGGACAACGCCAAGAACACACTGTATCTGCAGATGAACTCTCTGA AGCCAGAGGACACTGCCATGTACTACTGTGCCGCTCCTCTGTACGACTGT GATAGCGGCGCTGTGGGCAGAAATCCACCTTATTGGGGCCAAGGCACTCA AGTGACAGTCTCGTCTGCTAGCCACCATCACCATCACCAC |
| **296** | DNA Sequence Encoding SEQ ID NO: 198 | CAAGTGCAGCTGCAAGAGAGCGGAGGAGGAAGCGTGCAAGCCGGAGGCT CTCTGAGACTGAGCTGTGCCGCCTCTAGGTATCTGTACAGCATCGACTAC ATGGCTTGGTTCAGACAGAGCCCCGGCAAGGAGAGGGAGCCAGTGGCTG TCATCTACACTGCCTCCGGCGCCACATTCTATCCAGATAGCGTGAAGGGA AGGTTCACTATCAGCCAAGATAACGCCAAGATGACAGTGTATCTGCAGAT GAACTCTCTGAAGAGCGAGGACACTGCCATGTACTACTGTGCCGCCGTGA GGAAGACAGATAGCTACCTCTTCGACGCCCAGAGCTTCACATACTGGGGC CAAGGCACACAAGTGACAGTCTCGAGCGGCGGAGGATCCCAAGTGCAGC TGCAAGAGAGCGGAGGCGGCAGCGTGCAGACTGGAGGCTCTCTGAGACT GAGCTGTGCTGCCAGCGGCTACACTTATCTGAGGGGCTGTATGGGCTGGT TTAGGCAAGCCCCCGGCAAGGAGAGAGAGGGCGTGGCCGTCATGGATGT GGTGGGCGATAGGAGAAGCTACATCGACAGCGTGAAGGGAAGGTTCACA ATCTCTAGGGACAATGCCGCCAACAGCGTCTATCTGCAGATGGACAATCT GAAGCCAGAGGACACAGCCATGTACTACTGCACTGCCGGCCCTAACTGTG TGGGCTGGAGAAGCGGACTGGATTACTGGGGCCAAGGCACACAAGTGAC AGTCTCGTCTGCTAGCCACCATCACCATCACCAC |
| **297** | DNA Sequence Encoding SEQ ID NO: 199 | CAAGTGCAGCTGCAAGAGAGCGGAGGAGGAAGCGTGCAAGCCGGAGGCT CTCTGAGGCTGAGCTGTGCCGCCTCTAGGTTCACATACAGCAGCTACTGC ATGGGCTGGTTCAGACAAGCCCCCGGCAAAGAGAGAGAAGGCGTGGCCA GCATCGATAGCGATGGCTCCACTAGCTACACTGACAGCGTGAAGGGAAG GTTCACTATCAGCAAGGACAACGCCAAGAACACTCTGTATCTGCAGATGA ACTCTCTGAAGCCAGAGGACACAGCCATGTACTACTGTGCCCTCGATCTG ATGAGCACAGTGGTGCCCGGCTTCTGTGGCTTTCTGCTGAGCGCTGGCAT GGATTACTGGGGCAAGGGCACTCAAGTGACTGTCTCGAGCGGCGGAGGA TCCCAAGTGCAGCTGCAAGAGAGCGGCGGAGGAAGCGTGCAAGCTGGAG GCTCTCTGAGGCTGAGCTGTGCTGCCAGCGGCTACACTTATAGCAGCGGC TGTATGGGCTGGTTCAGACAAGCCCCCGGCAAGGAAAGGGAAGCCGTGG CCGCCATCAATTCCGATGGCAGCACAAGCTACGCCGACAGCGTGAAGGG AAGGTTCACAATCAGCAAGGACAACGCCAAGAACACACTCTATCTGCAG ATGAACTCTCTGAAGCCAGAGGACACAGCCATGTACTACTGCGCCGCTGA GCCTTACTGTAGCGGCGGCTACCCAAGATGGAGCGTCGCTGAGTTCGGCT ACTGGGGCCAAGGCACACAAGTGACTGTCTCGTCTGCTAGCCACCATCAC CATCACCAC |
| **298** | DNA Sequence Encoding SEQ ID NO: 200 | CAAGTGCAGCTGCAAGAGAGCGGAGGAGGAAGCGTGCAAGCCGGAGGCT CTCTGAGGCTGAGCTGTGCCGCCTCTAGGTTCACATACAGCAGCTACTGC ATGGGCTGGTTCAGACAAGCCCCCGGCAAAGAGAGAGAAGGCGTGGCCA GCATCGATAGCGATGGCTCCACTAGCTACACTGACAGCGTGAAGGGAAG GTTCACTATCAGCAAGGACAACGCCAAGAACACTCTGTATCTGCAGATGA ACTCTCTGAAGCCAGAGGACACAGCCATGTACTACTGTGCCCTCGATCTG ATGAGCACAGTGGTGCCCGGCTTCTGTGGCTTTCTGCTGAGCGCTGGCAT GGATTACTGGGGCAAGGGCACTCAAGTGACTGTCTCGAGCGGCGGAGGA TCCCAAGTGCAGCTGCAAGAGAGCGGAGGAGGCAGCGTCCAAGCCGGAG GCTCTCTGAGGCTGAGCTGTGCTGCCAGCGGCTACACTTACAGCAGCTAC TGCATGGGCTGGTTCAGACAAGCCCCCGGCAAGGAGAGAGAGGGCGTGG CTCACATCGACAGCGACGGCTCCACAAGCTACGCCGATAGCGTGAAGGG AAGGTTCACAATCTCCAAGGACAACGCCAAGAACACTCTGTACCTCCAGA TGAACTCTCTGAAGCCAGAGGACACTGCCATGTACTACTGTGCCGCCGAT CCAATTCCCGGCCCCGGCTACTGCGATGGCGGCCCTAACAAGTACTGGGG CCAAGGCACACAAGTGACTGTCTCGTCTGCTAGCCACCATCACCATCACC AC |
| **299** | DNA Sequence Encoding SEQ ID NO: 201 | CAAGTGCAGCTGCAAGAGAGCGGAGGAGGAAGCGTGCAAGCCGGAGGCT CTCTGAGGCTGAGCTGTGCCGCCTCTAGGTTCACATACAGCAGCTACTGC ATGGGCTGGTTCAGACAAGCCCCCGGCAAAGAGAGAGAAGGCGTGGCCA GCATCGATAGCGATGGCTCCACTAGCTACACTGACAGCGTGAAGGGAAG GTTCACTATCAGCAAGGACAACGCCAAGAACACTCTGTATCTGCAGATGA ACTCTCTGAAGCCAGAGGACACAGCCATGTACTACTGTGCCCTCGATCTG ATGAGCACAGTGGTGCCCGGCTTCTGTGGCTTTCTGCTGAGCGCTGGCAT GGATTACTGGGGCAAGGGCACTCAAGTGACTGTCTCGAGCGGCGGAGGA TCCCAAGTGCAGCTGCAAGAGTCCGGAGGAGGAAGCGTGCAAGCCGGCG GATCTCTGAGACTGAGCTGTGCCGCCTCTAGGTACACTTACAACAGCTAC TGCATGGGCTGGTTCAGACAAGCCCCCGGCAAGGAAAGAGAGGGCGTGG CCACTATCGATAGCGACGGCATGACTAGGTACGCTGATAGCGTCAAGGGA AGGTTCACAATCTCCAAGGACAATGCTAAGAACACTCTGTACCTCCAGAT GAACTCTCTGAAGCCAGAGGACACAGCCATGTACTACTGCGCTGCCGATG CCGACTGCACTATCGCCGCCATGACTACTAATCCTCTGGGCCAAGGCACA CAAGTGACTGTCTCGTCTGCTAGCCACCATCACCATCACCAC |
| **300** | DNA Sequence Encoding SEQ ID NO: 202 | CAAGTGCAGCTGCAAGAGAGCGGAGGAGGAAGCGTGCAAGCCGGAGGCT CTCTGAGGCTGAGCTGTGCCGCCTCTAGGTTCACATACAGCAGCTACTGC ATGGGCTGGTTCAGACAAGCCCCCGGCAAAGAGAGAGAAGGCGTGGCCA GCATCGATAGCGATGGCTCCACTAGCTACACTGACAGCGTGAAGGGAAG GTTCACTATCAGCAAGGACAACGCCAAGAACACTCTGTATCTGCAGATGA ACTCTCTGAAGCCAGAGGACACAGCCATGTACTACTGTGCCCTCGATCTG ATGAGCACAGTGGTGCCCGGCTTCTGTGGCTTTCTGCTGAGCGCTGGCAT GGATTACTGGGGCAAGGGCACTCAAGTGACTGTCTCGAGCGGCGGAGGA TCCCAAGTGCAGCTGCAAGAGTCCGGCGGAGGCAGCGTCCAAGCCGGAG GATCTCTGAGGCTGAGCTGTACAGTGAGCAGATACACTGCCAGCGTGAAC TACATGGGCTGGTTCAGACAAGCCCCCGGCAAAGAGAGAGAGGGCGTGG CCACAATCTTCACTGGCGCCGGCACAACATACTACGCCAACTCCGTCAAG GGAAGGTTCACAATCTCTAGGGACAACGCCAAGAACACTGCCTATCTGCA GATGAACTCCCTCAAGCCAGAGGACACTGCCATCTACTACTGCGCCGTGG ATTTCAGAGGCGGACTGCTGTATAGGCCAGCCTACGAGTACACTTATAGG GGCCAAGGCACACAAGTGACAGTCTCGTCTGCTAGCCACCATCACCATCA CCAC |
| **301** | DNA Sequence Encoding SEQ ID NO: 203 | CAAGTGCAGCTGCAAGAGAGCGGAGGAGGAAGCGTGCAAGCCGGAGGCT CTCTGAGGCTGAGCTGTGCCGCCTCTAGGTTCACATACAGCAGCTACTGC ATGGGCTGGTTCAGACAAGCCCCCGGCAAAGAGAGAGAAGGCGTGGCCA GCATCGATAGCGATGGCTCCACTAGCTACACTGACAGCGTGAAGGGAAG GTTCACTATCAGCAAGGACAACGCCAAGAACACTCTGTATCTGCAGATGA ACTCTCTGAAGCCAGAGGACACAGCCATGTACTACTGTGCCCTCGATCTG ATGAGCACAGTGGTGCCCGGCTTCTGTGGCTTTCTGCTGAGCGCTGGCAT GGATTACTGGGGCAAGGGCACTCAAGTGACTGTCTCGAGCGGCGGAGGA TCCCAAGTGCAGCTGCAAGAGAGCGGAGGAGGCAGCGTCGAAGCTGGAG GATCTCTGAGGCTGAGCTGTGCTGCCAGCGGCTACACTCACAGCAGCTAC TGTATGGGCTGGTTCAGACAAGCCCCCGGCAAGGAGAGGGAAGGCGTGG CTGCCATCGACGTGGATGGCAGCACTACTTACGCCGACAGCGTGAAGGGA AGGTTCACTATCAGCAAGGACAACGCCAAGAACACACTCTATCTGCAGAT GAACAGCCTCAAGCCAGAGGACACTGGCATGTACTACTGCGCCGCCGAGT TCGCCGATTGCAGCAGCAACTACTTTCTGCCTCCCGGCGCCGTCAGATATT GGGGCCAAGGCACTCAAGTGACAGTCTCGTCTGCTAGCCACCATCACCAT CACCAC |
| **302** | DNA Sequence Encoding SEQ ID NO: 204 | CAAGTGCAGCTGCAAGAGAGCGGAGGAGGAAGCGTGCAAGCCGGAGGCT CTCTGAGGCTGAGCTGTGCCGCCTCTAGGTTCACATACAGCAGCTACTGC ATGGGCTGGTTCAGACAAGCCCCCGGCAAAGAGAGAGAAGGCGTGGCCA GCATCGATAGCGATGGCTCCACTAGCTACACTGACAGCGTGAAGGGAAG GTTCACTATCAGCAAGGACAACGCCAAGAACACTCTGTATCTGCAGATGA ACTCTCTGAAGCCAGAGGACACAGCCATGTACTACTGTGCCCTCGATCTG ATGAGCACAGTGGTGCCCGGCTTCTGTGGCTTTCTGCTGAGCGCTGGCAT GGATTACTGGGGCAAGGGCACTCAAGTGACTGTCTCGAGCGGCGGAGGA TCCCAAGTGCAGCTGCAAGAGAGCGGAGGAGGAAGCGTCCAAGCCGGAG GATCTCTGAGACTGAGCTGCGCCGCTAGTGGCTACTCCTACAGCAGCTAC TGCATGGGCTGGTTTAGGCAAGCCCCCGGCAAGGAGAGAGAAGGCGTGG CCACTATCGACAGCGACGGCATGACAAGGTACGCCGACAGCGTGAAGGG AAGGTTCACAATCAGCAAGGACAACGCCAAGAACACACTGTATCTGCAG ATGAACTCTCTGAAGCCAGAGGACACTGCCATGTACTACTGTGCCGCTCC TCTGTACGACTGTGATAGCGGCGCTGTGGGCAGAAATCCACCTTATTGGG GCCAAGGCACTCAAGTGACAGTCTCGTCTGCTAGCCACCATCACCATCAC CAC |
| **303** | DNA Sequence Encoding SEQ ID NO: 205 | CAAGTGCAGCTGCAAGAGAGCGGAGGAGGAAGCGTGCAAGCCGGAGGCT CTCTGAGGCTGAGCTGTGCCGCCTCTAGGTTCACATACAGCAGCTACTGC ATGGGCTGGTTCAGACAAGCCCCCGGCAAAGAGAGAGAAGGCGTGGCCA GCATCGATAGCGATGGCTCCACTAGCTACACTGACAGCGTGAAGGGAAG GTTCACTATCAGCAAGGACAACGCCAAGAACACTCTGTATCTGCAGATGA ACTCTCTGAAGCCAGAGGACACAGCCATGTACTACTGTGCCCTCGATCTG ATGAGCACAGTGGTGCCCGGCTTCTGTGGCTTTCTGCTGAGCGCTGGCAT GGATTACTGGGGCAAGGGCACTCAAGTGACTGTCTCGAGCGGCGGAGGA TCCCAAGTGCAGCTGCAAGAGAGCGGAGGCGGCAGCGTGCAGACTGGAG GCTCTCTGAGACTGAGCTGTGCTGCCAGCGGCTACACTTATCTGAGGGGC TGTATGGGCTGGTTTAGGCAAGCCCCCGGCAAGGAGAGAGAGGGCGTGG CCGTCATGGATGTGGTGGGCGATAGGAGAAGCTACATCGACAGCGTGAA GGGAAGGTTCACAATCTCTAGGGACAATGCCGCCAACAGCGTCTATCTGC AGATGGACAATCTGAAGCCAGAGGACACAGCCATGTACTACTGCACTGCC GGCCCTAACTGTGTGGGCTGGAGAAGCGGACTGGATTACTGGGGCCAAG GCACACAAGTGACAGTCTCGTCTGCTAGCCACCATCACCATCACCAC |
| **304** | DNA Sequence Encoding SEQ ID NO: 206 | CAAGTGCAGCTGCAAGAGTCCGGAGGAGGCAGCGTCCAAGCCGGAGGCT CTCTGAGGCTGAGCTGTGCTGCCAGCGGCTACACTTACAGCATGTACTGC ATGGGCTGGTTCAGACAAGCCCCCGGCAAGGAAAGAGAGGGCGTGGCCC AGATCAATAGCGATGGCAGCACAAGCTACGCCGACAGCGTGAAGGGAAG GTTCACTATCTCCAAGGACAACGCCAAGAACACTCTGTATCTGCAGATGA ACTCTCTGAAGCCAGAGGACACTGCCATGTACTACTGCGCTGCCGATTCT AGGGTGTACGGCGGCAGCTGGTATGAGAGGCTCTGCGGCCCTTACACATA CGAGTACAACTACTGGGGCCAAGGCACACAAGTGACTGTCTCGAGCGGC GGAGGATCCCAAGTGCAGCTGCAAGAGAGCGGCGGAGGAAGCGTGCAAG CTGGAGGCTCTCTGAGGCTGAGCTGTGCTGCCAGCGGCTACACTTATAGC AGCGGCTGTATGGGCTGGTTCAGACAAGCCCCCGGCAAGGAAAGGGAAG CCGTGGCCGCCATCAATTCCGATGGCAGCACAAGCTACGCCGACAGCGTG AAGGGAAGGTTCACAATCAGCAAGGACAACGCCAAGAACACACTCTATC TGCAGATGAACTCTCTGAAGCCAGAGGACACAGCCATGTACTACTGCGCC GCTGAGCCTTACTGTAGCGGCGGCTACCCAAGATGGAGCGTCGCTGAGTT CGGCTACTGGGGCCAAGGCACACAAGTGACTGTCTCGTCTGCTAGCCACC ATCACCATCACCAC |
| **305** | DNA Sequence Encoding SEQ ID NO: 207 | CAAGTGCAGCTGCAAGAGTCCGGAGGAGGCAGCGTCCAAGCCGGAGGCT CTCTGAGGCTGAGCTGTGCTGCCAGCGGCTACACTTACAGCATGTACTGC ATGGGCTGGTTCAGACAAGCCCCCGGCAAGGAAAGAGAGGGCGTGGCCC AGATCAATAGCGATGGCAGCACAAGCTACGCCGACAGCGTGAAGGGAAG GTTCACTATCTCCAAGGACAACGCCAAGAACACTCTGTATCTGCAGATGA ACTCTCTGAAGCCAGAGGACACTGCCATGTACTACTGCGCTGCCGATTCT AGGGTGTACGGCGGCAGCTGGTATGAGAGGCTCTGCGGCCCTTACACATA CGAGTACAACTACTGGGGCCAAGGCACACAAGTGACTGTCTCGAGCGGC GGAGGATCCCAAGTGCAGCTGCAAGAGAGCGGAGGAGGCAGCGTCCAAG CCGGAGGCTCTCTGAGGCTGAGCTGTGCTGCCAGCGGCTACACTTACAGC AGCTACTGCATGGGCTGGTTCAGACAAGCCCCCGGCAAGGAGAGAGAGG GCGTGGCTCACATCGACAGCGACGGCTCCACAAGCTACGCCGATAGCGTG AAGGGAAGGTTCACAATCTCCAAGGACAACGCCAAGAACACTCTGTACCT CCAGATGAACTCTCTGAAGCCAGAGGACACTGCCATGTACTACTGTGCCG CCGATCCAATTCCCGGCCCCGGCTACTGCGATGGCGGCCCTAACAAGTAC TGGGGCCAAGGCACACAAGTGACTGTCTCGTCTGCTAGCCACCATCACCA TCACCAC |
| **306** | DNA Sequence Encoding SEQ ID NO: 208 | CAAGTGCAGCTGCAAGAGTCCGGAGGAGGCAGCGTCCAAGCCGGAGGCT CTCTGAGGCTGAGCTGTGCTGCCAGCGGCTACACTTACAGCATGTACTGC ATGGGCTGGTTCAGACAAGCCCCCGGCAAGGAAAGAGAGGGCGTGGCCC AGATCAATAGCGATGGCAGCACAAGCTACGCCGACAGCGTGAAGGGAAG GTTCACTATCTCCAAGGACAACGCCAAGAACACTCTGTATCTGCAGATGA ACTCTCTGAAGCCAGAGGACACTGCCATGTACTACTGCGCTGCCGATTCT AGGGTGTACGGCGGCAGCTGGTATGAGAGGCTCTGCGGCCCTTACACATA CGAGTACAACTACTGGGGCCAAGGCACACAAGTGACTGTCTCGAGCGGC GGAGGATCCCAAGTGCAGCTGCAAGAGTCCGGAGGAGGAAGCGTGCAAG CCGGCGGATCTCTGAGACTGAGCTGTGCCGCCTCTAGGTACACTTACAAC AGCTACTGCATGGGCTGGTTCAGACAAGCCCCCGGCAAGGAAAGAGAGG GCGTGGCCACTATCGATAGCGACGGCATGACTAGGTACGCTGATAGCGTC AAGGGAAGGTTCACAATCTCCAAGGACAATGCTAAGAACACTCTGTACCT CCAGATGAACTCTCTGAAGCCAGAGGACACAGCCATGTACTACTGCGCTG CCGATGCCGACTGCACTATCGCCGCCATGACTACTAATCCTCTGGGCCAA GGCACACAAGTGACTGTCTCGTCTGCTAGCCACCATCACCATCACCAC |
| **307** | DNA Sequence Encoding SEQ ID NO: 209 | CAAGTGCAGCTGCAAGAGTCCGGAGGAGGCAGCGTCCAAGCCGGAGGCT CTCTGAGGCTGAGCTGTGCTGCCAGCGGCTACACTTACAGCATGTACTGC ATGGGCTGGTTCAGACAAGCCCCCGGCAAGGAAAGAGAGGGCGTGGCCC AGATCAATAGCGATGGCAGCACAAGCTACGCCGACAGCGTGAAGGGAAG GTTCACTATCTCCAAGGACAACGCCAAGAACACTCTGTATCTGCAGATGA ACTCTCTGAAGCCAGAGGACACTGCCATGTACTACTGCGCTGCCGATTCT AGGGTGTACGGCGGCAGCTGGTATGAGAGGCTCTGCGGCCCTTACACATA CGAGTACAACTACTGGGGCCAAGGCACACAAGTGACTGTCTCGAGCGGC GGAGGATCCCAAGTGCAGCTGCAAGAGTCCGGCGGAGGCAGCGTCCAAG CCGGAGGATCTCTGAGGCTGAGCTGTACAGTGAGCAGATACACTGCCAGC GTGAACTACATGGGCTGGTTCAGACAAGCCCCCGGCAAAGAGAGAGAGG GCGTGGCCACAATCTTCACTGGCGCCGGCACAACATACTACGCCAACTCC GTCAAGGGAAGGTTCACAATCTCTAGGGACAACGCCAAGAACACTGCCT ATCTGCAGATGAACTCCCTCAAGCCAGAGGACACTGCCATCTACTACTGC GCCGTGGATTTCAGAGGCGGACTGCTGTATAGGCCAGCCTACGAGTACAC TTATAGGGGCCAAGGCACACAAGTGACAGTCTCGTCTGCTAGCCACCATC ACCATCACCAC |
| **308** | DNA Sequence Encoding SEQ ID NO: 210 | CAAGTGCAGCTGCAAGAGTCCGGAGGAGGCAGCGTCCAAGCCGGAGGCT CTCTGAGGCTGAGCTGTGCTGCCAGCGGCTACACTTACAGCATGTACTGC ATGGGCTGGTTCAGACAAGCCCCCGGCAAGGAAAGAGAGGGCGTGGCCC AGATCAATAGCGATGGCAGCACAAGCTACGCCGACAGCGTGAAGGGAAG GTTCACTATCTCCAAGGACAACGCCAAGAACACTCTGTATCTGCAGATGA ACTCTCTGAAGCCAGAGGACACTGCCATGTACTACTGCGCTGCCGATTCT AGGGTGTACGGCGGCAGCTGGTATGAGAGGCTCTGCGGCCCTTACACATA CGAGTACAACTACTGGGGCCAAGGCACACAAGTGACTGTCTCGAGCGGC GGAGGATCCCAAGTGCAGCTGCAAGAGAGCGGAGGAGGCAGCGTCGAAG CTGGAGGATCTCTGAGGCTGAGCTGTGCTGCCAGCGGCTACACTCACAGC AGCTACTGTATGGGCTGGTTCAGACAAGCCCCCGGCAAGGAGAGGGAAG GCGTGGCTGCCATCGACGTGGATGGCAGCACTACTTACGCCGACAGCGTG AAGGGAAGGTTCACTATCAGCAAGGACAACGCCAAGAACACACTCTATC TGCAGATGAACAGCCTCAAGCCAGAGGACACTGGCATGTACTACTGCGCC GCCGAGTTCGCCGATTGCAGCAGCAACTACTTTCTGCCTCCCGGCGCCGT CAGATATTGGGGCCAAGGCACTCAAGTGACAGTCTCGTCTGCTAGCCACC ATCACCATCACCAC |
| **309** | DNA Sequence Encoding SEQ ID NO: 211 | CAAGTGCAGCTGCAAGAGTCCGGAGGAGGCAGCGTCCAAGCCGGAGGCT CTCTGAGGCTGAGCTGTGCTGCCAGCGGCTACACTTACAGCATGTACTGC ATGGGCTGGTTCAGACAAGCCCCCGGCAAGGAAAGAGAGGGCGTGGCCC AGATCAATAGCGATGGCAGCACAAGCTACGCCGACAGCGTGAAGGGAAG GTTCACTATCTCCAAGGACAACGCCAAGAACACTCTGTATCTGCAGATGA ACTCTCTGAAGCCAGAGGACACTGCCATGTACTACTGCGCTGCCGATTCT AGGGTGTACGGCGGCAGCTGGTATGAGAGGCTCTGCGGCCCTTACACATA CGAGTACAACTACTGGGGCCAAGGCACACAAGTGACTGTCTCGAGCGGC GGAGGATCCCAAGTGCAGCTGCAAGAGAGCGGAGGAGGAAGCGTCCAAG CCGGAGGATCTCTGAGACTGAGCTGCGCCGCTAGTGGCTACTCCTACAGC AGCTACTGCATGGGCTGGTTTAGGCAAGCCCCCGGCAAGGAGAGAGAAG GCGTGGCCACTATCGACAGCGACGGCATGACAAGGTACGCCGACAGCGT GAAGGGAAGGTTCACAATCAGCAAGGACAACGCCAAGAACACACTGTAT CTGCAGATGAACTCTCTGAAGCCAGAGGACACTGCCATGTACTACTGTGC CGCTCCTCTGTACGACTGTGATAGCGGCGCTGTGGGCAGAAATCCACCTT ATTGGGGCCAAGGCACTCAAGTGACAGTCTCGTCTGCTAGCCACCATCAC CATCACCAC |
| **310** | DNA Sequence Encoding SEQ ID NO: 212 | CAAGTGCAGCTGCAAGAGTCCGGAGGAGGCAGCGTCCAAGCCGGAGGCT CTCTGAGGCTGAGCTGTGCTGCCAGCGGCTACACTTACAGCATGTACTGC ATGGGCTGGTTCAGACAAGCCCCCGGCAAGGAAAGAGAGGGCGTGGCCC AGATCAATAGCGATGGCAGCACAAGCTACGCCGACAGCGTGAAGGGAAG GTTCACTATCTCCAAGGACAACGCCAAGAACACTCTGTATCTGCAGATGA ACTCTCTGAAGCCAGAGGACACTGCCATGTACTACTGCGCTGCCGATTCT AGGGTGTACGGCGGCAGCTGGTATGAGAGGCTCTGCGGCCCTTACACATA CGAGTACAACTACTGGGGCCAAGGCACACAAGTGACTGTCTCGAGCGGC GGAGGATCCCAAGTGCAGCTGCAAGAGAGCGGAGGCGGCAGCGTGCAGA CTGGAGGCTCTCTGAGACTGAGCTGTGCTGCCAGCGGCTACACTTATCTG AGGGGCTGTATGGGCTGGTTTAGGCAAGCCCCCGGCAAGGAGAGAGAGG GCGTGGCCGTCATGGATGTGGTGGGCGATAGGAGAAGCTACATCGACAG CGTGAAGGGAAGGTTCACAATCTCTAGGGACAATGCCGCCAACAGCGTCT ATCTGCAGATGGACAATCTGAAGCCAGAGGACACAGCCATGTACTACTGC ACTGCCGGCCCTAACTGTGTGGGCTGGAGAAGCGGACTGGATTACTGGGG CCAAGGCACACAAGTGACAGTCTCGTCTGCTAGCCACCATCACCATCACC AC |
| **311** | DNA Sequence Encoding SEQ ID NO: 213 | CAAGTGCAGCTGCAAGAGAGCGGCGGAGGAAGCGTGCAAGCCGGAGGAT CTCTGAGACTGAGCTGCGCTGTGAGCGGCTACGCCTACTCCACATACTGC ATGGGCTGGTTTAGGCAAGCCCCCGGCAAAGAGAGAGAGGGCGTGGCTG CTATCGATAGCGGCGGCAGCACAAGCTACGCCGATAGCGTGAAGGGAAG GTTCACAATCAGCAAGGACAACGCCAAGAACACACTGTATCTGAGGATG AACTCTCTGAAGCCAGAGGACACAGCCATGTACTACTGTGCTGCTGTGCC TCCTCCTCCAGATGGCGGCAGCTGTCTGTTTCTGGGACCAGAGATCAAGG TCAGCAAGGCCGATTTTAGGTACTGGGGCCAAGGCACACAAGTGACAGTC TCGAGCGGCGGAGGATCCCAAGTGCAGCTGCAAGAGAGCGGCGGAGGAA GCGTGCAAGCTGGAGGCTCTCTGAGGCTGAGCTGTGCTGCCAGCGGCTAC ACTTATAGCAGCGGCTGTATGGGCTGGTTCAGACAAGCCCCCGGCAAGGA AAGGGAAGCCGTGGCCGCCATCAATTCCGATGGCAGCACAAGCTACGCC GACAGCGTGAAGGGAAGGTTCACAATCAGCAAGGACAACGCCAAGAACA CACTCTATCTGCAGATGAACTCTCTGAAGCCAGAGGACACAGCCATGTAC TACTGCGCCGCTGAGCCTTACTGTAGCGGCGGCTACCCAAGATGGAGCGT CGCTGAGTTCGGCTACTGGGGCCAAGGCACACAAGTGACTGTCTCGTCTG CTAGCCACCATCACCATCACCAC |
| **312** | DNA Sequence Encoding SEQ ID NO: 214 | CAAGTGCAGCTGCAAGAGAGCGGCGGAGGAAGCGTGCAAGCCGGAGGAT CTCTGAGACTGAGCTGCGCTGTGAGCGGCTACGCCTACTCCACATACTGC ATGGGCTGGTTTAGGCAAGCCCCCGGCAAAGAGAGAGAGGGCGTGGCTG CTATCGATAGCGGCGGCAGCACAAGCTACGCCGATAGCGTGAAGGGAAG GTTCACAATCAGCAAGGACAACGCCAAGAACACACTGTATCTGAGGATG AACTCTCTGAAGCCAGAGGACACAGCCATGTACTACTGTGCTGCTGTGCC TCCTCCTCCAGATGGCGGCAGCTGTCTGTTTCTGGGACCAGAGATCAAGG TCAGCAAGGCCGATTTTAGGTACTGGGGCCAAGGCACACAAGTGACAGTC TCGAGCGGCGGAGGATCCCAAGTGCAGCTGCAAGAGAGCGGAGGAGGCA GCGTCCAAGCCGGAGGCTCTCTGAGGCTGAGCTGTGCTGCCAGCGGCTAC ACTTACAGCAGCTACTGCATGGGCTGGTTCAGACAAGCCCCCGGCAAGGA GAGAGAGGGCGTGGCTCACATCGACAGCGACGGCTCCACAAGCTACGCC GATAGCGTGAAGGGAAGGTTCACAATCTCCAAGGACAACGCCAAGAACA CTCTGTACCTCCAGATGAACTCTCTGAAGCCAGAGGACACTGCCATGTAC TACTGTGCCGCCGATCCAATTCCCGGCCCCGGCTACTGCGATGGCGGCCC TAACAAGTACTGGGGCCAAGGCACACAAGTGACTGTCTCGTCTGCTAGCC ACCATCACCATCACCAC |
| **313** | DNA Sequence Encoding SEQ ID NO: 215 | CAAGTGCAGCTGCAAGAGAGCGGCGGAGGAAGCGTGCAAGCCGGAGGAT CTCTGAGACTGAGCTGCGCTGTGAGCGGCTACGCCTACTCCACATACTGC ATGGGCTGGTTTAGGCAAGCCCCCGGCAAAGAGAGAGAGGGCGTGGCTG CTATCGATAGCGGCGGCAGCACAAGCTACGCCGATAGCGTGAAGGGAAG GTTCACAATCAGCAAGGACAACGCCAAGAACACACTGTATCTGAGGATG AACTCTCTGAAGCCAGAGGACACAGCCATGTACTACTGTGCTGCTGTGCC TCCTCCTCCAGATGGCGGCAGCTGTCTGTTTCTGGGACCAGAGATCAAGG TCAGCAAGGCCGATTTTAGGTACTGGGGCCAAGGCACACAAGTGACAGTC TCGAGCGGCGGAGGATCCCAAGTGCAGCTGCAAGAGTCCGGAGGAGGAA GCGTGCAAGCCGGCGGATCTCTGAGACTGAGCTGTGCCGCCTCTAGGTAC ACTTACAACAGCTACTGCATGGGCTGGTTCAGACAAGCCCCCGGCAAGGA AAGAGAGGGCGTGGCCACTATCGATAGCGACGGCATGACTAGGTACGCT GATAGCGTCAAGGGAAGGTTCACAATCTCCAAGGACAATGCTAAGAACA CTCTGTACCTCCAGATGAACTCTCTGAAGCCAGAGGACACAGCCATGTAC TACTGCGCTGCCGATGCCGACTGCACTATCGCCGCCATGACTACTAATCCT CTGGGCCAAGGCACACAAGTGACTGTCTCGTCTGCTAGCCACCATCACCA TCACCAC |
| **314** | DNA Sequence Encoding SEQ ID NO: 216 | CAAGTGCAGCTGCAAGAGAGCGGCGGAGGAAGCGTGCAAGCCGGAGGAT CTCTGAGACTGAGCTGCGCTGTGAGCGGCTACGCCTACTCCACATACTGC ATGGGCTGGTTTAGGCAAGCCCCCGGCAAAGAGAGAGAGGGCGTGGCTG CTATCGATAGCGGCGGCAGCACAAGCTACGCCGATAGCGTGAAGGGAAG GTTCACAATCAGCAAGGACAACGCCAAGAACACACTGTATCTGAGGATG AACTCTCTGAAGCCAGAGGACACAGCCATGTACTACTGTGCTGCTGTGCC TCCTCCTCCAGATGGCGGCAGCTGTCTGTTTCTGGGACCAGAGATCAAGG TCAGCAAGGCCGATTTTAGGTACTGGGGCCAAGGCACACAAGTGACAGTC TCGAGCGGCGGAGGATCCCAAGTGCAGCTGCAAGAGTCCGGCGGAGGCA GCGTCCAAGCCGGAGGATCTCTGAGGCTGAGCTGTACAGTGAGCAGATAC ACTGCCAGCGTGAACTACATGGGCTGGTTCAGACAAGCCCCCGGCAAAG AGAGAGAGGGCGTGGCCACAATCTTCACTGGCGCCGGCACAACATACTA CGCCAACTCCGTCAAGGGAAGGTTCACAATCTCTAGGGACAACGCCAAG AACACTGCCTATCTGCAGATGAACTCCCTCAAGCCAGAGGACACTGCCAT CTACTACTGCGCCGTGGATTTCAGAGGCGGACTGCTGTATAGGCCAGCCT ACGAGTACACTTATAGGGGCCAAGGCACACAAGTGACAGTCTCGTCTGCT AGCCACCATCACCATCACCAC |
| **315** | DNA Sequence Encoding SEQ ID NO: 217 | CAAGTGCAGCTGCAAGAGAGCGGCGGAGGAAGCGTGCAAGCCGGAGGAT CTCTGAGACTGAGCTGCGCTGTGAGCGGCTACGCCTACTCCACATACTGC ATGGGCTGGTTTAGGCAAGCCCCCGGCAAAGAGAGAGAGGGCGTGGCTG CTATCGATAGCGGCGGCAGCACAAGCTACGCCGATAGCGTGAAGGGAAG GTTCACAATCAGCAAGGACAACGCCAAGAACACACTGTATCTGAGGATG AACTCTCTGAAGCCAGAGGACACAGCCATGTACTACTGTGCTGCTGTGCC TCCTCCTCCAGATGGCGGCAGCTGTCTGTTTCTGGGACCAGAGATCAAGG TCAGCAAGGCCGATTTTAGGTACTGGGGCCAAGGCACACAAGTGACAGTC TCGAGCGGCGGAGGATCCCAAGTGCAGCTGCAAGAGAGCGGAGGAGGCA GCGTCGAAGCTGGAGGATCTCTGAGGCTGAGCTGTGCTGCCAGCGGCTAC ACTCACAGCAGCTACTGTATGGGCTGGTTCAGACAAGCCCCCGGCAAGGA GAGGGAAGGCGTGGCTGCCATCGACGTGGATGGCAGCACTACTTACGCC GACAGCGTGAAGGGAAGGTTCACTATCAGCAAGGACAACGCCAAGAACA CACTCTATCTGCAGATGAACAGCCTCAAGCCAGAGGACACTGGCATGTAC TACTGCGCCGCCGAGTTCGCCGATTGCAGCAGCAACTACTTTCTGCCTCCC GGCGCCGTCAGATATTGGGGCCAAGGCACTCAAGTGACAGTCTCGTCTGC TAGCCACCATCACCATCACCAC |
| **316** | DNA Sequence Encoding SEQ ID NO: 218 | CAAGTGCAGCTGCAAGAGAGCGGCGGAGGAAGCGTGCAAGCCGGAGGAT CTCTGAGACTGAGCTGCGCTGTGAGCGGCTACGCCTACTCCACATACTGC ATGGGCTGGTTTAGGCAAGCCCCCGGCAAAGAGAGAGAGGGCGTGGCTG CTATCGATAGCGGCGGCAGCACAAGCTACGCCGATAGCGTGAAGGGAAG GTTCACAATCAGCAAGGACAACGCCAAGAACACACTGTATCTGAGGATG AACTCTCTGAAGCCAGAGGACACAGCCATGTACTACTGTGCTGCTGTGCC TCCTCCTCCAGATGGCGGCAGCTGTCTGTTTCTGGGACCAGAGATCAAGG TCAGCAAGGCCGATTTTAGGTACTGGGGCCAAGGCACACAAGTGACAGTC TCGAGCGGCGGAGGATCCCAAGTGCAGCTGCAAGAGAGCGGAGGAGGAA GCGTCCAAGCCGGAGGATCTCTGAGACTGAGCTGCGCCGCTAGTGGCTAC TCCTACAGCAGCTACTGCATGGGCTGGTTTAGGCAAGCCCCCGGCAAGGA GAGAGAAGGCGTGGCCACTATCGACAGCGACGGCATGACAAGGTACGCC GACAGCGTGAAGGGAAGGTTCACAATCAGCAAGGACAACGCCAAGAACA CACTGTATCTGCAGATGAACTCTCTGAAGCCAGAGGACACTGCCATGTAC TACTGTGCCGCTCCTCTGTACGACTGTGATAGCGGCGCTGTGGGCAGAAA TCCACCTTATTGGGGCCAAGGCACTCAAGTGACAGTCTCGTCTGCTAGCC ACCATCACCATCACCAC |
| **317** | DNA Sequence Encoding SEQ ID NO: 219 | CAAGTGCAGCTGCAAGAGAGCGGCGGAGGAAGCGTGCAAGCCGGAGGAT CTCTGAGACTGAGCTGCGCTGTGAGCGGCTACGCCTACTCCACATACTGC ATGGGCTGGTTTAGGCAAGCCCCCGGCAAAGAGAGAGAGGGCGTGGCTG CTATCGATAGCGGCGGCAGCACAAGCTACGCCGATAGCGTGAAGGGAAG GTTCACAATCAGCAAGGACAACGCCAAGAACACACTGTATCTGAGGATG AACTCTCTGAAGCCAGAGGACACAGCCATGTACTACTGTGCTGCTGTGCC TCCTCCTCCAGATGGCGGCAGCTGTCTGTTTCTGGGACCAGAGATCAAGG TCAGCAAGGCCGATTTTAGGTACTGGGGCCAAGGCACACAAGTGACAGTC TCGAGCGGCGGAGGATCCCAAGTGCAGCTGCAAGAGAGCGGAGGCGGCA GCGTGCAGACTGGAGGCTCTCTGAGACTGAGCTGTGCTGCCAGCGGCTAC ACTTATCTGAGGGGCTGTATGGGCTGGTTTAGGCAAGCCCCCGGCAAGGA GAGAGAGGGCGTGGCCGTCATGGATGTGGTGGGCGATAGGAGAAGCTAC ATCGACAGCGTGAAGGGAAGGTTCACAATCTCTAGGGACAATGCCGCCA ACAGCGTCTATCTGCAGATGGACAATCTGAAGCCAGAGGACACAGCCAT GTACTACTGCACTGCCGGCCCTAACTGTGTGGGCTGGAGAAGCGGACTGG ATTACTGGGGCCAAGGCACACAAGTGACAGTCTCGTCTGCTAGCCACCAT CACCATCACCAC |
| **318** | DNA Sequence Encoding SEQ ID NO: 220 | CAAGTGCAGCTGCAAGAGAGCGGAGGAGGAAGCGTGCAAGCCGGAGGCT CTCTGAGGCTGAGCTGTACAGTGTCCGGCTACACTTACAGCTCCAATTGC ATGGGCTGGTTTAGGCAAGCCCCCGGCAAGGAAAGAGAGGGCGTGGCCA CTATCTACACTGGCGGCGGCAACACATACTACGCCGATAGCGTGAAGGGA AGGTTCACTATCAGCCAAGATAACGCCAAGAACACAGTGTATCTGCAGAT GAACAATCTGAAGCCAGAGGACACTGCCATGTACTACTGTGCTGCTGAGC CACTGTCTAGGGTGTACGGCGGCAGCTGCCCAACTCCTACATTCGACTAC TGGGGCCAAGGCACACAAGTGACTGTCTCGAGCGGCGGAGGATCCCAAG TGCAGCTGCAAGAGAGCGGCGGAGGAAGCGTGCAAGCTGGAGGCTCTCT GAGGCTGAGCTGTGCTGCCAGCGGCTACACTTATAGCAGCGGCTGTATGG GCTGGTTCAGACAAGCCCCCGGCAAGGAAAGGGAAGCCGTGGCCGCCAT CAATTCCGATGGCAGCACAAGCTACGCCGACAGCGTGAAGGGAAGGTTC ACAATCAGCAAGGACAACGCCAAGAACACACTCTATCTGCAGATGAACT CTCTGAAGCCAGAGGACACAGCCATGTACTACTGCGCCGCTGAGCCTTAC TGTAGCGGCGGCTACCCAAGATGGAGCGTCGCTGAGTTCGGCTACTGGGG CCAAGGCACACAAGTGACTGTCTCGTCTGCTAGCCACCATCACCATCACC AC |
| **319** | DNA Sequence Encoding SEQ ID NO: 221 | CAAGTGCAGCTGCAAGAGAGCGGAGGAGGAAGCGTGCAAGCCGGAGGCT CTCTGAGGCTGAGCTGTACAGTGTCCGGCTACACTTACAGCTCCAATTGC ATGGGCTGGTTTAGGCAAGCCCCCGGCAAGGAAAGAGAGGGCGTGGCCA CTATCTACACTGGCGGCGGCAACACATACTACGCCGATAGCGTGAAGGGA AGGTTCACTATCAGCCAAGATAACGCCAAGAACACAGTGTATCTGCAGAT GAACAATCTGAAGCCAGAGGACACTGCCATGTACTACTGTGCTGCTGAGC CACTGTCTAGGGTGTACGGCGGCAGCTGCCCAACTCCTACATTCGACTAC TGGGGCCAAGGCACACAAGTGACTGTCTCGAGCGGCGGAGGATCCCAAG TGCAGCTGCAAGAGAGCGGAGGAGGCAGCGTCCAAGCCGGAGGCTCTCT GAGGCTGAGCTGTGCTGCCAGCGGCTACACTTACAGCAGCTACTGCATGG GCTGGTTCAGACAAGCCCCCGGCAAGGAGAGAGAGGGCGTGGCTCACAT CGACAGCGACGGCTCCACAAGCTACGCCGATAGCGTGAAGGGAAGGTTC ACAATCTCCAAGGACAACGCCAAGAACACTCTGTACCTCCAGATGAACTC TCTGAAGCCAGAGGACACTGCCATGTACTACTGTGCCGCCGATCCAATTC CCGGCCCCGGCTACTGCGATGGCGGCCCTAACAAGTACTGGGGCCAAGGC ACACAAGTGACTGTCTCGTCTGCTAGCCACCATCACCATCACCAC |
| **320** | DNA Sequence Encoding SEQ ID NO: 222 | CAAGTGCAGCTGCAAGAGAGCGGAGGAGGAAGCGTGCAAGCCGGAGGCT CTCTGAGGCTGAGCTGTACAGTGTCCGGCTACACTTACAGCTCCAATTGC ATGGGCTGGTTTAGGCAAGCCCCCGGCAAGGAAAGAGAGGGCGTGGCCA CTATCTACACTGGCGGCGGCAACACATACTACGCCGATAGCGTGAAGGGA AGGTTCACTATCAGCCAAGATAACGCCAAGAACACAGTGTATCTGCAGAT GAACAATCTGAAGCCAGAGGACACTGCCATGTACTACTGTGCTGCTGAGC CACTGTCTAGGGTGTACGGCGGCAGCTGCCCAACTCCTACATTCGACTAC TGGGGCCAAGGCACACAAGTGACTGTCTCGAGCGGCGGAGGATCCCAAG TGCAGCTGCAAGAGTCCGGAGGAGGAAGCGTGCAAGCCGGCGGATCTCT GAGACTGAGCTGTGCCGCCTCTAGGTACACTTACAACAGCTACTGCATGG GCTGGTTCAGACAAGCCCCCGGCAAGGAAAGAGAGGGCGTGGCCACTAT CGATAGCGACGGCATGACTAGGTACGCTGATAGCGTCAAGGGAAGGTTC ACAATCTCCAAGGACAATGCTAAGAACACTCTGTACCTCCAGATGAACTC TCTGAAGCCAGAGGACACAGCCATGTACTACTGCGCTGCCGATGCCGACT GCACTATCGCCGCCATGACTACTAATCCTCTGGGCCAAGGCACACAAGTG ACTGTCTCGTCTGCTAGCCACCATCACCATCACCAC |
| **321** | DNA Sequence Encoding SEQ ID NO: 223 | CAAGTGCAGCTGCAAGAGAGCGGAGGAGGAAGCGTGCAAGCCGGAGGCT CTCTGAGGCTGAGCTGTACAGTGTCCGGCTACACTTACAGCTCCAATTGC ATGGGCTGGTTTAGGCAAGCCCCCGGCAAGGAAAGAGAGGGCGTGGCCA CTATCTACACTGGCGGCGGCAACACATACTACGCCGATAGCGTGAAGGGA AGGTTCACTATCAGCCAAGATAACGCCAAGAACACAGTGTATCTGCAGAT GAACAATCTGAAGCCAGAGGACACTGCCATGTACTACTGTGCTGCTGAGC CACTGTCTAGGGTGTACGGCGGCAGCTGCCCAACTCCTACATTCGACTAC TGGGGCCAAGGCACACAAGTGACTGTCTCGAGCGGCGGAGGATCCCAAG TGCAGCTGCAAGAGTCCGGCGGAGGCAGCGTCCAAGCCGGAGGATCTCT GAGGCTGAGCTGTACAGTGAGCAGATACACTGCCAGCGTGAACTACATG GGCTGGTTCAGACAAGCCCCCGGCAAAGAGAGAGAGGGCGTGGCCACAA TCTTCACTGGCGCCGGCACAACATACTACGCCAACTCCGTCAAGGGAAGG TTCACAATCTCTAGGGACAACGCCAAGAACACTGCCTATCTGCAGATGAA CTCCCTCAAGCCAGAGGACACTGCCATCTACTACTGCGCCGTGGATTTCA GAGGCGGACTGCTGTATAGGCCAGCCTACGAGTACACTTATAGGGGCCAA GGCACACAAGTGACAGTCTCGTCTGCTAGCCACCATCACCATCACCAC |
| **322** | DNA Sequence Encoding SEQ ID NO: 224 | CAAGTGCAGCTGCAAGAGAGCGGAGGAGGAAGCGTGCAAGCCGGAGGCT CTCTGAGGCTGAGCTGTACAGTGTCCGGCTACACTTACAGCTCCAATTGC ATGGGCTGGTTTAGGCAAGCCCCCGGCAAGGAAAGAGAGGGCGTGGCCA CTATCTACACTGGCGGCGGCAACACATACTACGCCGATAGCGTGAAGGGA AGGTTCACTATCAGCCAAGATAACGCCAAGAACACAGTGTATCTGCAGAT GAACAATCTGAAGCCAGAGGACACTGCCATGTACTACTGTGCTGCTGAGC CACTGTCTAGGGTGTACGGCGGCAGCTGCCCAACTCCTACATTCGACTAC TGGGGCCAAGGCACACAAGTGACTGTCTCGAGCGGCGGAGGATCCCAAG TGCAGCTGCAAGAGAGCGGAGGAGGCAGCGTCGAAGCTGGAGGATCTCT GAGGCTGAGCTGTGCTGCCAGCGGCTACACTCACAGCAGCTACTGTATGG GCTGGTTCAGACAAGCCCCCGGCAAGGAGAGGGAAGGCGTGGCTGCCAT CGACGTGGATGGCAGCACTACTTACGCCGACAGCGTGAAGGGAAGGTTC ACTATCAGCAAGGACAACGCCAAGAACACACTCTATCTGCAGATGAACA GCCTCAAGCCAGAGGACACTGGCATGTACTACTGCGCCGCCGAGTTCGCC GATTGCAGCAGCAACTACTTTCTGCCTCCCGGCGCCGTCAGATATTGGGG CCAAGGCACTCAAGTGACAGTCTCGTCTGCTAGCCACCATCACCATCACC AC |
| **323** | DNA Sequence Encoding SEQ ID NO: 225 | CAAGTGCAGCTGCAAGAGAGCGGAGGAGGAAGCGTGCAAGCCGGAGGCT CTCTGAGGCTGAGCTGTACAGTGTCCGGCTACACTTACAGCTCCAATTGC ATGGGCTGGTTTAGGCAAGCCCCCGGCAAGGAAAGAGAGGGCGTGGCCA CTATCTACACTGGCGGCGGCAACACATACTACGCCGATAGCGTGAAGGGA AGGTTCACTATCAGCCAAGATAACGCCAAGAACACAGTGTATCTGCAGAT GAACAATCTGAAGCCAGAGGACACTGCCATGTACTACTGTGCTGCTGAGC CACTGTCTAGGGTGTACGGCGGCAGCTGCCCAACTCCTACATTCGACTAC TGGGGCCAAGGCACACAAGTGACTGTCTCGAGCGGCGGAGGATCCCAAG TGCAGCTGCAAGAGAGCGGAGGAGGAAGCGTCCAAGCCGGAGGATCTCT GAGACTGAGCTGCGCCGCTAGTGGCTACTCCTACAGCAGCTACTGCATGG GCTGGTTTAGGCAAGCCCCCGGCAAGGAGAGAGAAGGCGTGGCCACTAT CGACAGCGACGGCATGACAAGGTACGCCGACAGCGTGAAGGGAAGGTTC ACAATCAGCAAGGACAACGCCAAGAACACACTGTATCTGCAGATGAACT CTCTGAAGCCAGAGGACACTGCCATGTACTACTGTGCCGCTCCTCTGTAC GACTGTGATAGCGGCGCTGTGGGCAGAAATCCACCTTATTGGGGCCAAGG CACTCAAGTGACAGTCTCGTCTGCTAGCCACCATCACCATCACCAC |
| **324** | DNA Sequence Encoding SEQ ID NO: 226 | CAAGTGCAGCTGCAAGAGAGCGGAGGAGGAAGCGTGCAAGCCGGAGGCT CTCTGAGGCTGAGCTGTACAGTGTCCGGCTACACTTACAGCTCCAATTGC ATGGGCTGGTTTAGGCAAGCCCCCGGCAAGGAAAGAGAGGGCGTGGCCA CTATCTACACTGGCGGCGGCAACACATACTACGCCGATAGCGTGAAGGGA AGGTTCACTATCAGCCAAGATAACGCCAAGAACACAGTGTATCTGCAGAT GAACAATCTGAAGCCAGAGGACACTGCCATGTACTACTGTGCTGCTGAGC CACTGTCTAGGGTGTACGGCGGCAGCTGCCCAACTCCTACATTCGACTAC TGGGGCCAAGGCACACAAGTGACTGTCTCGAGCGGCGGAGGATCCCAAG TGCAGCTGCAAGAGAGCGGAGGCGGCAGCGTGCAGACTGGAGGCTCTCT GAGACTGAGCTGTGCTGCCAGCGGCTACACTTATCTGAGGGGCTGTATGG GCTGGTTTAGGCAAGCCCCCGGCAAGGAGAGAGAGGGCGTGGCCGTCAT GGATGTGGTGGGCGATAGGAGAAGCTACATCGACAGCGTGAAGGGAAGG TTCACAATCTCTAGGGACAATGCCGCCAACAGCGTCTATCTGCAGATGGA CAATCTGAAGCCAGAGGACACAGCCATGTACTACTGCACTGCCGGCCCTA ACTGTGTGGGCTGGAGAAGCGGACTGGATTACTGGGGCCAAGGCACACA AGTGACAGTCTCGTCTGCTAGCCACCATCACCATCACCAC |
| **325** | DNA Sequence Encoding SEQ ID NO: 227 | CAAGTGCAGCTGCAAGAGAGCGGAGGAGGAAGCGTCCAAGCCGGAGGCT CTCTGAGGCTGAGCTGTGGAGCCAGCGGCTACACTTACAGCAGCTACTGT ATGGGCTGGTTTAGGCAAGTGCCCGGCAAGGAGAGAGAGGGCGTGGCCG TGATCGATTCCGATGGCAGCACAAGCTACGCTGACAGCGTGAAGGGAAG GTTCACAATCAGCAAGGACAACGGCAAGAACACACTCTATCTGCAGATG AACAGCCTCAAGCCAGAGGACACAGCCATGTACTACTGCGCCGCTGATCT GGGCCACTATAGGCCTCCTTGTGGCGTGCTGTATCTGGGCATGGATTACT GGGGCAAGGGCACACAAGTGACAGTCTCGAGCGGCGGAGGATCCCAAGT GCAGCTGCAAGAGAGCGGCGGAGGAAGCGTGCAAGCTGGAGGCTCTCTG AGGCTGAGCTGTGCTGCCAGCGGCTACACTTATAGCAGCGGCTGTATGGG CTGGTTCAGACAAGCCCCCGGCAAGGAAAGGGAAGCCGTGGCCGCCATC AATTCCGATGGCAGCACAAGCTACGCCGACAGCGTGAAGGGAAGGTTCA CAATCAGCAAGGACAACGCCAAGAACACACTCTATCTGCAGATGAACTCT CTGAAGCCAGAGGACACAGCCATGTACTACTGCGCCGCTGAGCCTTACTG TAGCGGCGGCTACCCAAGATGGAGCGTCGCTGAGTTCGGCTACTGGGGCC AAGGCACACAAGTGACTGTCTCGTCTGCTAGCCACCATCACCATCACCAC |
| **326** | DNA Sequence Encoding SEQ ID NO: 228 | CAAGTGCAGCTGCAAGAGAGCGGAGGAGGAAGCGTCCAAGCCGGAGGCT CTCTGAGGCTGAGCTGTGGAGCCAGCGGCTACACTTACAGCAGCTACTGT ATGGGCTGGTTTAGGCAAGTGCCCGGCAAGGAGAGAGAGGGCGTGGCCG TGATCGATTCCGATGGCAGCACAAGCTACGCTGACAGCGTGAAGGGAAG GTTCACAATCAGCAAGGACAACGGCAAGAACACACTCTATCTGCAGATG AACAGCCTCAAGCCAGAGGACACAGCCATGTACTACTGCGCCGCTGATCT GGGCCACTATAGGCCTCCTTGTGGCGTGCTGTATCTGGGCATGGATTACT GGGGCAAGGGCACACAAGTGACAGTCTCGAGCGGCGGAGGATCCCAAGT GCAGCTGCAAGAGAGCGGAGGAGGCAGCGTCCAAGCCGGAGGCTCTCTG AGGCTGAGCTGTGCTGCCAGCGGCTACACTTACAGCAGCTACTGCATGGG CTGGTTCAGACAAGCCCCCGGCAAGGAGAGAGAGGGCGTGGCTCACATC GACAGCGACGGCTCCACAAGCTACGCCGATAGCGTGAAGGGAAGGTTCA CAATCTCCAAGGACAACGCCAAGAACACTCTGTACCTCCAGATGAACTCT CTGAAGCCAGAGGACACTGCCATGTACTACTGTGCCGCCGATCCAATTCC CGGCCCCGGCTACTGCGATGGCGGCCCTAACAAGTACTGGGGCCAAGGC ACACAAGTGACTGTCTCGTCTGCTAGCCACCATCACCATCACCAC |
| **327** | DNA Sequence Encoding SEQ ID NO: 229 | CAAGTGCAGCTGCAAGAGAGCGGAGGAGGAAGCGTCCAAGCCGGAGGCT CTCTGAGGCTGAGCTGTGGAGCCAGCGGCTACACTTACAGCAGCTACTGT ATGGGCTGGTTTAGGCAAGTGCCCGGCAAGGAGAGAGAGGGCGTGGCCG TGATCGATTCCGATGGCAGCACAAGCTACGCTGACAGCGTGAAGGGAAG GTTCACAATCAGCAAGGACAACGGCAAGAACACACTCTATCTGCAGATG AACAGCCTCAAGCCAGAGGACACAGCCATGTACTACTGCGCCGCTGATCT GGGCCACTATAGGCCTCCTTGTGGCGTGCTGTATCTGGGCATGGATTACT GGGGCAAGGGCACACAAGTGACAGTCTCGAGCGGCGGAGGATCCCAAGT GCAGCTGCAAGAGTCCGGAGGAGGAAGCGTGCAAGCCGGCGGATCTCTG AGACTGAGCTGTGCCGCCTCTAGGTACACTTACAACAGCTACTGCATGGG CTGGTTCAGACAAGCCCCCGGCAAGGAAAGAGAGGGCGTGGCCACTATC GATAGCGACGGCATGACTAGGTACGCTGATAGCGTCAAGGGAAGGTTCA CAATCTCCAAGGACAATGCTAAGAACACTCTGTACCTCCAGATGAACTCT CTGAAGCCAGAGGACACAGCCATGTACTACTGCGCTGCCGATGCCGACTG CACTATCGCCGCCATGACTACTAATCCTCTGGGCCAAGGCACACAAGTGA CTGTCTCGTCTGCTAGCCACCATCACCATCACCAC |
| **328** | DNA Sequence Encoding SEQ ID NO: 230 | CAAGTGCAGCTGCAAGAGAGCGGAGGAGGAAGCGTCCAAGCCGGAGGCT CTCTGAGGCTGAGCTGTGGAGCCAGCGGCTACACTTACAGCAGCTACTGT ATGGGCTGGTTTAGGCAAGTGCCCGGCAAGGAGAGAGAGGGCGTGGCCG TGATCGATTCCGATGGCAGCACAAGCTACGCTGACAGCGTGAAGGGAAG GTTCACAATCAGCAAGGACAACGGCAAGAACACACTCTATCTGCAGATG AACAGCCTCAAGCCAGAGGACACAGCCATGTACTACTGCGCCGCTGATCT GGGCCACTATAGGCCTCCTTGTGGCGTGCTGTATCTGGGCATGGATTACT GGGGCAAGGGCACACAAGTGACAGTCTCGAGCGGCGGAGGATCCCAAGT GCAGCTGCAAGAGTCCGGCGGAGGCAGCGTCCAAGCCGGAGGATCTCTG AGGCTGAGCTGTACAGTGAGCAGATACACTGCCAGCGTGAACTACATGG GCTGGTTCAGACAAGCCCCCGGCAAAGAGAGAGAGGGCGTGGCCACAAT CTTCACTGGCGCCGGCACAACATACTACGCCAACTCCGTCAAGGGAAGGT TCACAATCTCTAGGGACAACGCCAAGAACACTGCCTATCTGCAGATGAAC TCCCTCAAGCCAGAGGACACTGCCATCTACTACTGCGCCGTGGATTTCAG AGGCGGACTGCTGTATAGGCCAGCCTACGAGTACACTTATAGGGGCCAAG GCACACAAGTGACAGTCTCGTCTGCTAGCCACCATCACCATCACCAC |
| **329** | DNA Sequence Encoding SEQ ID NO: 231 | CAAGTGCAGCTGCAAGAGAGCGGAGGAGGAAGCGTCCAAGCCGGAGGCT CTCTGAGGCTGAGCTGTGGAGCCAGCGGCTACACTTACAGCAGCTACTGT ATGGGCTGGTTTAGGCAAGTGCCCGGCAAGGAGAGAGAGGGCGTGGCCG TGATCGATTCCGATGGCAGCACAAGCTACGCTGACAGCGTGAAGGGAAG GTTCACAATCAGCAAGGACAACGGCAAGAACACACTCTATCTGCAGATG AACAGCCTCAAGCCAGAGGACACAGCCATGTACTACTGCGCCGCTGATCT GGGCCACTATAGGCCTCCTTGTGGCGTGCTGTATCTGGGCATGGATTACT GGGGCAAGGGCACACAAGTGACAGTCTCGAGCGGCGGAGGATCCCAAGT GCAGCTGCAAGAGAGCGGAGGAGGCAGCGTCGAAGCTGGAGGATCTCTG AGGCTGAGCTGTGCTGCCAGCGGCTACACTCACAGCAGCTACTGTATGGG CTGGTTCAGACAAGCCCCCGGCAAGGAGAGGGAAGGCGTGGCTGCCATC GACGTGGATGGCAGCACTACTTACGCCGACAGCGTGAAGGGAAGGTTCA CTATCAGCAAGGACAACGCCAAGAACACACTCTATCTGCAGATGAACAG CCTCAAGCCAGAGGACACTGGCATGTACTACTGCGCCGCCGAGTTCGCCG ATTGCAGCAGCAACTACTTTCTGCCTCCCGGCGCCGTCAGATATTGGGGC CAAGGCACTCAAGTGACAGTCTCGTCTGCTAGCCACCATCACCATCACCA C |
| **330** | DNA Sequence Encoding SEQ ID NO: 232 | CAAGTGCAGCTGCAAGAGAGCGGAGGAGGAAGCGTCCAAGCCGGAGGCT CTCTGAGGCTGAGCTGTGGAGCCAGCGGCTACACTTACAGCAGCTACTGT ATGGGCTGGTTTAGGCAAGTGCCCGGCAAGGAGAGAGAGGGCGTGGCCG TGATCGATTCCGATGGCAGCACAAGCTACGCTGACAGCGTGAAGGGAAG GTTCACAATCAGCAAGGACAACGGCAAGAACACACTCTATCTGCAGATG AACAGCCTCAAGCCAGAGGACACAGCCATGTACTACTGCGCCGCTGATCT GGGCCACTATAGGCCTCCTTGTGGCGTGCTGTATCTGGGCATGGATTACT GGGGCAAGGGCACACAAGTGACAGTCTCGAGCGGCGGAGGATCCCAAGT GCAGCTGCAAGAGAGCGGAGGAGGAAGCGTCCAAGCCGGAGGATCTCTG AGACTGAGCTGCGCCGCTAGTGGCTACTCCTACAGCAGCTACTGCATGGG CTGGTTTAGGCAAGCCCCCGGCAAGGAGAGAGAAGGCGTGGCCACTATC GACAGCGACGGCATGACAAGGTACGCCGACAGCGTGAAGGGAAGGTTCA CAATCAGCAAGGACAACGCCAAGAACACACTGTATCTGCAGATGAACTCT CTGAAGCCAGAGGACACTGCCATGTACTACTGTGCCGCTCCTCTGTACGA CTGTGATAGCGGCGCTGTGGGCAGAAATCCACCTTATTGGGGCCAAGGCA CTCAAGTGACAGTCTCGTCTGCTAGCCACCATCACCATCACCAC |
| **331** | DNA Sequence Encoding SEQ ID NO: 233 | CAAGTGCAGCTGCAAGAGAGCGGAGGAGGAAGCGTCCAAGCCGGAGGCT CTCTGAGGCTGAGCTGTGGAGCCAGCGGCTACACTTACAGCAGCTACTGT ATGGGCTGGTTTAGGCAAGTGCCCGGCAAGGAGAGAGAGGGCGTGGCCG TGATCGATTCCGATGGCAGCACAAGCTACGCTGACAGCGTGAAGGGAAG GTTCACAATCAGCAAGGACAACGGCAAGAACACACTCTATCTGCAGATG AACAGCCTCAAGCCAGAGGACACAGCCATGTACTACTGCGCCGCTGATCT GGGCCACTATAGGCCTCCTTGTGGCGTGCTGTATCTGGGCATGGATTACT GGGGCAAGGGCACACAAGTGACAGTCTCGAGCGGCGGAGGATCCCAAGT GCAGCTGCAAGAGAGCGGAGGCGGCAGCGTGCAGACTGGAGGCTCTCTG AGACTGAGCTGTGCTGCCAGCGGCTACACTTATCTGAGGGGCTGTATGGG CTGGTTTAGGCAAGCCCCCGGCAAGGAGAGAGAGGGCGTGGCCGTCATG GATGTGGTGGGCGATAGGAGAAGCTACATCGACAGCGTGAAGGGAAGGT TCACAATCTCTAGGGACAATGCCGCCAACAGCGTCTATCTGCAGATGGAC AATCTGAAGCCAGAGGACACAGCCATGTACTACTGCACTGCCGGCCCTAA CTGTGTGGGCTGGAGAAGCGGACTGGATTACTGGGGCCAAGGCACACAA GTGACAGTCTCGTCTGCTAGCCACCATCACCATCACCAC |
| **332** | DNA Sequence Encoding SEQ ID NO: 234 | CAAGTGCAGCTGCAAGAGAGCGGAGGAGGAAGCGTCCAAGCCGGAGGCT CTCTGAGACTGAGCTGTGCCGCCAGCGGCTACTCCAACTGCAGCTACGAC ATGACTTGGTATAGGCAAGCCCCCGGCAAGGAGAGGGAGTTCGTGTCCGC CATCCACAGCGACGGCAGCACTAGATACGCCGACAGCGTGAAGGGAAGG TTCTTCATCAGCCAAGATAACGCCAAGAACACAGTGTATCTGCAGATGAA CTCCCTCAAGCCAGAGGACACTGCCATGTACTACTGCAAGACAGACCCAC TGCACTGCAGAGCCCATGGCGGCAGCTGGTATAGCGTGAGGGCCAACTAC TGGGGCCAAGGCACACAAGTGACAGTCTCGAGCGGCGGAGGATCCCAAG TGCAGCTGCAAGAGAGCGGCGGAGGAAGCGTGCAAGCTGGAGGCTCTCT GAGGCTGAGCTGTGCTGCCAGCGGCTACACTTATAGCAGCGGCTGTATGG GCTGGTTCAGACAAGCCCCCGGCAAGGAAAGGGAAGCCGTGGCCGCCAT CAATTCCGATGGCAGCACAAGCTACGCCGACAGCGTGAAGGGAAGGTTC ACAATCAGCAAGGACAACGCCAAGAACACACTCTATCTGCAGATGAACT CTCTGAAGCCAGAGGACACAGCCATGTACTACTGCGCCGCTGAGCCTTAC TGTAGCGGCGGCTACCCAAGATGGAGCGTCGCTGAGTTCGGCTACTGGGG CCAAGGCACACAAGTGACTGTCTCGTCTGCTAGCCACCATCACCATCACC AC |
| **333** | DNA Sequence Encoding SEQ ID NO: 235 | CAAGTGCAGCTGCAAGAGAGCGGAGGAGGAAGCGTCCAAGCCGGAGGCT CTCTGAGACTGAGCTGTGCCGCCAGCGGCTACTCCAACTGCAGCTACGAC ATGACTTGGTATAGGCAAGCCCCCGGCAAGGAGAGGGAGTTCGTGTCCGC CATCCACAGCGACGGCAGCACTAGATACGCCGACAGCGTGAAGGGAAGG TTCTTCATCAGCCAAGATAACGCCAAGAACACAGTGTATCTGCAGATGAA CTCCCTCAAGCCAGAGGACACTGCCATGTACTACTGCAAGACAGACCCAC TGCACTGCAGAGCCCATGGCGGCAGCTGGTATAGCGTGAGGGCCAACTAC TGGGGCCAAGGCACACAAGTGACAGTCTCGAGCGGCGGAGGATCCCAAG TGCAGCTGCAAGAGAGCGGAGGAGGCAGCGTCCAAGCCGGAGGCTCTCT GAGGCTGAGCTGTGCTGCCAGCGGCTACACTTACAGCAGCTACTGCATGG GCTGGTTCAGACAAGCCCCCGGCAAGGAGAGAGAGGGCGTGGCTCACAT CGACAGCGACGGCTCCACAAGCTACGCCGATAGCGTGAAGGGAAGGTTC ACAATCTCCAAGGACAACGCCAAGAACACTCTGTACCTCCAGATGAACTC TCTGAAGCCAGAGGACACTGCCATGTACTACTGTGCCGCCGATCCAATTC CCGGCCCCGGCTACTGCGATGGCGGCCCTAACAAGTACTGGGGCCAAGGC ACACAAGTGACTGTCTCGTCTGCTAGCCACCATCACCATCACCAC |
| **334** | DNA Sequence Encoding SEQ ID NO: 236 | CAAGTGCAGCTGCAAGAGAGCGGAGGAGGAAGCGTCCAAGCCGGAGGCT CTCTGAGACTGAGCTGTGCCGCCAGCGGCTACTCCAACTGCAGCTACGAC ATGACTTGGTATAGGCAAGCCCCCGGCAAGGAGAGGGAGTTCGTGTCCGC CATCCACAGCGACGGCAGCACTAGATACGCCGACAGCGTGAAGGGAAGG TTCTTCATCAGCCAAGATAACGCCAAGAACACAGTGTATCTGCAGATGAA CTCCCTCAAGCCAGAGGACACTGCCATGTACTACTGCAAGACAGACCCAC TGCACTGCAGAGCCCATGGCGGCAGCTGGTATAGCGTGAGGGCCAACTAC TGGGGCCAAGGCACACAAGTGACAGTCTCGAGCGGCGGAGGATCCCAAG TGCAGCTGCAAGAGTCCGGAGGAGGAAGCGTGCAAGCCGGCGGATCTCT GAGACTGAGCTGTGCCGCCTCTAGGTACACTTACAACAGCTACTGCATGG GCTGGTTCAGACAAGCCCCCGGCAAGGAAAGAGAGGGCGTGGCCACTAT CGATAGCGACGGCATGACTAGGTACGCTGATAGCGTCAAGGGAAGGTTC ACAATCTCCAAGGACAATGCTAAGAACACTCTGTACCTCCAGATGAACTC TCTGAAGCCAGAGGACACAGCCATGTACTACTGCGCTGCCGATGCCGACT GCACTATCGCCGCCATGACTACTAATCCTCTGGGCCAAGGCACACAAGTG ACTGTCTCGTCTGCTAGCCACCATCACCATCACCAC |
| **335** | DNA Sequence Encoding SEQ ID NO: 237 | CAAGTGCAGCTGCAAGAGAGCGGAGGAGGAAGCGTCCAAGCCGGAGGCT CTCTGAGACTGAGCTGTGCCGCCAGCGGCTACTCCAACTGCAGCTACGAC ATGACTTGGTATAGGCAAGCCCCCGGCAAGGAGAGGGAGTTCGTGTCCGC CATCCACAGCGACGGCAGCACTAGATACGCCGACAGCGTGAAGGGAAGG TTCTTCATCAGCCAAGATAACGCCAAGAACACAGTGTATCTGCAGATGAA CTCCCTCAAGCCAGAGGACACTGCCATGTACTACTGCAAGACAGACCCAC TGCACTGCAGAGCCCATGGCGGCAGCTGGTATAGCGTGAGGGCCAACTAC TGGGGCCAAGGCACACAAGTGACAGTCTCGAGCGGCGGAGGATCCCAAG TGCAGCTGCAAGAGTCCGGCGGAGGCAGCGTCCAAGCCGGAGGATCTCT GAGGCTGAGCTGTACAGTGAGCAGATACACTGCCAGCGTGAACTACATG GGCTGGTTCAGACAAGCCCCCGGCAAAGAGAGAGAGGGCGTGGCCACAA TCTTCACTGGCGCCGGCACAACATACTACGCCAACTCCGTCAAGGGAAGG TTCACAATCTCTAGGGACAACGCCAAGAACACTGCCTATCTGCAGATGAA CTCCCTCAAGCCAGAGGACACTGCCATCTACTACTGCGCCGTGGATTTCA GAGGCGGACTGCTGTATAGGCCAGCCTACGAGTACACTTATAGGGGCCAA GGCACACAAGTGACAGTCTCGTCTGCTAGCCACCATCACCATCACCAC |
| **336** | DNA Sequence Encoding SEQ ID NO: 238 | CAAGTGCAGCTGCAAGAGAGCGGAGGAGGAAGCGTCCAAGCCGGAGGCT CTCTGAGACTGAGCTGTGCCGCCAGCGGCTACTCCAACTGCAGCTACGAC ATGACTTGGTATAGGCAAGCCCCCGGCAAGGAGAGGGAGTTCGTGTCCGC CATCCACAGCGACGGCAGCACTAGATACGCCGACAGCGTGAAGGGAAGG TTCTTCATCAGCCAAGATAACGCCAAGAACACAGTGTATCTGCAGATGAA CTCCCTCAAGCCAGAGGACACTGCCATGTACTACTGCAAGACAGACCCAC TGCACTGCAGAGCCCATGGCGGCAGCTGGTATAGCGTGAGGGCCAACTAC TGGGGCCAAGGCACACAAGTGACAGTCTCGAGCGGCGGAGGATCCCAAG TGCAGCTGCAAGAGAGCGGAGGAGGCAGCGTCGAAGCTGGAGGATCTCT GAGGCTGAGCTGTGCTGCCAGCGGCTACACTCACAGCAGCTACTGTATGG GCTGGTTCAGACAAGCCCCCGGCAAGGAGAGGGAAGGCGTGGCTGCCAT CGACGTGGATGGCAGCACTACTTACGCCGACAGCGTGAAGGGAAGGTTC ACTATCAGCAAGGACAACGCCAAGAACACACTCTATCTGCAGATGAACA GCCTCAAGCCAGAGGACACTGGCATGTACTACTGCGCCGCCGAGTTCGCC GATTGCAGCAGCAACTACTTTCTGCCTCCCGGCGCCGTCAGATATTGGGG CCAAGGCACTCAAGTGACAGTCTCGTCTGCTAGCCACCATCACCATCACC AC |
| **337** | DNA Sequence Encoding SEQ ID NO: 239 | CAAGTGCAGCTGCAAGAGAGCGGAGGAGGAAGCGTCCAAGCCGGAGGCT CTCTGAGACTGAGCTGTGCCGCCAGCGGCTACTCCAACTGCAGCTACGAC ATGACTTGGTATAGGCAAGCCCCCGGCAAGGAGAGGGAGTTCGTGTCCGC CATCCACAGCGACGGCAGCACTAGATACGCCGACAGCGTGAAGGGAAGG TTCTTCATCAGCCAAGATAACGCCAAGAACACAGTGTATCTGCAGATGAA CTCCCTCAAGCCAGAGGACACTGCCATGTACTACTGCAAGACAGACCCAC TGCACTGCAGAGCCCATGGCGGCAGCTGGTATAGCGTGAGGGCCAACTAC TGGGGCCAAGGCACACAAGTGACAGTCTCGAGCGGCGGAGGATCCCAAG TGCAGCTGCAAGAGAGCGGAGGAGGAAGCGTCCAAGCCGGAGGATCTCT GAGACTGAGCTGCGCCGCTAGTGGCTACTCCTACAGCAGCTACTGCATGG GCTGGTTTAGGCAAGCCCCCGGCAAGGAGAGAGAAGGCGTGGCCACTAT CGACAGCGACGGCATGACAAGGTACGCCGACAGCGTGAAGGGAAGGTTC ACAATCAGCAAGGACAACGCCAAGAACACACTGTATCTGCAGATGAACT CTCTGAAGCCAGAGGACACTGCCATGTACTACTGTGCCGCTCCTCTGTAC GACTGTGATAGCGGCGCTGTGGGCAGAAATCCACCTTATTGGGGCCAAGG CACTCAAGTGACAGTCTCGTCTGCTAGCCACCATCACCATCACCAC |
| **338** | DNA Sequence Encoding SEQ ID NO: 240 | CAAGTGCAGCTGCAAGAGAGCGGAGGAGGAAGCGTCCAAGCCGGAGGCT CTCTGAGACTGAGCTGTGCCGCCAGCGGCTACTCCAACTGCAGCTACGAC ATGACTTGGTATAGGCAAGCCCCCGGCAAGGAGAGGGAGTTCGTGTCCGC CATCCACAGCGACGGCAGCACTAGATACGCCGACAGCGTGAAGGGAAGG TTCTTCATCAGCCAAGATAACGCCAAGAACACAGTGTATCTGCAGATGAA CTCCCTCAAGCCAGAGGACACTGCCATGTACTACTGCAAGACAGACCCAC TGCACTGCAGAGCCCATGGCGGCAGCTGGTATAGCGTGAGGGCCAACTAC TGGGGCCAAGGCACACAAGTGACAGTCTCGAGCGGCGGAGGATCCCAAG TGCAGCTGCAAGAGAGCGGAGGCGGCAGCGTGCAGACTGGAGGCTCTCT GAGACTGAGCTGTGCTGCCAGCGGCTACACTTATCTGAGGGGCTGTATGG GCTGGTTTAGGCAAGCCCCCGGCAAGGAGAGAGAGGGCGTGGCCGTCAT GGATGTGGTGGGCGATAGGAGAAGCTACATCGACAGCGTGAAGGGAAGG TTCACAATCTCTAGGGACAATGCCGCCAACAGCGTCTATCTGCAGATGGA CAATCTGAAGCCAGAGGACACAGCCATGTACTACTGCACTGCCGGCCCTA ACTGTGTGGGCTGGAGAAGCGGACTGGATTACTGGGGCCAAGGCACACA AGTGACAGTCTCGTCTGCTAGCCACCATCACCATCACCAC |
| **339** | DNA Sequence Encoding SEQ ID NO: 241 | CAAGTGCAGCTGCAAGAGAGCGGCGGAGGAAGCGTGCAAGCTGGAGGCT CTCTGAGGCTGAGCTGTGCTGCCAGCGGCTACACTTATAGCAGCGGCTGT ATGGGCTGGTTCAGACAAGCCCCCGGCAAGGAAAGGGAAGCCGTGGCCG CCATCAATTCCGATGGCAGCACAAGCTACGCCGACAGCGTGAAGGGAAG GTTCACAATCAGCAAGGACAACGCCAAGAACACACTCTATCTGCAGATGA ACTCTCTGAAGCCAGAGGACACAGCCATGTACTACTGCGCCGCTGAGCCT TACTGTAGCGGCGGCTACCCAAGATGGAGCGTCGCTGAGTTCGGCTACTG GGGCCAAGGCACACAAGTGACTGTCTCGAGCGGCGGAGGATCCCAAGTG CAGCTGCAAGAGAGCGGAGGAGGAAGCGTGCAAGCCGGAGGCTCTCTGA GACTGAGCTGTGCCGCCTCTAGGTATCTGTACAGCATCGACTACATGGCT TGGTTCAGACAGAGCCCCGGCAAGGAGAGGGAGCCAGTGGCTGTCATCT ACACTGCCTCCGGCGCCACATTCTATCCAGATAGCGTGAAGGGAAGGTTC ACTATCAGCCAAGATAACGCCAAGATGACAGTGTATCTGCAGATGAACTC TCTGAAGAGCGAGGACACTGCCATGTACTACTGTGCCGCCGTGAGGAAGA CAGATAGCTACCTCTTCGACGCCCAGAGCTTCACATACTGGGGCCAAGGC ACACAAGTGACAGTCTCGTCTGCTAGCCACCATCACCATCACCAC |
| **340** | DNA Sequence Encoding SEQ ID NO: 242 | CAAGTGCAGCTGCAAGAGAGCGGCGGAGGAAGCGTGCAAGCTGGAGGCT CTCTGAGGCTGAGCTGTGCTGCCAGCGGCTACACTTATAGCAGCGGCTGT ATGGGCTGGTTCAGACAAGCCCCCGGCAAGGAAAGGGAAGCCGTGGCCG CCATCAATTCCGATGGCAGCACAAGCTACGCCGACAGCGTGAAGGGAAG GTTCACAATCAGCAAGGACAACGCCAAGAACACACTCTATCTGCAGATGA ACTCTCTGAAGCCAGAGGACACAGCCATGTACTACTGCGCCGCTGAGCCT TACTGTAGCGGCGGCTACCCAAGATGGAGCGTCGCTGAGTTCGGCTACTG GGGCCAAGGCACACAAGTGACTGTCTCGAGCGGCGGAGGATCCCAAGTG CAGCTGCAAGAGAGCGGAGGAGGAAGCGTGCAAGCCGGAGGCTCTCTGA GGCTGAGCTGTGCCGCCTCTAGGTTCACATACAGCAGCTACTGCATGGGC TGGTTCAGACAAGCCCCCGGCAAAGAGAGAGAAGGCGTGGCCAGCATCG ATAGCGATGGCTCCACTAGCTACACTGACAGCGTGAAGGGAAGGTTCACT ATCAGCAAGGACAACGCCAAGAACACTCTGTATCTGCAGATGAACTCTCT GAAGCCAGAGGACACAGCCATGTACTACTGTGCCCTCGATCTGATGAGCA CAGTGGTGCCCGGCTTCTGTGGCTTTCTGCTGAGCGCTGGCATGGATTACT GGGGCAAGGGCACTCAAGTGACTGTCTCGTCTGCTAGCCACCATCACCAT CACCAC |
| **341** | DNA Sequence Encoding SEQ ID NO: 243 | CAAGTGCAGCTGCAAGAGAGCGGCGGAGGAAGCGTGCAAGCTGGAGGCT CTCTGAGGCTGAGCTGTGCTGCCAGCGGCTACACTTATAGCAGCGGCTGT ATGGGCTGGTTCAGACAAGCCCCCGGCAAGGAAAGGGAAGCCGTGGCCG CCATCAATTCCGATGGCAGCACAAGCTACGCCGACAGCGTGAAGGGAAG GTTCACAATCAGCAAGGACAACGCCAAGAACACACTCTATCTGCAGATGA ACTCTCTGAAGCCAGAGGACACAGCCATGTACTACTGCGCCGCTGAGCCT TACTGTAGCGGCGGCTACCCAAGATGGAGCGTCGCTGAGTTCGGCTACTG GGGCCAAGGCACACAAGTGACTGTCTCGAGCGGCGGAGGATCCCAAGTG CAGCTGCAAGAGTCCGGAGGAGGCAGCGTCCAAGCCGGAGGCTCTCTGA GGCTGAGCTGTGCTGCCAGCGGCTACACTTACAGCATGTACTGCATGGGC TGGTTCAGACAAGCCCCCGGCAAGGAAAGAGAGGGCGTGGCCCAGATCA ATAGCGATGGCAGCACAAGCTACGCCGACAGCGTGAAGGGAAGGTTCAC TATCTCCAAGGACAACGCCAAGAACACTCTGTATCTGCAGATGAACTCTC TGAAGCCAGAGGACACTGCCATGTACTACTGCGCTGCCGATTCTAGGGTG TACGGCGGCAGCTGGTATGAGAGGCTCTGCGGCCCTTACACATACGAGTA CAACTACTGGGGCCAAGGCACACAAGTGACTGTCTCGTCTGCTAGCCACC ATCACCATCACCAC |
| **342** | DNA Sequence Encoding SEQ ID NO: 244 | CAAGTGCAGCTGCAAGAGAGCGGCGGAGGAAGCGTGCAAGCTGGAGGCT CTCTGAGGCTGAGCTGTGCTGCCAGCGGCTACACTTATAGCAGCGGCTGT ATGGGCTGGTTCAGACAAGCCCCCGGCAAGGAAAGGGAAGCCGTGGCCG CCATCAATTCCGATGGCAGCACAAGCTACGCCGACAGCGTGAAGGGAAG GTTCACAATCAGCAAGGACAACGCCAAGAACACACTCTATCTGCAGATGA ACTCTCTGAAGCCAGAGGACACAGCCATGTACTACTGCGCCGCTGAGCCT TACTGTAGCGGCGGCTACCCAAGATGGAGCGTCGCTGAGTTCGGCTACTG GGGCCAAGGCACACAAGTGACTGTCTCGAGCGGCGGAGGATCCCAAGTG CAGCTGCAAGAGAGCGGCGGAGGAAGCGTGCAAGCCGGAGGATCTCTGA GACTGAGCTGCGCTGTGAGCGGCTACGCCTACTCCACATACTGCATGGGC TGGTTTAGGCAAGCCCCCGGCAAAGAGAGAGAGGGCGTGGCTGCTATCG ATAGCGGCGGCAGCACAAGCTACGCCGATAGCGTGAAGGGAAGGTTCAC AATCAGCAAGGACAACGCCAAGAACACACTGTATCTGAGGATGAACTCT CTGAAGCCAGAGGACACAGCCATGTACTACTGTGCTGCTGTGCCTCCTCC TCCAGATGGCGGCAGCTGTCTGTTTCTGGGACCAGAGATCAAGGTCAGCA AGGCCGATTTTAGGTACTGGGGCCAAGGCACACAAGTGACAGTCTCGTCT GCTAGCCACCATCACCATCACCAC |
| **343** | DNA Sequence Encoding SEQ ID NO: 245 | CAAGTGCAGCTGCAAGAGAGCGGCGGAGGAAGCGTGCAAGCTGGAGGCT CTCTGAGGCTGAGCTGTGCTGCCAGCGGCTACACTTATAGCAGCGGCTGT ATGGGCTGGTTCAGACAAGCCCCCGGCAAGGAAAGGGAAGCCGTGGCCG CCATCAATTCCGATGGCAGCACAAGCTACGCCGACAGCGTGAAGGGAAG GTTCACAATCAGCAAGGACAACGCCAAGAACACACTCTATCTGCAGATGA ACTCTCTGAAGCCAGAGGACACAGCCATGTACTACTGCGCCGCTGAGCCT TACTGTAGCGGCGGCTACCCAAGATGGAGCGTCGCTGAGTTCGGCTACTG GGGCCAAGGCACACAAGTGACTGTCTCGAGCGGCGGAGGATCCCAAGTG CAGCTGCAAGAGAGCGGAGGAGGAAGCGTGCAAGCCGGAGGCTCTCTGA GGCTGAGCTGTACAGTGTCCGGCTACACTTACAGCTCCAATTGCATGGGC TGGTTTAGGCAAGCCCCCGGCAAGGAAAGAGAGGGCGTGGCCACTATCT ACACTGGCGGCGGCAACACATACTACGCCGATAGCGTGAAGGGAAGGTT CACTATCAGCCAAGATAACGCCAAGAACACAGTGTATCTGCAGATGAAC AATCTGAAGCCAGAGGACACTGCCATGTACTACTGTGCTGCTGAGCCACT GTCTAGGGTGTACGGCGGCAGCTGCCCAACTCCTACATTCGACTACTGGG GCCAAGGCACACAAGTGACTGTCTCGTCTGCTAGCCACCATCACCATCAC CAC |
| **344** | DNA Sequence Encoding SEQ ID NO: 246 | CAAGTGCAGCTGCAAGAGAGCGGCGGAGGAAGCGTGCAAGCTGGAGGCT CTCTGAGGCTGAGCTGTGCTGCCAGCGGCTACACTTATAGCAGCGGCTGT ATGGGCTGGTTCAGACAAGCCCCCGGCAAGGAAAGGGAAGCCGTGGCCG CCATCAATTCCGATGGCAGCACAAGCTACGCCGACAGCGTGAAGGGAAG GTTCACAATCAGCAAGGACAACGCCAAGAACACACTCTATCTGCAGATGA ACTCTCTGAAGCCAGAGGACACAGCCATGTACTACTGCGCCGCTGAGCCT TACTGTAGCGGCGGCTACCCAAGATGGAGCGTCGCTGAGTTCGGCTACTG GGGCCAAGGCACACAAGTGACTGTCTCGAGCGGCGGAGGATCCCAAGTG CAGCTGCAAGAGAGCGGAGGAGGAAGCGTCCAAGCCGGAGGCTCTCTGA GGCTGAGCTGTGGAGCCAGCGGCTACACTTACAGCAGCTACTGTATGGGC TGGTTTAGGCAAGTGCCCGGCAAGGAGAGAGAGGGCGTGGCCGTGATCG ATTCCGATGGCAGCACAAGCTACGCTGACAGCGTGAAGGGAAGGTTCAC AATCAGCAAGGACAACGGCAAGAACACACTCTATCTGCAGATGAACAGC CTCAAGCCAGAGGACACAGCCATGTACTACTGCGCCGCTGATCTGGGCCA CTATAGGCCTCCTTGTGGCGTGCTGTATCTGGGCATGGATTACTGGGGCA AGGGCACACAAGTGACAGTCTCGTCTGCTAGCCACCATCACCATCACCAC |
| **345** | DNA Sequence Encoding SEQ ID NO: 247 | CAAGTGCAGCTGCAAGAGAGCGGCGGAGGAAGCGTGCAAGCTGGAGGCT CTCTGAGGCTGAGCTGTGCTGCCAGCGGCTACACTTATAGCAGCGGCTGT ATGGGCTGGTTCAGACAAGCCCCCGGCAAGGAAAGGGAAGCCGTGGCCG CCATCAATTCCGATGGCAGCACAAGCTACGCCGACAGCGTGAAGGGAAG GTTCACAATCAGCAAGGACAACGCCAAGAACACACTCTATCTGCAGATGA ACTCTCTGAAGCCAGAGGACACAGCCATGTACTACTGCGCCGCTGAGCCT TACTGTAGCGGCGGCTACCCAAGATGGAGCGTCGCTGAGTTCGGCTACTG GGGCCAAGGCACACAAGTGACTGTCTCGAGCGGCGGAGGATCCCAAGTG CAGCTGCAAGAGAGCGGAGGAGGAAGCGTCCAAGCCGGAGGCTCTCTGA GACTGAGCTGTGCCGCCAGCGGCTACTCCAACTGCAGCTACGACATGACT TGGTATAGGCAAGCCCCCGGCAAGGAGAGGGAGTTCGTGTCCGCCATCCA CAGCGACGGCAGCACTAGATACGCCGACAGCGTGAAGGGAAGGTTCTTC ATCAGCCAAGATAACGCCAAGAACACAGTGTATCTGCAGATGAACTCCCT CAAGCCAGAGGACACTGCCATGTACTACTGCAAGACAGACCCACTGCACT GCAGAGCCCATGGCGGCAGCTGGTATAGCGTGAGGGCCAACTACTGGGG CCAAGGCACACAAGTGACAGTCTCGTCTGCTAGCCACCATCACCATCACC AC |
| **346** | DNA Sequence Encoding SEQ ID NO: 248 | CAAGTGCAGCTGCAAGAGAGCGGAGGAGGCAGCGTCCAAGCCGGAGGCT CTCTGAGGCTGAGCTGTGCTGCCAGCGGCTACACTTACAGCAGCTACTGC ATGGGCTGGTTCAGACAAGCCCCCGGCAAGGAGAGAGAGGGCGTGGCTC ACATCGACAGCGACGGCTCCACAAGCTACGCCGATAGCGTGAAGGGAAG GTTCACAATCTCCAAGGACAACGCCAAGAACACTCTGTACCTCCAGATGA ACTCTCTGAAGCCAGAGGACACTGCCATGTACTACTGTGCCGCCGATCCA ATTCCCGGCCCCGGCTACTGCGATGGCGGCCCTAACAAGTACTGGGGCCA AGGCACACAAGTGACTGTCTCGAGCGGCGGAGGATCCCAAGTGCAGCTG CAAGAGAGCGGAGGAGGAAGCGTGCAAGCCGGAGGCTCTCTGAGACTGA GCTGTGCCGCCTCTAGGTATCTGTACAGCATCGACTACATGGCTTGGTTCA GACAGAGCCCCGGCAAGGAGAGGGAGCCAGTGGCTGTCATCTACACTGC CTCCGGCGCCACATTCTATCCAGATAGCGTGAAGGGAAGGTTCACTATCA GCCAAGATAACGCCAAGATGACAGTGTATCTGCAGATGAACTCTCTGAAG AGCGAGGACACTGCCATGTACTACTGTGCCGCCGTGAGGAAGACAGATA GCTACCTCTTCGACGCCCAGAGCTTCACATACTGGGGCCAAGGCACACAA GTGACAGTCTCGTCTGCTAGCCACCATCACCATCACCAC |
| **347** | DNA Sequence Encoding SEQ ID NO: 249 | CAAGTGCAGCTGCAAGAGAGCGGAGGAGGCAGCGTCCAAGCCGGAGGCT CTCTGAGGCTGAGCTGTGCTGCCAGCGGCTACACTTACAGCAGCTACTGC ATGGGCTGGTTCAGACAAGCCCCCGGCAAGGAGAGAGAGGGCGTGGCTC ACATCGACAGCGACGGCTCCACAAGCTACGCCGATAGCGTGAAGGGAAG GTTCACAATCTCCAAGGACAACGCCAAGAACACTCTGTACCTCCAGATGA ACTCTCTGAAGCCAGAGGACACTGCCATGTACTACTGTGCCGCCGATCCA ATTCCCGGCCCCGGCTACTGCGATGGCGGCCCTAACAAGTACTGGGGCCA AGGCACACAAGTGACTGTCTCGAGCGGCGGAGGATCCCAAGTGCAGCTG CAAGAGAGCGGAGGAGGAAGCGTGCAAGCCGGAGGCTCTCTGAGGCTGA GCTGTGCCGCCTCTAGGTTCACATACAGCAGCTACTGCATGGGCTGGTTC AGACAAGCCCCCGGCAAAGAGAGAGAAGGCGTGGCCAGCATCGATAGCG ATGGCTCCACTAGCTACACTGACAGCGTGAAGGGAAGGTTCACTATCAGC AAGGACAACGCCAAGAACACTCTGTATCTGCAGATGAACTCTCTGAAGCC AGAGGACACAGCCATGTACTACTGTGCCCTCGATCTGATGAGCACAGTGG TGCCCGGCTTCTGTGGCTTTCTGCTGAGCGCTGGCATGGATTACTGGGGCA AGGGCACTCAAGTGACTGTCTCGTCTGCTAGCCACCATCACCATCACCAC |
| **348** | DNA Sequence Encoding SEQ ID NO: 250 | CAAGTGCAGCTGCAAGAGAGCGGAGGAGGCAGCGTCCAAGCCGGAGGCT CTCTGAGGCTGAGCTGTGCTGCCAGCGGCTACACTTACAGCAGCTACTGC ATGGGCTGGTTCAGACAAGCCCCCGGCAAGGAGAGAGAGGGCGTGGCTC ACATCGACAGCGACGGCTCCACAAGCTACGCCGATAGCGTGAAGGGAAG GTTCACAATCTCCAAGGACAACGCCAAGAACACTCTGTACCTCCAGATGA ACTCTCTGAAGCCAGAGGACACTGCCATGTACTACTGTGCCGCCGATCCA ATTCCCGGCCCCGGCTACTGCGATGGCGGCCCTAACAAGTACTGGGGCCA AGGCACACAAGTGACTGTCTCGAGCGGCGGAGGATCCCAAGTGCAGCTG CAAGAGTCCGGAGGAGGCAGCGTCCAAGCCGGAGGCTCTCTGAGGCTGA GCTGTGCTGCCAGCGGCTACACTTACAGCATGTACTGCATGGGCTGGTTC AGACAAGCCCCCGGCAAGGAAAGAGAGGGCGTGGCCCAGATCAATAGCG ATGGCAGCACAAGCTACGCCGACAGCGTGAAGGGAAGGTTCACTATCTCC AAGGACAACGCCAAGAACACTCTGTATCTGCAGATGAACTCTCTGAAGCC AGAGGACACTGCCATGTACTACTGCGCTGCCGATTCTAGGGTGTACGGCG GCAGCTGGTATGAGAGGCTCTGCGGCCCTTACACATACGAGTACAACTAC TGGGGCCAAGGCACACAAGTGACTGTCTCGTCTGCTAGCCACCATCACCA TCACCAC |
| **349** | DNA Sequence Encoding SEQ ID NO: 251 | CAAGTGCAGCTGCAAGAGAGCGGAGGAGGCAGCGTCCAAGCCGGAGGCT CTCTGAGGCTGAGCTGTGCTGCCAGCGGCTACACTTACAGCAGCTACTGC ATGGGCTGGTTCAGACAAGCCCCCGGCAAGGAGAGAGAGGGCGTGGCTC ACATCGACAGCGACGGCTCCACAAGCTACGCCGATAGCGTGAAGGGAAG GTTCACAATCTCCAAGGACAACGCCAAGAACACTCTGTACCTCCAGATGA ACTCTCTGAAGCCAGAGGACACTGCCATGTACTACTGTGCCGCCGATCCA ATTCCCGGCCCCGGCTACTGCGATGGCGGCCCTAACAAGTACTGGGGCCA AGGCACACAAGTGACTGTCTCGAGCGGCGGAGGATCCCAAGTGCAGCTG CAAGAGAGCGGCGGAGGAAGCGTGCAAGCCGGAGGATCTCTGAGACTGA GCTGCGCTGTGAGCGGCTACGCCTACTCCACATACTGCATGGGCTGGTTT AGGCAAGCCCCCGGCAAAGAGAGAGAGGGCGTGGCTGCTATCGATAGCG GCGGCAGCACAAGCTACGCCGATAGCGTGAAGGGAAGGTTCACAATCAG CAAGGACAACGCCAAGAACACACTGTATCTGAGGATGAACTCTCTGAAG CCAGAGGACACAGCCATGTACTACTGTGCTGCTGTGCCTCCTCCTCCAGA TGGCGGCAGCTGTCTGTTTCTGGGACCAGAGATCAAGGTCAGCAAGGCCG ATTTTAGGTACTGGGGCCAAGGCACACAAGTGACAGTCTCGTCTGCTAGC CACCATCACCATCACCAC |
| **350** | DNA Sequence Encoding SEQ ID NO: 252 | CAAGTGCAGCTGCAAGAGAGCGGAGGAGGCAGCGTCCAAGCCGGAGGCT CTCTGAGGCTGAGCTGTGCTGCCAGCGGCTACACTTACAGCAGCTACTGC ATGGGCTGGTTCAGACAAGCCCCCGGCAAGGAGAGAGAGGGCGTGGCTC ACATCGACAGCGACGGCTCCACAAGCTACGCCGATAGCGTGAAGGGAAG GTTCACAATCTCCAAGGACAACGCCAAGAACACTCTGTACCTCCAGATGA ACTCTCTGAAGCCAGAGGACACTGCCATGTACTACTGTGCCGCCGATCCA ATTCCCGGCCCCGGCTACTGCGATGGCGGCCCTAACAAGTACTGGGGCCA AGGCACACAAGTGACTGTCTCGAGCGGCGGAGGATCCCAAGTGCAGCTG CAAGAGAGCGGAGGAGGAAGCGTGCAAGCCGGAGGCTCTCTGAGGCTGA GCTGTACAGTGTCCGGCTACACTTACAGCTCCAATTGCATGGGCTGGTTTA GGCAAGCCCCCGGCAAGGAAAGAGAGGGCGTGGCCACTATCTACACTGG CGGCGGCAACACATACTACGCCGATAGCGTGAAGGGAAGGTTCACTATC AGCCAAGATAACGCCAAGAACACAGTGTATCTGCAGATGAACAATCTGA AGCCAGAGGACACTGCCATGTACTACTGTGCTGCTGAGCCACTGTCTAGG GTGTACGGCGGCAGCTGCCCAACTCCTACATTCGACTACTGGGGCCAAGG CACACAAGTGACTGTCTCGTCTGCTAGCCACCATCACCATCACCAC |
| **351** | DNA Sequence Encoding SEQ ID NO: 253 | CAAGTGCAGCTGCAAGAGAGCGGAGGAGGCAGCGTCCAAGCCGGAGGCT CTCTGAGGCTGAGCTGTGCTGCCAGCGGCTACACTTACAGCAGCTACTGC ATGGGCTGGTTCAGACAAGCCCCCGGCAAGGAGAGAGAGGGCGTGGCTC ACATCGACAGCGACGGCTCCACAAGCTACGCCGATAGCGTGAAGGGAAG GTTCACAATCTCCAAGGACAACGCCAAGAACACTCTGTACCTCCAGATGA ACTCTCTGAAGCCAGAGGACACTGCCATGTACTACTGTGCCGCCGATCCA ATTCCCGGCCCCGGCTACTGCGATGGCGGCCCTAACAAGTACTGGGGCCA AGGCACACAAGTGACTGTCTCGAGCGGCGGAGGATCCCAAGTGCAGCTG CAAGAGAGCGGAGGAGGAAGCGTCCAAGCCGGAGGCTCTCTGAGGCTGA GCTGTGGAGCCAGCGGCTACACTTACAGCAGCTACTGTATGGGCTGGTTT AGGCAAGTGCCCGGCAAGGAGAGAGAGGGCGTGGCCGTGATCGATTCCG ATGGCAGCACAAGCTACGCTGACAGCGTGAAGGGAAGGTTCACAATCAG CAAGGACAACGGCAAGAACACACTCTATCTGCAGATGAACAGCCTCAAG CCAGAGGACACAGCCATGTACTACTGCGCCGCTGATCTGGGCCACTATAG GCCTCCTTGTGGCGTGCTGTATCTGGGCATGGATTACTGGGGCAAGGGCA CACAAGTGACAGTCTCGTCTGCTAGCCACCATCACCATCACCAC |
| **352** | DNA Sequence Encoding SEQ ID NO: 254 | CAAGTGCAGCTGCAAGAGAGCGGAGGAGGCAGCGTCCAAGCCGGAGGCT CTCTGAGGCTGAGCTGTGCTGCCAGCGGCTACACTTACAGCAGCTACTGC ATGGGCTGGTTCAGACAAGCCCCCGGCAAGGAGAGAGAGGGCGTGGCTC ACATCGACAGCGACGGCTCCACAAGCTACGCCGATAGCGTGAAGGGAAG GTTCACAATCTCCAAGGACAACGCCAAGAACACTCTGTACCTCCAGATGA ACTCTCTGAAGCCAGAGGACACTGCCATGTACTACTGTGCCGCCGATCCA ATTCCCGGCCCCGGCTACTGCGATGGCGGCCCTAACAAGTACTGGGGCCA AGGCACACAAGTGACTGTCTCGAGCGGCGGAGGATCCCAAGTGCAGCTG CAAGAGAGCGGAGGAGGAAGCGTCCAAGCCGGAGGCTCTCTGAGACTGA GCTGTGCCGCCAGCGGCTACTCCAACTGCAGCTACGACATGACTTGGTAT AGGCAAGCCCCCGGCAAGGAGAGGGAGTTCGTGTCCGCCATCCACAGCG ACGGCAGCACTAGATACGCCGACAGCGTGAAGGGAAGGTTCTTCATCAG CCAAGATAACGCCAAGAACACAGTGTATCTGCAGATGAACTCCCTCAAGC CAGAGGACACTGCCATGTACTACTGCAAGACAGACCCACTGCACTGCAGA GCCCATGGCGGCAGCTGGTATAGCGTGAGGGCCAACTACTGGGGCCAAG GCACACAAGTGACAGTCTCGTCTGCTAGCCACCATCACCATCACCAC |
| **353** | DNA Sequence Encoding SEQ ID NO: 255 | CAAGTGCAGCTGCAAGAGTCCGGAGGAGGAAGCGTGCAAGCCGGCGGAT CTCTGAGACTGAGCTGTGCCGCCTCTAGGTACACTTACAACAGCTACTGC ATGGGCTGGTTCAGACAAGCCCCCGGCAAGGAAAGAGAGGGCGTGGCCA CTATCGATAGCGACGGCATGACTAGGTACGCTGATAGCGTCAAGGGAAG GTTCACAATCTCCAAGGACAATGCTAAGAACACTCTGTACCTCCAGATGA ACTCTCTGAAGCCAGAGGACACAGCCATGTACTACTGCGCTGCCGATGCC GACTGCACTATCGCCGCCATGACTACTAATCCTCTGGGCCAAGGCACACA AGTGACTGTCTCGAGCGGCGGAGGATCCCAAGTGCAGCTGCAAGAGAGC GGAGGAGGAAGCGTGCAAGCCGGAGGCTCTCTGAGACTGAGCTGTGCCG CCTCTAGGTATCTGTACAGCATCGACTACATGGCTTGGTTCAGACAGAGC CCCGGCAAGGAGAGGGAGCCAGTGGCTGTCATCTACACTGCCTCCGGCGC CACATTCTATCCAGATAGCGTGAAGGGAAGGTTCACTATCAGCCAAGATA ACGCCAAGATGACAGTGTATCTGCAGATGAACTCTCTGAAGAGCGAGGA CACTGCCATGTACTACTGTGCCGCCGTGAGGAAGACAGATAGCTACCTCT TCGACGCCCAGAGCTTCACATACTGGGGCCAAGGCACACAAGTGACAGTC TCGTCTGCTAGCCACCATCACCATCACCAC |
| **354** | DNA Sequence Encoding SEQ ID NO: 256 | CAAGTGCAGCTGCAAGAGTCCGGAGGAGGAAGCGTGCAAGCCGGCGGAT CTCTGAGACTGAGCTGTGCCGCCTCTAGGTACACTTACAACAGCTACTGC ATGGGCTGGTTCAGACAAGCCCCCGGCAAGGAAAGAGAGGGCGTGGCCA CTATCGATAGCGACGGCATGACTAGGTACGCTGATAGCGTCAAGGGAAG GTTCACAATCTCCAAGGACAATGCTAAGAACACTCTGTACCTCCAGATGA ACTCTCTGAAGCCAGAGGACACAGCCATGTACTACTGCGCTGCCGATGCC GACTGCACTATCGCCGCCATGACTACTAATCCTCTGGGCCAAGGCACACA AGTGACTGTCTCGAGCGGCGGAGGATCCCAAGTGCAGCTGCAAGAGAGC GGAGGAGGAAGCGTGCAAGCCGGAGGCTCTCTGAGGCTGAGCTGTGCCG CCTCTAGGTTCACATACAGCAGCTACTGCATGGGCTGGTTCAGACAAGCC CCCGGCAAAGAGAGAGAAGGCGTGGCCAGCATCGATAGCGATGGCTCCA CTAGCTACACTGACAGCGTGAAGGGAAGGTTCACTATCAGCAAGGACAA CGCCAAGAACACTCTGTATCTGCAGATGAACTCTCTGAAGCCAGAGGACA CAGCCATGTACTACTGTGCCCTCGATCTGATGAGCACAGTGGTGCCCGGC TTCTGTGGCTTTCTGCTGAGCGCTGGCATGGATTACTGGGGCAAGGGCAC TCAAGTGACTGTCTCGTCTGCTAGCCACCATCACCATCACCAC |
| **355** | DNA Sequence Encoding SEQ ID NO: 257 | CAAGTGCAGCTGCAAGAGTCCGGAGGAGGAAGCGTGCAAGCCGGCGGAT CTCTGAGACTGAGCTGTGCCGCCTCTAGGTACACTTACAACAGCTACTGC ATGGGCTGGTTCAGACAAGCCCCCGGCAAGGAAAGAGAGGGCGTGGCCA CTATCGATAGCGACGGCATGACTAGGTACGCTGATAGCGTCAAGGGAAG GTTCACAATCTCCAAGGACAATGCTAAGAACACTCTGTACCTCCAGATGA ACTCTCTGAAGCCAGAGGACACAGCCATGTACTACTGCGCTGCCGATGCC GACTGCACTATCGCCGCCATGACTACTAATCCTCTGGGCCAAGGCACACA AGTGACTGTCTCGAGCGGCGGAGGATCCCAAGTGCAGCTGCAAGAGTCC GGAGGAGGCAGCGTCCAAGCCGGAGGCTCTCTGAGGCTGAGCTGTGCTG CCAGCGGCTACACTTACAGCATGTACTGCATGGGCTGGTTCAGACAAGCC CCCGGCAAGGAAAGAGAGGGCGTGGCCCAGATCAATAGCGATGGCAGCA CAAGCTACGCCGACAGCGTGAAGGGAAGGTTCACTATCTCCAAGGACAA CGCCAAGAACACTCTGTATCTGCAGATGAACTCTCTGAAGCCAGAGGACA CTGCCATGTACTACTGCGCTGCCGATTCTAGGGTGTACGGCGGCAGCTGG TATGAGAGGCTCTGCGGCCCTTACACATACGAGTACAACTACTGGGGCCA AGGCACACAAGTGACTGTCTCGTCTGCTAGCCACCATCACCATCACCAC |
| **356** | DNA Sequence Encoding SEQ ID NO: 258 | CAAGTGCAGCTGCAAGAGTCCGGAGGAGGAAGCGTGCAAGCCGGCGGAT CTCTGAGACTGAGCTGTGCCGCCTCTAGGTACACTTACAACAGCTACTGC ATGGGCTGGTTCAGACAAGCCCCCGGCAAGGAAAGAGAGGGCGTGGCCA CTATCGATAGCGACGGCATGACTAGGTACGCTGATAGCGTCAAGGGAAG GTTCACAATCTCCAAGGACAATGCTAAGAACACTCTGTACCTCCAGATGA ACTCTCTGAAGCCAGAGGACACAGCCATGTACTACTGCGCTGCCGATGCC GACTGCACTATCGCCGCCATGACTACTAATCCTCTGGGCCAAGGCACACA AGTGACTGTCTCGAGCGGCGGAGGATCCCAAGTGCAGCTGCAAGAGAGC GGCGGAGGAAGCGTGCAAGCCGGAGGATCTCTGAGACTGAGCTGCGCTG TGAGCGGCTACGCCTACTCCACATACTGCATGGGCTGGTTTAGGCAAGCC CCCGGCAAAGAGAGAGAGGGCGTGGCTGCTATCGATAGCGGCGGCAGCA CAAGCTACGCCGATAGCGTGAAGGGAAGGTTCACAATCAGCAAGGACAA CGCCAAGAACACACTGTATCTGAGGATGAACTCTCTGAAGCCAGAGGAC ACAGCCATGTACTACTGTGCTGCTGTGCCTCCTCCTCCAGATGGCGGCAG CTGTCTGTTTCTGGGACCAGAGATCAAGGTCAGCAAGGCCGATTTTAGGT ACTGGGGCCAAGGCACACAAGTGACAGTCTCGTCTGCTAGCCACCATCAC CATCACCAC |
| **357** | DNA Sequence Encoding SEQ ID NO: 259 | CAAGTGCAGCTGCAAGAGTCCGGAGGAGGAAGCGTGCAAGCCGGCGGAT CTCTGAGACTGAGCTGTGCCGCCTCTAGGTACACTTACAACAGCTACTGC ATGGGCTGGTTCAGACAAGCCCCCGGCAAGGAAAGAGAGGGCGTGGCCA CTATCGATAGCGACGGCATGACTAGGTACGCTGATAGCGTCAAGGGAAG GTTCACAATCTCCAAGGACAATGCTAAGAACACTCTGTACCTCCAGATGA ACTCTCTGAAGCCAGAGGACACAGCCATGTACTACTGCGCTGCCGATGCC GACTGCACTATCGCCGCCATGACTACTAATCCTCTGGGCCAAGGCACACA AGTGACTGTCTCGAGCGGCGGAGGATCCCAAGTGCAGCTGCAAGAGAGC GGAGGAGGAAGCGTGCAAGCCGGAGGCTCTCTGAGGCTGAGCTGTACAG TGTCCGGCTACACTTACAGCTCCAATTGCATGGGCTGGTTTAGGCAAGCC CCCGGCAAGGAAAGAGAGGGCGTGGCCACTATCTACACTGGCGGCGGCA ACACATACTACGCCGATAGCGTGAAGGGAAGGTTCACTATCAGCCAAGAT AACGCCAAGAACACAGTGTATCTGCAGATGAACAATCTGAAGCCAGAGG ACACTGCCATGTACTACTGTGCTGCTGAGCCACTGTCTAGGGTGTACGGC GGCAGCTGCCCAACTCCTACATTCGACTACTGGGGCCAAGGCACACAAGT GACTGTCTCGTCTGCTAGCCACCATCACCATCACCAC |
| **358** | DNA Sequence Encoding SEQ ID NO: 260 | CAAGTGCAGCTGCAAGAGTCCGGAGGAGGAAGCGTGCAAGCCGGCGGAT CTCTGAGACTGAGCTGTGCCGCCTCTAGGTACACTTACAACAGCTACTGC ATGGGCTGGTTCAGACAAGCCCCCGGCAAGGAAAGAGAGGGCGTGGCCA CTATCGATAGCGACGGCATGACTAGGTACGCTGATAGCGTCAAGGGAAG GTTCACAATCTCCAAGGACAATGCTAAGAACACTCTGTACCTCCAGATGA ACTCTCTGAAGCCAGAGGACACAGCCATGTACTACTGCGCTGCCGATGCC GACTGCACTATCGCCGCCATGACTACTAATCCTCTGGGCCAAGGCACACA AGTGACTGTCTCGAGCGGCGGAGGATCCCAAGTGCAGCTGCAAGAGAGC GGAGGAGGAAGCGTCCAAGCCGGAGGCTCTCTGAGGCTGAGCTGTGGAG CCAGCGGCTACACTTACAGCAGCTACTGTATGGGCTGGTTTAGGCAAGTG CCCGGCAAGGAGAGAGAGGGCGTGGCCGTGATCGATTCCGATGGCAGCA CAAGCTACGCTGACAGCGTGAAGGGAAGGTTCACAATCAGCAAGGACAA CGGCAAGAACACACTCTATCTGCAGATGAACAGCCTCAAGCCAGAGGAC ACAGCCATGTACTACTGCGCCGCTGATCTGGGCCACTATAGGCCTCCTTGT GGCGTGCTGTATCTGGGCATGGATTACTGGGGCAAGGGCACACAAGTGAC AGTCTCGTCTGCTAGCCACCATCACCATCACCAC |
| **359** | DNA Sequence Encoding SEQ ID NO: 261 | CAAGTGCAGCTGCAAGAGTCCGGAGGAGGAAGCGTGCAAGCCGGCGGAT CTCTGAGACTGAGCTGTGCCGCCTCTAGGTACACTTACAACAGCTACTGC ATGGGCTGGTTCAGACAAGCCCCCGGCAAGGAAAGAGAGGGCGTGGCCA CTATCGATAGCGACGGCATGACTAGGTACGCTGATAGCGTCAAGGGAAG GTTCACAATCTCCAAGGACAATGCTAAGAACACTCTGTACCTCCAGATGA ACTCTCTGAAGCCAGAGGACACAGCCATGTACTACTGCGCTGCCGATGCC GACTGCACTATCGCCGCCATGACTACTAATCCTCTGGGCCAAGGCACACA AGTGACTGTCTCGAGCGGCGGAGGATCCCAAGTGCAGCTGCAAGAGAGC GGAGGAGGAAGCGTCCAAGCCGGAGGCTCTCTGAGACTGAGCTGTGCCG CCAGCGGCTACTCCAACTGCAGCTACGACATGACTTGGTATAGGCAAGCC CCCGGCAAGGAGAGGGAGTTCGTGTCCGCCATCCACAGCGACGGCAGCA CTAGATACGCCGACAGCGTGAAGGGAAGGTTCTTCATCAGCCAAGATAAC GCCAAGAACACAGTGTATCTGCAGATGAACTCCCTCAAGCCAGAGGACA CTGCCATGTACTACTGCAAGACAGACCCACTGCACTGCAGAGCCCATGGC GGCAGCTGGTATAGCGTGAGGGCCAACTACTGGGGCCAAGGCACACAAG TGACAGTCTCGTCTGCTAGCCACCATCACCATCACCAC |
| **360** | DNA Sequence Encoding SEQ ID NO: 262 | CAAGTGCAGCTGCAAGAGTCCGGCGGAGGCAGCGTCCAAGCCGGAGGAT CTCTGAGGCTGAGCTGTACAGTGAGCAGATACACTGCCAGCGTGAACTAC ATGGGCTGGTTCAGACAAGCCCCCGGCAAAGAGAGAGAGGGCGTGGCCA CAATCTTCACTGGCGCCGGCACAACATACTACGCCAACTCCGTCAAGGGA AGGTTCACAATCTCTAGGGACAACGCCAAGAACACTGCCTATCTGCAGAT GAACTCCCTCAAGCCAGAGGACACTGCCATCTACTACTGCGCCGTGGATT TCAGAGGCGGACTGCTGTATAGGCCAGCCTACGAGTACACTTATAGGGGC CAAGGCACACAAGTGACAGTCTCGAGCGGCGGAGGATCCCAAGTGCAGC TGCAAGAGAGCGGAGGAGGAAGCGTGCAAGCCGGAGGCTCTCTGAGACT GAGCTGTGCCGCCTCTAGGTATCTGTACAGCATCGACTACATGGCTTGGTT CAGACAGAGCCCCGGCAAGGAGAGGGAGCCAGTGGCTGTCATCTACACT GCCTCCGGCGCCACATTCTATCCAGATAGCGTGAAGGGAAGGTTCACTAT CAGCCAAGATAACGCCAAGATGACAGTGTATCTGCAGATGAACTCTCTGA AGAGCGAGGACACTGCCATGTACTACTGTGCCGCCGTGAGGAAGACAGA TAGCTACCTCTTCGACGCCCAGAGCTTCACATACTGGGGCCAAGGCACAC AAGTGACAGTCTCGTCTGCTAGCCACCATCACCATCACCAC |
| **361** | DNA Sequence Encoding SEQ ID NO: 263 | CAAGTGCAGCTGCAAGAGTCCGGCGGAGGCAGCGTCCAAGCCGGAGGAT CTCTGAGGCTGAGCTGTACAGTGAGCAGATACACTGCCAGCGTGAACTAC ATGGGCTGGTTCAGACAAGCCCCCGGCAAAGAGAGAGAGGGCGTGGCCA CAATCTTCACTGGCGCCGGCACAACATACTACGCCAACTCCGTCAAGGGA AGGTTCACAATCTCTAGGGACAACGCCAAGAACACTGCCTATCTGCAGAT GAACTCCCTCAAGCCAGAGGACACTGCCATCTACTACTGCGCCGTGGATT TCAGAGGCGGACTGCTGTATAGGCCAGCCTACGAGTACACTTATAGGGGC CAAGGCACACAAGTGACAGTCTCGAGCGGCGGAGGATCCCAAGTGCAGC TGCAAGAGAGCGGAGGAGGAAGCGTGCAAGCCGGAGGCTCTCTGAGGCT GAGCTGTGCCGCCTCTAGGTTCACATACAGCAGCTACTGCATGGGCTGGT TCAGACAAGCCCCCGGCAAAGAGAGAGAAGGCGTGGCCAGCATCGATAG CGATGGCTCCACTAGCTACACTGACAGCGTGAAGGGAAGGTTCACTATCA GCAAGGACAACGCCAAGAACACTCTGTATCTGCAGATGAACTCTCTGAAG CCAGAGGACACAGCCATGTACTACTGTGCCCTCGATCTGATGAGCACAGT GGTGCCCGGCTTCTGTGGCTTTCTGCTGAGCGCTGGCATGGATTACTGGG GCAAGGGCACTCAAGTGACTGTCTCGTCTGCTAGCCACCATCACCATCAC CAC |
| **362** | DNA Sequence Encoding SEQ ID NO: 264 | CAAGTGCAGCTGCAAGAGTCCGGCGGAGGCAGCGTCCAAGCCGGAGGAT CTCTGAGGCTGAGCTGTACAGTGAGCAGATACACTGCCAGCGTGAACTAC ATGGGCTGGTTCAGACAAGCCCCCGGCAAAGAGAGAGAGGGCGTGGCCA CAATCTTCACTGGCGCCGGCACAACATACTACGCCAACTCCGTCAAGGGA AGGTTCACAATCTCTAGGGACAACGCCAAGAACACTGCCTATCTGCAGAT GAACTCCCTCAAGCCAGAGGACACTGCCATCTACTACTGCGCCGTGGATT TCAGAGGCGGACTGCTGTATAGGCCAGCCTACGAGTACACTTATAGGGGC CAAGGCACACAAGTGACAGTCTCGAGCGGCGGAGGATCCCAAGTGCAGC TGCAAGAGTCCGGAGGAGGCAGCGTCCAAGCCGGAGGCTCTCTGAGGCT GAGCTGTGCTGCCAGCGGCTACACTTACAGCATGTACTGCATGGGCTGGT TCAGACAAGCCCCCGGCAAGGAAAGAGAGGGCGTGGCCCAGATCAATAG CGATGGCAGCACAAGCTACGCCGACAGCGTGAAGGGAAGGTTCACTATC TCCAAGGACAACGCCAAGAACACTCTGTATCTGCAGATGAACTCTCTGAA GCCAGAGGACACTGCCATGTACTACTGCGCTGCCGATTCTAGGGTGTACG GCGGCAGCTGGTATGAGAGGCTCTGCGGCCCTTACACATACGAGTACAAC TACTGGGGCCAAGGCACACAAGTGACTGTCTCGTCTGCTAGCCACCATCA CCATCACCAC |
| **363** | DNA Sequence Encoding SEQ ID NO: 265 | CAAGTGCAGCTGCAAGAGTCCGGCGGAGGCAGCGTCCAAGCCGGAGGAT CTCTGAGGCTGAGCTGTACAGTGAGCAGATACACTGCCAGCGTGAACTAC ATGGGCTGGTTCAGACAAGCCCCCGGCAAAGAGAGAGAGGGCGTGGCCA CAATCTTCACTGGCGCCGGCACAACATACTACGCCAACTCCGTCAAGGGA AGGTTCACAATCTCTAGGGACAACGCCAAGAACACTGCCTATCTGCAGAT GAACTCCCTCAAGCCAGAGGACACTGCCATCTACTACTGCGCCGTGGATT TCAGAGGCGGACTGCTGTATAGGCCAGCCTACGAGTACACTTATAGGGGC CAAGGCACACAAGTGACAGTCTCGAGCGGCGGAGGATCCCAAGTGCAGC TGCAAGAGAGCGGCGGAGGAAGCGTGCAAGCCGGAGGATCTCTGAGACT GAGCTGCGCTGTGAGCGGCTACGCCTACTCCACATACTGCATGGGCTGGT TTAGGCAAGCCCCCGGCAAAGAGAGAGAGGGCGTGGCTGCTATCGATAG CGGCGGCAGCACAAGCTACGCCGATAGCGTGAAGGGAAGGTTCACAATC AGCAAGGACAACGCCAAGAACACACTGTATCTGAGGATGAACTCTCTGA AGCCAGAGGACACAGCCATGTACTACTGTGCTGCTGTGCCTCCTCCTCCA GATGGCGGCAGCTGTCTGTTTCTGGGACCAGAGATCAAGGTCAGCAAGGC CGATTTTAGGTACTGGGGCCAAGGCACACAAGTGACAGTCTCGTCTGCTA GCCACCATCACCATCACCAC |
| **364** | DNA Sequence Encoding SEQ ID NO: 266 | CAAGTGCAGCTGCAAGAGTCCGGCGGAGGCAGCGTCCAAGCCGGAGGAT CTCTGAGGCTGAGCTGTACAGTGAGCAGATACACTGCCAGCGTGAACTAC ATGGGCTGGTTCAGACAAGCCCCCGGCAAAGAGAGAGAGGGCGTGGCCA CAATCTTCACTGGCGCCGGCACAACATACTACGCCAACTCCGTCAAGGGA AGGTTCACAATCTCTAGGGACAACGCCAAGAACACTGCCTATCTGCAGAT GAACTCCCTCAAGCCAGAGGACACTGCCATCTACTACTGCGCCGTGGATT TCAGAGGCGGACTGCTGTATAGGCCAGCCTACGAGTACACTTATAGGGGC CAAGGCACACAAGTGACAGTCTCGAGCGGCGGAGGATCCCAAGTGCAGC TGCAAGAGAGCGGAGGAGGAAGCGTGCAAGCCGGAGGCTCTCTGAGGCT GAGCTGTACAGTGTCCGGCTACACTTACAGCTCCAATTGCATGGGCTGGT TTAGGCAAGCCCCCGGCAAGGAAAGAGAGGGCGTGGCCACTATCTACAC TGGCGGCGGCAACACATACTACGCCGATAGCGTGAAGGGAAGGTTCACT ATCAGCCAAGATAACGCCAAGAACACAGTGTATCTGCAGATGAACAATCT GAAGCCAGAGGACACTGCCATGTACTACTGTGCTGCTGAGCCACTGTCTA GGGTGTACGGCGGCAGCTGCCCAACTCCTACATTCGACTACTGGGGCCAA GGCACACAAGTGACTGTCTCGTCTGCTAGCCACCATCACCATCACCAC |
| **365** | DNA Sequence Encoding SEQ ID NO: 267 | CAAGTGCAGCTGCAAGAGTCCGGCGGAGGCAGCGTCCAAGCCGGAGGAT CTCTGAGGCTGAGCTGTACAGTGAGCAGATACACTGCCAGCGTGAACTAC ATGGGCTGGTTCAGACAAGCCCCCGGCAAAGAGAGAGAGGGCGTGGCCA CAATCTTCACTGGCGCCGGCACAACATACTACGCCAACTCCGTCAAGGGA AGGTTCACAATCTCTAGGGACAACGCCAAGAACACTGCCTATCTGCAGAT GAACTCCCTCAAGCCAGAGGACACTGCCATCTACTACTGCGCCGTGGATT TCAGAGGCGGACTGCTGTATAGGCCAGCCTACGAGTACACTTATAGGGGC CAAGGCACACAAGTGACAGTCTCGAGCGGCGGAGGATCCCAAGTGCAGC TGCAAGAGAGCGGAGGAGGAAGCGTCCAAGCCGGAGGCTCTCTGAGGCT GAGCTGTGGAGCCAGCGGCTACACTTACAGCAGCTACTGTATGGGCTGGT TTAGGCAAGTGCCCGGCAAGGAGAGAGAGGGCGTGGCCGTGATCGATTC CGATGGCAGCACAAGCTACGCTGACAGCGTGAAGGGAAGGTTCACAATC AGCAAGGACAACGGCAAGAACACACTCTATCTGCAGATGAACAGCCTCA AGCCAGAGGACACAGCCATGTACTACTGCGCCGCTGATCTGGGCCACTAT AGGCCTCCTTGTGGCGTGCTGTATCTGGGCATGGATTACTGGGGCAAGGG CACACAAGTGACAGTCTCGTCTGCTAGCCACCATCACCATCACCAC |
| **366** | DNA Sequence Encoding SEQ ID NO: 268 | CAAGTGCAGCTGCAAGAGTCCGGCGGAGGCAGCGTCCAAGCCGGAGGAT CTCTGAGGCTGAGCTGTACAGTGAGCAGATACACTGCCAGCGTGAACTAC ATGGGCTGGTTCAGACAAGCCCCCGGCAAAGAGAGAGAGGGCGTGGCCA CAATCTTCACTGGCGCCGGCACAACATACTACGCCAACTCCGTCAAGGGA AGGTTCACAATCTCTAGGGACAACGCCAAGAACACTGCCTATCTGCAGAT GAACTCCCTCAAGCCAGAGGACACTGCCATCTACTACTGCGCCGTGGATT TCAGAGGCGGACTGCTGTATAGGCCAGCCTACGAGTACACTTATAGGGGC CAAGGCACACAAGTGACAGTCTCGAGCGGCGGAGGATCCCAAGTGCAGC TGCAAGAGAGCGGAGGAGGAAGCGTCCAAGCCGGAGGCTCTCTGAGACT GAGCTGTGCCGCCAGCGGCTACTCCAACTGCAGCTACGACATGACTTGGT ATAGGCAAGCCCCCGGCAAGGAGAGGGAGTTCGTGTCCGCCATCCACAG CGACGGCAGCACTAGATACGCCGACAGCGTGAAGGGAAGGTTCTTCATC AGCCAAGATAACGCCAAGAACACAGTGTATCTGCAGATGAACTCCCTCAA GCCAGAGGACACTGCCATGTACTACTGCAAGACAGACCCACTGCACTGCA GAGCCCATGGCGGCAGCTGGTATAGCGTGAGGGCCAACTACTGGGGCCA AGGCACACAAGTGACAGTCTCGTCTGCTAGCCACCATCACCATCACCAC |
| **367** | DNA Sequence Encoding SEQ ID NO: 269 | CAAGTGCAGCTGCAAGAGAGCGGAGGAGGCAGCGTCGAAGCTGGAGGAT CTCTGAGGCTGAGCTGTGCTGCCAGCGGCTACACTCACAGCAGCTACTGT ATGGGCTGGTTCAGACAAGCCCCCGGCAAGGAGAGGGAAGGCGTGGCTG CCATCGACGTGGATGGCAGCACTACTTACGCCGACAGCGTGAAGGGAAG GTTCACTATCAGCAAGGACAACGCCAAGAACACACTCTATCTGCAGATGA ACAGCCTCAAGCCAGAGGACACTGGCATGTACTACTGCGCCGCCGAGTTC GCCGATTGCAGCAGCAACTACTTTCTGCCTCCCGGCGCCGTCAGATATTG GGGCCAAGGCACTCAAGTGACAGTCTCGAGCGGCGGAGGATCCCAAGTG CAGCTGCAAGAGAGCGGAGGAGGAAGCGTGCAAGCCGGAGGCTCTCTGA GACTGAGCTGTGCCGCCTCTAGGTATCTGTACAGCATCGACTACATGGCT TGGTTCAGACAGAGCCCCGGCAAGGAGAGGGAGCCAGTGGCTGTCATCT ACACTGCCTCCGGCGCCACATTCTATCCAGATAGCGTGAAGGGAAGGTTC ACTATCAGCCAAGATAACGCCAAGATGACAGTGTATCTGCAGATGAACTC TCTGAAGAGCGAGGACACTGCCATGTACTACTGTGCCGCCGTGAGGAAGA CAGATAGCTACCTCTTCGACGCCCAGAGCTTCACATACTGGGGCCAAGGC ACACAAGTGACAGTCTCGTCTGCTAGCCACCATCACCATCACCAC |
| **368** | DNA Sequence Encoding SEQ ID NO: 270 | CAAGTGCAGCTGCAAGAGAGCGGAGGAGGCAGCGTCGAAGCTGGAGGAT CTCTGAGGCTGAGCTGTGCTGCCAGCGGCTACACTCACAGCAGCTACTGT ATGGGCTGGTTCAGACAAGCCCCCGGCAAGGAGAGGGAAGGCGTGGCTG CCATCGACGTGGATGGCAGCACTACTTACGCCGACAGCGTGAAGGGAAG GTTCACTATCAGCAAGGACAACGCCAAGAACACACTCTATCTGCAGATGA ACAGCCTCAAGCCAGAGGACACTGGCATGTACTACTGCGCCGCCGAGTTC GCCGATTGCAGCAGCAACTACTTTCTGCCTCCCGGCGCCGTCAGATATTG GGGCCAAGGCACTCAAGTGACAGTCTCGAGCGGCGGAGGATCCCAAGTG CAGCTGCAAGAGAGCGGAGGAGGAAGCGTGCAAGCCGGAGGCTCTCTGA GGCTGAGCTGTGCCGCCTCTAGGTTCACATACAGCAGCTACTGCATGGGC TGGTTCAGACAAGCCCCCGGCAAAGAGAGAGAAGGCGTGGCCAGCATCG ATAGCGATGGCTCCACTAGCTACACTGACAGCGTGAAGGGAAGGTTCACT ATCAGCAAGGACAACGCCAAGAACACTCTGTATCTGCAGATGAACTCTCT GAAGCCAGAGGACACAGCCATGTACTACTGTGCCCTCGATCTGATGAGCA CAGTGGTGCCCGGCTTCTGTGGCTTTCTGCTGAGCGCTGGCATGGATTACT GGGGCAAGGGCACTCAAGTGACTGTCTCGTCTGCTAGCCACCATCACCAT CACCAC |
| **369** | DNA Sequence Encoding SEQ ID NO: 271 | CAAGTGCAGCTGCAAGAGAGCGGAGGAGGCAGCGTCGAAGCTGGAGGAT CTCTGAGGCTGAGCTGTGCTGCCAGCGGCTACACTCACAGCAGCTACTGT ATGGGCTGGTTCAGACAAGCCCCCGGCAAGGAGAGGGAAGGCGTGGCTG CCATCGACGTGGATGGCAGCACTACTTACGCCGACAGCGTGAAGGGAAG GTTCACTATCAGCAAGGACAACGCCAAGAACACACTCTATCTGCAGATGA ACAGCCTCAAGCCAGAGGACACTGGCATGTACTACTGCGCCGCCGAGTTC GCCGATTGCAGCAGCAACTACTTTCTGCCTCCCGGCGCCGTCAGATATTG GGGCCAAGGCACTCAAGTGACAGTCTCGAGCGGCGGAGGATCCCAAGTG CAGCTGCAAGAGTCCGGAGGAGGCAGCGTCCAAGCCGGAGGCTCTCTGA GGCTGAGCTGTGCTGCCAGCGGCTACACTTACAGCATGTACTGCATGGGC TGGTTCAGACAAGCCCCCGGCAAGGAAAGAGAGGGCGTGGCCCAGATCA ATAGCGATGGCAGCACAAGCTACGCCGACAGCGTGAAGGGAAGGTTCAC TATCTCCAAGGACAACGCCAAGAACACTCTGTATCTGCAGATGAACTCTC TGAAGCCAGAGGACACTGCCATGTACTACTGCGCTGCCGATTCTAGGGTG TACGGCGGCAGCTGGTATGAGAGGCTCTGCGGCCCTTACACATACGAGTA CAACTACTGGGGCCAAGGCACACAAGTGACTGTCTCGTCTGCTAGCCACC ATCACCATCACCAC |
| **370** | DNA Sequence Encoding SEQ ID NO: 272 | CAAGTGCAGCTGCAAGAGAGCGGAGGAGGCAGCGTCGAAGCTGGAGGAT CTCTGAGGCTGAGCTGTGCTGCCAGCGGCTACACTCACAGCAGCTACTGT ATGGGCTGGTTCAGACAAGCCCCCGGCAAGGAGAGGGAAGGCGTGGCTG CCATCGACGTGGATGGCAGCACTACTTACGCCGACAGCGTGAAGGGAAG GTTCACTATCAGCAAGGACAACGCCAAGAACACACTCTATCTGCAGATGA ACAGCCTCAAGCCAGAGGACACTGGCATGTACTACTGCGCCGCCGAGTTC GCCGATTGCAGCAGCAACTACTTTCTGCCTCCCGGCGCCGTCAGATATTG GGGCCAAGGCACTCAAGTGACAGTCTCGAGCGGCGGAGGATCCCAAGTG CAGCTGCAAGAGAGCGGCGGAGGAAGCGTGCAAGCCGGAGGATCTCTGA GACTGAGCTGCGCTGTGAGCGGCTACGCCTACTCCACATACTGCATGGGC TGGTTTAGGCAAGCCCCCGGCAAAGAGAGAGAGGGCGTGGCTGCTATCG ATAGCGGCGGCAGCACAAGCTACGCCGATAGCGTGAAGGGAAGGTTCAC AATCAGCAAGGACAACGCCAAGAACACACTGTATCTGAGGATGAACTCT CTGAAGCCAGAGGACACAGCCATGTACTACTGTGCTGCTGTGCCTCCTCC TCCAGATGGCGGCAGCTGTCTGTTTCTGGGACCAGAGATCAAGGTCAGCA AGGCCGATTTTAGGTACTGGGGCCAAGGCACACAAGTGACAGTCTCGTCT GCTAGCCACCATCACCATCACCAC |
| **371** | DNA Sequence Encoding SEQ ID NO: 273 | CAAGTGCAGCTGCAAGAGAGCGGAGGAGGCAGCGTCGAAGCTGGAGGAT CTCTGAGGCTGAGCTGTGCTGCCAGCGGCTACACTCACAGCAGCTACTGT ATGGGCTGGTTCAGACAAGCCCCCGGCAAGGAGAGGGAAGGCGTGGCTG CCATCGACGTGGATGGCAGCACTACTTACGCCGACAGCGTGAAGGGAAG GTTCACTATCAGCAAGGACAACGCCAAGAACACACTCTATCTGCAGATGA ACAGCCTCAAGCCAGAGGACACTGGCATGTACTACTGCGCCGCCGAGTTC GCCGATTGCAGCAGCAACTACTTTCTGCCTCCCGGCGCCGTCAGATATTG GGGCCAAGGCACTCAAGTGACAGTCTCGAGCGGCGGAGGATCCCAAGTG CAGCTGCAAGAGAGCGGAGGAGGAAGCGTGCAAGCCGGAGGCTCTCTGA GGCTGAGCTGTACAGTGTCCGGCTACACTTACAGCTCCAATTGCATGGGC TGGTTTAGGCAAGCCCCCGGCAAGGAAAGAGAGGGCGTGGCCACTATCT ACACTGGCGGCGGCAACACATACTACGCCGATAGCGTGAAGGGAAGGTT CACTATCAGCCAAGATAACGCCAAGAACACAGTGTATCTGCAGATGAAC AATCTGAAGCCAGAGGACACTGCCATGTACTACTGTGCTGCTGAGCCACT GTCTAGGGTGTACGGCGGCAGCTGCCCAACTCCTACATTCGACTACTGGG GCCAAGGCACACAAGTGACTGTCTCGTCTGCTAGCCACCATCACCATCAC CAC |
| **372** | DNA Sequence Encoding SEQ ID NO: 274 | CAAGTGCAGCTGCAAGAGAGCGGAGGAGGCAGCGTCGAAGCTGGAGGAT CTCTGAGGCTGAGCTGTGCTGCCAGCGGCTACACTCACAGCAGCTACTGT ATGGGCTGGTTCAGACAAGCCCCCGGCAAGGAGAGGGAAGGCGTGGCTG CCATCGACGTGGATGGCAGCACTACTTACGCCGACAGCGTGAAGGGAAG GTTCACTATCAGCAAGGACAACGCCAAGAACACACTCTATCTGCAGATGA ACAGCCTCAAGCCAGAGGACACTGGCATGTACTACTGCGCCGCCGAGTTC GCCGATTGCAGCAGCAACTACTTTCTGCCTCCCGGCGCCGTCAGATATTG GGGCCAAGGCACTCAAGTGACAGTCTCGAGCGGCGGAGGATCCCAAGTG CAGCTGCAAGAGAGCGGAGGAGGAAGCGTCCAAGCCGGAGGCTCTCTGA GGCTGAGCTGTGGAGCCAGCGGCTACACTTACAGCAGCTACTGTATGGGC TGGTTTAGGCAAGTGCCCGGCAAGGAGAGAGAGGGCGTGGCCGTGATCG ATTCCGATGGCAGCACAAGCTACGCTGACAGCGTGAAGGGAAGGTTCAC AATCAGCAAGGACAACGGCAAGAACACACTCTATCTGCAGATGAACAGC CTCAAGCCAGAGGACACAGCCATGTACTACTGCGCCGCTGATCTGGGCCA CTATAGGCCTCCTTGTGGCGTGCTGTATCTGGGCATGGATTACTGGGGCA AGGGCACACAAGTGACAGTCTCGTCTGCTAGCCACCATCACCATCACCAC |
| **373** | DNA Sequence Encoding SEQ ID NO: 275 | CAAGTGCAGCTGCAAGAGAGCGGAGGAGGCAGCGTCGAAGCTGGAGGAT CTCTGAGGCTGAGCTGTGCTGCCAGCGGCTACACTCACAGCAGCTACTGT ATGGGCTGGTTCAGACAAGCCCCCGGCAAGGAGAGGGAAGGCGTGGCTG CCATCGACGTGGATGGCAGCACTACTTACGCCGACAGCGTGAAGGGAAG GTTCACTATCAGCAAGGACAACGCCAAGAACACACTCTATCTGCAGATGA ACAGCCTCAAGCCAGAGGACACTGGCATGTACTACTGCGCCGCCGAGTTC GCCGATTGCAGCAGCAACTACTTTCTGCCTCCCGGCGCCGTCAGATATTG GGGCCAAGGCACTCAAGTGACAGTCTCGAGCGGCGGAGGATCCCAAGTG CAGCTGCAAGAGAGCGGAGGAGGAAGCGTCCAAGCCGGAGGCTCTCTGA GACTGAGCTGTGCCGCCAGCGGCTACTCCAACTGCAGCTACGACATGACT TGGTATAGGCAAGCCCCCGGCAAGGAGAGGGAGTTCGTGTCCGCCATCCA CAGCGACGGCAGCACTAGATACGCCGACAGCGTGAAGGGAAGGTTCTTC ATCAGCCAAGATAACGCCAAGAACACAGTGTATCTGCAGATGAACTCCCT CAAGCCAGAGGACACTGCCATGTACTACTGCAAGACAGACCCACTGCACT GCAGAGCCCATGGCGGCAGCTGGTATAGCGTGAGGGCCAACTACTGGGG CCAAGGCACACAAGTGACAGTCTCGTCTGCTAGCCACCATCACCATCACC AC |
| **374** | DNA Sequence Encoding SEQ ID NO: 276 | CAAGTGCAGCTGCAAGAGAGCGGAGGAGGAAGCGTCCAAGCCGGAGGAT CTCTGAGACTGAGCTGCGCCGCTAGTGGCTACTCCTACAGCAGCTACTGC ATGGGCTGGTTTAGGCAAGCCCCCGGCAAGGAGAGAGAAGGCGTGGCCA CTATCGACAGCGACGGCATGACAAGGTACGCCGACAGCGTGAAGGGAAG GTTCACAATCAGCAAGGACAACGCCAAGAACACACTGTATCTGCAGATG AACTCTCTGAAGCCAGAGGACACTGCCATGTACTACTGTGCCGCTCCTCT GTACGACTGTGATAGCGGCGCTGTGGGCAGAAATCCACCTTATTGGGGCC AAGGCACTCAAGTGACAGTCTCGAGCGGCGGAGGATCCCAAGTGCAGCT GCAAGAGAGCGGAGGAGGAAGCGTGCAAGCCGGAGGCTCTCTGAGACTG AGCTGTGCCGCCTCTAGGTATCTGTACAGCATCGACTACATGGCTTGGTTC AGACAGAGCCCCGGCAAGGAGAGGGAGCCAGTGGCTGTCATCTACACTG CCTCCGGCGCCACATTCTATCCAGATAGCGTGAAGGGAAGGTTCACTATC AGCCAAGATAACGCCAAGATGACAGTGTATCTGCAGATGAACTCTCTGAA GAGCGAGGACACTGCCATGTACTACTGTGCCGCCGTGAGGAAGACAGAT AGCTACCTCTTCGACGCCCAGAGCTTCACATACTGGGGCCAAGGCACACA AGTGACAGTCTCGTCTGCTAGCCACCATCACCATCACCAC |
| **375** | DNA Sequence Encoding SEQ ID NO: 277 | CAAGTGCAGCTGCAAGAGAGCGGAGGAGGAAGCGTCCAAGCCGGAGGAT CTCTGAGACTGAGCTGCGCCGCTAGTGGCTACTCCTACAGCAGCTACTGC ATGGGCTGGTTTAGGCAAGCCCCCGGCAAGGAGAGAGAAGGCGTGGCCA CTATCGACAGCGACGGCATGACAAGGTACGCCGACAGCGTGAAGGGAAG GTTCACAATCAGCAAGGACAACGCCAAGAACACACTGTATCTGCAGATG AACTCTCTGAAGCCAGAGGACACTGCCATGTACTACTGTGCCGCTCCTCT GTACGACTGTGATAGCGGCGCTGTGGGCAGAAATCCACCTTATTGGGGCC AAGGCACTCAAGTGACAGTCTCGAGCGGCGGAGGATCCCAAGTGCAGCT GCAAGAGAGCGGAGGAGGAAGCGTGCAAGCCGGAGGCTCTCTGAGGCTG AGCTGTGCCGCCTCTAGGTTCACATACAGCAGCTACTGCATGGGCTGGTT CAGACAAGCCCCCGGCAAAGAGAGAGAAGGCGTGGCCAGCATCGATAGC GATGGCTCCACTAGCTACACTGACAGCGTGAAGGGAAGGTTCACTATCAG CAAGGACAACGCCAAGAACACTCTGTATCTGCAGATGAACTCTCTGAAGC CAGAGGACACAGCCATGTACTACTGTGCCCTCGATCTGATGAGCACAGTG GTGCCCGGCTTCTGTGGCTTTCTGCTGAGCGCTGGCATGGATTACTGGGGC AAGGGCACTCAAGTGACTGTCTCGTCTGCTAGCCACCATCACCATCACCA C |
| **376** | DNA Sequence Encoding SEQ ID NO: 278 | CAAGTGCAGCTGCAAGAGAGCGGAGGAGGAAGCGTCCAAGCCGGAGGAT CTCTGAGACTGAGCTGCGCCGCTAGTGGCTACTCCTACAGCAGCTACTGC ATGGGCTGGTTTAGGCAAGCCCCCGGCAAGGAGAGAGAAGGCGTGGCCA CTATCGACAGCGACGGCATGACAAGGTACGCCGACAGCGTGAAGGGAAG GTTCACAATCAGCAAGGACAACGCCAAGAACACACTGTATCTGCAGATG AACTCTCTGAAGCCAGAGGACACTGCCATGTACTACTGTGCCGCTCCTCT GTACGACTGTGATAGCGGCGCTGTGGGCAGAAATCCACCTTATTGGGGCC AAGGCACTCAAGTGACAGTCTCGAGCGGCGGAGGATCCCAAGTGCAGCT GCAAGAGTCCGGAGGAGGCAGCGTCCAAGCCGGAGGCTCTCTGAGGCTG AGCTGTGCTGCCAGCGGCTACACTTACAGCATGTACTGCATGGGCTGGTT CAGACAAGCCCCCGGCAAGGAAAGAGAGGGCGTGGCCCAGATCAATAGC GATGGCAGCACAAGCTACGCCGACAGCGTGAAGGGAAGGTTCACTATCT CCAAGGACAACGCCAAGAACACTCTGTATCTGCAGATGAACTCTCTGAAG CCAGAGGACACTGCCATGTACTACTGCGCTGCCGATTCTAGGGTGTACGG CGGCAGCTGGTATGAGAGGCTCTGCGGCCCTTACACATACGAGTACAACT ACTGGGGCCAAGGCACACAAGTGACTGTCTCGTCTGCTAGCCACCATCAC CATCACCAC |
| **377** | DNA Sequence Encoding SEQ ID NO: 279 | CAAGTGCAGCTGCAAGAGAGCGGAGGAGGAAGCGTCCAAGCCGGAGGAT CTCTGAGACTGAGCTGCGCCGCTAGTGGCTACTCCTACAGCAGCTACTGC ATGGGCTGGTTTAGGCAAGCCCCCGGCAAGGAGAGAGAAGGCGTGGCCA CTATCGACAGCGACGGCATGACAAGGTACGCCGACAGCGTGAAGGGAAG GTTCACAATCAGCAAGGACAACGCCAAGAACACACTGTATCTGCAGATG AACTCTCTGAAGCCAGAGGACACTGCCATGTACTACTGTGCCGCTCCTCT GTACGACTGTGATAGCGGCGCTGTGGGCAGAAATCCACCTTATTGGGGCC AAGGCACTCAAGTGACAGTCTCGAGCGGCGGAGGATCCCAAGTGCAGCT GCAAGAGAGCGGCGGAGGAAGCGTGCAAGCCGGAGGATCTCTGAGACTG AGCTGCGCTGTGAGCGGCTACGCCTACTCCACATACTGCATGGGCTGGTT TAGGCAAGCCCCCGGCAAAGAGAGAGAGGGCGTGGCTGCTATCGATAGC GGCGGCAGCACAAGCTACGCCGATAGCGTGAAGGGAAGGTTCACAATCA GCAAGGACAACGCCAAGAACACACTGTATCTGAGGATGAACTCTCTGAA GCCAGAGGACACAGCCATGTACTACTGTGCTGCTGTGCCTCCTCCTCCAG ATGGCGGCAGCTGTCTGTTTCTGGGACCAGAGATCAAGGTCAGCAAGGCC GATTTTAGGTACTGGGGCCAAGGCACACAAGTGACAGTCTCGTCTGCTAG CCACCATCACCATCACCAC |
| **378** | DNA Sequence Encoding SEQ ID NO: 280 | CAAGTGCAGCTGCAAGAGAGCGGAGGAGGAAGCGTCCAAGCCGGAGGAT CTCTGAGACTGAGCTGCGCCGCTAGTGGCTACTCCTACAGCAGCTACTGC ATGGGCTGGTTTAGGCAAGCCCCCGGCAAGGAGAGAGAAGGCGTGGCCA CTATCGACAGCGACGGCATGACAAGGTACGCCGACAGCGTGAAGGGAAG GTTCACAATCAGCAAGGACAACGCCAAGAACACACTGTATCTGCAGATG AACTCTCTGAAGCCAGAGGACACTGCCATGTACTACTGTGCCGCTCCTCT GTACGACTGTGATAGCGGCGCTGTGGGCAGAAATCCACCTTATTGGGGCC AAGGCACTCAAGTGACAGTCTCGAGCGGCGGAGGATCCCAAGTGCAGCT GCAAGAGAGCGGAGGAGGAAGCGTGCAAGCCGGAGGCTCTCTGAGGCTG AGCTGTACAGTGTCCGGCTACACTTACAGCTCCAATTGCATGGGCTGGTTT AGGCAAGCCCCCGGCAAGGAAAGAGAGGGCGTGGCCACTATCTACACTG GCGGCGGCAACACATACTACGCCGATAGCGTGAAGGGAAGGTTCACTAT CAGCCAAGATAACGCCAAGAACACAGTGTATCTGCAGATGAACAATCTG AAGCCAGAGGACACTGCCATGTACTACTGTGCTGCTGAGCCACTGTCTAG GGTGTACGGCGGCAGCTGCCCAACTCCTACATTCGACTACTGGGGCCAAG GCACACAAGTGACTGTCTCGTCTGCTAGCCACCATCACCATCACCAC |
| **379** | DNA Sequence Encoding SEQ ID NO: 281 | CAAGTGCAGCTGCAAGAGAGCGGAGGAGGAAGCGTCCAAGCCGGAGGAT CTCTGAGACTGAGCTGCGCCGCTAGTGGCTACTCCTACAGCAGCTACTGC ATGGGCTGGTTTAGGCAAGCCCCCGGCAAGGAGAGAGAAGGCGTGGCCA CTATCGACAGCGACGGCATGACAAGGTACGCCGACAGCGTGAAGGGAAG GTTCACAATCAGCAAGGACAACGCCAAGAACACACTGTATCTGCAGATG AACTCTCTGAAGCCAGAGGACACTGCCATGTACTACTGTGCCGCTCCTCT GTACGACTGTGATAGCGGCGCTGTGGGCAGAAATCCACCTTATTGGGGCC AAGGCACTCAAGTGACAGTCTCGAGCGGCGGAGGATCCCAAGTGCAGCT GCAAGAGAGCGGAGGAGGAAGCGTCCAAGCCGGAGGCTCTCTGAGGCTG AGCTGTGGAGCCAGCGGCTACACTTACAGCAGCTACTGTATGGGCTGGTT TAGGCAAGTGCCCGGCAAGGAGAGAGAGGGCGTGGCCGTGATCGATTCC GATGGCAGCACAAGCTACGCTGACAGCGTGAAGGGAAGGTTCACAATCA GCAAGGACAACGGCAAGAACACACTCTATCTGCAGATGAACAGCCTCAA GCCAGAGGACACAGCCATGTACTACTGCGCCGCTGATCTGGGCCACTATA GGCCTCCTTGTGGCGTGCTGTATCTGGGCATGGATTACTGGGGCAAGGGC ACACAAGTGACAGTCTCGTCTGCTAGCCACCATCACCATCACCAC |
| **380** | DNA Sequence Encoding SEQ ID NO: 282 | CAAGTGCAGCTGCAAGAGAGCGGAGGAGGAAGCGTCCAAGCCGGAGGAT CTCTGAGACTGAGCTGCGCCGCTAGTGGCTACTCCTACAGCAGCTACTGC ATGGGCTGGTTTAGGCAAGCCCCCGGCAAGGAGAGAGAAGGCGTGGCCA CTATCGACAGCGACGGCATGACAAGGTACGCCGACAGCGTGAAGGGAAG GTTCACAATCAGCAAGGACAACGCCAAGAACACACTGTATCTGCAGATG AACTCTCTGAAGCCAGAGGACACTGCCATGTACTACTGTGCCGCTCCTCT GTACGACTGTGATAGCGGCGCTGTGGGCAGAAATCCACCTTATTGGGGCC AAGGCACTCAAGTGACAGTCTCGAGCGGCGGAGGATCCCAAGTGCAGCT GCAAGAGAGCGGAGGAGGAAGCGTCCAAGCCGGAGGCTCTCTGAGACTG AGCTGTGCCGCCAGCGGCTACTCCAACTGCAGCTACGACATGACTTGGTA TAGGCAAGCCCCCGGCAAGGAGAGGGAGTTCGTGTCCGCCATCCACAGC GACGGCAGCACTAGATACGCCGACAGCGTGAAGGGAAGGTTCTTCATCA GCCAAGATAACGCCAAGAACACAGTGTATCTGCAGATGAACTCCCTCAAG CCAGAGGACACTGCCATGTACTACTGCAAGACAGACCCACTGCACTGCAG AGCCCATGGCGGCAGCTGGTATAGCGTGAGGGCCAACTACTGGGGCCAA GGCACACAAGTGACAGTCTCGTCTGCTAGCCACCATCACCATCACCAC |
| **381** | DNA Sequence Encoding SEQ ID NO: 283 | CAAGTGCAGCTGCAAGAGAGCGGAGGCGGCAGCGTGCAGACTGGAGGCT CTCTGAGACTGAGCTGTGCTGCCAGCGGCTACACTTATCTGAGGGGCTGT ATGGGCTGGTTTAGGCAAGCCCCCGGCAAGGAGAGAGAGGGCGTGGCCG TCATGGATGTGGTGGGCGATAGGAGAAGCTACATCGACAGCGTGAAGGG AAGGTTCACAATCTCTAGGGACAATGCCGCCAACAGCGTCTATCTGCAGA TGGACAATCTGAAGCCAGAGGACACAGCCATGTACTACTGCACTGCCGGC CCTAACTGTGTGGGCTGGAGAAGCGGACTGGATTACTGGGGCCAAGGCA CACAAGTGACAGTCTCGAGCGGCGGAGGATCCCAAGTGCAGCTGCAAGA GAGCGGAGGAGGAAGCGTGCAAGCCGGAGGCTCTCTGAGACTGAGCTGT GCCGCCTCTAGGTATCTGTACAGCATCGACTACATGGCTTGGTTCAGACA GAGCCCCGGCAAGGAGAGGGAGCCAGTGGCTGTCATCTACACTGCCTCCG GCGCCACATTCTATCCAGATAGCGTGAAGGGAAGGTTCACTATCAGCCAA GATAACGCCAAGATGACAGTGTATCTGCAGATGAACTCTCTGAAGAGCGA GGACACTGCCATGTACTACTGTGCCGCCGTGAGGAAGACAGATAGCTACC TCTTCGACGCCCAGAGCTTCACATACTGGGGCCAAGGCACACAAGTGACA GTCTCGTCTGCTAGCCACCATCACCATCACCAC |
| **382** | DNA Sequence Encoding SEQ ID NO: 284 | CAAGTGCAGCTGCAAGAGAGCGGAGGCGGCAGCGTGCAGACTGGAGGCT CTCTGAGACTGAGCTGTGCTGCCAGCGGCTACACTTATCTGAGGGGCTGT ATGGGCTGGTTTAGGCAAGCCCCCGGCAAGGAGAGAGAGGGCGTGGCCG TCATGGATGTGGTGGGCGATAGGAGAAGCTACATCGACAGCGTGAAGGG AAGGTTCACAATCTCTAGGGACAATGCCGCCAACAGCGTCTATCTGCAGA TGGACAATCTGAAGCCAGAGGACACAGCCATGTACTACTGCACTGCCGGC CCTAACTGTGTGGGCTGGAGAAGCGGACTGGATTACTGGGGCCAAGGCA CACAAGTGACAGTCTCGAGCGGCGGAGGATCCCAAGTGCAGCTGCAAGA GAGCGGAGGAGGAAGCGTGCAAGCCGGAGGCTCTCTGAGGCTGAGCTGT GCCGCCTCTAGGTTCACATACAGCAGCTACTGCATGGGCTGGTTCAGACA AGCCCCCGGCAAAGAGAGAGAAGGCGTGGCCAGCATCGATAGCGATGGC TCCACTAGCTACACTGACAGCGTGAAGGGAAGGTTCACTATCAGCAAGGA CAACGCCAAGAACACTCTGTATCTGCAGATGAACTCTCTGAAGCCAGAGG ACACAGCCATGTACTACTGTGCCCTCGATCTGATGAGCACAGTGGTGCCC GGCTTCTGTGGCTTTCTGCTGAGCGCTGGCATGGATTACTGGGGCAAGGG CACTCAAGTGACTGTCTCGTCTGCTAGCCACCATCACCATCACCAC |
| **383** | DNA Sequence Encoding SEQ ID NO: 285 | CAAGTGCAGCTGCAAGAGAGCGGAGGCGGCAGCGTGCAGACTGGAGGCT CTCTGAGACTGAGCTGTGCTGCCAGCGGCTACACTTATCTGAGGGGCTGT ATGGGCTGGTTTAGGCAAGCCCCCGGCAAGGAGAGAGAGGGCGTGGCCG TCATGGATGTGGTGGGCGATAGGAGAAGCTACATCGACAGCGTGAAGGG AAGGTTCACAATCTCTAGGGACAATGCCGCCAACAGCGTCTATCTGCAGA TGGACAATCTGAAGCCAGAGGACACAGCCATGTACTACTGCACTGCCGGC CCTAACTGTGTGGGCTGGAGAAGCGGACTGGATTACTGGGGCCAAGGCA CACAAGTGACAGTCTCGAGCGGCGGAGGATCCCAAGTGCAGCTGCAAGA GTCCGGAGGAGGCAGCGTCCAAGCCGGAGGCTCTCTGAGGCTGAGCTGT GCTGCCAGCGGCTACACTTACAGCATGTACTGCATGGGCTGGTTCAGACA AGCCCCCGGCAAGGAAAGAGAGGGCGTGGCCCAGATCAATAGCGATGGC AGCACAAGCTACGCCGACAGCGTGAAGGGAAGGTTCACTATCTCCAAGG ACAACGCCAAGAACACTCTGTATCTGCAGATGAACTCTCTGAAGCCAGAG GACACTGCCATGTACTACTGCGCTGCCGATTCTAGGGTGTACGGCGGCAG CTGGTATGAGAGGCTCTGCGGCCCTTACACATACGAGTACAACTACTGGG GCCAAGGCACACAAGTGACTGTCTCGTCTGCTAGCCACCATCACCATCAC CAC |
| **384** | DNA Sequence Encoding SEQ ID NO: 286 | CAAGTGCAGCTGCAAGAGAGCGGAGGCGGCAGCGTGCAGACTGGAGGCT CTCTGAGACTGAGCTGTGCTGCCAGCGGCTACACTTATCTGAGGGGCTGT ATGGGCTGGTTTAGGCAAGCCCCCGGCAAGGAGAGAGAGGGCGTGGCCG TCATGGATGTGGTGGGCGATAGGAGAAGCTACATCGACAGCGTGAAGGG AAGGTTCACAATCTCTAGGGACAATGCCGCCAACAGCGTCTATCTGCAGA TGGACAATCTGAAGCCAGAGGACACAGCCATGTACTACTGCACTGCCGGC CCTAACTGTGTGGGCTGGAGAAGCGGACTGGATTACTGGGGCCAAGGCA CACAAGTGACAGTCTCGAGCGGCGGAGGATCCCAAGTGCAGCTGCAAGA GAGCGGCGGAGGAAGCGTGCAAGCCGGAGGATCTCTGAGACTGAGCTGC GCTGTGAGCGGCTACGCCTACTCCACATACTGCATGGGCTGGTTTAGGCA AGCCCCCGGCAAAGAGAGAGAGGGCGTGGCTGCTATCGATAGCGGCGGC AGCACAAGCTACGCCGATAGCGTGAAGGGAAGGTTCACAATCAGCAAGG ACAACGCCAAGAACACACTGTATCTGAGGATGAACTCTCTGAAGCCAGA GGACACAGCCATGTACTACTGTGCTGCTGTGCCTCCTCCTCCAGATGGCG GCAGCTGTCTGTTTCTGGGACCAGAGATCAAGGTCAGCAAGGCCGATTTT AGGTACTGGGGCCAAGGCACACAAGTGACAGTCTCGTCTGCTAGCCACCA TCACCATCACCAC |
| **385** | DNA Sequence Encoding SEQ ID NO: 287 | CAAGTGCAGCTGCAAGAGAGCGGAGGCGGCAGCGTGCAGACTGGAGGCT CTCTGAGACTGAGCTGTGCTGCCAGCGGCTACACTTATCTGAGGGGCTGT ATGGGCTGGTTTAGGCAAGCCCCCGGCAAGGAGAGAGAGGGCGTGGCCG TCATGGATGTGGTGGGCGATAGGAGAAGCTACATCGACAGCGTGAAGGG AAGGTTCACAATCTCTAGGGACAATGCCGCCAACAGCGTCTATCTGCAGA TGGACAATCTGAAGCCAGAGGACACAGCCATGTACTACTGCACTGCCGGC CCTAACTGTGTGGGCTGGAGAAGCGGACTGGATTACTGGGGCCAAGGCA CACAAGTGACAGTCTCGAGCGGCGGAGGATCCCAAGTGCAGCTGCAAGA GAGCGGAGGAGGAAGCGTGCAAGCCGGAGGCTCTCTGAGGCTGAGCTGT ACAGTGTCCGGCTACACTTACAGCTCCAATTGCATGGGCTGGTTTAGGCA AGCCCCCGGCAAGGAAAGAGAGGGCGTGGCCACTATCTACACTGGCGGC GGCAACACATACTACGCCGATAGCGTGAAGGGAAGGTTCACTATCAGCC AAGATAACGCCAAGAACACAGTGTATCTGCAGATGAACAATCTGAAGCC AGAGGACACTGCCATGTACTACTGTGCTGCTGAGCCACTGTCTAGGGTGT ACGGCGGCAGCTGCCCAACTCCTACATTCGACTACTGGGGCCAAGGCACA CAAGTGACTGTCTCGTCTGCTAGCCACCATCACCATCACCAC |
| **386** | DNA Sequence Encoding SEQ ID NO: 288 | CAAGTGCAGCTGCAAGAGAGCGGAGGCGGCAGCGTGCAGACTGGAGGCT CTCTGAGACTGAGCTGTGCTGCCAGCGGCTACACTTATCTGAGGGGCTGT ATGGGCTGGTTTAGGCAAGCCCCCGGCAAGGAGAGAGAGGGCGTGGCCG TCATGGATGTGGTGGGCGATAGGAGAAGCTACATCGACAGCGTGAAGGG AAGGTTCACAATCTCTAGGGACAATGCCGCCAACAGCGTCTATCTGCAGA TGGACAATCTGAAGCCAGAGGACACAGCCATGTACTACTGCACTGCCGGC CCTAACTGTGTGGGCTGGAGAAGCGGACTGGATTACTGGGGCCAAGGCA CACAAGTGACAGTCTCGAGCGGCGGAGGATCCCAAGTGCAGCTGCAAGA GAGCGGAGGAGGAAGCGTCCAAGCCGGAGGCTCTCTGAGGCTGAGCTGT GGAGCCAGCGGCTACACTTACAGCAGCTACTGTATGGGCTGGTTTAGGCA AGTGCCCGGCAAGGAGAGAGAGGGCGTGGCCGTGATCGATTCCGATGGC AGCACAAGCTACGCTGACAGCGTGAAGGGAAGGTTCACAATCAGCAAGG ACAACGGCAAGAACACACTCTATCTGCAGATGAACAGCCTCAAGCCAGA GGACACAGCCATGTACTACTGCGCCGCTGATCTGGGCCACTATAGGCCTC CTTGTGGCGTGCTGTATCTGGGCATGGATTACTGGGGCAAGGGCACACAA GTGACAGTCTCGTCTGCTAGCCACCATCACCATCACCAC |
| **387** | DNA Sequence Encoding SEQ ID NO: 289 | CAAGTGCAGCTGCAAGAGAGCGGAGGCGGCAGCGTGCAGACTGGAGGCT CTCTGAGACTGAGCTGTGCTGCCAGCGGCTACACTTATCTGAGGGGCTGT ATGGGCTGGTTTAGGCAAGCCCCCGGCAAGGAGAGAGAGGGCGTGGCCG TCATGGATGTGGTGGGCGATAGGAGAAGCTACATCGACAGCGTGAAGGG AAGGTTCACAATCTCTAGGGACAATGCCGCCAACAGCGTCTATCTGCAGA TGGACAATCTGAAGCCAGAGGACACAGCCATGTACTACTGCACTGCCGGC CCTAACTGTGTGGGCTGGAGAAGCGGACTGGATTACTGGGGCCAAGGCA CACAAGTGACAGTCTCGAGCGGCGGAGGATCCCAAGTGCAGCTGCAAGA GAGCGGAGGAGGAAGCGTCCAAGCCGGAGGCTCTCTGAGACTGAGCTGT GCCGCCAGCGGCTACTCCAACTGCAGCTACGACATGACTTGGTATAGGCA AGCCCCCGGCAAGGAGAGGGAGTTCGTGTCCGCCATCCACAGCGACGGC AGCACTAGATACGCCGACAGCGTGAAGGGAAGGTTCTTCATCAGCCAAG ATAACGCCAAGAACACAGTGTATCTGCAGATGAACTCCCTCAAGCCAGAG GACACTGCCATGTACTACTGCAAGACAGACCCACTGCACTGCAGAGCCCA TGGCGGCAGCTGGTATAGCGTGAGGGCCAACTACTGGGGCCAAGGCACA CAAGTGACAGTCTCGTCTGCTAGCCACCATCACCATCACCAC |
| **388** | SEQ ID NO: 44 CDR 1 | IDYMA |
| **389** | SEQ ID NO: 44 CDR 2 | VIYTASGATFYPDSVKG |
| **390** | SEQ ID NO: 44 CDR 3 | VRKTDSYLFDAQSFTY |
| **391** | SEQ ID NO: 45 CDR 1 | SYCMG |
| **392** | SEQ ID NO: 45 CDR 2 | SIDSDGSTSYTDSVKG |
| **393** | SEQ ID NO: 45 CDR 3 | DLMSTVVPGFCGFLLSAGMDY |
| **394** | SEQ ID NO: 46 CDR 1 | MYCMG |
| **395** | SEQ ID NO: 46 CDR 2 | QINSDGSTSYADSVKG |
| **396** | SEQ ID NO: 46 CDR 3 | DSRVYGGSWYERLCGPYTYEYNY |
| **397** | SEQ ID NO: 47 CDR 1 | TYCMG |
| **398** | SEQ ID NO: 47 CDR 2 | AIDSGGSTSYADSVKG |
| **399** | SEQ ID NO: 47 CDR 3 | VPPPPDGGSCLFLGPEIKVSKADFRY |
| **400** | SEQ ID NO: 48 CDR 1 | SNCMG |
| **401** | SEQ ID NO: 48 CDR 2 | TIYTGGGNTYYADSVKG |
| **402** | SEQ ID NO: 48 CDR 3 | EPLSRVYGGSCPTPTFDY |
| **403** | SEQ ID NO: 49 CDR 1 | SYCMG |
| **404** | SEQ ID NO: 49 CDR 2 | VIDSDGSTSYADSVKG |
| **405** | SEQ ID NO: 49 CDR 3 | DLGHYRPPCGVLYLGMDY |
| **406** | SEQ ID NO: 50 CDR 1 | SYDMT |
| **407** | SEQ ID NO: 50 CDR 2 | AIHSDGSTRYADSVKG |
| **408** | SEQ ID NO: 50 CDR 3 | DPLHCRAHGGSWYSVRANY |
| **409** | SEQ ID NO: 51 CDR 1 | SGCMG |
| **410** | SEQ ID NO: 51 CDR 2 | AINSDGSTSYADSVKG |
| **411** | SEQ ID NO: 51 CDR 3 | EPYCSGGYPR |
| **412** | SEQ ID NO: 52 CDR 1 | SYCMG |
| **413** | SEQ ID NO: 52 CDR 2 | HIDSDGSTSYADSVKG |
| **414** | SEQ ID NO: 52 CDR 3 | DPIPGPGYCDGGPNKY |
| **415** | SEQ ID NO: 53 CDR 1 | SYCMG |
| **416** | SEQ ID NO: 53 CDR 2 | TIDSDGMTRY ADSVKG |
| **417** | SEQ ID NO: 53 CDR 3 | DADCTIAAMTTNPL |
| **418** | SEQ ID NO: 54 CDR 1 | VNYMG |
| **419** | SEQ ID NO: 54 CDR 2 | TIFTGAGTTYYANSVKG |
| **420** | SEQ ID NO: 54 CDR 3 | DFRGGLLYRPAYEYTYR |
| **421** | SEQ ID NO: 55 CDR 1 | VNYMG |
| **422** | SEQ ID NO: 55 CDR 2 | TIFTGAGTTYYANSVKG |
| **423** | SEQ ID NO: 55 CDR 3 | DFRGGLLYRPAYEYTYR |
| **424** | SEQ ID NO: 56 CDR 1 | SYCMG |
| **425** | SEQ ID NO: 56 CDR 2 | TIDSDGMTRY ADSVKG |
| **426** | SEQ ID NO: 56 CDR 3 | PLYDCDSGAVGRNPPY |
| **427** | SEQ ID NO: 57 CDR 1 | RGCMG |
| **428** | SEQ ID NO: 57 CDR 2 | VMDVVGDRRSYIDSVKG |
| **429** | SEQ ID NO: 57 CDR 3 | GPNCVGWRSGLDY |
| **430** | ASH6 purification handle | ASHHHHHH |

It is understood that the embodiments described herein are for illustrative purposes only and that various modifications or changes in light thereof will be suggested to persons skilled in the art and are to be included within the spirit and purview of this application and scope of the appended claims. The sequences of the sequence accession numbers cited herein are hereby incorporated by reference.
The following numbered aspects represent particular embodiments of the invention:
1. An IL12 receptor (IL12R) binding protein that specifically binds to IL12Rβ1 and IL12Rβ2,
wherein the binding protein causes the multimerization of IL12Rβ1 and IL12Rβ2 and downstream signaling, and
wherein the binding protein comprises a single-domain antibody (sdAb) that specifically binds to IL12Rβ1 (an anti-IL12Rβ1 sdAb) and a sdAb that specifically binds to IL12Rβ2 (an anti-IL12Rβ2 sdAb).
2. The IL12R binding protein of aspect 1, wherein the anti-IL12Rβ1 sdAb is a V_{H}H antibody (an anti-IL12Rβ1 V_{H}H antibody) and/or the anti-IL12Rβ2 sdAb is a V_{H}H antibody (an anti-IL12Rβ2 V_{H}H antibody).
3. The IL12R binding protein of aspect 1 or 2, wherein the anti-IL12Rβ1 sdAb and the anti-IL12Rβ2 sdAb are joined by a peptide linker.
4. The IL12R binding protein of aspect 3, wherein the peptide linker comprises between 1 and 50 amino acids.
5. The IL12R binding protein of any one of aspects 1 to 4, wherein the IL12R binding protein has a reduced Eₘₐₓ compared to IL12.
6. The IL12R binding protein of any one of aspects 1 to 5, wherein the IL12R binding protein has a similar potency compared to that of IL12.
7. A method for treating cancer in a subject in need thereof, comprising administering to the subject the IL12R binding protein of any one of aspects 1 to 6, wherein the IL12R binding protein binds to and activates natural killer, CD4⁺ T cells, and/or CD8⁺ T cells.
8. The method of aspect 7, wherein the cancer is a solid tumor cancer.
9. An IL27 receptor (IL27R) binding protein that specifically binds to IL27Rα subunit (IL27Rα) and glycoprotein 130 subunit (gp130),
wherein the binding protein causes the multimerization of IL27Rα and gp130 and downstream signaling, and
wherein the binding protein comprises a single-domain antibody (sdAb) that specifically binds to IL27Rα (an anti-IL27Rα sdAb) and a sdAb that specifically binds to gp130 (an anti-gp130 sdAb).
10. The IL27R binding protein of aspect 9, wherein the anti-IL27Rα sdAb is a V_{H}H antibody (an anti-IL27Rα V_{H}H antibody) and/or the anti-gp130 sdAb is a V_{H}H antibody (an anti-gp130 V_{H}H antibody).
11. The IL27R binding protein of any one of aspects 9 to 10, wherein the anti-IL27Rα sdAb and the anti-gp130 sdAb are joined by a peptide linker.
12. The IL27R binding protein of aspect 11, wherein the peptide linker comprises between 1 and 50 amino acids.
13. A method for treating cancer in a subject in need thereof, comprising administering to the subject the IL27R binding protein of any one of aspects 9 to 12, wherein the IL27R binding protein binds to and activates CD8⁺ T cells, CD4⁺ T cells, and/or T-regulatory (Treg) cells.
14. The method of aspect 13, wherein the IL27R binding protein binds to and activates CD8⁺ T cells.
15. The method of aspect 13 or 14, wherein the IL27R binding protein binds to and activates CXCR5⁺ CD8⁺ T cells.
16. The method of any one of aspects 13 to 15, wherein the cancer is a solid tumor cancer.
17. An ILI0 receptor (IL10R) binding protein that specifically binds to IL10R α subunit (IL10Rα) and IL10Rβ,
wherein the binding protein causes the multimerization of IL10Rα and IL10Rβ and downstream signaling, and
wherein the binding protein comprises a single-domain antibody (sdAb) that specifically binds to IL10Rα (an anti-IL10Rα sdAb) and a sdAb that specifically binds to IL10Rβ (an anti-IL10Rβ sdAb).
18. The IL10R binding protein of aspect 17, wherein the anti-IL10Rα sdAb is a V_{H}H antibody (an anti-IL10Rα V_{H}H antibody) and/or the anti-IL10Rβ sdAb is a V_{H}H antibody (an anti-IL10Rβ V_{H}H antibody).
19. The IL10R binding protein of any one of aspects 17 to 18, wherein the anti-IL10Rα sdAb and the anti-IL10Rβ sdAb are joined by a peptide linker.
20. The IL10R binding protein of aspect 19, wherein the peptide linker comprises between 1 and 50 amino acids.
21. A method for treating cancer in a subject in need thereof, comprising administering to the subject the IL10R binding protein of any one of aspects 17 to 20, wherein the IL10R binding protein binds to and activates CD8⁺ T cells, CD4⁺ T cells, macrophages, and/or Treg cells.
22. The method of aspect 21, wherein the IL10R binding protein provides longer therapeutic efficacy than a pegylated IL10.
23. The method of aspect 21 or 22, wherein the cancer is a solid tumor cancer.
24. An interferon (IFN) λ receptor (IFNλR) binding protein that specifically binds to IL10Rβ and IL28 receptor (IL28R) α subunit (IL28Rα),
wherein the binding protein causes the multimerization of IL10Rβ and IL28Rα and downstream signaling, and
wherein the binding protein comprises a single-domain antibody (sdAb) that specifically binds to IL10Rβ (an anti-IL10Rβ sdAb) and a sdAb that specifically binds to IL28Rα (an anti-IL28Rα sdAb).
25. The IFNλR binding protein of aspect 24, wherein the anti-IL10Rβ sdAb is a V_{H}H antibody (an anti-IL10Rβ V_{H}H antibody) and/or the anti-IL28Rα sdAb is a V_{H}H antibody (an anti-IL28Rα V_{H}H antibody).
26. The IFNλR binding protein of any one of aspects 24 to 25, wherein the anti-IL10Rβ sdAb and the anti-IL28Rα sdAb are joined by a peptide linker.
27. The IFNλR binding protein of aspect 26, wherein the peptide linker comprises between 1 and 50 amino acids.
28. A method for treating an infectious disease in a subject in need thereof, comprising administering to the subject an IFNλR binding protein of any one of aspects 24 to 27, wherein the IFNλR binding protein binds to and activates macrophages, CD8⁺ T cells, CD4⁺ T cells, Treg cells, dendritic cells, and/or epithelial cells.
29. The method of aspect 28, wherein the IFNλR binding protein binds to and activates macrophages.
30. The method of aspect 28 or 29, wherein the infectious disease is influenza, hepatitis B, hepatitis C, or human immunodeficiency virus (HIV) infection.
31. A binding protein that specifically binds to IL10Rα and IL2Rγ,
wherein the binding protein causes the multimerization of IL10Rα and IL2Rγ and downstream signaling, and
wherein the binding protein comprises a sdAb that specifically binds to IL10Rα (an anti-IL10Rα sdAb) and a sdAb that specifically binds to IL2Rγ (an anti-IL2Rγ sdAb).
32. The binding protein of aspect 31, wherein the anti-IL10Rα sdAb is a V_{H}H antibody (an anti-IL10Rα V_{H}H antibody) and/or the anti-IL2Rγ sdAb is a V_{H}H antibody (an anti-IL2Rγ V_{H}H antibody).
33. The binding protein of any one of aspects 31 to 32, wherein the anti-IL10Rα sdAb and the anti-IL2Rγ sdAb are joined by a peptide linker.
34. The binding protein of aspect 33, wherein the peptide linker comprises between 1 and 50 amino acids.
35. A method for treating cancer in a subject in need thereof, comprising administering to the subject the binding protein of any one of aspects 31 to 34, wherein the binding protein binds to and activates CD8⁺ T cells and/or CD4⁺ T cells.
36. The method of aspect 35, wherein the method does not cause anemia.
37. A binding protein that specifically binds to a first receptor and a second receptor,
wherein the first receptor is interferon γ receptor 1 (IFNγR1) or IL28Rα and the second receptor is preferentially expressed on myeloid cells and/or T cells,
wherein the binding protein causes the multimerization of the first receptor and the second receptor and their downstream signaling, and
wherein the binding protein comprises a single-domain antibody (sdAb) that specifically binds to the first receptor and a sdAb that specifically binds to the second receptor.
38. The binding protein of aspect 37, wherein the sdAb that specifically binds to the first receptor is an anti-IFNγR1 V_{H}H antibody.
39. The binding protein of aspect 37, wherein the sdAb that specifically binds to the first receptor is an anti-IL28Rα V_{H}H antibody.
40. The binding protein of any one of aspects 37 to 39, wherein the first receptor is IFNγR1 and the second receptor is IL2Rγ.
41. The binding protein of any one of aspects 37 to 39, wherein the first receptor is IL28Rα and the second receptor is IL2Rγ.
42. The binding protein of any one of aspects 37 to 41, wherein the sdAb that specifically binds to the first receptor and the sdAb that specifically binds to the second receptor are joined by a peptide linker.
43. The binding protein of aspect 42, wherein the peptide linker comprises between 5 and 50 amino acids.
44. A method for treating cancer in a subject in need thereof, comprising administering to the subject the binding protein of any one of aspects 37 to 43, wherein the binding protein binds to and activates myeloid cells and/or T cells.
45. The method of aspect 44, wherein the binding protein binds to and activates macrophages.
46. The method of aspect 44, wherein the binding protein binds to and activates CD8⁺ T cells and/or CD4⁺ T cells.
47. The IL10R binding protein of any one of aspects 17 to 20 wherein the anti-IL10Rα sdAb is selected from the group consisting of SEQ ID NOs: 44-50 and the anti-IL10Rβ sdAb is selected from the group consisting of SEQ ID Nos: 51-57.
48. The IL10R binding protein of aspect 47 wherein the anti-IL10Rα sdAb is joined to the anti-IL10Rβ sdAb via a linker selected from the group consisting of SEQ ID Nos:1-23.
49. The ILR binding protein of aspect 47 wherein the IL10R binding protein comprises, from amino to carboxy, a first anti-IL10R sdAb joined via a linker to a second anti-IL10R sdAb, according to the following:

| first anti-IL10R sdAb SEQ ID | second anti-IL10R sdAb SEQ ID |
|---|---|
| 48 | 57 |
| 49 | 56 |
| 50 | 55 |
| 52 | 46 |
| 47 | 51 |
| 51 | 47 |
| 46 | 55 |
| 46 | 56 |
| 47 | 56 |
| 46 | 54 |
| 44 | 53 |
| 55 | 44 |
| 46 | 52 |
| 45 | 57 |
| 45 | 55 |
| 47 | 55 |
| 50 | 54 |
| 48 | 55 |
| 46 | 57 |
| 47 | 57 |
| 50 | 56 |
| 49 | 51 |
| 52 | 45 |
| 53 | 44 |
| 54 | 47 |

and wherein said linker is selected from the group consisting of SEQ ID Nos:1-23.
50. The IL-10 receptor binding protein of aspect 17 selected from the group consisting of SEQ ID Nos: 194, 209, 210, 211, 213, 218, 226, 233, 238, 244, 250, 203, 205, 207, 269, 212, 217, 219, 224, 227, 237, 239, and 249.

## Claims

1. An IL 22 receptor (IL22R) binding protein that specifically binds to IL22R1 subunit (IL22R1) and IL10Rβ subunit (IL10Rβ), wherein the binding protein causes the multimerization of IL22R1 and IL10RB and downstream signaling, and wherein the binding protein comprises a single-domain antibody (sdAb) that specifically binds to IL22R1 (an anti-IL22R1 sdAb) and a sdAb that specifically binds to IL10Rβ (an anti-IL10Rβ sdAb).

2. The IL22R binding protein of claim 1, wherein the anti-IL22R1 sdAb is a VHH antibody (an anti-IL22R1 VHH antibody) and/or the anti-IL10Rβ sdAb is a VHH antibody (an anti-IL10Rβ VHH antibody).

3. The IL22R binding protein of claim 1 or 2, wherein the anti-IL22R1 sdAb and the anti-IL10Rβ sdAb are joined by a peptide linker.

4. The IL22R binding protein of claim 3, wherein the peptide linker comprises between 1 and 50 amino acids.

5. The IL22R binding protein of any one of claims 1 to 4, wherein the IL22R binding protein has a reduced Emax compared to IL22.

6. The IL22R binding protein of any one of claims 1 to 5, wherein the IL22R binding protein has a similar potency compared to that of IL22.

7. The IL22R binding protein of any one of claims 1-6 for use in a method for treating a disease in a subject in need thereof, wherein the disease is selected from the group consisting of: ulcerative colitis, Crohn's disease, psoriasis, graft-versus-host disease, inflammatory diseases of the lung and airway such as lung fibrosis, ventilator induced lung injury, neoplastic disease (e.g., IL22R1 -expressing tumors), liver fibrosis, diseases associated with liver injury such as alcohol toxicity (acute or chronic) steatosis,, and pancreatitis, lupus, and type-2 diabetes.

8. An IL 12 receptor (IL12R) binding protein that specifically binds to IL12Rβ1 and IL12Rβ2, wherein the binding protein causes the multimerization of IL12Rβ1 and IL12Rβ2 and downstream signaling, and wherein the binding protein comprises a single-domain antibody (sdAb) that specifically binds to IL12Rβ1 (an anti-IL12Rβ1 sdAb) and a sdAb that specifically binds to IL12Rβ2 (an anti-IL12Rβ2 sdAb).

9. The IL12R binding protein of claim 8, wherein the anti-IL12Rβ1 sdAb is a VHH antibody (an anti-IL12Rβ1 VHH antibody) and/or the anti-IL12Rβ2 sdAb is a VHH antibody (an anti-IL12Rβ2 VHH antibody).

10. The IL12R binding protein of claim 8 or 9, wherein the anti-IL12Rβ1 sdAb and the anti-IL12Rβ2 sdAb are joined by a peptide linker.

11. The IL12R binding protein of claim 10, wherein the peptide linker comprises between 1 and 50 amino acids.

12. The IL12R binding protein of any one of claims 8 to 11, wherein the IL12R binding protein has a reduced Emax compared to IL12.

13. The IL12R binding protein of any one of claims 8 to 12, wherein the IL12R binding protein has a similar potency compared to that of IL12.

14. The IL12R binding protein of any one of claims 8 to 13 for use in a method for treating cancer in a subject in need thereof, wherein the IL12R binding protein binds to and activates natural killer, CD4+ T cells, and/or CD8+ T cells.

15. The IL12R binding protein for use of claim 14, wherein the cancer is a solid tumor cancer.
